# EUROPEAN PATENT SPECIFICATION

(11) **EP 1 578 919 B1**
(45) Date of publication and mention of the grant of the patent: **17.02.2010**
(21) Application number: 02807929.1
(22) Date of filing: 09.12.2002
(51) Int. Cl.: C07K 14/47, C12Q 1/68, C12Q 1/70, A61K 39/00, C07K 14/15, G01N 33/574

(54) **ENDOGENOUS RETROVIRUS UP-REGULATED IN PROSTATE CANCER**
BEI PROSTATAKREBS HOCHREGULIERTES ENDOGENES RETROVIRUS
RETROVIRUS ENDOGENE REGULE POSITIVEMENT DANS LE CANCER DE LA PROSTATE

(30) Priority: 07.12.2001 US 16604; 07.12.2001 US 340640 P; 07.12.2001 WO PCT/US01/47824; 12.06.2002 US 388046 P
(43) Date of publication of application: 28.09.2005
(73) Proprietor: Novartis Vaccines and Diagnostics, Inc., Emeryville, CA 94608 (US)
(72) Inventor: HARDY, Stephen, F., San Francisco, CA 94121 (US); GARCIA, Pablo, San Francisco, CA 94131 (US); WILLIAMS, Lewis, T., Mill Valley, CA 94902 (US); ESCOBEDO, Jaime, Alamo, CA 94507 (US)
(74) Representative: Marshall, Cameron John
(86) International application number: PCT/US2002/039136
(87) International publication number: WO 2004/037972

(56) References cited:
- WO-A2-00/73801
- WO-A2-01/60860
- DATABASE Geneseq [Online] 26 March 2001 (2001-03-26), "Human prostate cancer associated antigen nucleotide sequence SEQ ID:506." XP002424336 retrieved from EBI accession no. GSN:AAF22927 Database accession no. AAF22927
- DATABASE Geneseq [Online] 26 March 2001 (2001-03-26), "Human prostate cancer associated antigen protein sequence SEQ ID NO:1220." XP002424337 retrieved from EBI accession no. GSP:AAB63858 Database accession no. AAB63858
- DATABASE EMBL [Online] 16 June 1999 (1999-06-16), "Homo sapiens genomic DNA, chromosome 22q11.2, clone KB1572G7." XP002424338 retrieved from EBI accession no. EMBL:AP000346 Database accession no. AP000346
- ARTAMONOVA I I ET AL: "Nonrandom distribution of endogenous retroviral regulatory elements, HERV-K LTRs, on human Chr22" DOKLADY AKADEMII NAUK, vol. 372, no. 3, May 2000 (2000-05), pages 401-403, XP008076288 ISSN: 0869-5652
- SMITH R D ET AL: "THE HUMAN ENDOGENOUS RETROVIRUS HERV-K IN PROSTATE CANCER" AMERICAN JOURNAL OF HUMAN GENETICS, AMERICAN SOCIETY OF HUMAN GENETICS, CHICAGO, IL, US, vol. 69, no. 4, SUPPL, 12 October 2001 (2001-10-12), page 275, XP009009588 ISSN: 0002-9297
- TURNER G ET AL: "Insertional polymorphisms of full-length endogenous retroviruses in humans" CURRENT BIOLOGY, CURRENT SCIENCE,, GB, vol. 11, no. 19, 2 October 2001 (2001-10-02), pages 1531-1535, XP002233323 ISSN: 0960-9822
- KURDYUKOV SERGEY G ET AL: "Full-sized HERV-K (HML-2) human endogenous retroviral LTR sequences on human chromosome 21: Map locations and evolutionary history" GENE: AN INTERNATIONAL JOURNAL ON GENES AND GENOMES, ELSEVIER, AMSTERDAM, NL, vol. 273, no. 1, 25 July 2001 (2001-07-25), pages 51-61, XP002361442 ISSN: 0378-1119
- ANDERSSON M.-L. ET AL: 'Diversity of human endogenous retrovirus class II-like sequences' JOURNAL OF GENERAL VIROLOGY vol. 80, no. 2, January 1999, pages 255 - 260, XP002996131
- GRIFFITHS D.J.: 'Endogenous retroviruses in the human genome sequence' GENOME BIOLOGY vol. 2, no. 6, 05 June 2001, pages 1017.1 - 1017.5, XP002996132
- GOEDERT J.J. ET AL: 'High prevalence of antibodies against HERV-K10 in patients with testicular cancer but not with AIDS' CANCER EPIDEMIOLOGY , BIOMARKERS & PREVENTION vol. 8, no. 4, April 1999, pages 293 - 296, XP002983119
- TRISTEM M.: 'Identification and characterization of novel human endogenous retrovirus families by phylogenetic screening of the human genome mapping project database' JOURNAL OF VIROLOGY vol. 74, no. 8, April 2000, pages 3715 - 3730, XP002996133
- DATABASE GENSEQ [Online] DERWENT 13 September 2002 SCHLEGEL R. ET AL: 'Human prostate expression marker cDNA 21509' Database accession no. ABV21518
- DATABASE GENSEQ [Online] DERWENT 13 September 2002 SCHLEGEL ET AL: 'Human prostate expression marker cDNA 21999' Database accession no. ABV22008
- DATABASE EMBL [Online] HINXTON, UK 13 January 1997 TOENTJES ET AL: 'Human endogenous retrovirus HERV-K, LTR R and U% region and gag gene' Database accession no. Y10390
- ONO ET AL: 'Nucleotide Sequence of Human Endogenous Retrovirus Genome Related to the Mouse Mammary Tumor Virus Genome' J. VIROL. vol. 60, no. 2, November 1986, pages 589 - 598
- SAUTER ET AL: 'Human Endogenous Retrovirus K10: Expression of Gag Protein and Detection of Antibodies in Patients with Seminomas' J. VIROL. vol. 69, no. 1, January 1995, pages 414 - 421
- DATABASE GENSEQ [Online] DERWENT 23 August 2001 SCHLEGEL ET AL: 'Human prostate expression marker cDNA 24381'
- TOMLINS ET AL: 'Distinct classes of chromosomal rearrangements create oncogenic ETS gene fusions in prostate cancer' NATURE vol. 448, pages 595 - 601

## Description

This application claims the benifit of: international patent application PCT/US01/47824 (published in English on June 13, 2002, as WO02/46477), filed December 7th 2001; US patent application 10/016,604; filed December 7th 2001; US provisional patent pplication 60/340,064, filed December 7, 2001; and US provisional patent application 60/388,046, filed June 12th 2002.

### TECHNICAL FIELD

The present invention relates to the diagnosis of cancer, particularly prostate cancer. In particular, it relates to a human endogenous retrovirus (HERV) located on chromosome 22 which shows up-regulated expression in tumors, particularly prostate tumors.

### BACKGROUND ART

Prostate cancer is the most common type of cancer in men in the USA. Benign prostatic hyperplasia (BPH) is the abnormal growth of benign prostate cells in which the prostate grows and pushes against the urethra and bladder, blocking the normal flow of urine. More than half of the men in the USA aged 60-70 and as many as 90% percent aged 70-90 have symptoms of BPH. Although BPH is seldom a threat to life, it may require treatment to relieve symptoms.

Cancer that begins in the prostate is called primary prostate cancer (or prostatic cancer). Prostate cancer may remain in the prostate gland, or it may spread to nearby lymph nodes and may also spread to the bones, bladder, rectum, and other organs. Prostate cancer is currently diagnosed by measuring levels of prostate-specific antigen (PSA) and prostatic acid phosphatase (PAP) in the blood. The level of PSA in blood may rise in men who have prostate cancer, BPH, or an infection in the prostate. The level of PAP rises above normal in many prostate cancer patients, especially if the cancer has spread beyond the prostate. However, prostate cancer cannot be diagnosed using these tests alone because elevated PSA or PAP levels may also indicate other, non-cancerous problems.

In order to help determine whether conditions of the prostate are benign or malignant further tests such as transrectal ultrasonography, intravenous pyelogram, and cystoscopy are usually performed. If these test results suggest that cancer may be present, the patient must undergo a biopsy as the only sure way of diagnosis. Consequently, it is desirable to provide a simple and direct test for the early detection and diagnosis of prostate cancer without having to undergo multiple rounds of cumbersome testing procedures. It is also desirable and necessary to provide compositions and methods for the prevention and/or treatment of prostate cancer.

References 1 and 2 disclose that human endogenous retroviruses (HERVs) of the HML-2 subgroup of the HERV-K family show up-regulated expression in prostate tumors. This finding is disclosed as being useful in prostate cancer screening, diagnosis and therapy. In particular, higher levels of an HML-2 expression product relative to normal tissue are said to indicate that the patient from whom the sample was taken has cancer.

WO00/73801 describes nucleic acids and encoded polypeptides which are cancer associated antigens expressed in patients afflicted with breast, gastric or prostate cancer.

WO01/60860 relates to novel genes associated with prostate cancer as well as methods of assessing whether a patient is afflicted with or has higher than normal risk for developing prostate cancer.

Smith et al., (American Journal of Human Genetics, vol. 69, no. 4, suppl., 2001) describes an investigation into HERV-K expression and possible retrotransposition in prostate cancer.

It is an object of the invention to provide additional and improved materials and methods that can be used in the diagnosis, prevention and treatment of prostate cancer.

### DISCLOSURE OF THE INVENTION

A specific member of the HERV-K family located in chromosome 22 at 20.428 megabases (22q11.2) has been found to be preferentially and significantly up-regulated in prostate tumors. This endogenous retrovirus (named 'PCAV' herein) has several features not found in other members of the HERV-K family and these features can be exploited in prostate cancer screening, diagnosis and therapy (*e.g.* adjuvant therapy).

The invention provides a method for diagnosing prostate cancer, the method comprising the step of detecting in a patient sample the level of an expression product of a human endogenous retrovirus located at megabase 20.428 on chromosome 22 wherein the level of expression product in the patient sample is compared to a control sample. Higher levels of expression product relative to normal tissue indicate that the patient from whom the sample was taken has cancer.

The expression product which is detected is preferably a mRNA transcript, but may alternatively be a polypeptide translated from such a transcript. These expression products may be detected directly or indirectly. A direct test uses an assay which detects PCAV RNA or polypeptide in a patient sample. An indirect test uses an assay which detects biomolecules which are not directly expressed *in vivo* from PCAV *e.g.* an assay to detect cDNA which has been reverse-transcribed from PCAV mRNA, or an assay to detect an antibody which has been raised in response to a PCAV polypeptide.

### A - THE HUMAN CHROMOSOME 22 ENDOGENOUS RETROVIRUS

Many regions within the published human genome sequence are annotated as endogenous retroviruses and, even before its sequence was determined, it was known that the human genome contained multiple HERVs. One of the many HERVs is a HERV-K located at megabase 20.428 of chromosome 22, referred to herein as 'PCAV'. Expression of this HERV has been found to be up-regulated in cancer tissue. Furthermore, PCAV has five specific features not found in other HERVs. These five features are manifested in PCAV mRNA transcripts and can be exploited in screening and diagnosis: (1) it has a specific nucleotide sequence which distinguishes it from other HERVs within the genome, although the sequence shares significant identity with the other HERVs; (2) it has tandem 5' LTRs; (3) it has a fragmented 3' LTR; (4) its *env* gene is interrupted by an alu insertion; and (5) its gag contains a unique insertion.

### A.1 - Nucleotide sequence

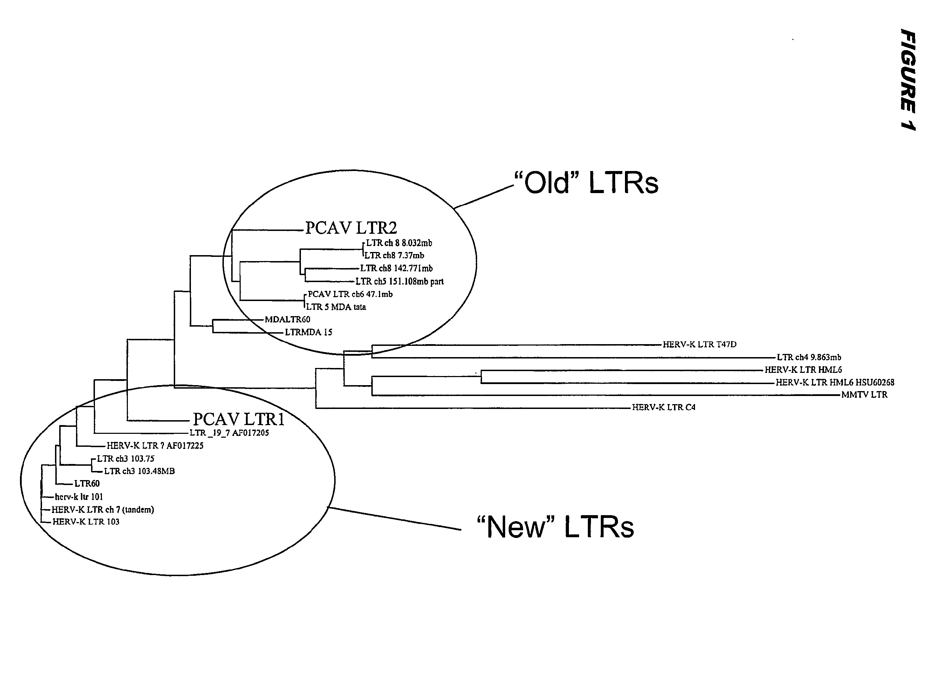

PCAV is a member of the HERV-K sub-family HML2.0. There are roughly 30 to 50 copies of HML2.0 viruses per haploid human genome. HML2 viruses appear to have inserted at least twice in human ancestry: 30 million years ago, before the ape lineage (including humans) split off from monkeys; and 20 million years ago, after the split. The viruses from the 30 million year insertion are sometimes referred to as "old type" viruses and the 20 million insertion as "new type" {3}. Old and new virus proteins are very highly related at the amino acid sequence level, but there are some distinguishing epitopes. DNA sequence identity is high at some regions of the genome but in others, particularly the LTRs, conservation is only about 70%. Most of the differences between old and new LTRs are clustered near the start of transcription, where old viruses have oen or two insertions relative to the new viruses. Old and new LTRs cluster as two separate groups in phylogenetic analyses (figure 1). In keeping with their relative genetic ages, old viruses also contain more interruptions and deletions than new viruses.

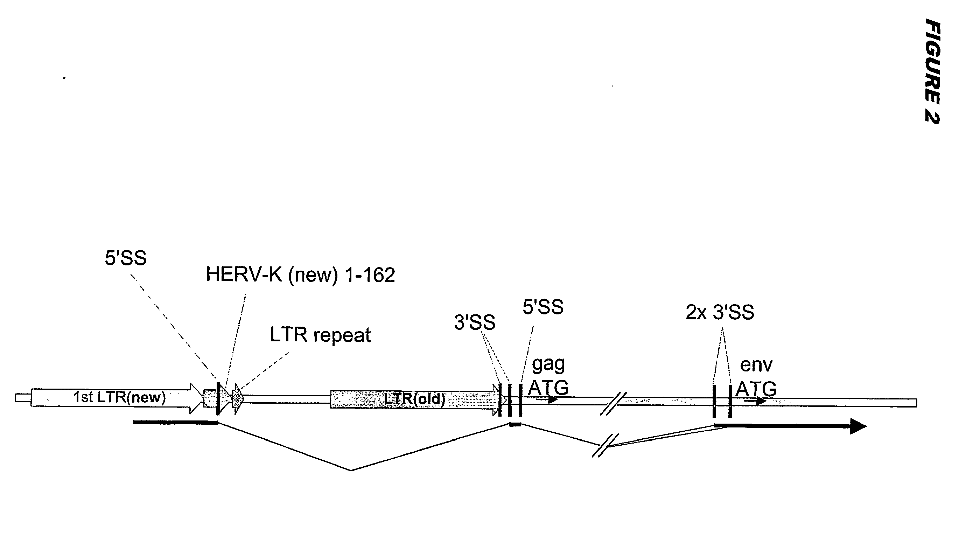

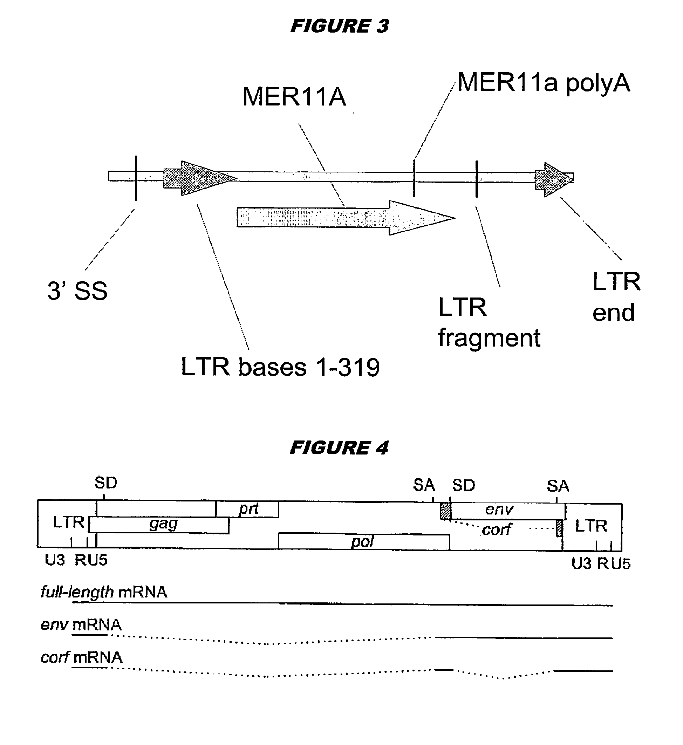

PCAV appears to have arisen from a rearrangement between a new and an old virus. The 5' region of the virus (figure 2) starts with a new LTR followed by 162 bp from a new virus. The rest of the new virus seems to be missing, as the 162 bp is followed by a 552 bp of non-viral sequence and then an almost-complete old virus. The 3' LTR of the old virus (figure 3) is fragmented and includes a MER11 a insertion.

SEQ ID 1 is the 12366bp sequence of PCAV, based on available human chromosome 22 sequence {4}, from the beginning of its first 5' LTR to the end of its fragmented 3' LTR. It is the sense strand of the double-stranded genomic DNA. SEQ ID 10 is the 11101bp sequence of PCAV from nucleotide 559 in SEQ ID 1 (a possible transcription start site) to its polyadenylation site (up to nucleotide 11735 in SEQ ID 1), although a more downstream transcription start site (*e.g.* nucleotide 635±5) is more likely.

The specific sequence of PCAV is manifested at both the mRNA and amino acid levels, and can be used to distinguish it from other HERVs within the genome.

### A.2 - Tandem 5'LTRs

Downstream of the 5' LTR of a HERV-K, before the start of the gag open reading frame, there is a conserved splice donor site (5'SS). This splice donor can join to splice acceptor sites (3'SS) at the start of the env open reading frame (Figure 4).

HERV-K genomes also include two splice acceptor sequences near the 3' end of the LTR, but these are not ordinarily used because they have no upstream viral splice donor partner. However, PCAV has two LTRs at its 5' end: the first is from a new HERV-K and the second is from an old HERV-K. The normally-unused splice acceptors in the old LTR can thus co-operate with the splice donor in the new LTR (Figure 2), and transcripts resulting from these splice donor/acceptor pairings are specific to PCAV.

Transcripts formed by using a splice acceptor site near the 3' end of the second 5' LTR comprise (i) a sequence transcribed from the transcription start site in the first 5'LTR, continuing to a splice donor site closely downstream of the first 5' LTR, joined to (ii) a sequence transcribed from one of the splice acceptor sites near the 3' end of the second 5' LTR. Detection of such transcripts indicates that PCAV is being transcribed.

In SEQ ID 1: the transcription start site in the first 5' LTR would be at nucleotide 559 by homology to other viruses, but seems to be further downstream (*e.g.* at around 635±2) empirically; the conserved splice donor site downstream of the first 5' LTR is at nucleotides 1076-1081; the two splice acceptor sites near the 3' end of the second 5' LTR are at nucleotides 2593-2611 and 2680-2699. SEQ ID 2 is the sequence between the predicted transcription start site and the splice donor site. SEQ ID 3 is the first 10 nucleotides following the first splice acceptor site. SEQ ID 4 is the first 10 nucleotides following the second splice acceptor site. SEQ ID 5 is SEQ ID 2 fused to SEQ ID 3. SEQ ID 6 is SEQ ID 2 fused to SEQ ID 4.

### A.3 - Fragmented 3' LTR

The 3' LTR of PCAV is fragmented, including insertion of a MER11a repetitive element (figure 3). PCAV mRNAs terminate using a polyadenylation signal within the MER11a insertion, rather than using the signal within the viral LTR. Transcripts which terminate with a partial copy of a 3'HERV-K LTR followed by a MER11a sequence are specific to PCAV.

The 3' ends of transcripts from PCAV include copies of a partial LTR and a partial MER11a (figure 3). Detection of such transcripts indicates that PCAV is being transcribed.

In SEQ ID 1: the 3' LTR begins at nucleotide 10520 and continues until nucleotide 10838, where it is interrupted by a MER11a insertion; the MER11a insertion starts at nucleotide 10839 and continues to nucleotide 11834; after nucleotides 11835-11928, the 3' LTR continues from nucleotide 11929 to 12366. Within the MER11a insertion is its polyadenylation signal (located between nucleotides 11654 to 11659). SEQ ID 7 is the sequence of the first 319nt fragment of the 3' LTR. SEQ ID 8 is the sequence of the MER11a insertion up to its polyA site. SEQ ID 9 is SEQ ID 7 fused to SEQ ID 8.

### A.4 - Alu in env

As well as being disrupted by mutations due to genetic age, the *env* gene of PCAV is interrupted by an alu sequence. Detection of transcripts containing both env and alu sequence indicates that PCAV is being transcribed.

In SEQ ID 1, the alu is at nucleotides 9938 to 10244 (SEQ ID 32). The 100 nucleotides immediately preceding the alu sequence (9838-9937) are SEQ ID 37, the last 10mer of which (9928-9937) is SEQ ID 33. The 100 nucleotides immediately following the alu sequence are SEQ ID 40, the first 10mer of which (10244-10253) is SEQ ID 34. The first 10 nucleotides of the alu sequence are SEQ ID 35 and the last 10 are SEQ ID 41. SEQ ID 36 is the 20mer bridging the alu/env boundary and SEQ ID 45 is the 20mer bridging the end of the alu sequence. SEQ ID 39 is the 8mer bridging the alu/env boundary, and SEQ ID 44 is the 8mer bridging the end of the alu sequence. SEQ ID 38 is SEQ ID 37 + SEQ ID 32, SEQ ID 42 is SEQ ID 41 + SEQ ID 40, and SEQ ID 43 is SEQ ID 32 + SEQ ID 40.

### A.5 - Unique gag sequences

The PCAV gag gene contains a 48 nucleotide sequence (SEQ ID 53) which is not found in other HERV-Ks. The 48mer encodes 16mer SEQ ID 110, which is not found in gag proteins from new or in other old HERV-Ks. Detection of transcripts containing SEQ ID 53, or of polypeptides containing SEQ ID 110, or antibodies which recognize epitope within or including SEQ ID 110 thus indicates that PCAV is being transcribed.

The PCAV gag gene also contains a 69 nucleotide sequence (SEQ ID 111) which is not found in new HERV-Ks. The 69mer encodes 23mer SEQ ID 55. Detection of transcripts containing SEQ ID 111, or of polypeptides containing SEQ ID 55, or antibodies which recognize epitope within or including SEQ ID 55 thus indicates that an old HERV-K, typically PCAV, is being transcribed.

### B - DETECTING mRNA EXPRESSION PRODUCTS

The diagnostic method of the invention may be based on mRNA detection. PCAV mRNA may be detected directly or indirectly. It is preferred to detect a mRNA directly, thereby avoiding the need for separate preparation of mRNA-derived material (*e.g.* cDNA).

### B.1 - PCAV mRNA transcripts of the invention

mRNA transcripts for use according to the present invention are transcribed from PCAV. Three preferred types of transcript are: (1) transcripts spliced using a splice acceptor site near the 3' end of the second 5' LTR; (2) transcripts comprising both 3' LTR and MER11a sequences; (3) transcripts comprising the alu-interrupted *env* gene; and (4) transcripts comprising a PCAV-specific gag sequence.

The invention provides a mRNA transcript transcribed from a human endogenous retrovirus located at megabase 20.428 on chromosome 22.

The invention also provides a mRNA transcript comprising a nucleotide sequence with *n%* or more sequence identity to SEQ ID 23, or to a nucleotide sequence lacking up to 100 nucleotides (*e.g*. 10, 20, 30, 40, 50, 60, 70, 71, 72, 73, 74, 75, 76, 77, 78, 79, 80, 81, 82, 83, 84, 85, 86, 87, 88, 89, 90 or 100) from the 5' end of SEQ ID 23 *e.g. n%* or more sequence identity to SEQ ID 1197 or 1198. The nucleotide sequence is preferably at the 5' end of the RNA, although upstream sequences may be present. The nucleotide sequence may be at the 3' end of the RNA, but there will typically be further downstream elements such as a poly-A tail. These mRNA transcripts include allelic variants, SNP variants, homologs, orthologs, paralogs, mutants, *etc*. of SEQ ID 23, SEQ ID 1197 and SEQ ID 1198.

The invention provides a mRNA transcript formed by splicing involving a splice acceptor site near the 3' end of the second 5' LTR. Thus the invention provides a mRNA transcript comprising the sequence -N₁-N₂- (*e.g*. SEQ ID 24, SEQ ID 25, SEQ ID 1199 or SEQ ID 1200), where: N₁ is a nucleotide sequence (*e.g*. SEQ ID 26, SEQ ID 1201) from (i) the 5' end of a mRNA transcribed from the first 5' LTR of a human endogenous retrovirus located at megabase 20.428 on chromosome 22, to (ii) a first splice donor site downstream of the U5 region of said mRNA transcribed from the first 5' LTR; and N₂ is a nucleotide sequence (*e.g*. SEQ ID 27 or SEQ ID 28) immediately downstream of a splice acceptor site located (i) downstream of said first splice donor site and (ii) upstream of a second splice donor site, the second splice donor site being downstream of the second 5' LTR of said endogenous retrovirus. The first splice donor site is preferably the site conserved in the HML2 sub-family, located about 100 nucleotides downstream of the first 5' LTR (after nucleotide 1075 in SEQ ID 1). The second splice donor site is preferably the site conserved in the HML2 sub-family, located about 100 nucleotides downstream of the second 5' LTR (after SEQ ID 1 nucleotide 2778). The splice acceptor is preferably downstream of the second 5'LTR.

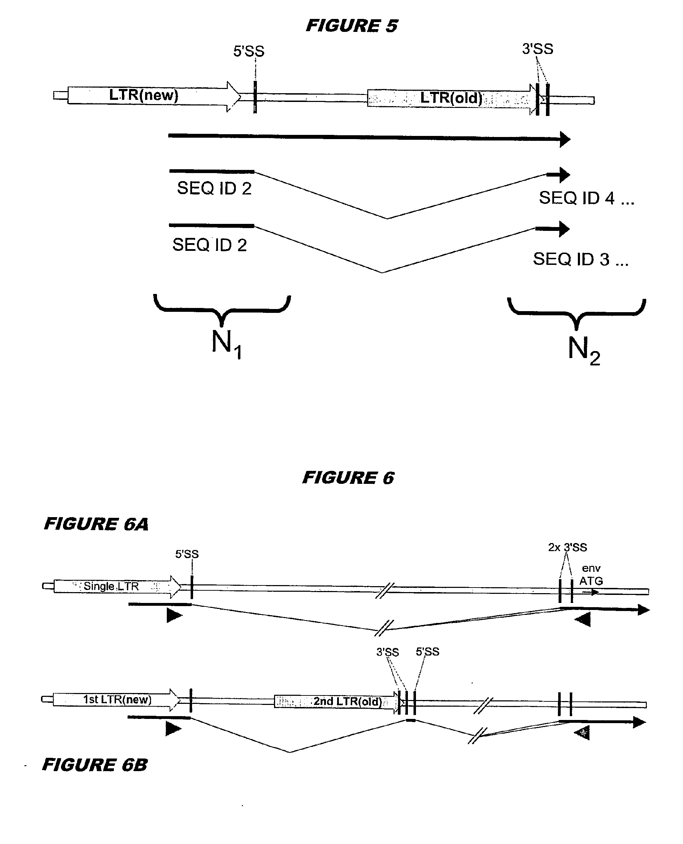

The invention also provides a mRNA transcript comprising the sequence -N₁-N₂-, where: N₁ is a nucleotide sequence with *a%* or more sequence identity to SEQ ID 26 and/or SEQ ID 1201 and N₂ is a nucleotide sequence with *b*% or more sequence identity to SEQ ID 27 or SEQ ID 28. These mRNA transcripts of the invention are illustrated in figure 5. Transcripts which use the second splice site (*i.e*. N₂ is SEQ ID 28) are preferred.

In both cases, N₁ is preferably at the 5' end of the RNA, although upstream sequences may be present. N₂ may be at the 3' end of the RNA, but downstream sequences will usually be present.

The invention also provides a mRNA transcript comprising a nucleotide sequence with *c%* or more sequence identity to SEQ ID 24, SEQ ID 25, SEQ ID 1199 or SEQ ID 1200.

The invention provides a mRNA transcript comprising the sequence -N₃-N₄- (*e.g.* SEQ ID 29), where: N₃ is a nucleotide sequence (*e.g*. SEQ ID 30) from the 3' end of the 5' fragment of the 3' LTR of a human endogenous retrovirus located at megabase 20.428 on chromosome 22, and N₄ is a nucleotide sequence (*e.g*. SEQ ID 31) from 5' end of the MER11a insertion in a human endogenous retrovirus located at megabase 20.428 on chromosome 22.

The invention also provides a mRNA transcript comprising the sequence -N₃-N₄-, where: N₃ is a nucleotide sequence with *d*% or more sequence identity to SEQ ID 30 and N₄ is a nucleotide sequence with *e%* or more sequence identity to SEQ ID 31. The RNA may comprise the sequence -N₃-N₄-N₅-N₆-, wherein: N₅ is a nucleotide sequence between the polyA signal and the polyA site of a MER11a sequence; and N₆ is a polyA tail.

In both cases, the transcript will generally include sequence upstream of N₃. The transcript will generally include sequence downstream of N₄, such as a polyA tail.

The invention also provides a mRNA transcript comprising a nucleotide sequence wich *f*% or more sequence identity to SEQ ID 29.

The invention provides a mRNA transcript comprising the sequence -N₇-N₈- (*e.g*. SEQ ID 38), where: N₇ is a nucleotide sequence (*e.g.* SEQ ID 37) preceding the alu insertion within the *env* gene of a human endogenous retrovirus located at megabase 20.428 on chromosome 22, and N₈ is a nucleotide sequence (*e.g*. SEQ ID 32) beginning at the 5' end of said alu insertion.

The invention also provides a mRNA transcript comprising the sequence -N₇-N₈-, where: N₇ is a nucleotide sequence with *mm%* or more sequence identity to SEQ ID 37 and N₈ is a nucleotide sequence with *nn%* or more sequence identity to SEQ ID 32.

The transcript will generally include sequence upstream of N₇ and downstream of N₈.

The invention also provides a mRNA transcript comprising a nucleotide sequence with *pp%* or more sequence identity to SEQ ID 38.

The invention provides a mRNA transcript comprising the sequence -N₉-N₁₀- (*e.g.* SEQ ID 43), where: N₉ is a nucleotide sequence (*e.g.* SEQ ID 32) at the end of the alu insertion within the env gene of a human endogenous retrovirus located at megabase 20.428 on chromosome 22, and N₁₀ is a nucleotide sequence (*e.g.* SEQ ID 40) immediately downstream of said alu insertion.

The invention also provides a mRNA transcript comprising the sequence -N₉-N₁₀-, where: N₉ is a nucleotide sequence with *uu%* or more sequence identity to SEQ ID 41 and N₁₀ is a nucleotide sequence with *vv%* or more sequence identity to SEQ ID 40.

The transcript will generally include sequence upstream of N₉ and downstream of N₁₀.

The invention also provides a mRNA transcript comprising a nucleotide sequence with *ww%* or more sequence identity to SEQ ID 42.

The invention provides a mRNA transcript comprising a nucleotide sequence with *uu%* or more sequence identity to SEQ ID 41.

The transcript will generally include sequence upstream of N₉ and downstream of N₁₀.

The invention also provides a mRNA transcript comprising a nucleotide sequence with *ii%* or more sequence identity to SEQ ID 53.

The invention also provides a mRNA transcript comprising a nucleotide sequence with *ii%* or more sequence identity to SEQ ID 111.

The invention also provides a mRNA transcript comprising a nucleotide sequence with *ii%* or more sequence identity to SEQ ID 1191. The invention also provides a mRNA transcript which encodes a polypeptide having at least *ii%* sequence identity to SEQ ID 98.

### B.2 - Direct and indirect detection of mRNA

PCAV mRNA transcripts of the invention may be detected directly, for example by sequencing of the mRNA or by hybridization to mRNA transcripts (*e.g.* by Northern blot). Various techniques are available for detecting the presence or absence of a particular RNA sequence in a sample {*e.g.* refs. 20 & 21}.

Indirect detection of mRNA transcripts is also possible and is performed on nucleic acid derived from a PCAV mRNA transcript *e.g.* detection of a cDNA copy of PCAV mRNA, detection of nucleic acids amplified from a PCAV mRNA template, *etc.*

A preferred method for detecting RNA is RT-PCR (reverse transcriptase polymerase chain reaction) {*e.g.* refs. 5 to 13}. RT-PCR of mRNA from prostate cells is reported in, for example, references 14 to 19. It is preferred to use PCAV-specific probes in RT-PCR.

Whether direct or indirect detection is used, the method of the invention involves detection of a single-stranded or double-stranded PCAV nucleic acid target, either (a) in the form of PCAV mRNA or (b) in the form of nucleic acid comprising a copy of at least a portion of a PCAV mRNA and/or a sequence complementary to at least a portion of a PCAV mRNA.

The method of the invention does not involve the detection of PCAV genomic DNA, as this is present in all human cells and its presence is therefore not characteristic of tumors. If a sample contains PCAV DNA, it is preferred to use a RNA-specific detection technique or to focus on sequences present in PCAV mRNA transcripts but not in PCAV genomic DNA (*e.g.* splice junctions, polyA tail *etc.*)*.* The method of the invention may therefore comprise an initial step of: (a) extracting mRNA from a patient sample; (b) removing DNA from a patient sample without removing mRNA; and/or (c) removing or disrupting PCAV DNA, but not PCAV mRNA, in a patient sample. As an alternative, a RNA-specific assay can be used which is not affected by the presence of homologous DNA. For RT-PCR, genomic DNA should be removed.

Methods for selectively extracting RNA from biological samples are well known {*e.g.* refs. 20 & 21} and include methods based on guanidinium buffers, lithium chloride, acid phenol:chloroform extraction, SDS/potassium acetate *etc.* After total cellular RNA has been extracted, mRNA may be enriched *e.g*. using oligo-dT techniques.

Methods for removing DNA from biological samples without removing mRNA are well known {*e.g.* appendix C of ref. 20} and include DNase digestion. If DNase is used then it must be removed or inactivated (*e.g*. by chelation with EDTA, by heating, or by proteinase K treatment followed by phenol/chloroform extraction and NH₄OAc/EtOH precipitation) prior to subsequent DNA synthesis or amplification, in order to avoid digestion of the newly-synthesized DNA.

Methods for removing PCAV DNA, but not PCAV RNA, will use a reagent which is specific to a sequence within a PCAV DNA *e.g*. a restriction enzyme which recognizes a DNA sequence within the PCAV genome, but which does not cleave the corresponding RNA sequence.

Methods for specifically purifying PCAV mRNAs from a sample may also be used. One such method uses an affinity support which binds to PCAV mRNAs. The affinity support may include a polypeptide sequence which binds to the PCAV mRNA *e.g.* the cORF polypeptide, which binds to the LTR of HERV-K mRNAs in a sequence-specific manner, or HIV Rev protein, which has been shown to recognize the HERV-K LTR in RNA transcripts {22}.

PCAV mRNA need not be maintained in a wild-type form for detection. It may, for example, be fragmented, provided that the fragmentation maintains PCAV-specific sequences within the mRNA.

### B.3 - PCAV nucleic acid targets for detection

There is described nucleic acid comprising (a) the nucleotide sequence of a mRNA transcript transcribed from a human endogenous retrovirus located at megabase 20.428 on chromosome 22, and/or (b) the complement of (a). There is also described nucleic acid comprising a nucleotide sequence with *qq%* or more sequence identity to SEQ ID 10, SEQ ID 1197 and/or SEQ ID 1198. PCAV is approximately 87.5% identical to the HERV-K found at megabase 47.1 on chromosome 6 and approximately 86% identical to the HERV-K found at megabase 103.75 on chromosome 3.

There is described nucleic acid comprising (a) nucleotide sequence -N₁-N₂- as defined above, and/or (b) the complement of (a). There is described nucleic acid comprising (a) a nucleotide sequence with *c%* or more sequence identity to SEQ ID 5, SEQ ID 6, SEQ ID 1199 or SEQ ID 1200 and/or (b) the complement of (a).

There is described nucleic acid comprising (a) nucleotide sequence -N₃-N₄- as defined above, and/or (b) the complement of (a). There is described nucleic acid comprising (a) a nucleotide sequence with *f%* or more sequence identity to SEQ ID 9, and/or (b) the complement of (a).

There is described nucleic acid comprising (a) nucleotide sequence -N₃-N₄-N₅-N₆- as defined above, and/or (b) the complement of (a).

There is described nucleic acid comprising (a) nucleotide sequence -N₇-N₈- as defined above, and/or (b) the complement of (a). There is described nucleic acid comprising (a) a nucleotide sequence with *aa%* or more sequence identity to SEQ ID 38, and/or (b) the complement of (a).

There is described nucleic acid comprising (a) nucleotide sequence -N₉-N₁₀- as defined above, and/or (b) the complement of (a). There is described nucleic acid comprising (a) a nucleotide sequence with *hh%* or more sequence identity to SEQ ID 42, and/or (b) the complement of (a).

There is described nucleic acid comprising a nucleotide sequence with *bbb%* or more sequence identity to SEQ ID 53, and/or (b) the complement of (a).

There is described nucleic acid comprising a nucleotide sequence with *fff%* or more sequence identity to SEQ ID 111, and/or (b) the complement of (a).

Specific nucleic acid targets include SEQ IDs 99 to 109, which are splice variant cDNA sequences assuming a transcription start site in SEQ ID 1 at 559 and including four A residues at the 3' end. Assuming a more downstream transcription start site (*e.g.* nucleotide 635 of SEQ ID 1), these nucleic targets would not include a stretch of nucleotides at the 5' end of SEQ IDs 99 to 109 *e.g.* they would not include 10, 20, 30, 40, 50, 60, 70, 71, 72, 73, 74, 75, 76, 77, 78, 79, 80, 81, 82, 83, 84, 85, 86, 87, 88, 89, 90, 100 or more of the 5' nucleotides. 25mer sequences based on cDNA sequences are given as SEQ IDs 337 to 599.

### B.4 - Nucleic acid materials for direct or indirect mRNA detection

There is described nucleic acid which can hybridize to a PCAV nucleic acid target.

Hybridization reactions can be performed under conditions of different "stringency". Conditions that increase stringency of a hybridization reaction of widely known and published in the art {*e.g.* page 7.52 of reference 21}. Examples of relevant conditions include (in order of increasing stringency): incubation temperatures of 25°C, 37°C, 50°C, 55°C and 68°C; buffer concentrations of 10 x SSC, 6 x SSC, 1 x SSC, 0.1 x SSC (where SSC is 0.15 M NaCl and 15mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or de-ionized water. Hybridization techniques and their optimization are well known in the art {*e.g.* see references 20, 21, 23, 24, 28 *etc.*}.

In some embodiments, nucleic acid hybridizes to a target under low stringency conditions; in other embodiments it hybridizes under intermediate stringency conditions; in preferred embodiments, it hybridizes under high stringency conditions. An exemplary set of low stringency hybridization conditions is 50°C and 10 x SSC. An exemplary set of intermediate stringency hybridization conditions is 55°C and 1 x SSC. An exemplary set of high stringency hybridization conditions is 68°C and 0.1 x SSC.

Preferred nucleic acids hybridize to PCAV nucleic acid targets but not to nucleic acid targets from other HERV-Ks. PCAV-specific hybridization is favored by exploiting features found within PCAV transcripts but not in other HERV-K transcripts *e.g.* specific nucleotide sequences, features arising from the tandem 5' LTRs, features arising from the MER11a insertion within the 3'. LTR, or features arising from the alu interruption of env. Sequence alignments can be used to locate regions of PCAV which are most divergent from other HERV-K genomes and in which PCAV-specific hybridization can occur. Specificity for PCAV is desirable in order to detect its up-regulation above the low-level of natural background expression of other new HERV-Ks seen in most cells.

One group of preferred nucleic acids can specifically detect PCAV products in which a splice acceptor site near the 3' end of the second 5' LTR has been used. As described above, such splicing brings together sequences N₁ and N₂, which are not juxtaposed in PCAV genomic DNA. There is described a nucleic acid which hybridizes to sequence -N₁-N₂- (or the complement thereof) within a PCAV nucleic acid target, but which does not hybridize to sequences N₁ or N₂ alone (or to their complements alone). The nucleic acid comprises a first sequence which can hybridize to N₁ (or to its complement) and a second sequence which can hybridize to N₂ (or to its complement), such that it will hybridize to a target in which N₁ and N₂ are adjacent, but will not hybridize to targets in which splicing has not brought N₁ and N₂ together. Such nucleic acids can identify PCAV transcripts in the presence of PCAV genomic DNA because of the difference in relative locations of N₁ and N₂.

Another group of preferred nucleic acids can specifically detect mRNAs containing 3' LTR and MER11a sequences. Thus there is described a nucleic acid which hybridizes to sequence -N₃-N₄- (or the complement thereof) within a PCAV nucleic acid target, but which does not hybridize to sequences N₃ or N₄ alone (or to their complements alone). The nucleic acid comprises a first sequence which can hybridize to N₃ (or to its complement) and a second sequence which can hybridize to N₄ (or to its complement), such that it will hybridize to targets which include both (i) a 3' LTR sequence and (ii) a MER11a sequence, but not to targets which include only one of (i) and (ii). The nucleic acid may inherently be able to hybridize to genomic DNA, although this property is not useful for detecting transcripts.

Another group of preferred nucleic acids can specifically detect mRNAs containing the alu-interrupted env gene. Thus there is described a nucleic acid which hybridizes to sequence -N₇-N₈- (or the complement thereof) within a PCAV nucleic acid target, but which does not hybridize to sequences N₇ or N₈ alone (or to their complements alone). The nucleic acid comprises a first sequence which can hybridize to N₇ (or to its complement) and a second sequence which can hybridize to N₈ (or to its complement), such that it will hybridize to targets which include both (i) the env sequence immediately preceding the alu interruption and (ii) an alu interruption, but not to targets which include only one of (i) and (ii). The nucleic acid may inherently be able to hybridize to genomic DNA, although this property is not useful for detecting transcripts.

There is described a nucleic acid which hybridizes to sequence -N₉-N₁₀- (or the complement thereof) within a PCAV nucleic acid target, but which does not hybridize to sequences N₉ or N₁₀ alone (or to their complements alone). The nucleic acid comprises a first sequence which can hybridize to N₉ (or to its complement) and a second sequence which can hybridize to N₁₀ (or to its complement), such that it will hybridize to targets which include both (i) the 3' region of the alu interruption within env and (ii) the sequence immediately downstream of the alu interruption, but not to targets which include only one of (i) and (ii). The nucleic acid may inherently be able to hybridize to genomic DNA, although this property is not useful for detecting transcripts.

The ability of a nucleic acid to hybridize to a PCAV nucleic acid target is related to its intrinsic features (*e.g.* the degree of sequence identity to the target) as well as extrinsic features (*e.g.* temperature, salt concentration *etc*.). A group of preferred nucleic acids of the invention have a good intrinsic ability to hybridize to PCAV nucleic acid targets.

Thus there is described a nucleic acid comprising a nucleotide sequence with *s%* or more sequence identity to a fragment of a PCAV nucleic acid target or to the complement of a fragment of a PCAV nucleic acid target. There is described a nucleic acid comprising a nucleotide sequence with *g%* or more sequence identity to a fragment of SEQ ID 10 or to the complement of a fragment of SEQ ID 10. There is described a nucleic acid comprising a nucleotide sequence with *h%* or more sequence identity to a fragment of SEQ ID 5 or to the complement of a fragment of SEQ ID 5. There is described a nucleic acid comprising a nucleotide sequence with *i%* or more sequence identity to a fragment of SEQ ID 6 or to the complement of a fragment of SEQ ID 6. There is described a nucleic acid comprising a nucleotide sequence with *j%* or more sequence identity to a fragment of SEQ ID 9 or to the complement of a fragment of SEQ ID 9. There is described a nucleic acid comprising a nucleotide sequence with *ccc%* or more sequence identity to a fragment of SEQ ID 53 or to the complement of a fragment of SEQ ID 53. There is described a nucleic acid comprising a nucleotide sequence with *kkk%* or more sequence identity to SEQ ID 1191. It also provides a nucleic acid comprising a nucleotide sequence which encodes a polypeptide having at least *mmm%* sequence identity to SEQ ID 98. There is described a nucleic acid comprising a nucleotide sequence with *nnn%* or more sequence identity to SEQ ID 1198. It also provides a nucleic acid comprising a nucleotide sequence which encodes a polypeptide having at least *qqq%* sequence identity to SEQ ID 1199. It also provides a nucleic acid comprising a nucleotide sequence which encodes a polypeptide having at least *rrr%* sequence identity to SEQ ID 1200.

There is described a nucleic acid comprising a fragment of at least k contiguous nucleotides of SEQ ID 10 or of the complement of SEQ ID 10. The fragment is preferably located within SEQ ID 1197 and/or 1198.

There is described a nucleic acid comprising a fragment of at least *l* contiguous nucleotides of SEQ ID 47 or of the complement of SEQ ID 47. The fragment preferably comprises nucleotide sequence B₁ₐ-B₂ₐ (or its complement), wherein B₁ₐ comprises *m* or more nucleotides from the 3' end of SEQ ID 2 and B₂ₐ comprises *p* or more nucleotides from the 5' end of SEQ ID 46. These nucleic acids thus span a splice junction which brings sequences N₁ and N₂ together and are thus able to identify PCAV transcripts in the presence of PCAV genomic DNA because of the difference in the relative locations of B₁ₐ and B₂ₐ, B₁ₐ-B₂ₐ preferably comprises SEQ ID 11 (or its complement), where m=p=4, and more preferably comprises SEQ ID 50 (or its complement), where m=p=10.

There is described a nucleic acid comprising a fragment of at least *q* contiguous nucleotides of SEQ ID 49 or of the complement of SEQ ID 49. The fragment preferably comprises nucleotide sequence B_{1b}-B_{2b} (or its complement), wherein B_{1b} comprises *r* or more nucleotides from the 3' end of SEQ ID 2 and B_{2b} comprises *t* or more nucleotides from the 5' end of SEQ ID 48. These nucleic acids thus span the splice junction which brings sequences N₁ and N₂ together and are thus able to identify PCAV transcripts in the presence of PCAV genomic DNA because of the difference in the relative locations of B_{1b} and B_{2b}. B_{1b}-B_{2b} preferably comprises SEQ ID 12 (or its complement), where r=t=4, and more preferably comprises SEQ ID 51 (or its complement), where r=t=10.

There is described a nucleic acid comprising a fragment of at least *u* contiguous nucleotides of SEQ ID 9 or of the complement of SEQ ID 9. The fragment preferably comprises nucleotide sequence B₃-B₄ (or its complement), wherein B₃ comprises *v* or more nucleotides from the 3' end of SEQ ID 7 and B₄ comprises w or more nucleotides from the 5' end of SEQ ID 8. These nucleic acids thus include part of both of N₃ and N₄. B₃-B₄ preferably comprises SEQ ID 13 (or its complement), where v=w=4, and more preferably comprises SEQ ID 52 (or its complement), where v=w=10.

There is described a nucleic acid comprising a fragment of at least *rr* contiguous nucleotides of SEQ ID 38 or of the complement of SEQ ID 38. The fragment preferably comprises nucleotide sequence B₇-B₈ (or its complement), wherein B₇ comprises *ss* or more nucleotides from the 3' end of SEQ ID 37 and B₄ comprises *tt* or more nucleotides from the 5' end of SEQ ID 32. These nucleic acids thus include part of both of N₇ and N₈. B₇-B₈ preferably comprises SEQ ID 39 (or its complement), where ss=tt=4, and more preferably comprises SEQ ID 36 (or its complement), where ss=tt=10.

There is described a nucleic acid comprising a fragment of at least *jj* contiguous nucleotides of SEQ ID 43 or of the complement of SEQ ID 43. The fragment preferably comprises nucleotide sequence B₉-B₁₀, or its complement, and wherein B₉ comprises *kk* or more nucleotides from the 3' end of SEQ ID 32 and B₁₀ comprises *ll* or more nucleotides from the *5*' end of SEQ ID 40. These nucleic acids thus include part of both of N₉ and N₁₀. B₉-B₁₀ preferably comprises SEQ ID 44 (or its complement), where kk=11=4, and more preferably comprises SEQ ID 45 (or its complement), where kk=11=10.

There is described a nucleic acid comprising a fragment of at least *ddd* contiguous nucleotides of SEQ ID 53 or of the complement of SEQ ID 53. The invention also provides a nucleic acid comprising a fragment of at least ggg contiguous nucleotides of SEQ ID 111 or of the complement of SEQ ID 111. The invention also provides a nucleic acid comprising a fragment of at least *hhh* contiguous nucleotides of SEQ ID 112 or of the complement of SEQ ID 112. There is described a nucleic acid comprising a fragment of at least *jjj* contiguous nucleotides of SEQ ID 1191 or of the complement of SEQ ID 1191.

There is described a nucleic acid of formula 5'-X-Y-Z-3', wherein: -X- is a nucleotide sequence consisting of *x* nucleotides; -Z- is a nucleotide sequence consisting of z nucleotides; -Y- is a nucleotide sequence consisting of either (a) a fragment of *y* nucleotides of any of SEQ IDs 1-13, 20-53, 57, 58, 63, 81, 86, 88-91, 99-109, 111, 112, 1191, 1197 or 1198, or (b) the complement of (a); and said nucleic acid 5'-X-Y-Z-3' is neither (i) a fragment of SEQ IDs 1-13, 20-53, 57, 58, 63, 81, 86, 88-91, 99-109, 111, 112, 1191, 1197 or 1198 or (ii) the complement of (i).

Where -Y- is (a), the nucleotide sequence of -X- preferably shares less than *bb*% sequence identity to the *x* nucleotides which are 5' of sequence -Y- in SEQ IDs 1-13, 20-53, 57, 58, 63, 81, 86, 88-91, 99-109, 111, 112, 1191, 1197 or 1198 and/or the nucleotide sequence of -Z-preferably shares less than cc% sequence identity to the z nucleotides which are 3' of sequence - Z- in SEQ IDs 1-13, 20-53, 57, 58, 63, 81, 86, 88-91,99-109,111, 112, 1191, 1197 or 1198.

Where -Y- is (b), the nucleotide sequence of -X- preferably shares less than *bb%* sequence identity to the complement of the *x* nucleotides which are 5' of the complement of sequence -Yin SEQ IDs 1-13, 20-53, 57, 58, 63, 81, 86, 88-91, 99-109, 111, 112, 1191, 1197 or 1198 and/or the nucleotide sequence of -Z- preferably shares less than *cc*% sequence identity to the complement of the z nucleotides which are 3' of the complement of sequence -Y- in SEQ IDs 1-13, 20-53, 57, 58, 63, 81, 86, 88-91, 99-109, 111, 112, 1191, 1197 or 1198.

The -X- and/or -Z- moieties may comprise a promoter sequence (or its complement).

There is described nucleic acid comprising nucleotide sequence SEQ ID 53. This sequence is specific within the human genome to PCAV. The invention also provides nucleic acid comprising nucleotide sequence SEQ ID 111.

There is described nucleic acid comprising nucleotide sequence SEQ ID 1191. Various PCAV nucleic acids are provided by the invention. 25mer fragments of PCAV sequences are given as SEQ IDs 120 to 1184. There is described these sequences as 25mers, as well as fragments thereof (*e*.*g*. the 2 x 24mers, the 3 x 23mers, the 4 x 22mers ... the 19 x 7mers in each) and as longer PCAV fragments comprising these 25mers.

Preferred nucleic acids of the invention comprise one or more of SEQ IDs 53 and 842-1184.

Nucleic acids are particularly useful as probes and/or as primers for use in hybridization and/or amplification reactions.

More than one nucleic acid can hybridize to the same target (*e*.*g*. more than one can hybridize to a single mRNA or cDNA).

### B.5 - Nucleic acid amplification

Nucleic acid in a sample can conveniently and sensitively be detected by nucleic acid amplification techniques such as PCR, SDA, SSSR, LCR, TMA, NASBA, T7 amplification *etc.* The technique preferably gives exponential amplification. A preferred technique for use with RNA is RT-PCR (*e*.*g*. see chapter 15 of ref. 20). The technique may be quantitative and/or real-time.

Amplification techniques generally involve the use of two primers. Where a target sequence is single-stranded, the techniques generally involve a preliminary step in which a complementary strand is made in order to give a double-stranded target. The two primers hybridize to different strands of the double-stranded target and are then extended. The extended products can serve as targets for further rounds of hybridization/extension. The net effect is to amplify a template sequence within the target, the 5' and 3' termini of the template being defined by the locations of the two primers in the target.

Primers and probes are preferably nucleic acids as described in section B.4 above. Particularly preferred primers are those based on SEQ IDs 600-1184. (or their complements) *e*.*g*. comprising primers comprising SEQ IDs 600-1184, or primers comprising fragments of *ppp* or more nucleotides from one of SEQ IDs 600-1184.

Further features of primers and probes are described in section B.6 below.

### B.6-General features of nucleic acids of the invention

Nucleic acids and transcripts are preferably provided in isolated or substantially isolated form *i*.*e*. substantially free from other nucleic acids (*e*.*g*. free from naturally-occurring nucleic acids), generally being at least about 50% pure (by weight), and usually at least about 90% pure.

Nucleic acids can take various forms.

Nucleic acids may be single-stranded or double-stranded. Unless otherwise specified or required, any embodiment of the invention that utilizes a nucleic acid may utilize both the double-stranded form and each of two complementary single-stranded forms which make up the double-stranded form. Primers and probes are generally single-stranded, as are antisense nucleic acids.

Nucleic acids may be circular or branched, but will generally be linear.

Nucleic acid may be attached to a solid support (*e*.*g*. a bead, plate, filter, film, slide, microarray support, resin, *etc*.)

For certain embodiments, nucleic acids are preferably at least 7 nucleotides in length (*e*.*g*. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 nucleotides or longer).

For certain embodiments, nucleic acids are preferably at most 500 nucleotides in length (*e*.*g*. 450, 400, 350, 300, 250, 200, 150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 nucleotides or shorter).

Primers and probes, and other nucleic acids used for hybridization, are preferably between 10 and 30 nucleotides in length (*e*.*g*. 10, 11, 12, 13, 14, 15, 16, 17, 18, 19, 20, 21, 22, 23, 24, 25, 26, 27, 28, 29, or 30 nucleotides).

Nucleic acids may be carry a detectable label *e*.*g*. a radioactive or fluorescent label, or a biotin label. This is particularly useful where the nucleic acid is to be used in nucleic acid detection techniques *e*.*g*. where the nucleic acid is a probe or a primer.

Nucleic acids comprise PCAV sequences, but they may also comprise non-PCAV sequences (*e*.*g*. in nucleic acids of formula 5'-X-Y-Z-3', as defined above). This is particularly useful for primers, which may thus comprise a first sequence complementary to a PCAV nucleic acid target and a second sequence which is not complementary to the nucleic acid target. Any such non-complementary sequences in the primer are preferably 5' to the complementary sequences. Typical non-complementary sequences comprise restriction sites {26} or promoter sequences {27}.

Nucleic acids can be prepared in many ways *e*.*g*. by chemical synthesis (at least in part), by digesting longer nucleic acids using nucleases (*e*.*g*. restriction enzymes), by joining shorter nucleic acids (*e*.*g*. using ligases or polymerases), from genomic or cDNA libraries, *etc*.

Nucleic acids may be part of a vector *i*.*e*. part of a nucleic acid construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, "viral vectors" which is designed to result in the production of a recombinant virus or virus-like particle, or "shuttle vectors", which comprise the attributes of more than one type of vector. A "host cell" includes an individual cell or cell culture which can be or has been a recipient of exogenous nucleic acid. Host cells include progeny of a single host cell, and the progeny may not necessarily be completely identical (in morphology or in total DNA complement) to the original parent cell due to natural, accidental, or deliberate mutation and/or change. Host cells include cells transfected or infected *in vivo* or *in vitro* with nucleic acid.

The term "nucleic acid" includes in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (*e*.*g*. peptide nucleic acids (PNAs) or phosphorothioates) or modified bases. The term "nucleic acid" is not intended to be limiting as to the length or structure of a nucleic acid unless specifically indicated, and the following are non-limiting examples of nucleic acids: a gene or gene fragment, mRNA, tRNA, rRNA, ribozymes, cDNA, recombinant nucleic acids, branched nucleic acids, plasmids, vectors, DNA from any source, RNA from any source, probes, and primers. Where nucleic acid of the invention takes the form of RNA, it may have a 5' cap.

Where a nucleic acid is DNA, it will be appreciated that "U" in a RNA sequence will be replaced by "T" in the DNA. Similarly, where a nucleic acid is RNA, it will be appreciated that "T" in a DNA sequence will be replaced by "U" in the RNA.

The term "complement" or "complementary" when used in relation to nucleic acids refers to Watson-Crick base pairing. Thus the complement of C is G, the complement of G is C, the complement of A is T (or U), and the complement of T (or U) is A. It is also possible to use bases such as I (the purine inosine) *e*.*g*. to complement pyrimidines (C or T). The terms also imply a direction- the complement of 5'-ACAGT-3' is 5'-ACTGT-3' rather than 5'-TGTCA-3'.

Nucleic acids can be used, for example: to produce polypeptides; as hybridization probes for the detection of nucleic acid in biological samples; to generate additional copies of the nucleic acids; to generate ribozymes or antisense oligonucleotides; as single-stranded DNA primers or probes; or as triple-strand forming oligonucleotides. The nucleic acids are preferably uses to detect PCAV nucleic acid targets such as PCAV mRNAs.

References to a percentage sequence identity between two nucleic acid sequences mean that, when aligned, that percentage of bases are the same in comparing the two sequences. This alignment and the percent homology or sequence identity can be determined using software programs known in the art, for example those described in section 7.7.18 of reference 28. A preferred alignment program is GCG Gap (Genetics Computer Group, Wisconsin, Suite Version 10.1), preferably using default parameters, which are as follows: open gap = 3; extend gap = 1.

The percentage values of *a, aa, b, bbb, c, ccc, d, e, eee, f, fff, g, h, hh, i, ii, j, kkk, mm, mmm; n, nn, nnn, pp, qq, qqq, rrr, s*, *uu, vv* and *ww* as used above may each independently be 50, 55, 60, 65, 70, 75, 80, 85, 90, 91, 92, 93, 94, 95, 96, 97, 98, 99, 99.5, 99.9 or 100. The values of each of *a, aa, b, bbb, c, ccc, d, e, eee, f, fff, g, h, hh, i, ii, j, mm, n*, *nn*, *pp, qq, s, uu, vv* and *ww* may be the same or different as each other. Nucleic acid sequences which include 'silent' changes (*i*.*e*. which do not affect the encoded amino acid for a codon) are examples of these nucleic acids.

The values of *ddd, ggg, hhh, jj, jjj, k kk, l*, *ll, m, p, ppp, q, r, rr, ss, t, tt, u, v, w* and *y* as used above may each independently be 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 60, 70, 80, 90, 100 or more. The values of each of *ddd, ggg, jj, k, kk*, *l*, *ll, m, p, q, r, rr, ss, t, tt, u, v, w* and *y* may be the same or different as each other.

The value of *x*+*z* is at least 1 (*e*.*g*. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100 *etc*.). It is preferred that the value of *x*+*y*+*z* is at least 8 (*e*.*g*. at least 9, 10, 11, 12, 13, 14, 15, 16, 17, 18, 19, 20, 21, 22, 23, 24, 25, 30, 35, 40, 45, 50, 60, 70, 80, 90, 100 *etc*.). It is preferred that the value of *x+y+z* is at most 500 (*e*.*g*. at most 450, 400, 350, 300, 250, 200, 190, 180, 170, 160, 150, 140, 130, 120, 110, 100, 90, 80, 70, 60, 50, 40, 30, 25, 20, 19, 18, 17, 16, 15, 14, 13, 12, 11, 10,9,8).

The percentage values of *bb* and *cc* as used above are independently each preferably less than 60 (*e*.*g*. 50, 40, 30, 20, 10), or may even be 0. The values of *bb* and *cc* may be the same or different as each other.

Preferred nucleic acids comprise nucleotide sequences which remain unmasked following application of a masking program for masking low complexity (*e*.*g*. XBLAST).

Where a nucleic acid is said to "encode" a polypeptide, it is not necessarily implied that the polynucleotide is translated, but it will include a series of codons which encode the amino acids of the polypeptide.

### C - DETECTING POLYPEPTIDE EXPRESSION PRODUCTS

Where the method is based on polypeptide detection, it will involve detecting expression of a polypeptide encoded by a PCAV mRNA transcript. This will typically involve detecting one or more of the following polypeptides: gag (*e*.*g*. SEQ ID 57) or PCAP3/mORF (*e*.*g*. SEQ ID 87). Although some PCAV mRNAs encode all of these polypeptides (*e*.*g*. ERVK6 {44}), PCAV is an old virus and its prt, pol and env genes are highly fragmented.

The transcripts which encode HML-2 polypeptides are generated by alternative splicing of the full-length mRNA copy of the endogenous genome {*e*.*g*. Figure 4 of ref. 45, Figure 1 of ref. 54}. PCAV gag polypeptide is encoded by the first long ORF in the genome (nucleotides 2813-4683 of SEQ ID 1; SEQ ID 54). Full-length gag polypeptide is proteolytically cleaved. PCAV prt polypeptide is encoded by the second long ORF in the genome and is translated as a gag-prt fusion polypeptide which is proteolytically cleaved to give the protease. PCAV pol polypeptide is encoded by the third long ORF in the genome and is translated as a gag-prt-pol fusion polypeptide which is proteolytically cleaved to give three pol products - reverse transcriptase, endonuclease and integrase {46}. PCAV env polypeptide is encoded by the fourth long ORF in the genome. The translated polypeptide is proteolytically cleaved. PCAV cORF polypeptide is encoded by an ORF which shares the same 5' region and start codon as env, but in which a splicing event removes env-coding sequences and shifts to a reading frame +1 relative to that of env {47, 48}. PCAP3 polypeptide is encoded by an ORF which shares the same 5' region and start codon as env, but in which a splicing event removes env-coding sequences and shifts to a reading frame +2 relative to that of env (the third reading frame).

### C.1 - Direct detection of HML-2 polypeptides

Various techniques are available for detecting the presence or absence of a particular polypeptides in a sample. These are generally immunoassay techniques which are based on the specific interaction between an antibody and an antigenic amino acid sequence in the polypeptide. Suitable techniques include standard immunohistological methods, ELISA, RIA, FIA, immunoprecipitation, immunofluorescence, *etc.*

Polypeptides of the invention can also be detected by functional assays *e*.*g*. assays to detect binding activity or enzymatic activity. For instance, functional assays for cORF are disclosed in references 48 to 50, and a functional assay for the protease is disclosed in reference 51. PCAP3 has been found to cause apoptosis in primary prostate epithelial cells and, when apoptosis is suppressed, to enable cells to expand beyond their normal senescence point.

Another way of detecting polypeptides of the invention is to use standard proteomics techniques *e*.*g*. purify or separate polypeptides and then use peptide sequencing. For example, polypeptides can be separated using 2D-PAGE and polypeptide spots can be sequenced (*e*.*g*. by mass spectroscopy) in order to identify if a sequence is present in a target polypeptide.

Techniques may require the enrichment of target polypeptides prior to detection. However, immunofluorescence assays can be easily performed on cells without the need for such enrichment. Cells may first be fixed onto a solid support, such as a microscope slide or microtiter well. The membranes of the cells can then be permeablized in order to permit entry of antibody (NB: fixing and permeabilization can be achieved together). Next, the fixed cells can be exposed to fluorescently-labeled antibody which is specific for the polypeptide. The presence of this label identifies cells which express the target PCAV polypeptide. To increase the sensitivity of the assay, it is possible to use a second antibody to bind to the anti-PCAV antibody, with the label being carried by the second antibody. {52}

### C.2 -Indirect detection of HML-2 polypeptides

Rather than detect polypeptides directly, it may be preferred to detect molecules which are produced by the body in response to a polypeptide (*i*.*e*. indirect detection of a polypeptide). This will typically involve the detection of antibodies, so the patient sample will generally be a blood sample. Antibodies can be detected by conventional immunoassay techniques *e*.*g*. using PCAV polypeptides of the invention, which will typically be immobilized.

Antibodies against HERV-K polypeptides have been detected in humans {*e*.*g*. 45, 53, 54} *e*.*g*. in seminoma or teratocarcinoma tissue.

### C.3-Polypeptide materials

The polypeptides which can be used in detection methods of the invention, are encoded by a human endogenous retrovirus located at megabase 20.428 on chromosome 22.

The polypeptides comprising an amino acid sequence selected from the group consisting of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. SEQ IDs 54, 55, 56, 87, 98 and 110 are preferred members of this group.

There is also described (a) a polypeptide comprising a fragment of at least *dd* amino acids of one or more of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188, and (b) a polypeptide comprising an amino acid sequence having at least ee% identity to one or more of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. These polypeptides include variants (*e*.*g*. allelic variants, homologs, orthologs, mutants, *etc.*)*.*

The fragment of (a) may comprise a T-cell or, preferably, a B-cell epitope of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. T- and B-cell epitopes can be identified empirically (*e*.*g*. Using PEPSCAN {55, 56} or similar methods), or they can be predicted (*e*.*g*. using the Jameson-Wolf antigenic index {57}, matrix-based approaches {58}, TEPITOPE {59}, neural networks {60}, OptiMer & EpiMer {61, 62}, ADEPT {63}, Tsites {64}, hydrophilicity {65}, antigenic index {66} or the methods disclosed in reference 67 *etc..*

Preferred fragments of (a) are SEQ IDs 55, 56 and 110, or are fragments of SEQ IDs 55, 56 or 110. SEQ IDs 55, 56 & 110 are found within the PCAV gag protein and are particularly useful for detecting PCAV expression above background expression of other HERV-Ks.

Within (b), the polypeptide may, compared to SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188, comprise one or more conservative amino acid replacements *i*.*e*. replacements of one amino acid with another which has a related side chain. Genetically-encoded amino acids are generally divided into four families: (1) acidic *i*.*e*. aspartate, glutamate; (2) basic *i*.*e*. lysine, arginine, histidine; (3) non-polar *i*.*e*. alanine, valine, leucine, isoleucine, proline, phenylalanine, methionine, tryptophan; and (4) uncharged polar *i*.*e*. glycine, asparagine, glutamine, cystine, serine, threonine, tyrosine. Phenylalanine, tryptophan, and tyrosine are sometimes classified jointly as aromatic amino acids. In general, substitution of single amino acids within these families does not have a major effect on the biological activity.

There is also described a polypeptide having formula NH₂-XX-YY-ZZ-COOH, wherein: XX is a polypeptide sequence consisting of *xx* amino acids; ZZ is a polypeptide sequence consisting of *zz* amino acids; YY is a polypeptide sequence consisting of a fragment of *yy* amino acids of an amino acid sequence selected from the group consisting of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188; and said polypeptide NH₂-YX-YY-ZZ-COOH is not a fragment of a polypeptide sequence selected from SEQ IDs 54, 55, 56, 59, 60, 61, 62, 64, 65, 66, 67, 69, 69, 70, 71, 72, 73, 74, 75, 76, 77, 78, 79, 80, 82, 83, 84, 85, 87, 92, 93, 94, 95, 96, 97, 98, 110, 1186 and 1188.

The sequence of -XX- preferably shares less than *ff*% sequence identity to the *xx* amino acids which are N-terminus to sequence -YY- in SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. The sequence of -ZZ- preferably shares less than *gg*% sequence identity to the zz amino acids which are C-terminus to sequence -YY- in SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc*.), as well as other modifications known in the art. Polypeptides can occur as single chains or associated chains. Polypeptides of the invention can be naturally or non-naturally glycosylated (*i*.*e*. the polypeptide has a glycosylation pattern that differs from the glycosylation pattern found in the corresponding naturally occurring polypeptide).

Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/hydrophilicity, and/or steric bulk of the amino acid substituted. Variants can be designed so as to retain or have enhanced biological activity of a particular region of the polypeptide (*e*.*g*. a functional domain and/or, where the polypeptide is a member of a polypeptide family, a region associated with a consensus sequence). Selection of amino acid alterations for production of variants can be based upon the accessibility (interior *vs.* exterior) of the amino acid (*e*.*g*. ref. 68), the thermostability of the variant polypeptide (*e*.*g*. ref. 69), desired glycosylation sites (*e*.*g*. ref. 70), desired disulfide bridges (*e*.*g*. refs. 71 & 72), desired metal binding sites (*e*.*g*. refs. 73 & 74), and desired substitutions with in proline loops (*e*.*g*. ref. 75). Cysteine-depleted muteins can be produced as disclosed in reference 76.

The percentage value of ee as used above may be 50, 60, 65, 70, 75, 80, 85, 90, 91, 92, 93, 94, 95, 96, 97, 98, 99, 99.5, 99.9 or 100.

The percentage values of *ff* and *gg* as used above are independently each preferably less than 60 (*e*.*g*. 50, 40, 30, 20, 10), or may even be 0. The values of *ff* and *gg* may be the same or different as each other.

The values of *dd, xx, yy* and *zz* as used above may each independently be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 60, 70, 75, 80, 90, 100 or more. The values of each of *dd, xx, yy* and *zz* may be the same or different as each other. The value of *dd* may be less than 2000 (*e*.*g*. less than 1000, 500, 100, or 50).

The value of *xx*+*zz* is at least 1 (*e*.*g*. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100 *etc.*). It is preferred that the value of *xx*+*yy*+*zz* is at least 8 (*e*.*g*. at least 9, 10, 11, 12, 13, 14, 15, 16, 17, 18, 19, 20, 21, 22, 23, 24, 25, 30, 35, 40, 45, 50, 60, 70, 80, 90, 100 *etc*.). It is preferred that the value of *xx*+*yy*+*zz* is at most 500 (*e*.*g*. at most 450, 400, 350, 300, 250, 200, 190, 180, 170, 160, 150, 140, 130, 120, 110, 100, 90, 80, 70, 60, 50, 40, 30, 25, 20, 19, 18, 17, 16, 15, 14, 13, 12, 11, 10, 9, 8).

Polypeptides are generally at least 7 amino acids in length (*e*.*g*. 8, 9, 10, 11, 12, 13, 14, 15, 16, 17, 18, 19, 20, 21, 22, 23, 24, 2S, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 amino acids or longer).

For certain embodiments, polypeptides are preferably at most 500 amino acids in length (*e*.*g*. 450, 400, 350, 300, 250, 200, 150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 amino acids or shorter).

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 28. A preferred alignment is determined by the Smith-Waterman homology search algorithm using an affine gap search with a gap open penalty of 12 and a gap extension penalty of 2, BLOSUM matrix of 62. The Smith-Wateiman homology search algorithm is taught in reference 77.

Preferred polypeptides comprise amino acid sequences which remain unmasked following application of a masking program for masking low complexity (*e*.*g*. XBLAST).

### C.4 - Anitibody materials

There is described antibody that binds to a polypeptide described herein. There is described antibody that binds to a polypeptide encoded by a nucleic acid described herein.

Preferred antibodies recognize epitopes within SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. More preferred antibodies recognize epitopes within SEQ IDs 54, 55, 56 or 110.

Other preferred antibodies recognize a HERV-K gag protein. The antibody may (a) recognize gag from PCAV and also from one or more further HERV-Ks, (b) recognize gag from PCAV but not from any other HERV-Ks, (c) recognize gag from PCAV and also from one or more old HERV-Ks, but not from new HERV-Ks, or (d) recognize gag from one or more HERV-Ks but not from PCAV. A preferred antibody in group (a) is 5G2; a preferred antibody in group (c) is 5A5.

Antibodies may be polyclonal or monoclonal.

Antibodies may be produced by any suitable means *e*.*g*. by recombinant expression, or by administering (*e*.*g*. injecting) a polypeptide of the invention to an appropriate animal (*e*.*g*. a rabbit, hamster, mouse or other rodent).

Antibodies may include a label. The label may be detectable directly, such as a radioactive or fluorescent label. Alternatively, the label may be detectable indirectly, such as an enzyme whose products are detectable (*e*.*g*. luciferase, β-galactosidase, peroxidase *etc*.).

Antibodies may be attached to a solid support.

In general, antibodies are provided in a non-naturally occurring environment *e*.*g*. they are separated from their naturally-occurring environment. In certain embodiments, the antibodies are present in a composition that is enriched for them as compared to a control. Antibodies are thus preferably provided in isolated or substantially isolated form *i*.*e*. the antibody is present in a composition that is substantially free of other antibodies, where by substantially free is meant that less than 75% (by weight), preferably less than 50%, and more preferably less than 10% (*e*.*g*. 5%) of the composition is made up of other antibodies.

The term "antibody" includes any suitable natural or artificial immunoglobulin or derivative thereof. In general, the antibody will comprise a Fv region which possesses specific antigen-binding activity. This includes, but is not limited to: whole immunoglobulins, antigen-binding immunoglobulin fragments (*e*.*g*. Fv, Fab, F(ab')₂ *etc*.), single-chain antibodies (*e*.*g*. scFv), oligobodies, chimeric antibodies, humanized antibodies, veneered antibodies, *etc*.

To increase compatibility with the human immune system, the antibodies may be chimeric or humanized {*e*.*g*. refs. 78 & 79}, or fully human antibodies may be used. Because humanized antibodies are far less immunogenic in humans than the original non-human monoclonal antibodies, they can be used for the treatment of humans with far less risk of anaphylaxis. Thus, these antibodies may be preferred in therapeutic applications that involve *in vivo* administration to a human such as, use as radiation sensitizers for the treatment of neoplastic disease or use in methods to reduce the side effects of cancer therapy.

Humanized antibodies may be achieved by a variety of methods including, for example: (1) grafting non-human complementarity determining regions (CDRs) onto a human framework and constant region ("humanizing"), with the optional transfer of one or more framework residues from the non-human antibody; (2) transplanting entire non-human variable domains, but "cloaking" them with a human-like surface by replacement of surface residues ("veneering"). In the present invention, humanized antibodies will include both "humanized" and "veneered" antibodies. {refs. 80 to 86}. CDRs are amino acid sequences which together define the binding affinity and specificity of a Fv region of a native immunoglobulin binding site {*e*.*g*. 87 & 88}.

The phrase "constant region" refers to the portion of the antibody molecule that confers effector functions. In chimeric antibodies, mouse constant regions are substituted by human constant regions. The constant regions of humanized antibodies are derived from human immunoglobulins. The heavy chain constant region can be selected from any of the 5 isotypes: alpha, delta, epsilon, gamma or mu, and thus antibody can be of any isotype (*e*.*g*. IgG, IgA, IgM, IgD, IgE). IgG is preferred, which may be of any subclass (*e*.*g*. IgG₁, IgG₂).

Humanized or fully-human antibodies can also be produced using transgenic animals that are engineered to contain human immunoglobulin loci. For example, ref. 89 discloses transgenic animals having a human Ig locus wherein the animals do not produce functional endogenous immunoglobulins due to the inactivation of endogenous heavy and light chain loci. Ref. 90 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. Ref. 91 discloses the use of the Cre/Lox system to modify the immunoglobulin locus in a mammal, such as to replace all or a portion of the constant or variable region to form a modified antibody molecule. Ref 92 discloses non-human mammalian hosts having inactivated endogenous Ig loci and functional human Ig loci. Ref. 93 discloses methods of making transgenic mice in which the mice lack endogenous heavy chains, and express an exogenous immunoglobulin locus comprising one or more xenogeneic constant regions.

Using a transgenic animal described above, an immune response can be produced to a PCAV polypeptide, and antibody-producilig cells can be removed from the animal and used to produce hybridomas that secrete human monoclonal antibodies. Immunization protocols, adjuvants, and the like are known in the art, and are used in immunization of, for example, a transgenic mouse as described in ref. 94. The monoclonal antibodies can be tested for the ability to inhibit or neutralize the biological activity or physiological effect of the corresponding polypeptide.

### D - PATIENT SAMPLES AND NORMAL SAMPLES

### D.1 - The patient sample

Where the diagnostic method of the invention is based on detecting mRNA expression, the patient sample will generally comprise cells (*e*.*g*. prostate cells, particularly those from the luminal epithelium). These may be present in a sample of tissue (*e*.*g*. prostate tissue), or may be cells which have escaped into circulation (*e*.*g*. during metastasis). Instead of or as well as comprising prostate cells, the sample may comprise virions which contain PCAV mRNA.

Where the diagnostic method of the invention is based on detecting polypeptide expression, the patient sample may comprise cells, preferably, prostate cells and/or virions (as described above for mRNA), or may comprise antibodies which recognize PCAV polypeptides. Such antibodies will typically be present in circulation.

In general, therefore, the patient sample is tissue sample, preferably, a prostate sample (*e*.*g*. a biopsy) or a blood sample. Other possible sources of patient samples include isolated cells, whole tissues, or bodily fluids (*e*.*g*. blood, plasma, serum, urine, pleural effusions, cerebro-spinal fluid, *etc*.). Another preferred patient sample is a semen sample.

The patient is generally a human, preferably a human male, and more preferably an adult human male.

Expression products may be detected in the patient sample itself, or may be detected in material derived from the sample (*e*.*g*. the supernatant of a cell lysate, a RNA extract, cDNA generated from a RNA extract, polypeptides translated from a RNA extract, cells derived from culturing cells extracted from a patient *etc*.). These are still considered to be "patient samples" within the meaning of the invention.

Detection methods of the invention can be conducted *in vitro* or *in vivo.*

### D.2 - Controls

PCAV transcripts are up-regulated in prostate tumors. To detect such up-regulation, a reference point is typically needed *i*.*e*. a control. Analysis of the control sample gives a standard level of mRNA and/or protein expression against which a patient sample can be compared. As PCAV transcription is negligible in normal cells and highly up-regulated in tumor cells, however, a reference point may not always be necessary - significant expression indicates disease. Even so, the use of controls is preferable, particularly for standardization or for quantitative assays.

A negative control gives a background or basal level of expression against which a patient sample can be compared. Higher levels of expression product relative to a negative control indicate that the patient from whom the sample was taken has a prostate tumor. Conversely, equivalent levels of expression product indicate that the patient does not have a PCAV-related cancer.

A negative control will generally comprise material from cells which are not tumor cells. The negative control could be a sample from the same patient as the patient sample, but from a tissue in which PCAV expression is not up-regulated *e*.*g*. a non-tumor non-prostate cell. The negative control could be a prostate cell from the same patient as the patient sample, but taken at an earlier stage in the patient's life (*e*.*g*. before the development of cancer, or from a BPH patient).. The negative control could be a cell from a patient without a prostate tumor, and this cell may or may not be a prostate cell. The negative control could be a suitable cell line. Typically, the negative control will be the same tissue or cell type as the patient sample being tested (*e*.*g*. a prostate cell or a blood sample).

A positive control gives a level of expression against which a patient sample can be compared. Equivalent or higher levels of expression product relative to a positive control indicate that the patient from whom the sample was taken has a prostate tumor. Conversely, lower levels of expression product indicate that the patient does not have a PCAV-related tumor.

A positive control will generally comprise material from tumor cells or from a blood sample taken from a patient known to have a tumor. The positive control could be a prostate tumor cell from the same patient as the patient sample, but taken at an earlier stage in the patient's life (*e*.*g*. to monitor remission). The positive control could be a cell from another patient with a prostate tumor. The positive control could be a suitable prostate cell line.

Other suitable positive and negative controls will be apparent to the skilled person.

PCAV expression in the control can be assessed at the same time as expression in the patient sample. Alternatively, PCAV expression in the control can be assessed separately (earlier or later). Rather than actually compare two samples, however, the control may be an absolute value *i*.*e*. a level of expression which has been empirically determined from samples taken from prostate tumor patients (*e*.*g*. under standard conditions). Examples of such negative controls for prostate tumors include lifetime baseline levels of expression or the expression level *e*.*g*. as observed in pooled normals.

### D.3 - Degree of up-regulation

The up-regulation relative to the control (100%) will usually be at least 150% (*e*.*g*. 200%, 250%, 300%, 400%, 500%, 600% or more). A twenty- to forty-fold up-regulation is not uncommon.

### E - DIAGNOSTIC METHODS AND DIAGNOSIS

The invention provides a method for diagnosing prostate cancer, comprising the step of detecting in a patient sample the level of an expression product of a human endogenous retrovirus located at megabase 20.428 on chromosome 22.

### E.1 - Products for use in diagnosis

Preferred expression products for detection in diagnostic methods of the invention are described in sections B.1, B.3 and C.3 above.

Preferred reagents for use in diagnostic methods of the invention are described in sections B.4, C.3 and C.4 above.

### E.2 - mRNA-based methods of the invention

The invention provides a method for analyzing a patient sample, comprising the steps of: (a) contacting the patient sample with nucleic acid described herein under hybridizing conditions; and (b) detecting the presence or absence of hybridization of nucleic acid described herein to nucleic acid present in the patient sample. The presence of hybridization in step (b) indicates that the patient from whom the sample was taken has a prostate tumor.

The invention also provides a method for analyzing a patient sample, comprising the steps of: (a) enriching mRNA in the sample relative to DNA to give a mRNA-enriehed sample; (b) contacting the mRNA-enriched sample with nucleic acid described herein under hybridizing conditions; and (c) detecting the presence or absence of hybridization of nucleic acid described herein to mRNA present in the mRNA-enriched sample. The presence of hybridization in step (c) indicates that the patient from whom the sample was taken has a prostate tumor. The enrichment in step (a) may take the form of extracting mRNA without extracting DNA, removing DNA without removing mRNA, or disrupting PCAV DNA without disrupting PCAV mRNA *etc.* (see section B.2 above).

The invention also provides a method for analyzing a patient sample, comprising the steps of: (a) preparing DNA copies of mRNA in the sample; (b) contacting the DNA copies with nucleic acid described herein under hybridizing conditions; and (c) detecting the presence or absence of hybridization of nucleic acid described herein to said DNA copies. The presence of hybridization in step (c) indicates that the patient from whom the sample was taken has a prostate tumor. Preparation of DNA in step (a) may be specific to PCAV (*e*.*g*. by using RT-PCR with appropriate primers) or may be non-specific (*e*.*g*. preparation of cellular cDNA).

In the above methods for analyzing a patient sample, the nucleic acid described herein contacted with the sample may be a probe described herein. As an alternative, it may comprise primers described herein, in which case the relevant step of the method will generally involve two or more (*e*.*g*. 3, 4, 5, 6, 7, 8, 9, 10 or more) cycles of amplification. Where primers are used, the method may involve the use of a probe for detecting hybridization to amplified DNA.

The invention also provides a method for analyzing a patient sample, comprising the steps of: (a) amplifying any PCAV nucleic acid targets in the sample; and (b) detecting the presence or absence of amplified targets. The presence of amplified targets in step (b) indicates that the patient from whom the sample was taken has a prostate tumor.

These methods of the invention may be qualitative, quantitative, or semi-quantitative.

### E.3 - Polypeptide-based methods of the invention

The invention provides an immunoassay method for diagnosing prostate cancer, comprising the step of contacting a patient sample with a polypeptide or antibody of the invention.

The invention also provides a method for analyzing a patient blood sample, comprising the steps of: (a) contacting the blood sample with a polypeptide of the invention; and (b) detecting the presence or absence of interaction between said polypeptide and antibodies in said sample. The presence of an interaction in step (b) indicates that the patient from whom the blood sample was taken has raised anti-PCAV antibodies, and thus that they have a prostate tumor. Step (a) may be preceded by a step wherein antibodies in the blood sample are enriched.

The invention also provides a method for analyzing a patient sample, comprising the steps of: (a) contacting the sample with antibody of the invention; and (b) detecting the presence or absence of interaction between said antibody and said sample. The presence of an interaction in step (b) indicates that the patient from whom the sample was taken is expressing PCAV polypeptides, and thus that they have a prostate tumor. Step (a) may be preceded by a step wherein cells in the sample are lysed or permeabilized and/or wherein polypeptides in the sample are enriched.

These methods of the invention may be qualitative, quantitative, or semi-quantitative.

The above methods may be adapted for use *in vivo* (*e*.*g*. to locate or identify sites where tumor cells are present). In these embodiments, an antibody specific for a target PCAV polypeptide is administered to an individual (*e*.*g*. by injection) and the antibody is located using standard imaging techniques (*e*.*g*. magnetic resonance imaging, computerized tomography scanning, *etc*.). Appropriate labels (*e*.*g*. spin labels *etc*.) will be used. Using these techniques, cancer cells are differentially labeled.

Other *in vivo* methods may detect PCAV polypeptides functionally. For instance, a construct comprising a PCAV LTR operatively linked to a reporter gene (*e*.*g*. a fluorescent protein such as GFP) will be expressed in parallel to native PCAV polypeptides.

To increase the sensitivity of immunoassays, it is possible to use a second antibody to bind to the anti-PCAV antibody, with a label being carried by the second antibody.

### E.4 - The meaning of "diagnosis"

The invention provides a method for diagnosing prostate cancer. It will be appreciated that "diagnosis" according to the invention can range from a definite clinical diagnosis of disease to an indication that the patient should undergo further testing which may lead to a definite diagnosis. For example, the method of the invention can be used as part of a screening process, with positive samples being subjected to further analysis.

Furthermore, diagnosis includes monitoring the progress of cancer in a patient already known to have the cancer. Cancer can also be staged by the methods of the invention. Preferably, the cancer is prostate cancer.

The efficacy of a treatment regimen (therametrics) of a cancer associated can also monitored by the method of the invention *e*.*g*. to determine its efficacy.

Susceptibility to a cancer can also be detected *e*.*g*. where up-regulation of expression has occurred, but before cancer has developed. Prognostic methods are also encompassed.

All of these techniques fall within the general meaning of "diagnosis" in the present invention.

### H - DEFINITIONS

The term "comprising" means "including" as well as "consisting" *e*.*g*. a composition "comprising" X may consist exclusively of X or may include something additional *e*.*g*. X + Y.

The term "about" in relation to a numerical value *x* means, for example, *x*±10%.

The terms "neoplastic cells", "neoplasia", "tumor", "tumor cells", "cancer" and "cancer cells" (used interchangeably) refer to cells which exhibit relatively autonomous growth, so that they exhibit an aberrant growth phenotype characterized by a significant loss of control of cell proliferation (*i*.*e*. de-regulated cell division). Neoplastic cells can be malignant or benign and include prostate cancer derived tissue.

The word "substantially" does not exclude "completely" *e*.*g*. a composition which is "substantially free" from Y may be completely free from Y. Where necessary, the word "substantially" may be omitted from the definition of the invention.

### BRIEF DESCRIPTION OF DRAWINGS

Figure 1 is a phylogenetic tree showing the relationship between various endogenous retroviral LTRs. "Old" and "new" HERV-K LTRs are highlighted.
Figure 2 illustrates the arrangement the PCAV genome at its 5' end.
Figure 3 illustrates the arrangement the PCAV genome at its 3' end.
Figure 4 shows splicing events which take place in a prior art HERV-K ('HTDV' {45}) to produce env and cORF proteins.
Figure 5 illustrates splicing events at the 5' LTRs of PCAV.
Figure 6 illustrates how splicing events at the tandem 5' LTRs of PCAV (Figure 6B) can be distinguished from those in other HERV-Ks (Figure 6A).

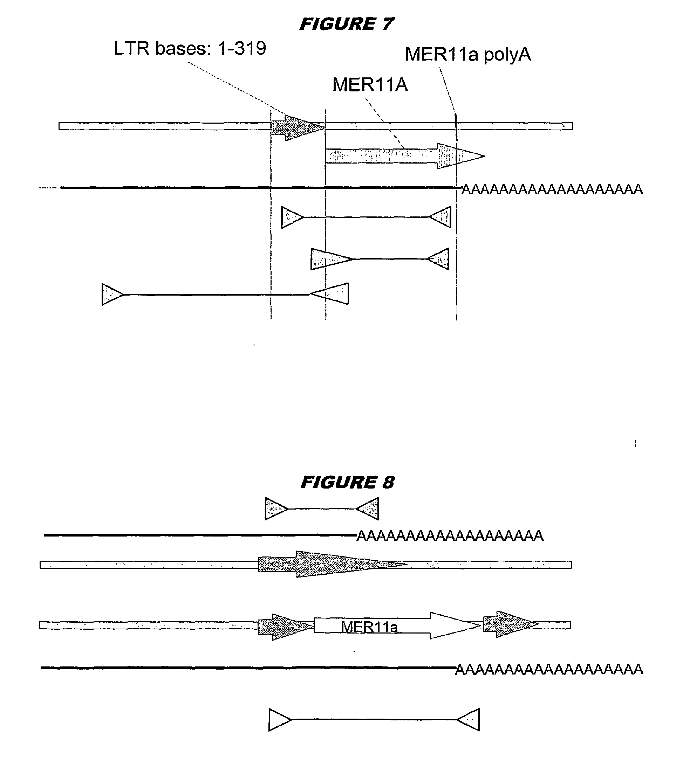

Figure 7 illustrates how primers can be used to specifically detect PCAV mRNA.
Figure 8 illustrates how insertions at the 3' end of PCAV can be exploited to distinguish it from other HERV-Ks.
Figure 9 maps the location of positive array features to the PCAV genome.
Figure 10 shows the results of RT-PCR analysis of the exon 1-2 splicing event in various tissues. Lanes are: (1) markers; (2) placenta; (3) & (4) brain; (5) testis; (6) prostate; (7) breast; (8) uterus; (9) thyroid; (10) cervix; and (11) lung.
Figure 11 shows the results of RT-PCR analysis of the exon 1-2 splicing event in cell lines. Lanes are: (1) and (12) markers; (2) Teral; (3) colo360; (4) PC3; (5) DU145; (6) 22RV1; (7) PCA 2B; (8) LNCaP; (9) RWPE1; (10) RWPE2; and (11) PrEC.
Figure 12 shows fluorescence results obtained using 5G2 monoclonal antibody against: (12B) MDA PCA 2b cells; (12C) PC3 cells; and (12D) NIH3T3 cells. Figure 12A shows MDA PCA 2b cells without 5G2 antibody.

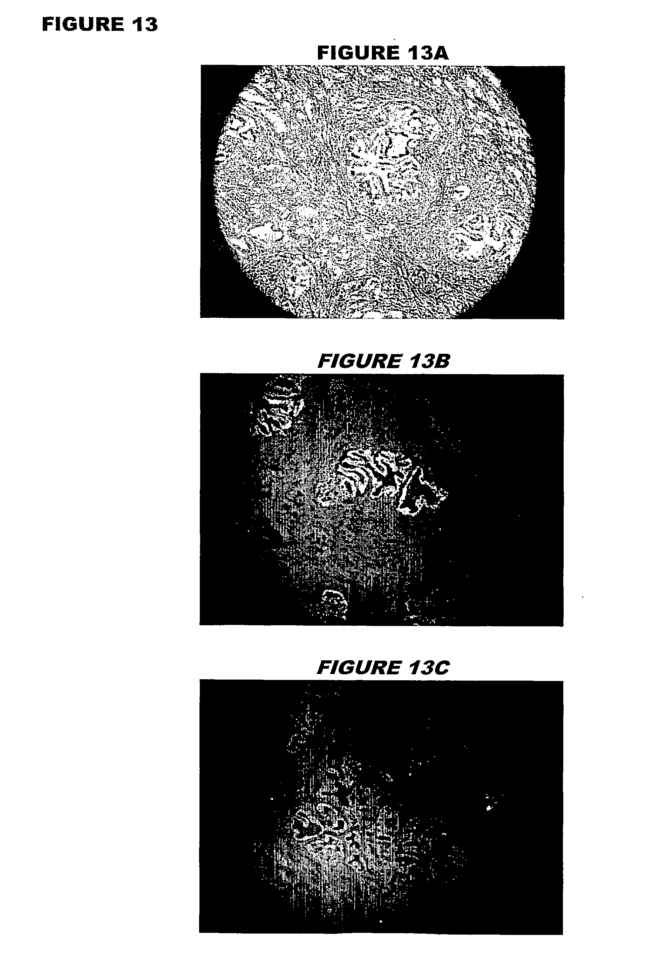

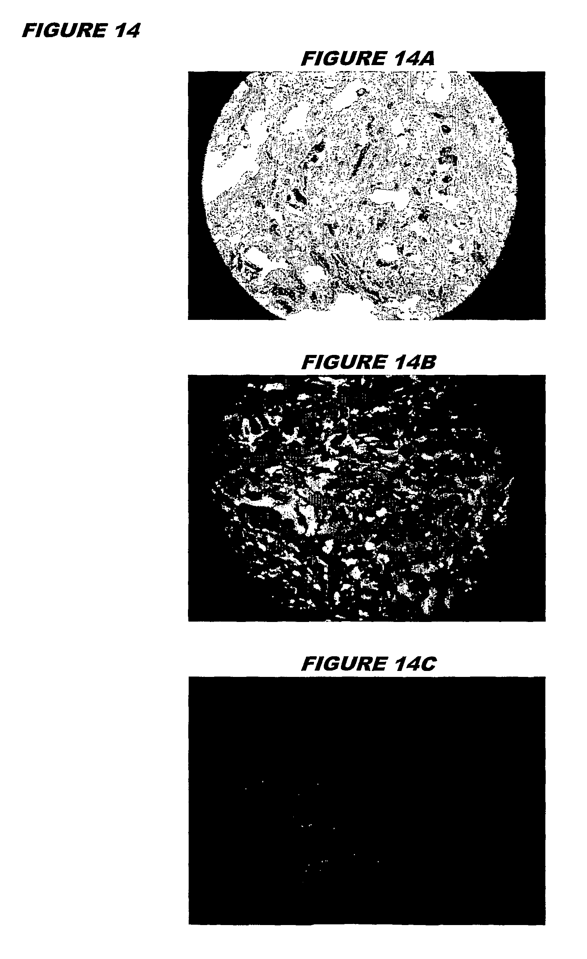

Figures 13 and 14 show staining of prostate tumor samples with (A) hematoxylin & eosin stained, (B) mAb 5G2 plus fluorescein-anti-mouse, or (C) fluorescein-anti-mouse only.
Figure 15 shows expression of HERV-K gag proteins in yeast, with 15A being a stained protein gel and 15B being a western blot.
Figure 16 shows western blots of gag proteins using eight monoclonal antibodies.

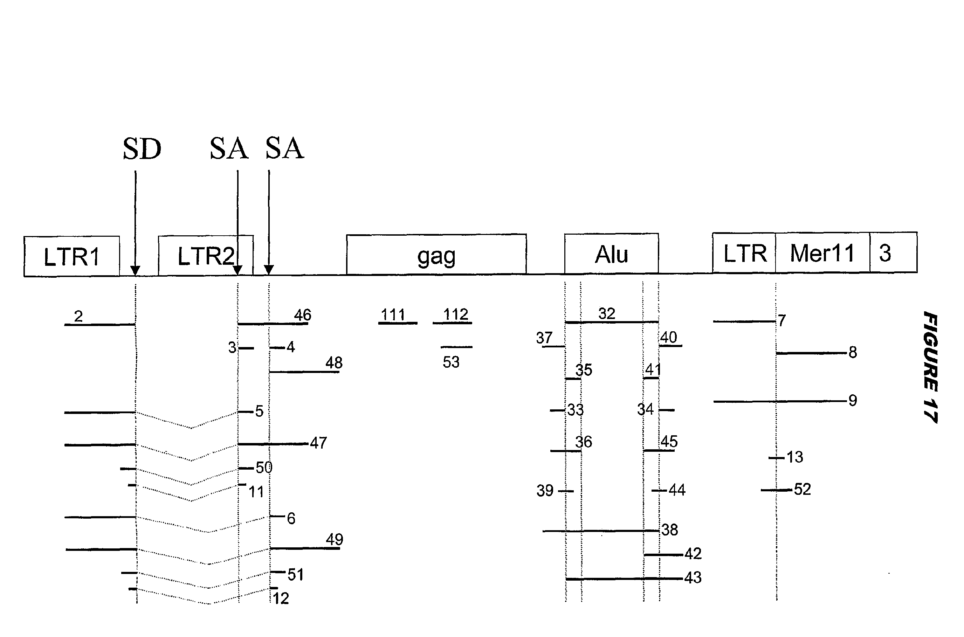

Figure 17 is a not-to-scale schematic of certain SEQ IDs mapped against the genome.
Figure 18 shows microarray analysis of PCAV expression in patient samples. In the expanded portion on the right, the headings indicate Gleason grades of the samples. Red identifies sequences up-regulated in cancer, green identifies those depressed in cancer, and black denotes unchanged spots. Individual sequences are arrayed vertically and patients are presented horizontally. The panel on the left shows all 6000 sequences assayed with RNA from 103 patients, and the region showing almost uniform up-regulation is expanded on the right.
Figure 19 shows the sub-cellular localization of PCAP3 using immuno-staining.
Figure 20 shows PIN staining using anti-gag immunofluorescence. A fresh frozen section of PIN tissue was used, and the assessment of PIN was made by a certified pathologist in an hemotoxylin and eosin stained serial section.
Figure 21 shows TUNEL for cells transfected with PCAP3-encoding adenovirus at moi 100 (top left), 50 (top right), 25 (bottom left), or an untransfected control (bottom right).
Figure 22 shows results from a cell division assay using bromo-deoxyuridine labeling.
Figure 23 shows splicing within the PCAV genome, particularly for env, cORF & PCAP3.
Figure 24 shows the adenovirus vector used in an expression assay to test for LTR activity, and Figure 25 shows the results of GFP expression driven from this vector.
Figure 26 shows the vector used to test the ability of PCAP3 to activate the PCAV LTR.
Figure 27 shows immunofluorescence experiments using an anti-gag monoclonal antibody 5G2 to stain sections of tissue taken from a prostate cancer patient. Figure 27A shows a normal prostate gland, 27B shows atrophied tissue, 27C shows a Gleason grade 3 cancer, and 27D shows a Gleason grade 4 cancer.
Figure 28 shows the position of PCAV-specific primers (*cf.* 5' region of Figure 2), and Figure 29 shows the results of PCR using these primers. 'P' is prostate tissue and 'B' is breast tissue. Figure 30 shows RT-PCR results using the primers. Pairs of matched normal ('N') or cancer ('C') prostate tissue was used, and the signal ratio is given above each pair.
Figure 31 shows quantitative PCR results for various tissues. The y-axis shows PCAV levels normalized to HPRT. The tissues are, from left to right: placenta, fetal brain, fetal heart, fetal liver, brain, heart, liver, pancreas, stomach, small intestine, colon, rectum, testicle, prostate (47 year old man), ovary, adrenal, thyroid, kidney, bladder, breast, uterus, cervix, skeletal muscle, lung, spleen, thymus, skin.
Figure 32 shows the age-related increase in PCAV mRNA expression in prostate tissue.
Figure 33 shows the results of a RT-PCR scanning assay used to map the 5' end of PCAV mRNAs.
Figure 34 gives details of a RNase protection assay. Two antisense probes were used - a long probe (24B) and a short probe (24C). Both probes protected the region shown in 24A. In 24B, the position of the band expected based on the 'usual' 5' end based on the position of the TATA signal is shown, plus the actual band achieved. The three lanes in 24B are: (1) Teral; (2) no RNA; (3) probe, no RNase. The two lanes in 24C are: (1) Tera1; (2) probe, no RNase.

### MODES FOR CARRYING OUT THE INVENTION

Certain aspects of the present invention are described in greater detail in the non-limiting examples that follow. The examples are put forth so as to provide those of ordinary skill in the art with a disclosure and description of how to make and use the present invention, and are not intended to limit the scope of what the inventors regard as their invention nor are they intended to represent that the experiments below are all and only experiments performed. Efforts have been made to ensure accuracy with respect to numbers used (*e*.*g*. amounts, temperature, *etc*.) but some experimental errors and deviations should be accounted for. Unless indicated otherwise, parts are parts by weight, molecular weight is weight average molecular weight, temperature is in degrees Celsius, and pressure is at or near atmospheric.

### Source of human prostate cell samples and isolation of nucleic acids expressed by them

Candidate nucleic acids that may represent genes differentially expressed in cancer were obtained from both publicly-available sources and from cDNA libraries generated from selected cell lines and patient tissues. A normalized cDNA library was prepared from one patient tumor tissue and cloned nucleic acids for spotting on microarrays were isolated from the library. Normal and tumor tissues from 100 patients were processed to generate T7 RNA polymerase transcribed nucleic acids, which were, in turn, assessed for expression in the microarrays.

Normalization: The objective of normalization is to generate a cDNA library in which all transcripts expressed in a particular cell type or tissue are equally represented {refs. 160 & 161}, and therefore isolation of as few as 30,000 recombinant clones in an optimally normalized library may represent the entire gene expression repertoire of a cell, estimated to number 10,000 per cell. The source materials for generating the normalized prostate libraries were cryopreserved prostate tumor tissue from a patient with Gleason grade 3+3 adenocarcinoma and normal prostate biopsies from a pool of at-risk subjects under medical surveillance. Prostate epithelia were harvested directly from frozen sections of tissue by laser capture microdissection (LCM, Arcturus Engineering Inc., Mountain View, CA), carried out according to methods well known in the art (*e*.*g*. ref. 162), to provide substantially homogenous cell samples.

Total RNA was extracted from LCM-harvested cells using RNeasy™ Protect Kit (Qiagen, Valencia, CA), following manufacturer's recommended procedures. RNA was quantified using RiboGreen™ RNA quantification kit (Molecular Probes, Inc. Eugene, OR). One µg of total RNA was reverse transcribed and PCR amplified using SMART™ PCR cDNA synthesis kit (ClonTech, Palo Alto, CA). The cDNA products were size-selected by agarose gel electrophoresis using standard procedures (ref. 21). The cDNA was extracted using Bio 101Geneclean® II kit (Qbiogene, Carlsbad, CA). Normalization of the cDNA was carried out using kinetics of hybridization principles: 1.0 µg of cDNA was denatured by heat at 100° C for 10 minutes, then incubated at 42°C for 42 hours in the presence of 120 mM NaCl, 10 mM Tris.HCl (pH=8.0), 5 mM EDTA.Na⁺ and 50% formamide. Single-stranded cDNA ("normalized" cDNA) was purified by hydroxyapatite chromatography (#130-0520, BioRad, Hercules, CA) following the manufacturer's recommended procedures, amplified and converted to double-stranded cDNA by three cycles of PCR amplification, and cloned into plasmid vectors using standard procedures (ref. **21).** All primers/adaptors used in the normalization and cloning process are provided by the manufacturer in the SMART™ PCR cDNA synthesis kit (ClonTech, Palo Alto, CA). Supercompetent cells (XL-2 Blue Ultracompetent Cells, Stratagene, California) were transfected with the normalized cDNA libraries, plated on plated on solid media and grown overnight at 36°C.

Characterization of normalized libraries: The sequences of 10,000 recombinants per library were analyzed by capillary sequencing using the ABI PRISM 3700 DNA Analyzer (Applied Biosystems, California). To determine the representation of transcripts in a library, BLAST analysis was performed on the clone sequences to assign transcript identity to each isolated clone, i.e. the sequences of the isolated nucleic acids were first masked to eliminate low complexity sequences using the XBLAST masking program (refs. 163, 164 and 165). Generally, masking does not influence the final search results, except to eliminate sequences of relative little interest due to their low complexity, and to eliminate multiple "hits" based on similarity to repetitive regions common to multiple sequences *e*.*g*. Alu repeats. The remaining sequences were then used in a BLASTN *vs*. GenBank search. The sequences were also used as query sequence in a BLASTX *vs*. NRP (non-redundant proteins) database search.

Automated sequencing reactions were performed using a Perkin-Elmer PRISM Dye Terminator Cycle Sequencing Ready Reaction Kit containing AmpliTaq DNA Polymerase, FS, according to the manufacturer's directions. The reactions were cycled on a GeneAmp PCR System 9600 as per manufacturer's instructions, except that they were annealed at 20° C. or 30° C. for one minute. Sequencing reactions were ethanol precipitated, pellets were resuspended in 8 microliters of loading buffer, 1.5 microliters was loaded on a sequencing gel, and the data was collected by an ABI PRISM 3700 DNA Sequencer. (Applied Biosystems, Foster City, CA).

The number of times a sequence is represented in a library is determined by performing sequence identity analysis on cloned cDNA sequences and assigning transcript identity to each isolated clone. First, each sequence was checked to see if it was a mitochondrial, bacterial or ribosomal contaminant. Such sequences were excluded from the subsequent analysis. Second, sequence artifacts (*e*.*g*. vector and repetitive elements) were masked and/or removed from each sequence.

The remaining sequences were compared via BLAST {166} to GenBank and EST databases for gene identification and were compared with each other via FastA {167} to calculate the frequency of cDNA appearance in the normalized cDNA library. The sequences were also searched against the GenBank and GeneSeq nucleotide databases using the BLASTN program (BLASTN 1.3MP {166}). Fourth, the sequences were analyzed against a non-redundant protein (NRP) database with the BLASTX program (BLASTX 1.3MP {166}). This protein database is a combination of the Swiss-Prot, PIR, and NCBI GenPept protein databases. The BLASTX program was run using the default BLOSUM-62 substitution matrix with the filter parameter: "xnu+seg". The score cutoff utilized was 75.

Assembly of overlapping clones into contigs was done using the program Sequencher (Gene Codes Corp.; Ann Arbor, Mich.). The assembled contigs were analyzed using the programs in the GCG package (Genetic Computer Group, University Research Park, 575 Science Drive, Madison, Wis. 53711) Suite Version 10.1.

### Detection of elevated levels of cDNA associated with prostate cancer using arrays

cDNA sequences representing a variety of candidate genes to be screened for differential expression in prostate cancer were assayed by hybridization on nucleic acid arrays. The cDNA sequences included cDNA clones isolated from cell lines or tissues as described above. The cDNA sequences analyzed also included nucleic acids comprising sequence overlap with sequences in the Unigene database, and which encode a variety gene products of various origins, functionality, and levels of characterization. cDNAs were spotted onto reflective slides (Amersham) according to methods well known in the art at a density of 9,216 spots per slide representing 4608 sequences (including controls) spotted in duplicate, with approximately 0.8 µl of an approximately 200ng/µl solution of cDNA.

PCR products of selected cDNA clones corresponding to the gene products of interest were prepared in a 50% DMSO solution. These PCR products were spotted onto Amersham aluminum microarray slides at a density of 9216 clones per array using a Molecular Dynamics Generation III spotting robot. Clones were spotted in duplicate, giving 4608 different sequences per array.

cDNA probes were prepared from total RNA obtained by laser capture microdissection (LCM, Arcturus Enginering Inc., Mountain View, CA) of tumor tissue samples and normal tissue samples isolated from the patients described above.

Total RNA was first reverse transcribed into cDNA using a primer containing a T7 RNA polymerase promoter, followed by second strand DNA synthesis. cDNA was then transcribed *in vitro* to produce antisense RNA using the T7 promoter-mediated expression (*e*.*g*. ref. 168), and the antisense RNA was then converted into cDNA. The second set of cDNAs were again transcribed *in vitro,* using the T7 promoter, to provide antisense RNA. This antisense RNA was then fluorescently labeled, or the RNA was again converted into cDNA, allowing for third round of T7-mediated amplification to produce more antisense RNA. Thus the procedure provided for two or three rounds of *in vitro* transcription to produce the final RNA used for fluorescent labeling. Probes were labeled by making fluorescently labeled cDNA from the RNA starting material. Fluorescently-labeled cDNAs prepared from the tumor RNA sample were compared to fluorescently labeled cDNAs prepared from normal cell RNA sample. For example, the cDNA probes from the normal cells were labeled with Cy3 fluorescent dye (green) and cDNA probes prepared from the tumor cells were labeled with Cy5 fluorescent dye (red).

The differential expression assay was performed by mixing equal amounts of probes from tumor cells and normal cells of the same patient. The arrays were pre-hybridized by incubation for about 2 hrs at 60°C in 5X SSC/0.2% SDS/1 mM EDTA, and then washed three times in water and twice in isopropanol. Following pre-hybridization of the array, the probe mixture was then hybridized to the array under conditions of high stringency (overnight at 42°C in 50% formamide, 5X SSC, and 0.2% SDS. After hybridization, the array was washed at 55°C three times as follows: 1) first wash in 1X SSC/0.2% SDS; 2) second wash in 0.1X SSC/0.2% SDS; and 3) third wash in 0.1X SSC.

The arrays were then scanned for green and red fluorescence using a Molecular Dynamics Generation III dual color laser-scanner/detector. The images were processed using BioDiscovery Autogene software, and the data from each scan set normalized. The experiment was repeated, this time labeling the two probes with the opposite color in order to perform the assay in both "color directions." Each experiment was sometimes repeated with two more slides (one in each color direction). The data from each scan was normalized, and the level fluorescence for each sequence on the array expressed as a ratio of the geometric mean of 8 replicate spots/genes from the four arrays or 4 replicate spots/gene from 2 arrays or some other permutation.

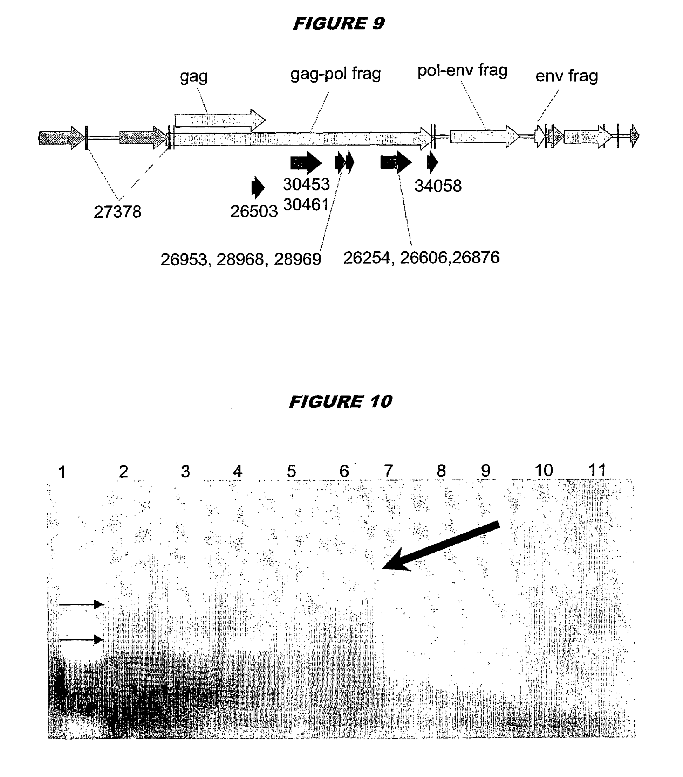

Array features which were found to give elevated signals using prostate tumor tissue were sequenced and mapped to the human genome sequence. The elevated array spots features span about 90% of PCAV and the locations of 11 such sequences on the PCAV genome are shown in figure 9, with five-digit numbers being the codes for individual array features.

Although some of the 11 elevated sequences come from regions in the genome which are highly conserved among the HERV-K HML2.0 family, and will thus not be specific for the virus at megabase 20.428 of chromosome 22, other spots are not.

### Sequence 27378

27378 (SEQ ID 14) is present at elevated levels in prostate tumors. It aligns to two separate regions of the genomic DNA sequence on chromosome 22 (nucleotides 977-1075 & 2700-2777 of SEQ ID 1):

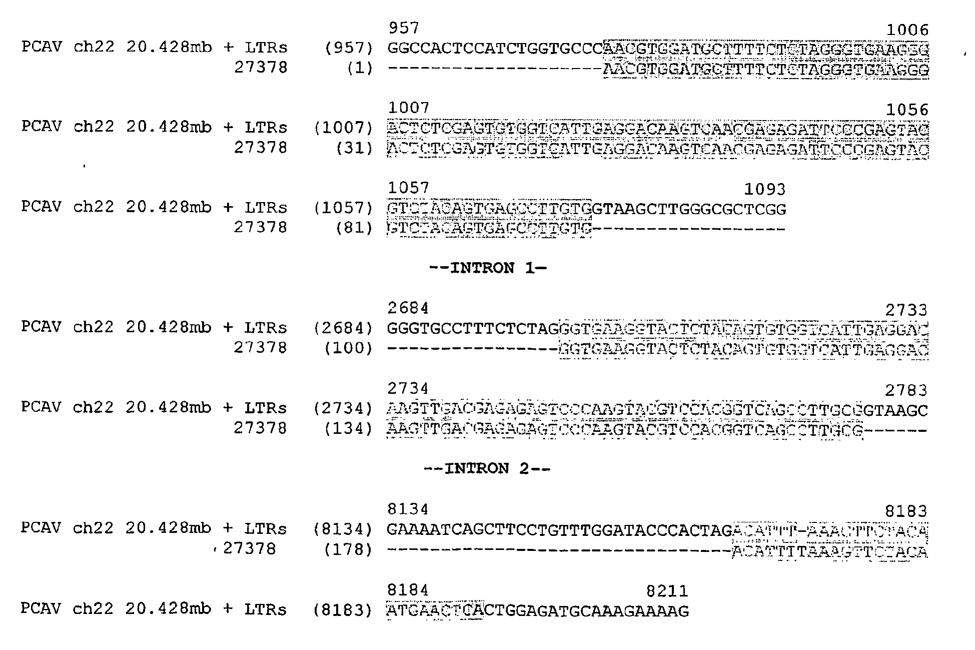

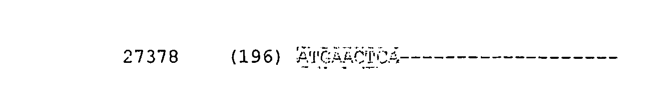

Within SEQ ID 1, nucleotides 1076-1077 are GT and nucleotides 2698-2699 are AG, these being consensus splice donor and acceptor sequences, respectively. Hybridization to 27378 thus verifies splicing in which the first 5' LTR is joined to the splice acceptor site near the 3' end of the second 5' LTR (joins nucleotide 1075 of SEQ ID 1 to nucleotide 2700). Because the sequences in the two exons are from two different viruses (old and new), and these are significantly different from other family new and old family members, it is unlikely that the 27378 product was transcribed from a HERV-K other than PCAV.

### Sequence 34058

Spot 34058 (SEQ ID 15) is highly elevated in prostate tumor tissue. Its sequence spans an alternative splice site that occurs in some "old" genomes and that connects the envelope ATG to a splice acceptor site near the 3' LTR. The sequence matches PCAV more closely (single mismatch at 2443) than the related HERV-Ks found on chromosomes 3 and 6:

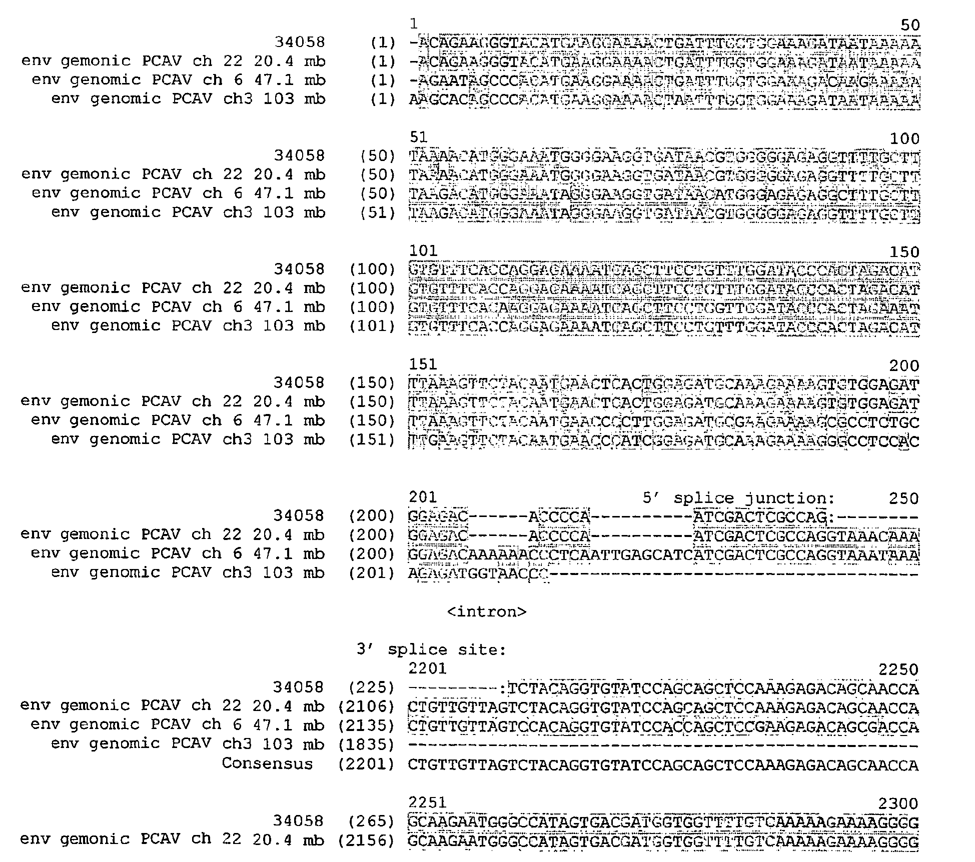

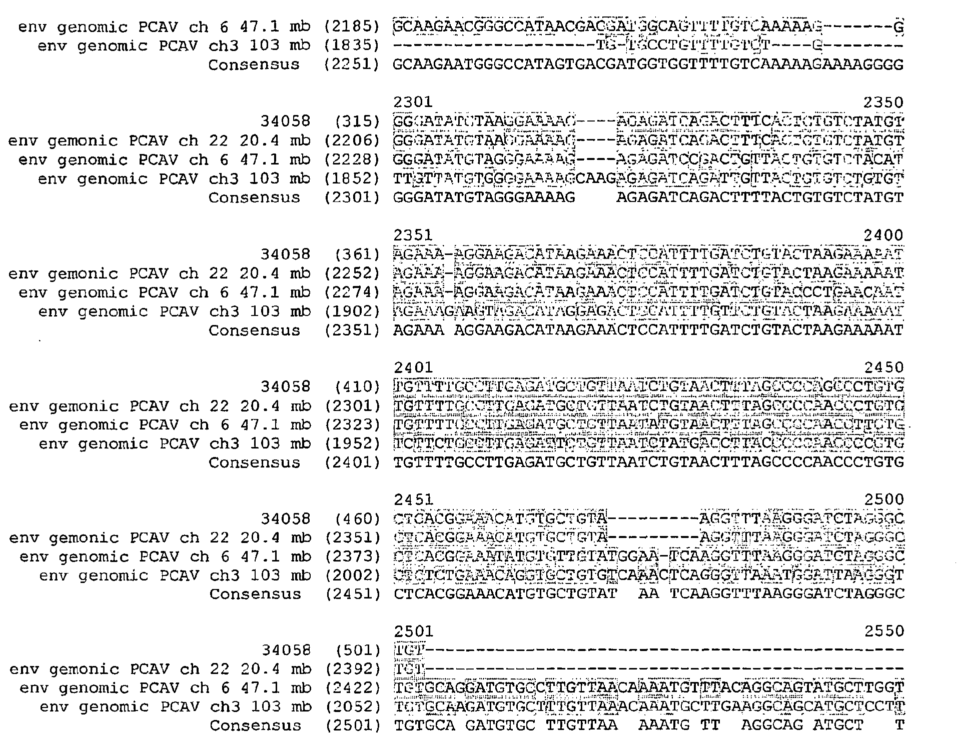

### Sequence 26254

Signal from sequence 26254 on the array was elevated in prostate tumor tissue compared to normal tissue. The 26254 sequence (SEQ ID 16) aligns almost perfectly to chromosome 22 contigs AP000345 (SEQ ID 17 = nucleotides 63683-64332 of AP000345) and AP000346 (SEQ ID 18 = nucleotides 26271-26920 of AP000346) (nucleotides 7065-7701 of SEQ ID 1):

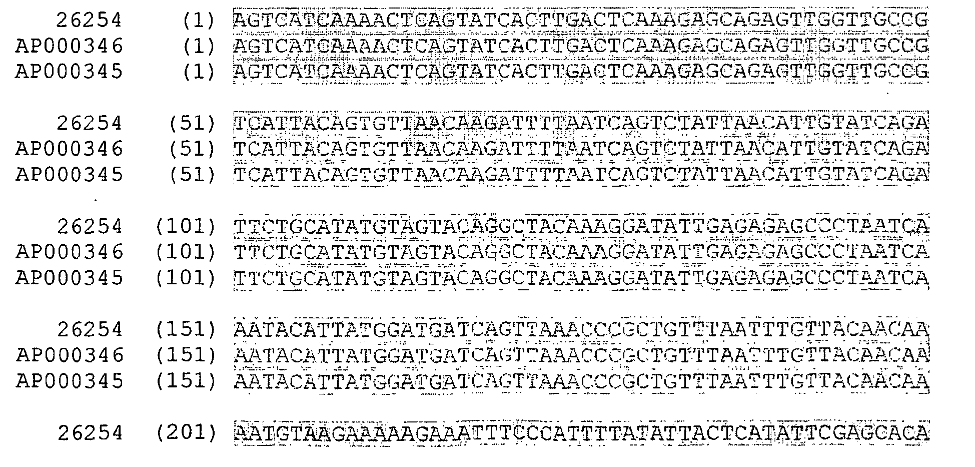

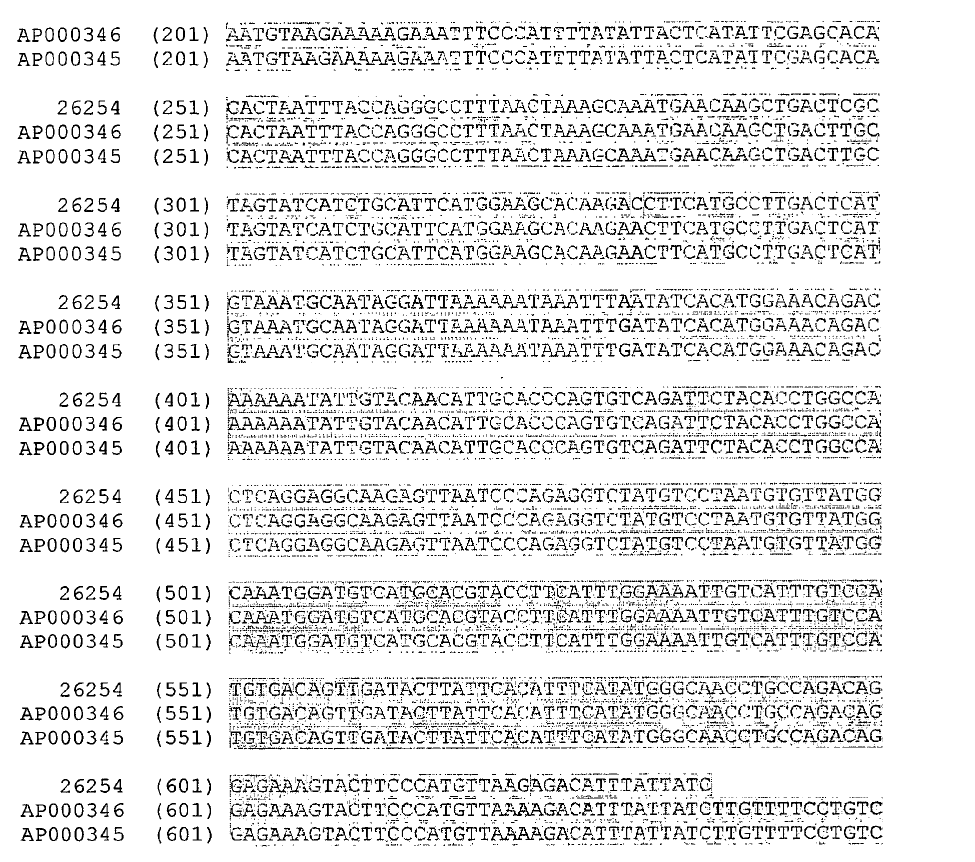

The four point mutations relative to the chromosome 22 sequence could represent sequencing errors (either for the chromosome or for 26254) or could, alternatively, be SNPs within the human genome.

PCAV is most closely related to HERV-Ks found on chromosomes 3 and 6. Alignment of the chromosome 3, 6 and 22 viruses in the region of 26254 shows that it is unlikely that 26254 is derived from chromosome 3 or 6 and that it is most likely derived from a chromosome 22 PCAV transcript:

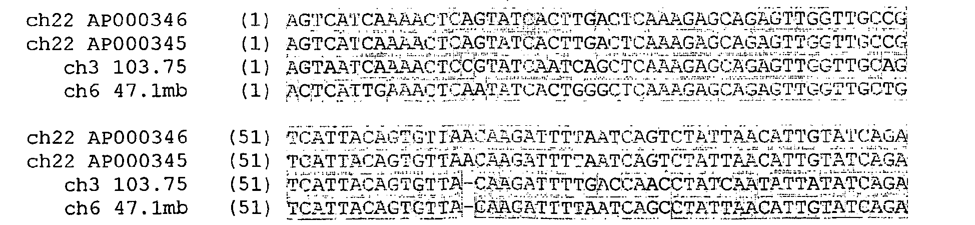

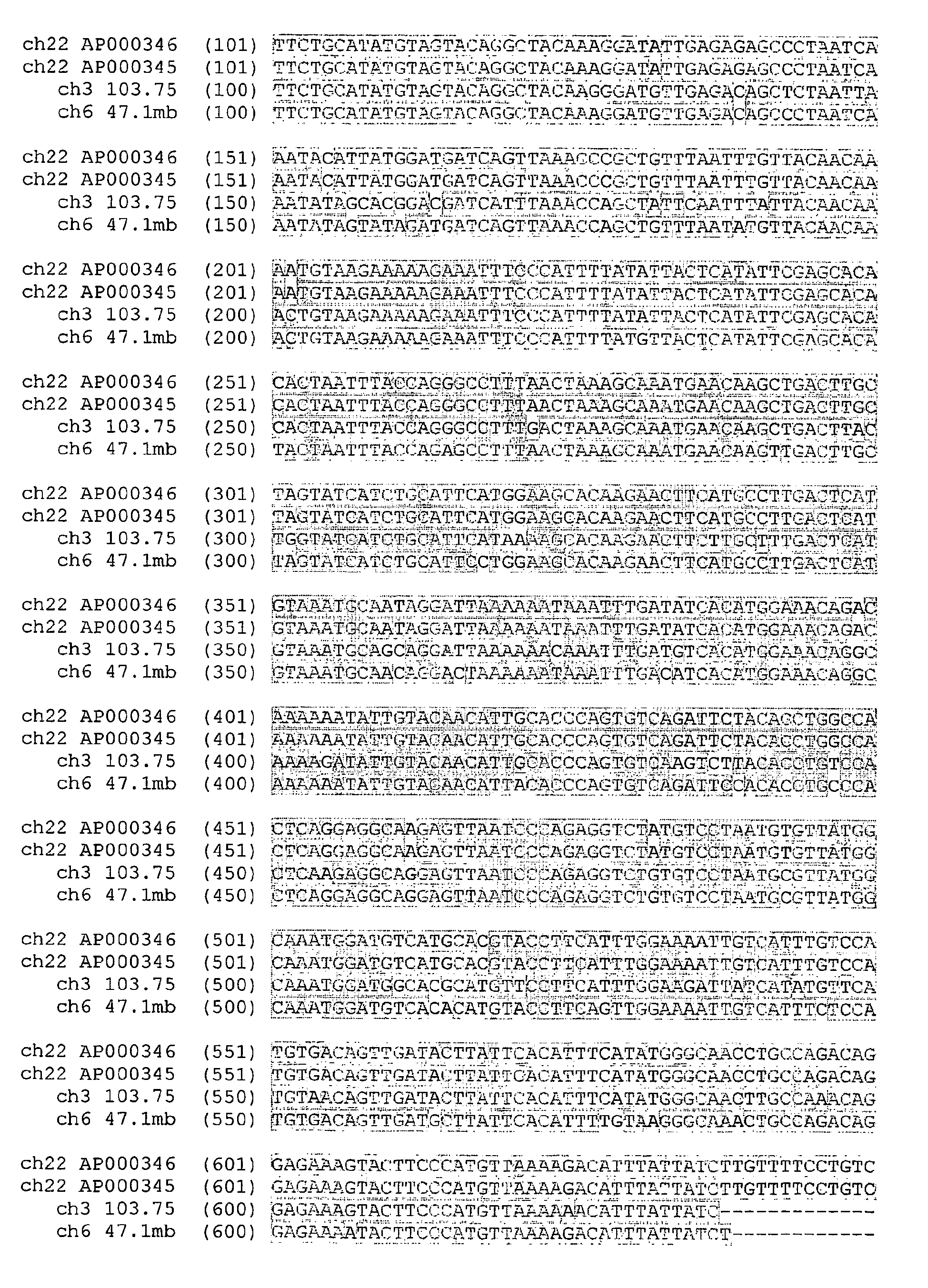

Although the HERVs on chromosomes 3, 6 and 22 are closely-related, therefore, they can be distinguished by hybridization.

### Sequence 30453

Signal from sequence 30453 on the array was elevated in prostate tumor tissue compared to normal tissue. The 30453 sequence (SEQ ID 113) aligns with chromosome 22:

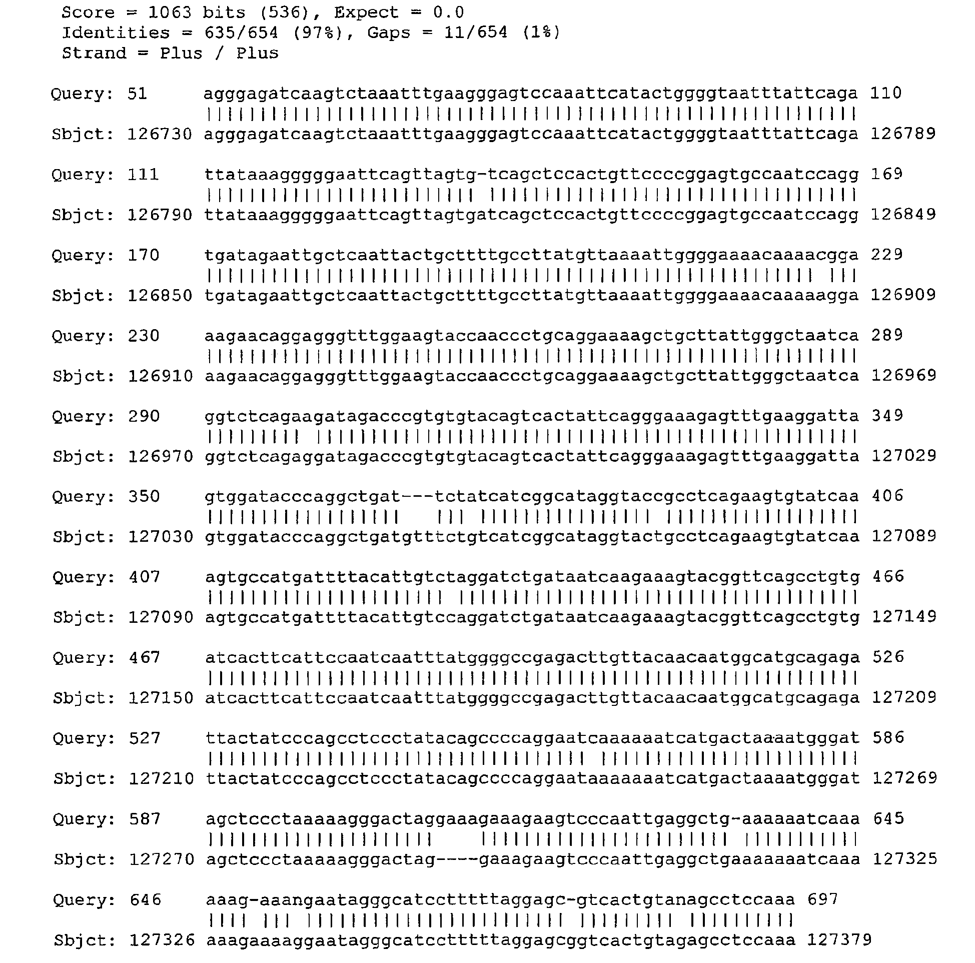

### Sequence 26503

Signal from sequence 26503 on the array was elevated in prostate tumor tissue compared to normal tissue. The 26503 sequence (SEQ ID 116) aligns with chromosome 22:

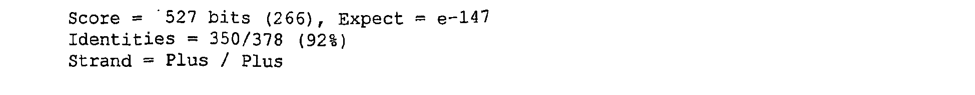

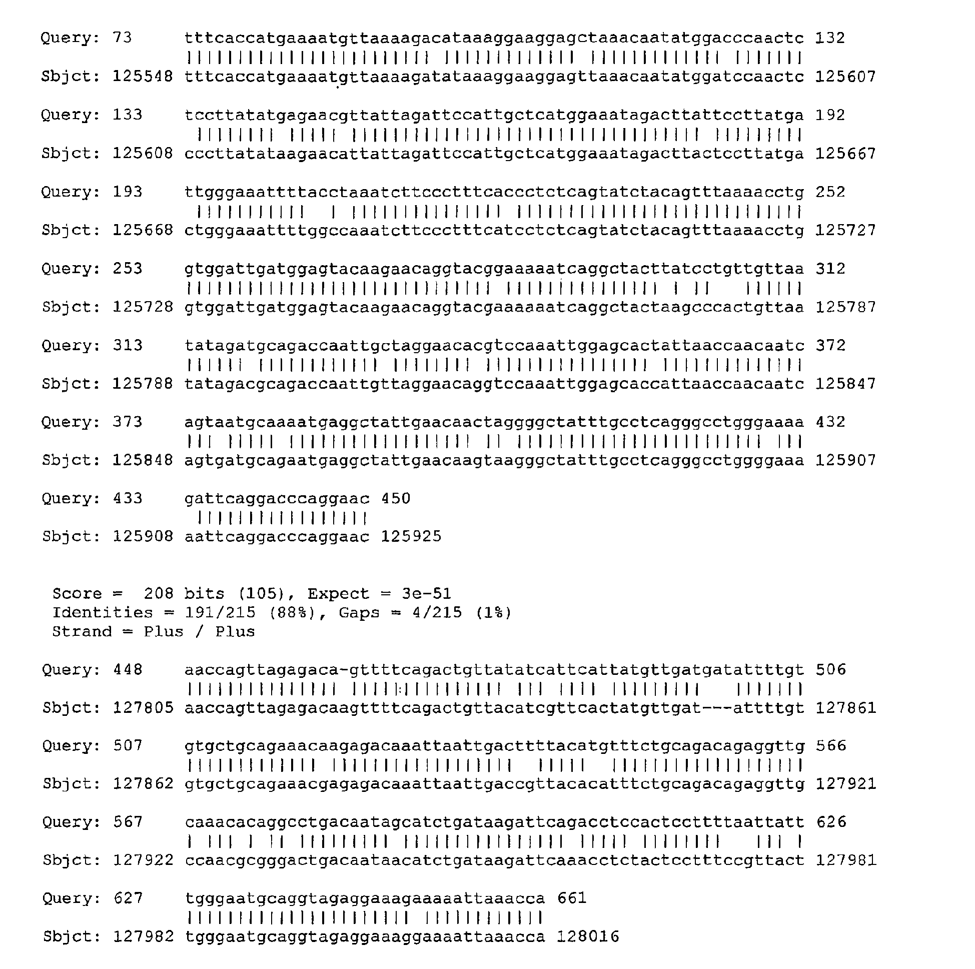

### Patient libraries

HERV-K HML2.0 cDNAs cloned from patient libraries align with PCAV. Clones from libraries derived from four patients align with >95% identity to PCAV.

SEQ ID 19 is from a cDNA which is present at elevated levels in prostate tumors. The first 463 of its 470 nucleotides align to four separate regions of the genomic DNA sequence on chromosome 22 (nucleotides 956-1075, 2700-2777, 8166-8244 & 10424-10609 of SEQ ID 1):

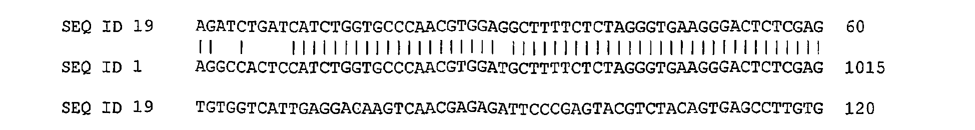

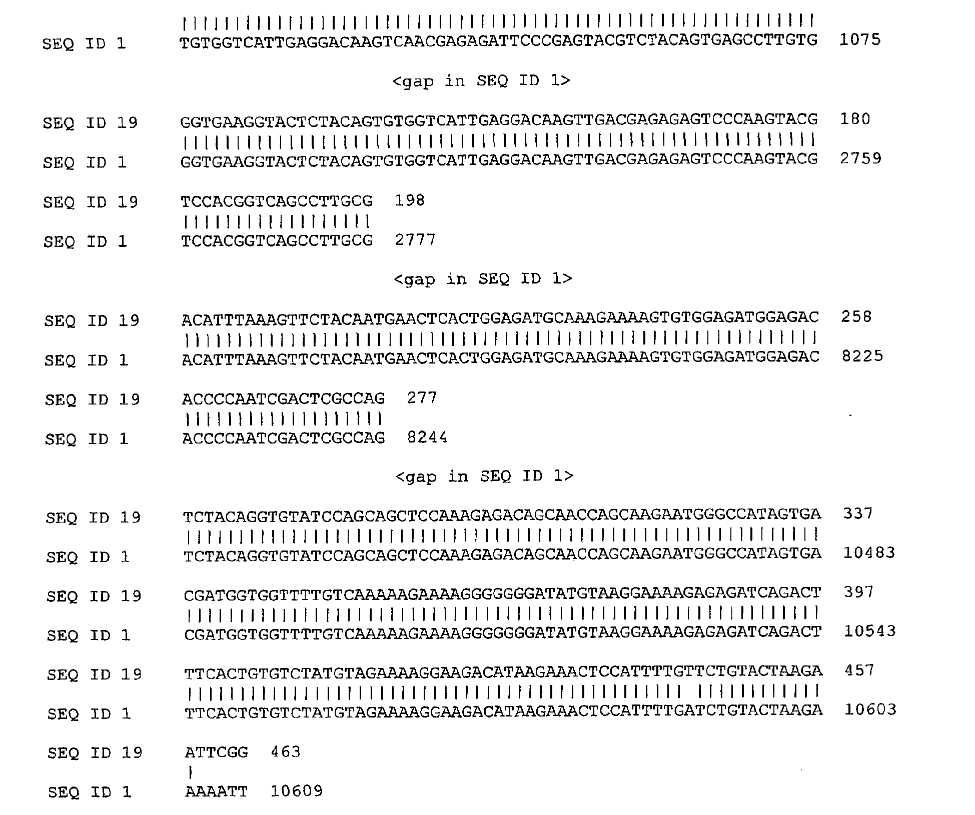

The dinucleotide sequences before and after the "gaps" in SEQ ID 1 are as follows:

| **SEQ ID 19** | **Exon** | **SEQ ID 1** | **Preceding and following dinucleotide in SEQ ID 1** | |
|---|---|---|---|---|
| 1-120 | 1 | 956-1075 | - | 1076-1077: GT |
| 121-198 | 2 | 2700-2777 | 2698-2699: AG | 2778-2779: GT |
| 199-277 | 3 | 8166-8244 | 8164-8165: AG | 8245-8246: GT |
| 278-463 | 4 | 10424-10609 | 10422-10423:AG | - |

The "gaps" in SEQ ID 1 thus begin and end with consensus splice donor and acceptor sequences. The presence of SEQ ID 19 in a cDNA thus verifies splicing in which the first 5' LTR is joined to the splice acceptor site near the 3' end of the second 5' LTR (nucleotide 1075 of SEQ ID 1 joined to nucleotide 2700), as well as other splicing events. Because the sequences in exons 1 and 2 are from two different viruses (old and new), and these are significantly different from other family new and old family members, it is unlikely that the SEQ ID 19 product was transcribed from a HERV-K other than PCAV.

SEQ ID 114 (035JN013.F03-FIS) aligns with available chromosome 22 sequence:

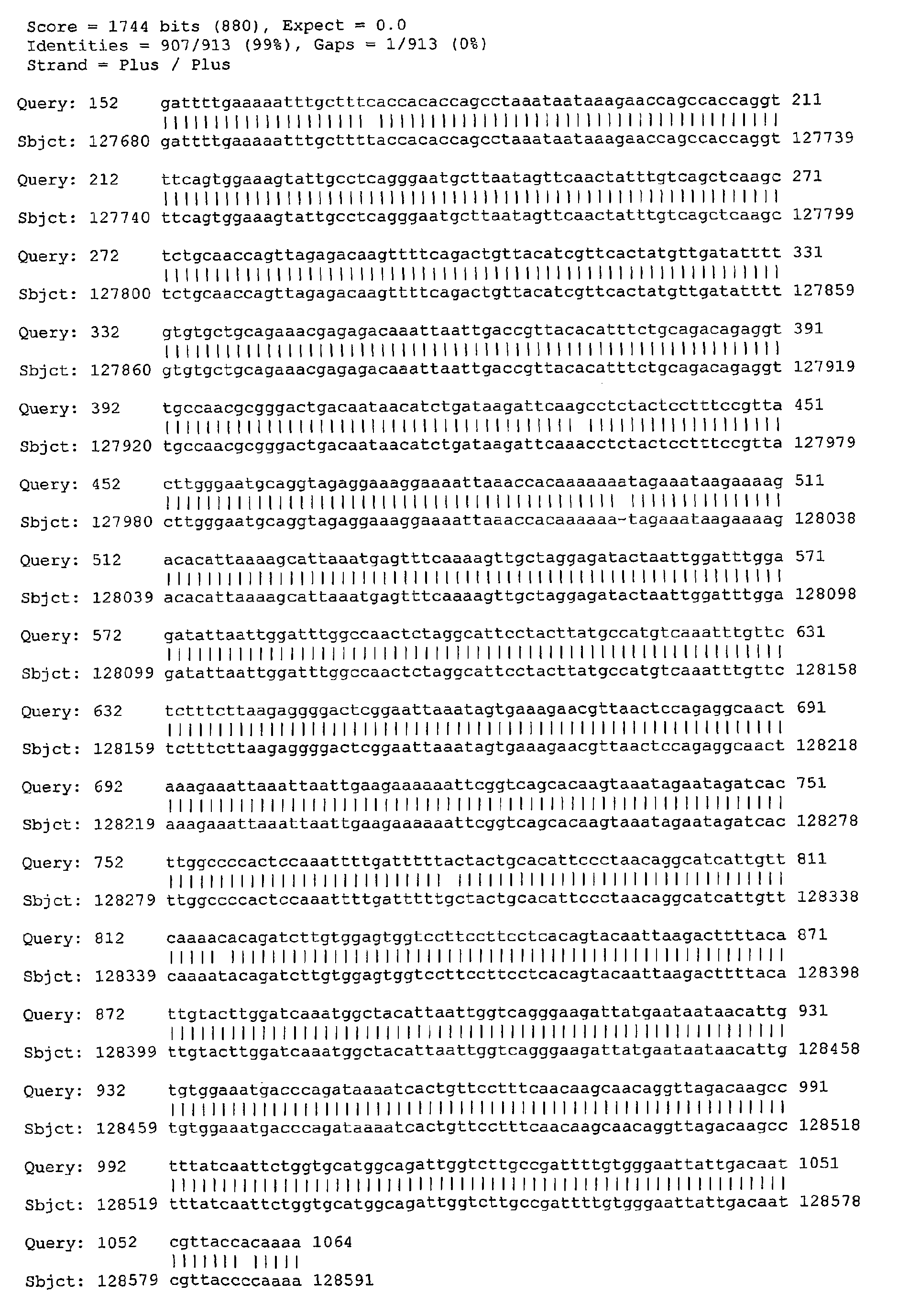

SEQ ID 115 (035JN015.H02-FIS) aligns with available chromosome 22 sequence:

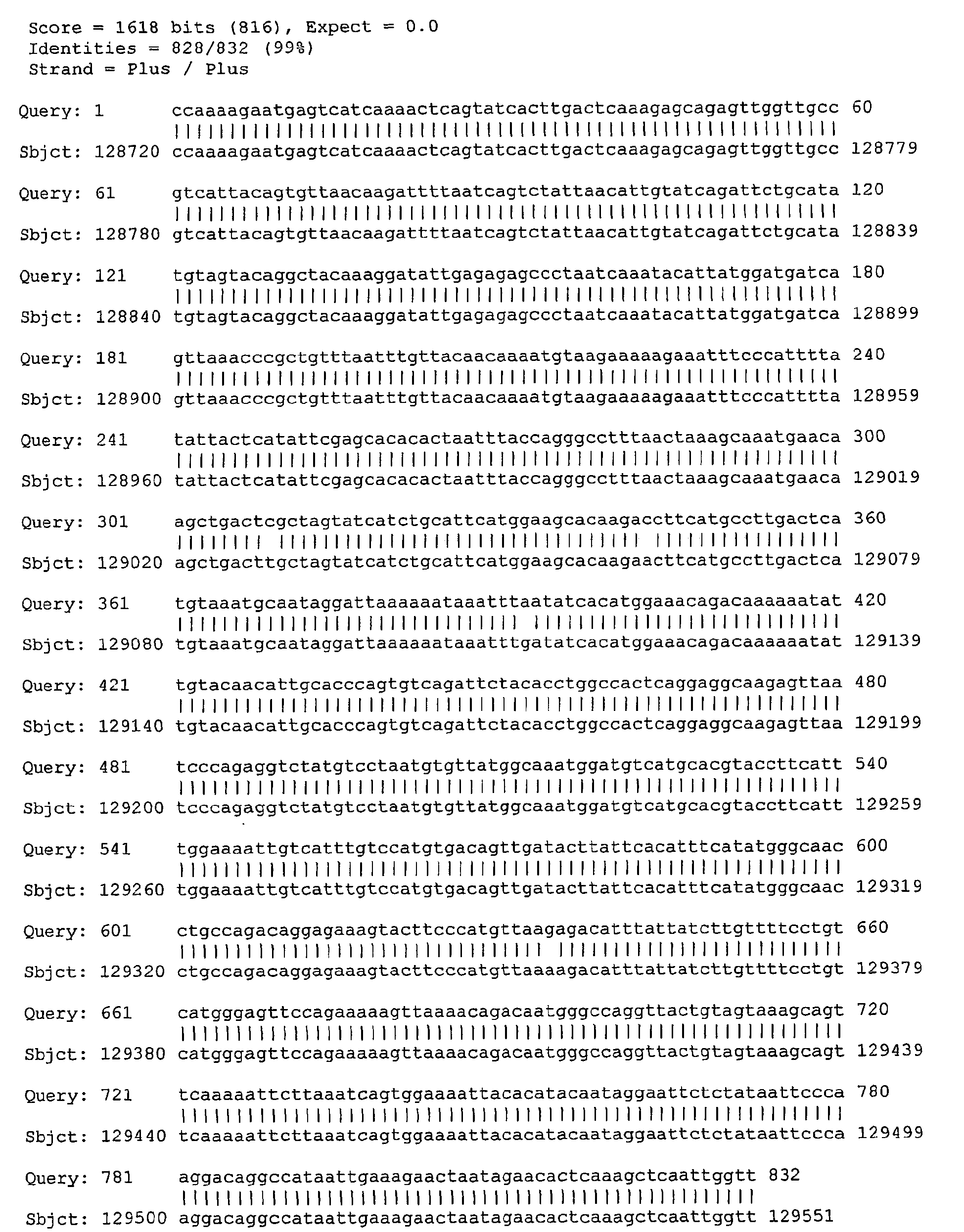

SEQ ID 117 (035JN003.E06-FIS) aligns with available chromosome 22 sequence:

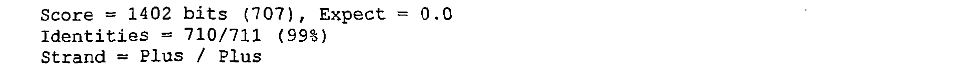

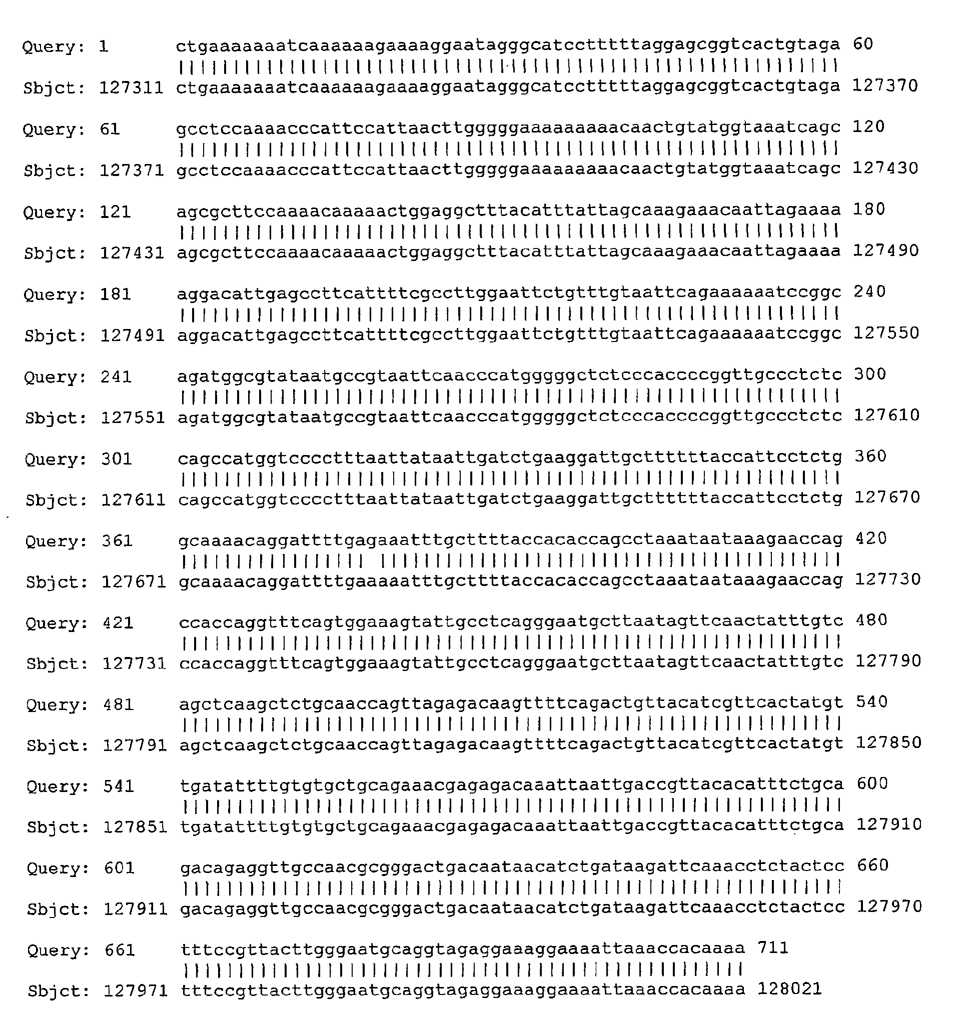

SEQ ID 118 (035JN013.C11) aligns with available chromosome 22 sequence:

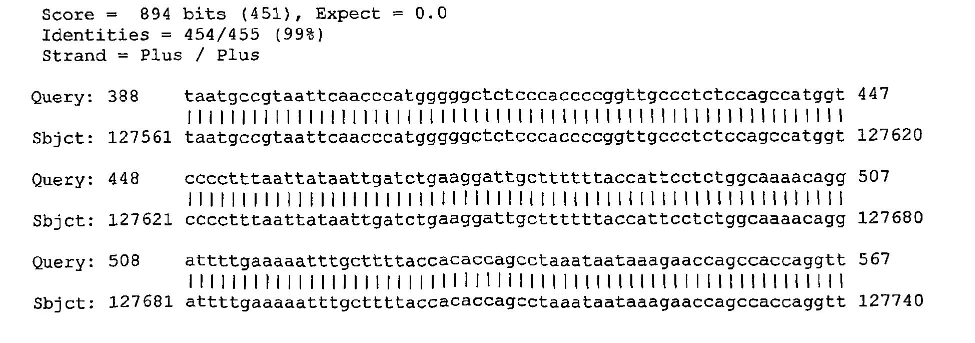

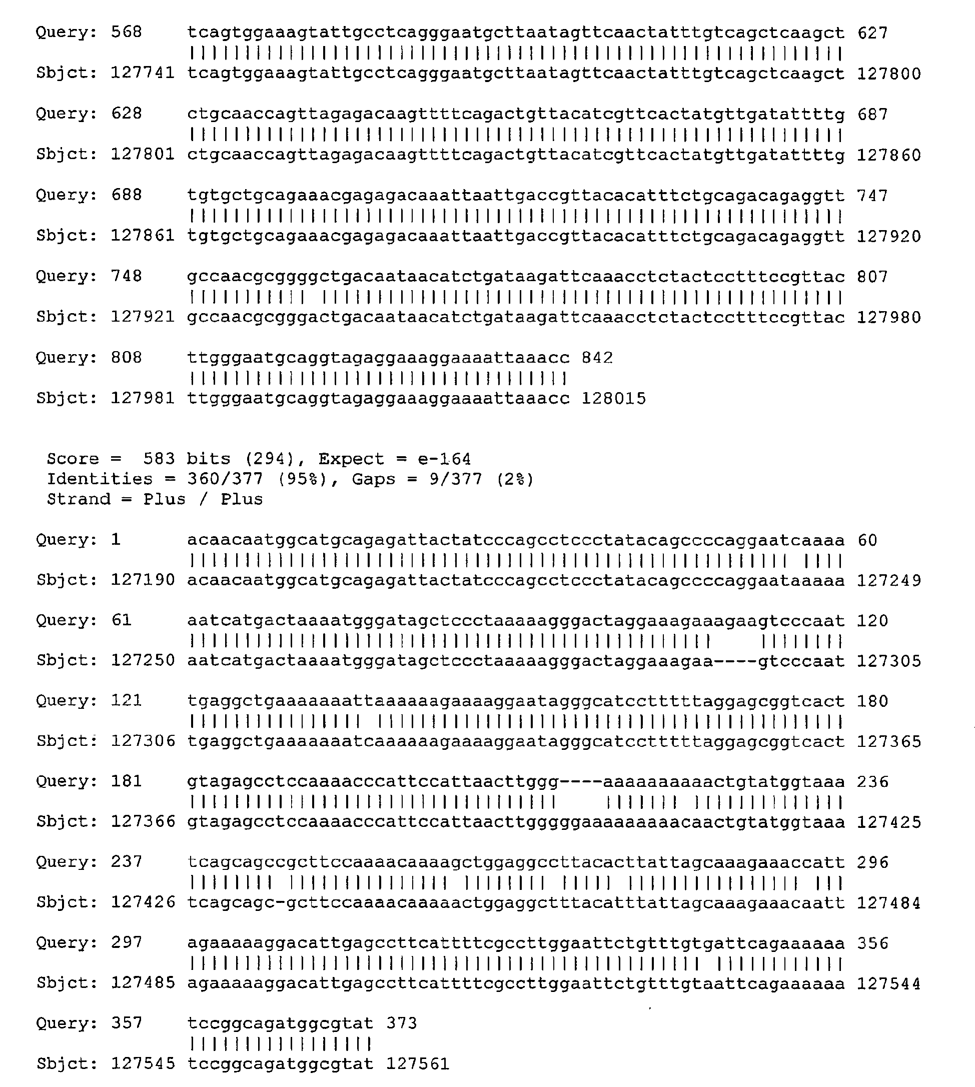

SEQ ID 119 (035JN001.F06) aligns with available chromosome 22 sequence:

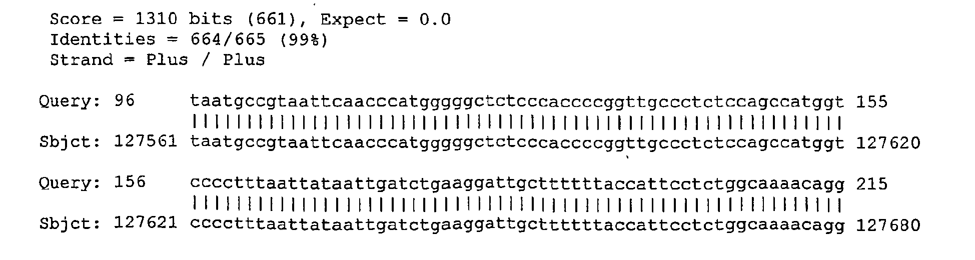

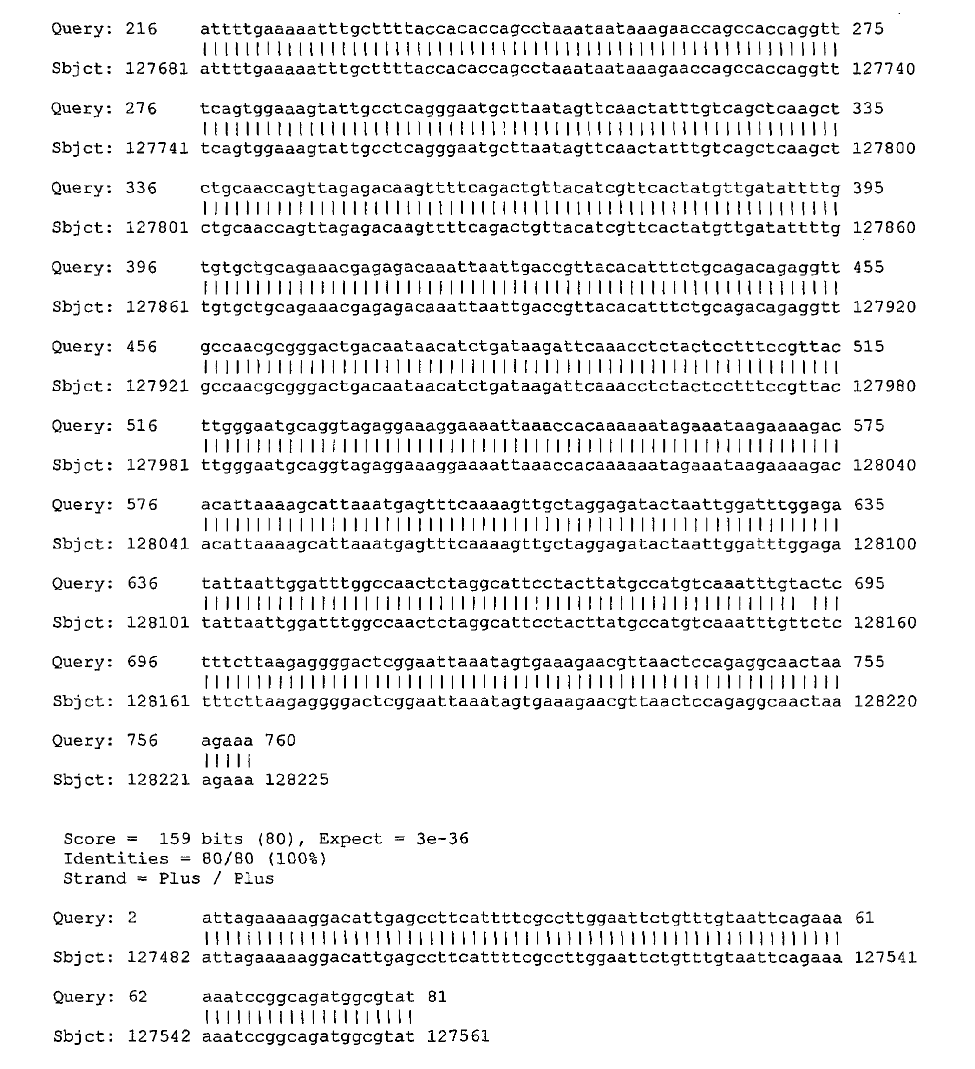

### Patient tumor samples

Fresh frozen prostate cancer tissue from two patients was cut in 10 micron sections, mounted on glass slides, and stained with murine monoclonal antibody 5G2. The staining was visualized with a second antibody (fluorescein-coupled goat anti-mouse). Staining was found to be specific for cancerous tissue. The samples were also analyzed by hybridization to 26254 and signal was 35-40 times stronger than in control samples from the same patient:

| **patient ID#** | **Gleason grade** | **5G2 staining** | **spot 26254 ratio** |
|---|---|---|---|
| 101 | 3+3 | + (Figure 13) | 35 |
| 153 | 3+3 | + (Figure 14) | 40 |

### RT-PCR

RNA extracts from various tissues were analyzed by RT-PCR. In particular, the splicing event between exons 1 and 2 was investigated using primers as shown in figure 6. Results are shown in figure 10. All lanes show background levels of HERV-K HML2.0 (*i*.*e*. new virus) expression (thin lines) but prostate tissue (lane 6) shows a longer product (thick line), indicating expression of a HERV-K with a longer sequence between the 5' LTR and the start of ENV. The difference in length between the long lane 6 product and the background product seen in other tissues (-80 bp) corresponds in length to the length of exon 2 illustrated in figure 6B.

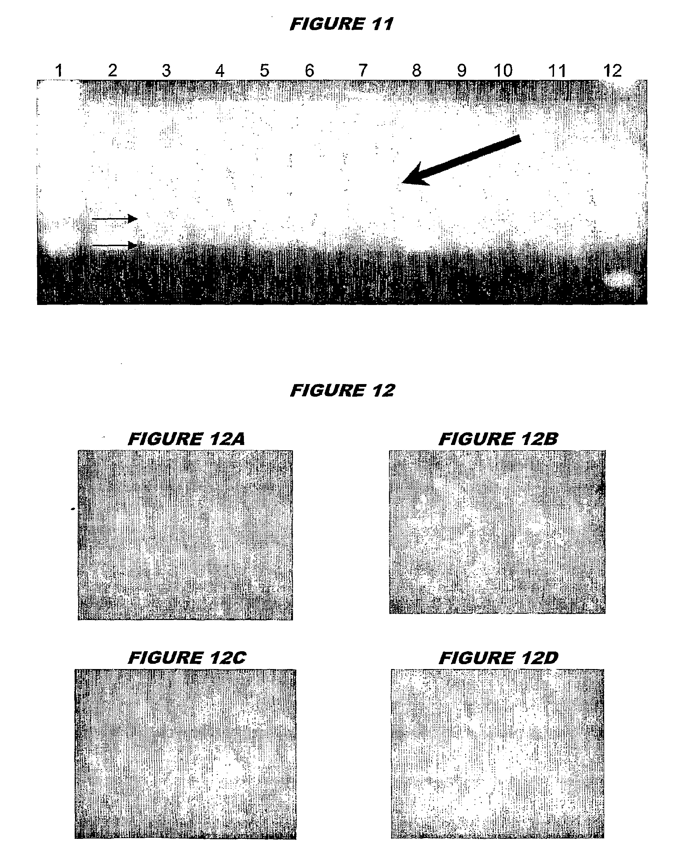

Extracts from cell lines were also tested (figure 11). Again, background levels of "ubiquitous" HERV-K expression were evident in most cell lines. Prostate cell lines MDA PCA 2b (lane 7) and, to a lesser extent, 22RV1 (lane 6), clearly showed longer RT-PCR products.

### MDA PCA 2b cell line

RNA was extracted from MDA PCA 2b cell lines. Spliced mRNAs were cloned and sequenced which confirm that splice acceptor sites near the 3' end of the second 5' LTR are used. These mRNAs have four exons with sequences exactly matching PCAV. They have exons adjacent to LTRs 1 and 2 followed by an exon containing the envelope ATG and a very short open reading frame and finally terminating in the final fragmentary 3' LTR.

The use of a splice acceptor site near the 3' end of the second 5' LTR was also seen in a cDNA present in a private prostate cancer library (Chiron clone ID 035JN024.B09).

The 3' end of MDA PCA 2b RNA was mapped by RACE. The forward PCR primer was SEQ ID 21, which matches PCAV and new HERV-Ks. The reverse PCR primer was SEQ ID 22. The primer for reverse transcription was SEQ ID 20. Using mRNA targets from MDA PCA 2b gave a major band at 1.3 kb. The bands were cloned and sequenced (using either T7 or SP6 sequencing primers) and an alignment is shown below:

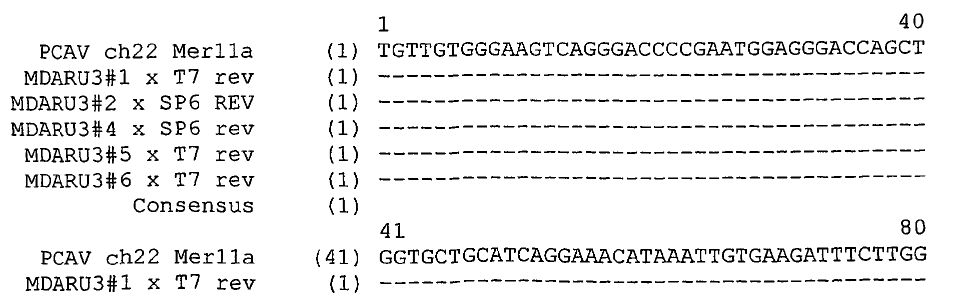

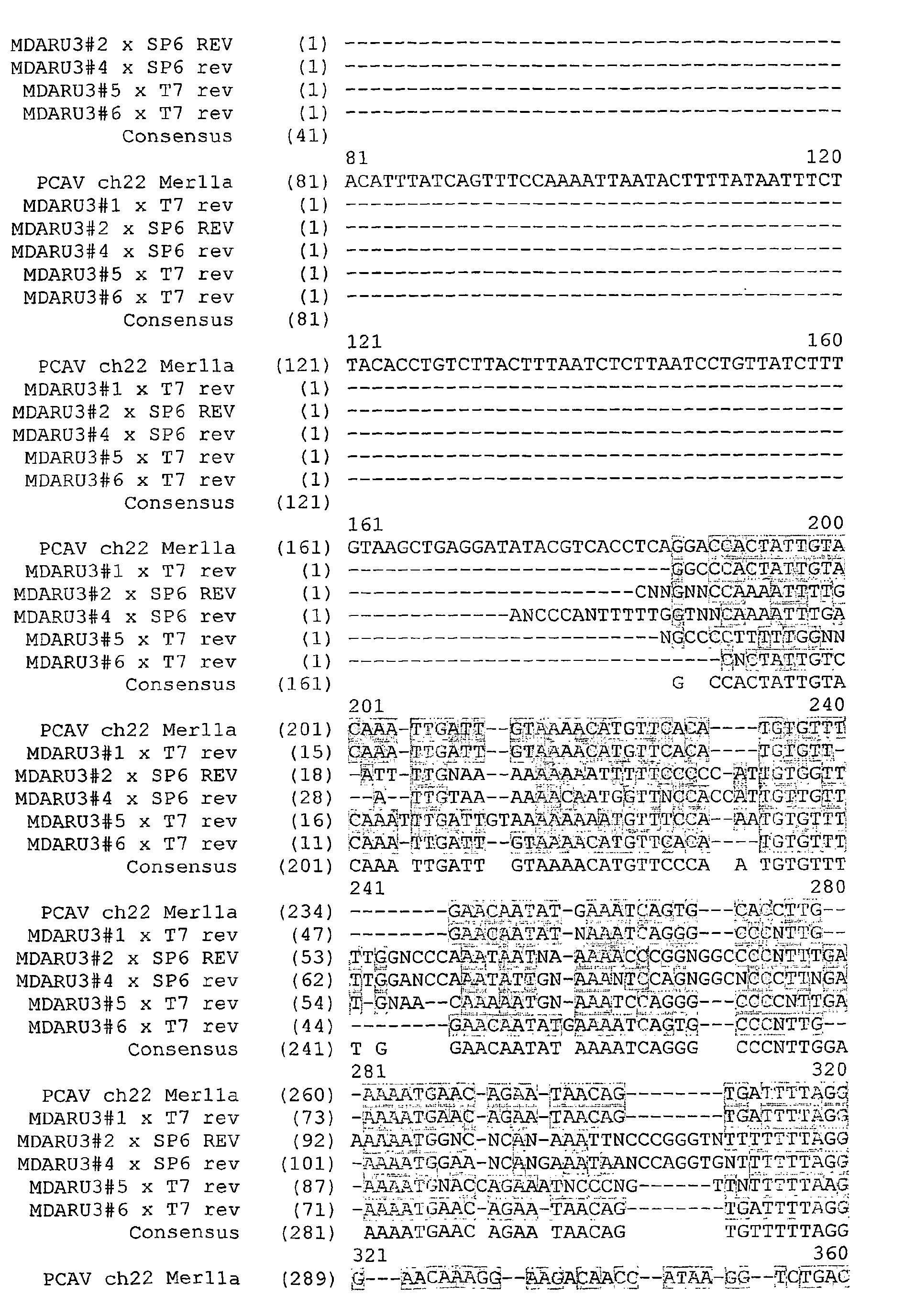

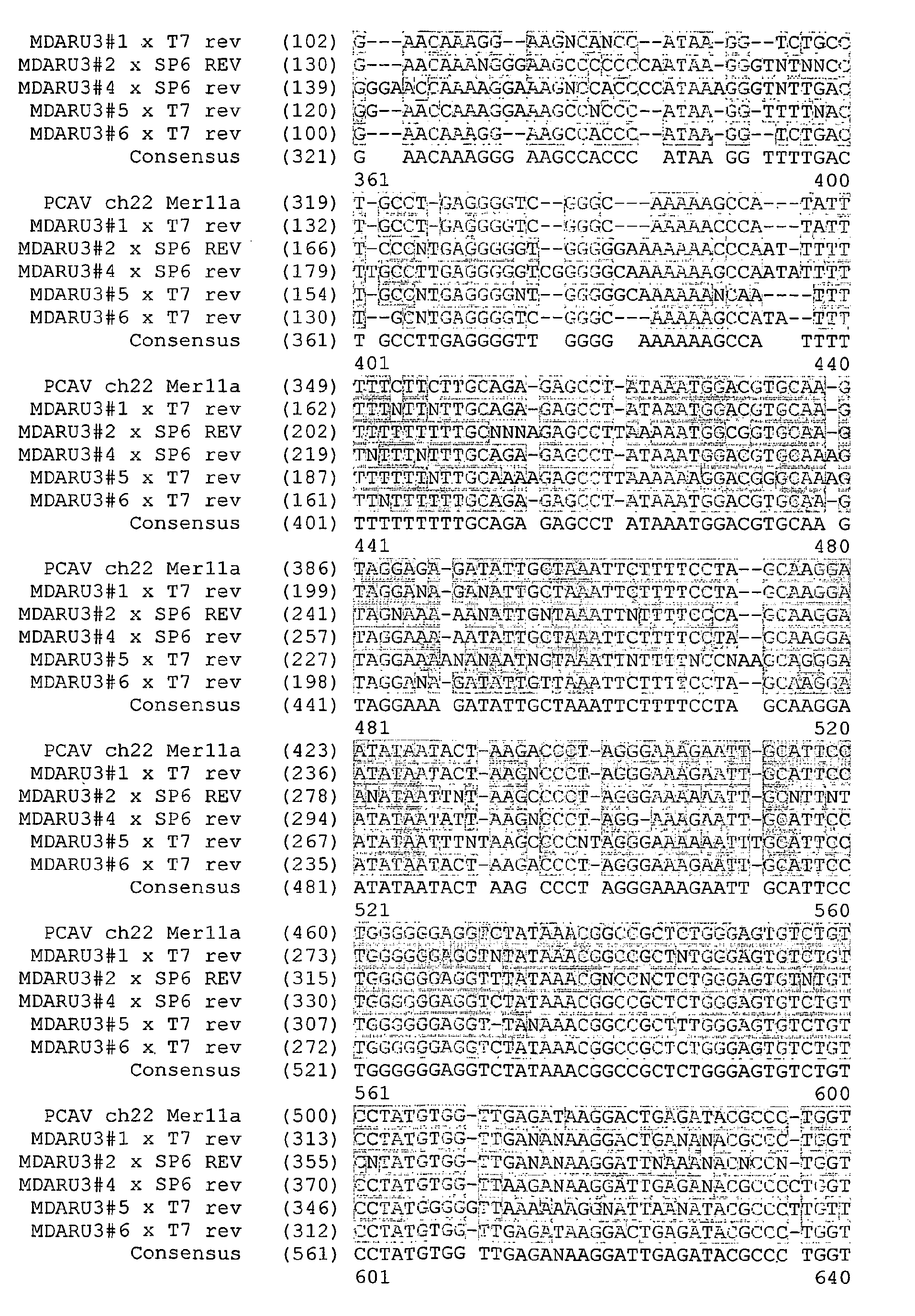

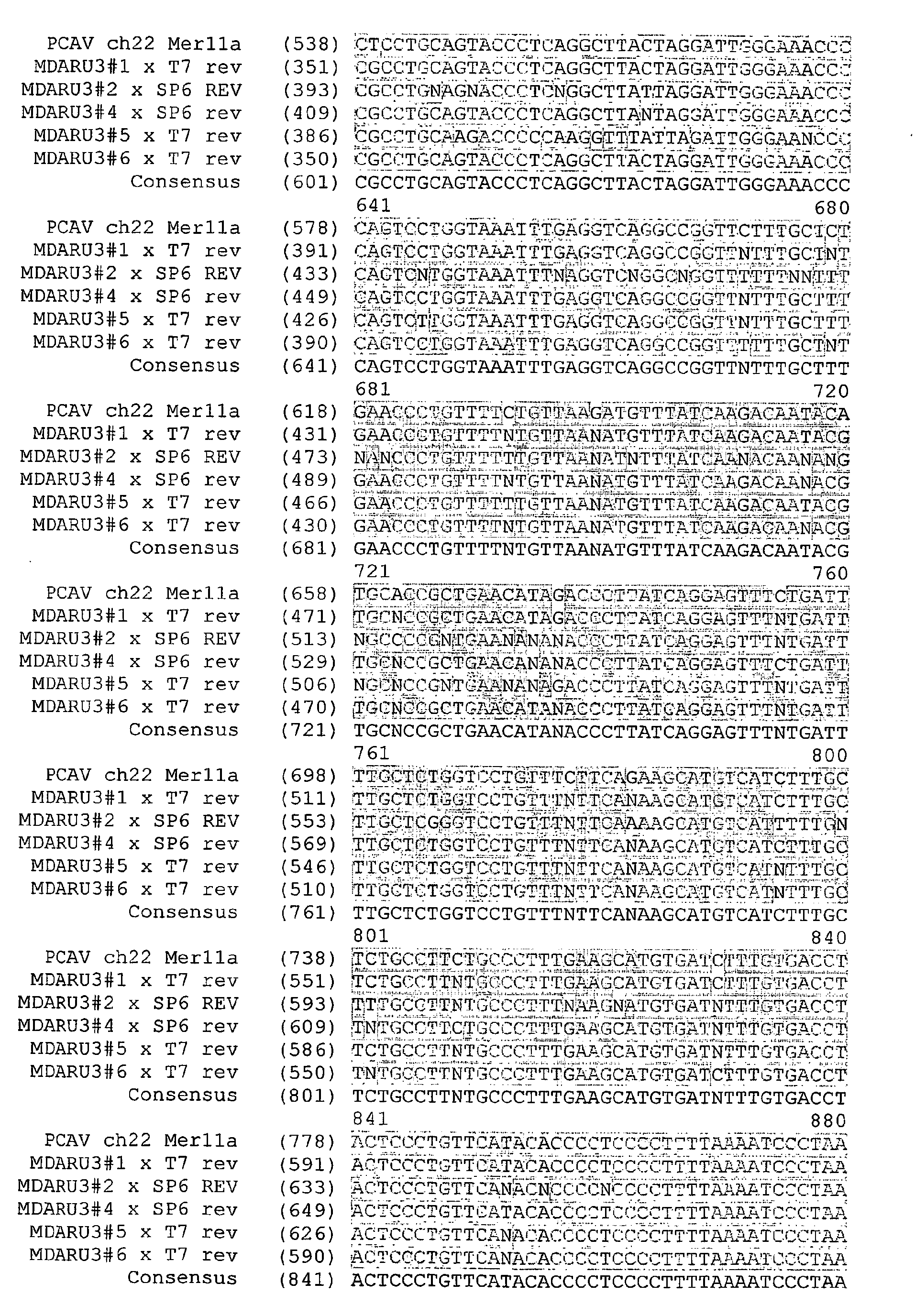

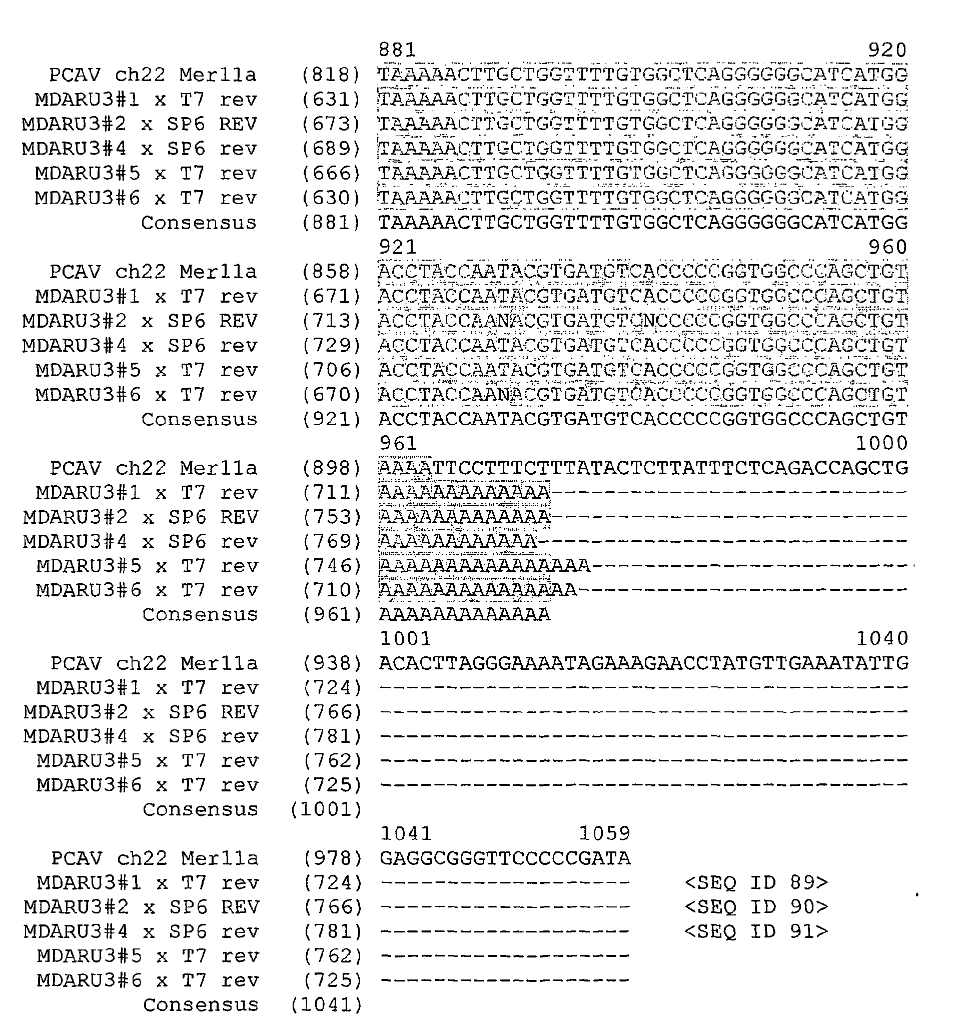

Sequencing of these amplification products shows that transcripts terminate using a polyA signal within a MER11a insertion (see row beginning with nucleotide 961). Again, this is a perfect match for PCAV.

### Anti-gag monoclonal antibodies

PCAV is an "old" HERV-K. Low-level expression of "new" HERV-Ks can also be detected. The gag open reading frames from PCAV and the "new" HERV-Ks are homologous at the primary sequence level, but with significant divergence. Gag protein was expressed in yeast and purified for both PCAV and "new" HERV-K, and mouse monoclonal antibodies were raised.

The "new" HERV-K gag sequence used for expression was isolated from the prostate cancer cell line LnCap and the PCAV gag sequence was isolated from the prostate cancer cell line MDA PCA 2b. These sequences were genetically engineered for expression in *Saccharomyces cerevisiae* AD3 strain, using the yeast expression vector pBS24.1. This vector contains the 2µ sequence for autonomous replication in yeast and the yeast genes leu2d and URA3 as selectable markers. The β-lactamase gene and the ColE1 origin of replication, required for plasmid replication in bacteria, are also present in this expression vector, as well as the a-factor terminator. Expression of the recombinant proteins is under the control of the hybrid ADH2/GAPDH promoter.

The coding sequences for "new" HERV-K and PCAV gag were cloned as *Hind*III*-SaI*I fragments of 2012bp and 2168bp respectively. Each gag was subcloned in two parts:
1. The "new" HERV-K gag was subcloned into pSP72. A 143bp synthetic oligonucleotide from the *Hind*III site adjoined the ADH/GAPDH promoter to a *Nco*I site within the gag coding sequence. The remaining 1869bp of "new" HERV-K gag sequence, from *Nco*I to *Sal*I, was derived by PCR using a cDNA clone obtained from LnCaP cells named orf-99 as the template.
2. PCR was used to create a 1715bp *Hin*dIII-*Ava*3 fragment PCAV gag, using a cDNA clone obtained from MDA PCa 2b cells named 2B11.12-44 as the template. The resulting PCR product was subcloned into pGEM7-Z. The *Ava*3*-SaI*I fragment encoding the 3' end of this construct was isolated from the "new" HERV-K gag clone above, since the 3' end of the gag protein was missing in the 2B 11.12-44 clone.

After sequence confirmation the respective fragments were ligated with the ADH2/GAPDH promoter into the yeast expression vector to create pd.LnCap.gag (encoding the "new" HERV-K gag) and pd.MDA.gag (encoding the hybrid PCAV/ "new" HERV-K gag) yeast expression plasmids.

The "new" expression construct is SEQ ID 1185 and encodes SEQ ID 1186:

HIND3 NCOI XMNI NAEI
AHA3 BGL2 ALWN1 ALWN1
ECORV
BSMI

KAS1 BGL2 BAMHI BALI MST2 ASE1
DRA3 AHA3 ALWN1
ASE1
NARI

AVA3 PFLM1 MST2 PVU2
BSTXI SALI
MST2

MetGlyGlnThrGluSerLysTyrAlaSerTyrLeuSerPheIle
2 AGCTTACAAAACAAAATGGGGCAAACTGAAAGTAAATATGCCTCTTATCTCAGCTTTATT
TCGAATGTTTTGTTTTACCCCGTTTGACTTTCATTTATACGGAGAATAGAGTCGAAATAA
^
1 HIND3,

LysIleLeuLeuLysArgGlyGlyValArgValSerThrLysAsnLeuIleLysLeuPhe
62 AAAATTCTTTTAAAAAGAGGGGGAGTTAGAGTATCTACAAAAAATCTAATCAAGCTATTT
TTTTAAGAAAATTTTTCTCCCCCTCAATCTCATAGATGTTTTTTAGATTAGTTCGATAAA
^
70 AHA3,

GlnIleIleGluGlnPheCysProTrpPheProGluGlnGlyThrLeuAspLeuLysAsp
122 CAAATAATAGAACAATTTTGCCCATGGTTTCCAGAACAAGGAACTTTAGATCTAAAAGAT
GTTTATTATCTTGTTAAAACGGGTACCAAAGGTCTTGTTCCTTGAAATCTAGATTTTCTA
^ ^
143 NCOI, 169 BGL2,

TrpLysArgIleGlyGluGluLeuLysGlnAlaGlyArgLysGlyAsnIleIleProLeu
182 TGGAAAAGAATTGGCGAGGAACTAAAACAAGCAGGTAGAAAGGGTAATATCATTCCACTT
ACCTTTTCTTAACCGCTCCTTGATTTTGTTCGTCCATCTTTCCCATTATAGTAAGGTGAA

ThrValTrpAsnAspTrpAlaIleIleLysAlaAlaLeuGluProPheGlnThrLysGlu
242 ACAGTATGGAATGATTGGGCCATTATTAAAGCAGCTTTAGAACCATTTCAAACAAAAGAA
TGTCATACCTTACTAACCCGGTAATAATTTCGTCGAAATCTTGGTAAAGTTTGTTTTCTT
^
281 XMNI,

AspSerValSerValSerAspAlaProGlySerCysValIleAspCysAsnGluLysThr
302 GATAGCGTTTCAGTTTCTGATGCCCCTGGAAGCTGTGTAATAGATTGTAATGAAAAGACA
CTATCGCAAAGTCAAAGACTACGGGGACCTTCGACACATTATCTAACATTACTTTTCTGT
^
312 ALWN1,

GlyArgLysSerGlnLysGluThrGluSerLeuHisCysGluTyrValThrGluProVal
362 GGGAGAAAATCCCAGAAAGAAACAGAAAGTTTACATTGCGAATATGTAACAGAGCCAGTA
CCCTCTTTTAGGGTCTTTCTTTGTCTTTCAAATGTAACGCTTATACATTGTCTCGGTCAT

MetAlaGlnSerThrGlnAsnValAspTyrAsnGlnLeuGlnGlyValIleTyrProGlu
422 ATGGCTCAGTCAACGCAAAATGTTGACTATAATCAATTACAGGGGGTGATATATCCTGAA
TACCGAGTCAGTTGCGTTTTACAACTGATATTAGTTAATGTCCCCCACTATATAGGACTT

ThrLeuLysLeuGluGlyLysGlyProGluLeuValGlyProSerGluSerLysProArg
482 ACGTTAAAATTAGAAGGAAAAGGTCCAGAATTAGTGGGGCCATCAGAGTCTAAACCACGA
TGCAATTTTAATCTTCCTTTTCCAGGTCTTAATCACCCCGGTAGTCTCAGATTTGGTGCT

GlyProSerProLeuProAlaGlyGlnValProValThrLeuGlnProGlnThrGlnVal
542 GGGCCAAGTCCTCTTCCAGCAGGTCAGGTGCCCGTAACATTACAACCTCAAACGCAGGTT
CCCGGTTCAGGAGAAGGTCGTCCAGTCCACGGGCATTGTAATGTTGGAGTTTGCGTCCAA

LysGluAsnLysThrGlnProProValAlaTyrGlnTyrTrpProProAlaGluLeuGln
602 AAAGAAAATAAGACCCAACCGCCAGTAGCTTATCAATACTGGCCGCCGGCTGAACTTCAG
TTTCTTTTATTCTGGGTTGGCGGTCATCGAATAGTTATGACCGGCGGCCGACTTGAAGTC
^ ^
646 NAEI, 659 ALWN1,

TyrLeuProProProGluSerGlnTyrGlyTyrProGlyMetProProAlaLeuGlnGly
662 TATCTGCCACCCCCAGAAAGTCAGTATGGATATCCAGGAATGCCCCCAGCACTACAGGGC
ATAGACGGTGGGGGTCTTTCAGTCATACCTATAGGTCCTTACGGGGGTCGTGATGTCCCG
^ ^

690 ECORV, 699 BSMI,

ArgAlaProTyrProGlnProProThrValArgLeuAsnProThrAlaSerArgSerGly
722 AGGGCGCCATATCCTCAGCCGCCCACTGTGAGACTTAATCCTACAGCATCACGTAGTGGA
TCCCGCGGTATAGGAGTCGGCGGGTGACACTCTGAATTAGGATGTCGTAGTGCATCACCT
^ ^

724 KAS1 NARI, 771 DRA3,

GlnGlyGlyThrLeuHisAlaValIleAspGluAlaArgLysGlnGlyAspLeuGluAla
782 CAAGGTGGTACACTGCACGCAGTCATTGATGAAGCCAGAAAACAGGGAGATCTTGAGGCA
GTTCCACCATGTGACGTGCGTCAGTAACTACTTCGGTCTTTTGTCCCTCTAGAACTCCGT
^

829 BGL2,

TrpArgPheLeuValIleLeuGlnLeuValGlnAlaGlyGluGluThrGlnValGlyAla
842 TGGCGGTTCCTGGTAATTTTACAACTGGTACAGGCCGGGGAAGAGACTCAAGTAGGAGCG
ACCGCCAAGGACCATTAAAATGTTGACCATGTCCGGCCCCTTCTCTGAGTTCATCCTCGC

ProAlaArgAlaGluThrArgCysGluProPheThrMetLysMetLeuLysAspIleLys
902 CCTGCCCGAGCTGAGACTAGATGTGAACCTTTCACCATGAAAATGTTAAAAGATATAAAG
GGACGGGCTCGACTCTGATCTACACTTGGAAAGTGGTACTTTTACAATTTTCTATATTTC

GluGlyValLysGlnTyrGlySerAsnSerProTyrIleArgThrLeuLeuAspSerIle
962 GAAGGAGTTAAACAATATGGATCCAACTCCCCTTATATAAGAACATTATTAGATTCCATT
CTTCCTCAATTTGTTATACCTAGGTTGAGGGGAATATATTCTTGTAATAATCTAAGGTAA
^

980 BAMHI,

AlaHisGlyAsnArgLeuThrProTyrAspTrpGluSerLeuAlaLysSerSerLeuSer
1022 GCTCATGGAAATAGACTTACTCCTTATGACTGGGAAAGTTTGGCCAAATCTTCCCTTTCA
CGAGTACCTTTATCTGAATGAGGAATACTGACCCTTTCAAACCGGTTTAGAAGGGAAAGT
^

1062 BALI,

SerSerGlnTyrLeuGlnPheLysThrTrpTrpIleAspGlyValGlnGluGlnValArg
1082 TCCTCTCAGTATCTACAGTTTAAAACCTGGTGGATTGATGGAGTACAAGAACAGGTACGA
AGGAGAGTCATAGATGTCAAATTTTGGACCACCTAACTACCTCATGTTCTTGTCCATGCT
^

1100 AHA3,

LysAsnGlnAlaThrLysProThrValAsnIleAspAlaAspGlnLeuLeuGlyThrGly
1142 AAAAATCAGGCTACTAAGCCCACTGTTAATATAGACGCAGACCAATTGTTAGGAACAGGT
TTTTTAGTCCGATGATTCGGGTGACAATTATATCTGCGTCTGGTTAACAATCCTTGTCCA

ProAsnTrpSerThrIleAsnGlnGlnSerValMetGlnAsnGluAlaIleGluGlnVal
1202 CCAAATTGGAGCACCATTAACCAACAATCAGTGATGCAGAATGAGGCTATTGAACAAGTA
GGTTTAACCTCGTGGTAATTGGTTGTTAGTCACTACGTCTTACTCCGATAACTTGTTCAT

ArgAlaIleCysLeuArgAlaTrpGlyLysIleGlnAspProGlyThrAlaPheProIle
1262 AGGGCTATTTGCCTCAGGGCCTGGGGAAAAATTCAGGACCCAGGAACAGCTTTCCCTATT
TCCCGATAAACGGAGTCCCGGACCCCTTTTTAAGTCCTGGGTCCTTGTCGAAAGGGATAA
^ ^ ^

1273 MST2, 1276 ALWN1, 1319 ASE1,

AsnSerIleArgGlnGlySerLysGluProTyrProAspPheValAlaArgLeuGlnAsp
1322 AATTCAATTAGACAAGGCTCTAAAGAGCCATATCCTGACTTTGTGGCAAGATTACAAGAT
TTAAGTTAATCTGTTCCGAGATTTCTCGGTATAGGACTGAAACACCGTTCTAATGTTCTA

AlaAlaGlnLysSerIleThrAspAspAsnAlaArgLysValIleValGluLeuMetAla
1382 GCTGCTCAAAAGTCTATTACAGATGACAATGCCCGAAAAGTTATTGTAGAATTAATGGCC
CGACGAGTTTTCAGATAATGTCTACTGTTACGGGCTTTTCAATAACATCTTAATTACCGG

^

1432 ASE1,

TyrGluAsnAlaAsnProGluCysGlnSerAlaIleLysProLeuLysGlyLysValPro
1442 TATGAAAATGCAAATCCAGAATGTCAGTCGGCCATAAAGCCATTAAAAGGAAAAGTTCCA
ATACTTTTACGTTTAGGTCTTACAGTCAGCCGGTATTTCGGTAATTTTCCTTTTCAAGGT

AlaGlyValAspValIleThrGluTyrValLysAlaCysAspGlyIleGlyGlyAlaMet
1502 GCAGGAGTTGATGTAATTACAGAATATGTGAAGGCTTGTGATGGATTGGAGGAGCTATG
CGTCCTCAACTACATTAATGTCTTATACACTTCCGAACACTACCCTAACCTCCTCGATAC
^

1559 AVA3,

HisLysAlaMetLeuMetAlaGlnAlaMetArgGlyLeuThrLeuGlyGlyGlnValArg
1562 CATAAGGCAATGCTAATGGCTCAAGCAATGAGGGGGCTCACTCTAGGAGGACAAGTTAGA
GTATTCCGTTACGATTACCGAGTTCGTTACTCCCCCGAGTGAGATCCTCCTGTTCAATCT

ThrPheGlyLysLysCysTyrAsnCysGlyGlnIleGlyHisLeuLysArgSerCysPro
1622 ACATTTGGGAAAAAATGTTATAATTGTGGTCAAATCGGTCATCTGAAAAGGAGTTGCCCA
TGTAAACCCTTTTTTACAATATTAACACCAGTTTAGCCAGTAGACTTTTCCTCAACGGGT

ValLeuAsnLysGlnAsnIleIleAsnGlnAlaIleThrAlaLysAsnLysLysProSer
1682 GTCTTAAATAAACAGAATATAATAAATCAAGCTATTACAGCAAAAAATAAAAAGCCATCT
CAGAATTTATTTGTCTTATATTATTTAGTTCGATAATGTCGTTTTTTATTTTTCGGTAGA

GlyLeuCysProLysCysGlyLysGlyLysHisTrpAlaAsnGlnCysHisSerLysPhe
1742 GGCCTGTGTCCAAAATGTGGAAAAGGAAAACATTGGGCCAATCAATGTCATTCTAAATTT
CCGGACACAGGTTTTACACCTTTTCCTTTTGTAACCCGGTTAGTTACAGTAAGATTTAAA
^

1751 PFLM1,

AspLysAspGlyGlnProLeuSerGlyAsnArgLysArgGlyGlnProGlnAlaProGln
1802 GATAAGGATGGGCAACCATTGTCGGGAAACAGGAAGAGGGGCCAGCCTCAGGCCCCCCAA
CTATTCCTACCCGTTGGTAACAGCCCTTTGTCCTTCTCCCCGGTCGGAGTCCGGGGGGTT
^ ^

1847 MST2, 1858 BSTXI,

GlnThrGlyAlaPheProValGlnLeuPheValProGlnGlyPheGlnGlyGlnGlnPro
1862 CAAACTGGGGCATTCCCAGTTCAACTGTTTGTTCCTCAGGGTTTTCAAGGACAACAACCC
GTTTGACCCCGTAAGGGTCAAGTTGACAAACAAGGAGTCCCAAAAGTTCCTGTTGTTGGG
^

1895 MST2,

LeuGlnLysIleProProLeuGlnGlyValSerGlnLeuGlnGlnSerAsnSerCysPro
1922 CTACAGAAAATACCACCACTTCAGGGAGTCAGCCAATTACAACAATCCAACAGCTGTCCC
GATGTCTTTTATGGTGGTGAAGTCCCTCAGTCGGTTAATGTTGTTAGGTTGTCGACAGGG
^

1972 PVU2,

AlaProGlnGlnAlaAlaProGlnAM OC
1982 GCGCCACAGCAGGCAGCACCGCAGTAGTAAGTCGAC
CGCGGTGTCGTCCGTCGTGGCGTCATCATTCAGCTG
^

2012 SALI,

The hybrid construct is SEQ ID 1187 and encodes SEQ ID 1188:

|_________|_|_________|_|_______|_________|________|____|_____
HIND3     NCOI        XMNI      ALWN1     PVUI     TTH3I-I
            BGL2        ALWN1             RSPI         PFLM1
                                          BSAB1

HGIE2
BSTXI
DRA3
APAL1
SPHI
BAMHI
BALI
AHA3
MST2
ALWN1
ASE1

ASE1
AVA3
PFLM1
MST2
BSTXI
MST2
PVU2
SALI

                              MetGlyGlnThrGluSerLysTyrAlaSerTyrLeuSerPheIle
  2  AGCTTACAAAACAAAATGGGGCAAACTGAAAGTAAATATGCCTCTTATCTCAGCTTTATT
     TCGAATGTTTTGTTTTACCCCGTTTGACTTTCATTTATACGGAGAATAGAGTCGAAATAA
     ^
     1 HIND3,

     LysIleLeuLeuArgArgGlyGlyValArgAlaSerThrGluAsnLeuIleThrLeuPhe
 62  AAAATTCTTTTAAGAAGAGGGGGAGTTAGAGCTTCTACAGAAAATCTAATTACGCTATTT
     TTTTAAGAAAATTCTTCTCCCCCTCAATCTCGAAGATGTCTTTTAGATTAATGCGATAAA

     GlnThrIleGluGlnPheCysProTrpPheProGluGlnGlyThrLeuAspLeuLysAsp
122  CAAACAATAGAACAATTCTGCCCATGGTTTCCAGAACAGGGAACTTTAGATCTAAAAGAT
     GTTTGTTATCTTGTTAAGACGGGTACCAAAGGTCTTGTCCCTTGAAATCTAGATTTTCTA
                         ^                         ^
     143 NCOI, 169 BGL2,

     TrpGluLysIleGlyLysGluLeuLysGlnAlaAsnArgGluGlyLysIleIleProLeu
182  TGGGAAAAAATTGGCAAAGAATTAAAACAAGCAAATAGGGAAGGTAAAATCATCCCACTT
     ACCCTTTTTTAACCGTTTCTTAATTTTGTTCGTTTATCCCTTCCATTTTAGTAGGGTGAA

     ThrValTrpAsnAspTrpAlaIleIleLysAlaThrLeuGluProPheGlnThrGlyGlu
242  ACAGTATGGAATGATTGGGCCATTATTAAAGCAACTTTAGAACCATTTCAAACAGGAGAA
     TGTCATACCTTACTAACCCGGTAATAATTTCGTTGAAATCTTGGTAAAGTTTGTCCTCTT
                                             ^
     281 XMNI,

     AspIleValSerValSerAspAlaProLysSerCysValThrAspCysGluGluGluAla
302  GATATTGTTTCAGTTTCTGATGCCCCTAAAGCTGTGTAACAGATTGTGAAGAAGAGGCA
     CTATAACAAAGTCAAAGACTACGGGGATTTCGACACATTGTCTAACACTTCTTCTCCGT
               ^
     312 ALWN1,

     GlyThrGluSerGlnGlnGlyThrGluSerSerHisCysLysTyrValAlaGluSerVal
362  GGGACAGAATCCCAGCAAGGAACGGAAAGTTCACATTGTAAATATGTAGCAGAGTCTGTA
     CCCTGTCTTAGGGTCGTTCCTTGCCTTTCAAGTGTAACATTTATACATCGTCTCAGACAT
                                                      ^
     411 ALWN1,

     MetAlaGlnSerThrGlnAsnValAspTyrSerGlnLeuGlnGluIleIleTyrProGlu
422  ATGGCTCAGTCAACGCAAAATGTTGACTACAGTCAATTACAGGAGATAATATACCCTGAA
     TACCGAGTCAGTTGCGTTTTACAACTGATGTCAGTTAATGTCCTCTATTATATGGGACTT

     SerSerLysLeuGlyGluGlyGlyProGluSerLeuGlyProSerGluProLysProArg
482  TCATCAAAATTGGGGGAAGGAGGTCCAGAATCATTGGGGCCATCAGAGCCTAAACCACGA
     AGTAGTTTTAACCCCCTTCCTCCAGGTCTTAGTAACCCCGGTAGTCTCGGATTTGGTGCT
                                                               ^^
     539 PVUI RSPI, 540 BSAB1,

     SerProSerThrProProProValValGlnMetProValThrLeuGlnProGlnThrGln

542 TCGCCATCAACTCCTCCTCCCGTGGTTCAGATGCCTGTAACATTACAACCTCAAACGCAG
AGCGGTAGTTGAGGAGGAGGGCACCAAGTCTACGGACATTGTAATGTTGGAGTTTGCGTC

ValArgGlnAlaGlnThrProArgGluAsnGlnValGluArgAspArgValSerIlePro
602 GTTAGACAAGCACAAACCCCAAGAGAAAATCAAGTAGAAAGGGACAGAGTCTCTATCCCG
CAATCTGTTCGTGTTTGGGGTTCTCTTTTAGTTCATCTTTCCCTGTCTCAGAGATAGGGC
^
644 TTH3I,

AlaMetProThrGlnIleGlnTyrProGlnTyrGlnProValGluAsnLysThrGlnPro
662 GCAATGCCAACTCAGATACAGTATCCACAATATCAGCCGGTAGAAAATAAGACCCAACCG
CGTTACGGTTGAGTCTATGTCATAGGTGTTATAGTCGGCCATCTTTTATTCTGGGTTGGC
^
715 PFLM1,

LeuValValTyrGlnTyrArgLeuProThrGluLeuGlnTyrArgProProSerGluVal
722 CTGGTAGTTTATCAATACCGGCTGCCAACCGAGCTTCAGTATCGGCCTCCTTCAGAGGTT
GACCATCAAATAGTTATGGCCGACGGTTGGCTCGAAGTCATAGCCGGAGGAAGTCTCCAA

GlnTyrArgProGlnAlaValCysProValProAsnSerThrAlaProTyrGlnGlnPro
782 CAATACAGACCTCAAGCGGTGTGTCCTGTGCCAAATAGCACGGCACCATACCAGCAACCC
GTTATGTCTGGAGTTCGCCACACAGGACACGGTTTATCGTGCCGTGGTATGGTCGTTGGG
^ ^
790 HGIE2, 840 BSTXI,

ThrAlaMetAlaSerAsnSerProAlaThrGlnAspAlaAlaLeuTyrProGlnProPro
842 ACAGCGATGGCGTCTAATTCACCAGCAACACAGGACGCGGCGCTGTATCCTCAGCCGCCC
TGTCGCTACCGCAGATTAAGTGGTCGTTGTGTCCTGCGCCGCGACATAGGAGTCGGCGGG

ThrValArgLeuAsnProThrAlaSerArgSerGlyGlnGlyGlyAlaLeuHisAlaVal
902 ACTGTGAGACTTAATCCTACAGCATCACGTAGTGGACAGGGTGGTGCACTGCATGCAGTC
TGACACTCTGAATTAGGATGTCGTAGTGCATCACCTGTCCCACCACGTGACGTACGTCAG
^ ^ ^
927 DRA3, 945 APAL1, 952 SPHI,

IleAspGluAlaArgLysGlnGlyAspLeuGluAlaTrpArgPheLeuValIleLeuGln
962 ATTGATGAAGCCAGAAAACAGGGCGATCTTGAGGCATGGCGGTTCCTGGTAATTTTACAA
TAACTACTTCGGTCTTTTGTCCCGCTAGAACTCCGTACCGCCAAGGACCATTAAAATGTT

LeuValGlnAlaGlyGluGluThrGlnValGlyAlaProAlaArgAlaGluThrArgCys
1022 CTGGTACAGGCCGGGGAAGAGACTCAAGTAGGAGCGCCTGCCCGAGCTGAGACTAGATGT
GACCATGTCCGGCCCCTTCTCTGAGTTCATCCTCGCGGACGGGCTCGACTCTGATCTACA

GluProPheThrMetLysMetLeuLysAspIleLysGluGlyValLysGlnTyrGlySer
1082 GAACCTTTCACCATGAAAATGTTAAAAGATATAAAGGAAGGAGTTAAACAATATGGATCC
CTTGGAAAGTGGTACTTTTACAATTTTCTATATTTCCTTCCTCAATTTGTTATACCTAGG
^
1136 BAMHI,

AsnSerProTyrIleArgThrLeuLeuAspSerIleAlaHisGlyAsnArgLeuThrPro
1142 AACTCCCCTTATATAAGAACATTATTAGATTCCATTGCTCATGGAAATAGACTTACTCCT
TTGAGGGGAATATATTCTTGTAATAATCTAAGGTAACGAGTACCTTTATCTGAATGAGGA

TyrAspTrpGluIleLeuAlaLysSerSerLeuSerSerSerGlnTyrLeuGlnPheLys
1202 TATGACTGGGAAATTTTGGCCAAATCTTCCCTTTCATCCTCTCAGTATCTACAGTTTAAA
ATACTGACCCTTTAAAACCGGTTTAGAAGGGAAAGTAGGAGAGTCATAGATGTCAAATTT
^ ^
1218 BALI, 1256 AHA3,

ThrTrpTrpIleAspGlyValGlnGluGlnValArgLysAsnGlnAlaThrLysProThr
1262 ACCTGGTGGATTGATGGAGTACAAGAACAGGTACGAAAAAATCAGGCTACTAAGCCCACT

TGGACCACCTAACTACCTCATGTTCTTGTCCATGCTTTTTAGTCCGATGATTCGGGTGA

VaLAsnIleAspAlaAspGlnLeuLeuGlyThrGlyProAsnTrpSerThrIleAsnGln
1322 GTTAATATAGACGCAGACCAATTGTTAGGAACAGGTCCAAATTGGAGCACCATTAACCAA
CAATTATATCTGCGTCTGGTTAACAATCCTTGTCCAGGTTTAACCTCGTGGTAATTGGTT

GlnSerValMetGlnAsnGluAlaIleGluGlnValArgAlaIleCysLeuArgAlaTrp
1382 CAATCAGTGATGCAGAATGAGGCTATTGAACAAGTAAGGGCTATTTGCCTCAGGGCCTGG
GTTAGTCACTACGTCTTACTCCGATAACTTGTTCATTCCCGATAAACGGAGTCCCGGACC
^ ^
1429 MST2, 1432 ALWN1,

GlyLysIleGlnAspProGlyThrAlaPheProIleAsnSerIleArgGlnGlySerLys
1442 GGAAAAATTCAGGACCCAGGAACAGCTTTCCCTATTAATTCAATTAGACAAGGCTCTAAA
CCTTTTTAAGTCCTGGGTCCTTGTCGAAAGGGATAATTAAGTTAATCTGTTCCGAGATTT
^
1475 ASE1,

GluProTyrProAspPheValAlaArgLeuGlnAspAlaAlaGlnLysSerIleThrAsp
1502 GAGCCATATCCTGACTTTGTGGCAAGATTACAAGATGCTGCTCAAAAGTCTATTACAGAT
CTCGGTATAGGACTGAAACACCGTTCTAATGTTCTACGACGAGTTTTCAGATAATGTCTA

AspAsnAlaArgLysValIleValGluLeuMetAlaTyrGluAsnAlaAsnProGluCys
1562 GACAATGCCCGAAAAGTTATTGTAGAATTAATGGCCTATGAAAATGCAAATCCAGAATGT
CTGTTACGGGCTTTTCAATAACATCTTAATTACCGGATACTTTTACGTTTAGGTCTTACA
^
1588 ASE1,

GlnSerAlaIleLysProLeuLysGlyLysValProAlaGlyValAspValIleThrGlu
1622 CAGTCGGCCATAAAGCCATTAAAAGGAAAAGTTCCAGCAGGAGTTGATGTAATTACAGAA
GTCAGCCGGTATTTCGGTAATTTTCCTTTTCAAGGTCGTCCTCAACTACATTAATGTCTT

TyrValLysAlaCysAspGlyIleGlyGlyAlaMetHisLysAlaMetLeuMetAlaGln
1682 TATGTGAAGGCTTGTGATGGGATTGGAGGAGCTATGCATAAGGCAATGCTAATGGCTCAA
ATACACTTCCGAACACTACCCTAACCTCCTCGATACGTATTCCGTTACGATTACCGAGTT
^
1715 AVA3,

AlaMetArgGlyLeuThrLeuGlyGlyGlnValArgThrPheGlyLysLysCysTyrAsn
1742 GCAATGAGGGGGCTCACTCTAGGAGGACAAGTTAGAACATTTGGGAAAAAATGTTATAAT
CGTTACTCCCCCGAGTGAGATCCTCCTGTTCAATCTTGTAAACCCTTTTTTACAATATTA

CysGlyGlnIleGlyHisLeuLysArgSerCysProValLeuAsnLysGlnAsnIleIle
1802 TGTGGTCAAATCGGTCATCTGAAAAGGAGTTGCCCAGTCTTAAATAAACAGAATATAATA
ACACCAGTTTAGCCAGTAGACTTTTCCTCAACGGGTCAGAATTTATTTGTCTTATATTAT

AsnGlnAlaIleThrAlaLysAsnLysLysProSerGlyLeuCysProLysCysGlyLys
1862 AATCAAGCTATTACAGCAAAAAATAAAAAGCCATCTGGCCTGTGTCCAAAATGTGGAAAA
TTAGTTCGATAATGTCGTTTTTTATTTTTCGGTAGACCGGACACAGGTTTTACACCTTTT
^
1907 PFLM1,

GlyLysHisTrpAlaAsnGlnCysHisSerLysPheAspLysAspGlyGlnProLeuSer
1922 GGAAAACATTGGGCCAATCAATGTCATTCTAAATTTGATAAGGATGGGCAACCATTGTCG
CCTTTTGTAACCCGGTTAGTTACAGTAAGATTTAAACTATTCCTACCCGTTGGTAACAGC

GlyAsnArgLysArgGlyGlnProGlnAlaProGlnGlnThrGlyAlaPheProValGln
1982 GGAAACAGGAAGAGGGGCCAGCCTCAGGCCCCCCAACAAACTGGGGCATTCCCAGTTCAA
CCTTTGTCCTTCTCCCCGGTCGGAGTCCGGGGGGTTGTTTGACCCCGTAAGGGTCAAGTT
^ ^
2003 MST2, 2014 BSTXI,

LeuPheValProGlnGlyPheGlnGlyGlnGlnProLeuGlnLysIleProProLeuGln
2042 CTGTTTGTTCCTCAGGGTTTTCAAGGACAACAACCCCTACAGAAAATACCACCACTTCAG
GACAAACAAGGAGTCCCAAAAGTTCCTGTTGTTGGGGATGTCTTTTATGGTGGTGAAGTC
^
2051 MST2,

GlyValSerGlnLeuGlnGlnSerAsnSerCysProAlaProGlnGlnAlaAlaProGln
2102 GGAGTCAGCCAATTACAACAATCCAACAGCTGTCCCGCGCCACAGCAGGCAGCACCGCAG
CCTCAGTCGGTTAATGTTGTTAGGTTGTCGACAGGGCGCGGTGTCGTCCGTCGTGGCGTC
^
2128 PVU2,

AM OC
2162 TAGTAAGTCGAC
ATCATTCAGCTG
^
2168 SALI,

An alignment of the encoded proteins is below:

# 1:  y.MDA.2b1112.44.aa          715          78.60%
# 2:  y.orf99.aa (LNCap)          663          84.77%

ALIGNMENT MAP     - showing sequences and aligned repeats {in brackets}
                  - in each given alphabet

In alphabet in which alignment was found:

     0   {MGQTESKYASYLSFIKILL} r {RGGVR} aste {NLI} t {LFQ} t {IEQFC
     0   {MGQTESKYASYLSFIKILL} k {RGGVR} vstk {NLI} k {LFQ} i {IEQFC

    42   PWFPEQGTLDLKDW} ekigk {ELKQA} nregk {IIPLTVWNDWAIIKA} t
    42   PWFPEQGTLDLKDW} krige {ELKQA} grkgn {IIPLTVWNDWAIIKA} a

    90   {LEPFQT} gedi {VSVSDAP} k {SCV} tdceeeagtesqqg {TES} shckyvaes
    90   {LEPFQT} keds {VSVSDAP} g {SCV} idcnektgrksqke {TES} lhceyvtep

   134   {VMAQSTQNVDY} s {QLQ} ei {IYPE} ssklgeg {GPE} sl {GPSE} p
   134   {VMAQSTQNVDY} n {QLQ} gv {IYPE} tlklegk {GPE} lv {GPSE} s

   174   {KPR} spstpppvvqm {PVTLQPQTQV} rqaqtprenqverdrvsipamptqiqypqyqp
   174   {KPR} gpsplpagqv. {PVTLQPQTQV} k...............................

   228   v  {ENKTQP} lvv {YQY} rlpt {ELQY} rppsevqyrpqavcpvpnstapyqqpt
   196   .  {ENKTQP} pva {YQY} wppa {ELQY} lpppesqygypgmppalqgrap.....

   276   amasnspatqdaal  {YPQPPTVRLNPTASRSGQGG} a {LHAVIDEARKQGDLEAWRF
   238   ..............  {YPQPPTVRLNPTASRSGQGG} t {LHAVIDEARKQGDLEAWRF

   330   LVILQLVQAGEETQVGAPARAETRCEPFTMKMLKDIKEGVKQYGSNSPYIRTLLDSIAHG
   278   LVILQLVQAGEETQVGAPARAETRCEPFTMKMLKDIKEGVKQYGSNSPYIRTLLDSIAHG

   390   NRLTPYDWE} i {LAKSSLSSSQYLQFKTWWIDGVQEQVRKNQATKPTVNIDADQLLGT
   338   NRLTPYDWE} s {LAKSSLSSSQYLQFKTWWIDGVQEQVRKNQATKPTVNIDADQLLGT

   446   GPNWSTINQQSVMQNEAIEQVRAICLRAWGKIQDPGTAFPINSIRQGSKEPYPDFVARLQ
   394   GPNWSTINQQSVMQNEAIEQVRAICLRAWGKIQDPGTAFPINSIRQGSKEPYPDFVARLQ

   506   DAAQKSITDDNARKVIVELMAYENANPECQSAIKPLKGKVPAGVDVITEYVKACDGIGGA
   454   DAAQKSITDDNARKVIVELMAYENANPECQSAIKPLKGKVPAGVDVITEYVKACDGIGGA

566 MHKAMLMAQAMRGLTLGGQVRTFGKKCYNCGQIGHLKRSCPVLNKQNIINQAITAKNKKP
514 MHKAMLMAQAMRGLTLGGQVRTFGKKCYNCGQIGHLKRSCPVLNKQNIINQAITAKNKKP

626 SGLCPKCGKGKHWANQCHSKFDKDGQPLSGNRKRGQPQAPQQTGAFPVQLFVPQGFQGQQ
574 SGLCPKCGKGKHWANQCHSKFDKDGQPLSGNRKRGQPQAPQQTGAFPVQLFVPQGFQGQQ

686 PLQKIPPLQGVSQLQQSNSCPAPQQAAPQ}
634 PLQKIPPLQGVSQLQQSNSCPAPQQAAPQ}

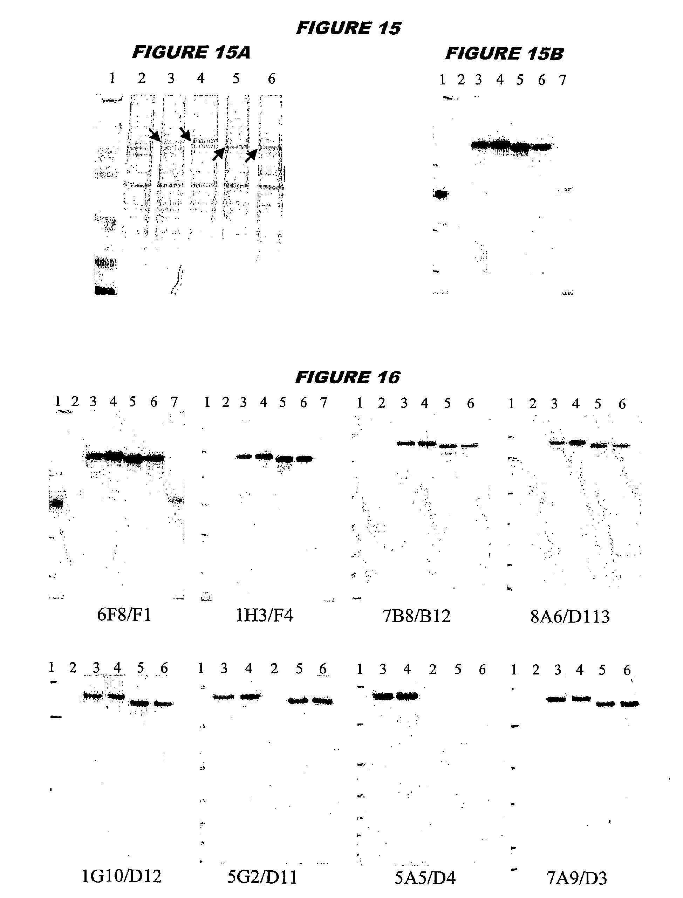

*S.cerevisiae* AD3 strain (mata,leu2,trp1,ura3-52,prb-1122,pep4-3,prcl-407,cir°,trp+ : DM15[GAP/ADR]) was transformed and single transformants were checked for expression after depletion of glucose in the medium. The recombinant proteins were expressed at high level in yeast, as detected in total yeast extracts by Coomassie blue staining (Figure 15A). The expressed proteins were easily observed in a total yeast extract (arrows), with "new" gag in lanes 5 & 6 and the hybrid gag in lanes 3 & 4. Un-transformed control cells are shown in lane 2.

After a large-scale fermentation, proteins were purified and used for monoclonal antibody production. Eight mAbs were obtained in large quantities and they were tested for their ability to recognize both gag proteins in Western blots (Figure 16). Of the 8 mAbs, 7 recognize both of the recombinant proteins and one (5A5/D4) recognizes only the PCAV/HERV-K hybrid gag protein. Antibody 5G2 cross-reacts with both old and new gag antigens:

| **mAb** | **Antigen** | **"New" HERV-K gag** | **PCAV / HERV-K liybrid gag** |
|---|---|---|---|
| 5G2/D11 | "New" HERV-K gag | POSITIVE | POSITIVE |
| 7B8/B12 | "New" HERV-K gag | POSITIVE | POSITIVE |
| 8A6/D113 | "New" HERV-K gag | POSITIVE | POSITIVE |
| 7A9/D3 | "New" HERV-K gag | POSITIVE | POSITIVE |
| 1G10/D12 | "New" HERV-K gag | POSITIVE | POSITIVE |
| 1H3/F4 | "New" HERV-K gag | POSITIVE | POSITIVE |
| 5A5/D4 | PCAV / HERV-K hybrid gag | NEGATIVE | POSITIVE |
| 6F8/F1 | PCAV / HERV-K hybrid gag | POSITIVE | POSITIVE |

mAb 6F8/F1 was used in a Western blot (Figure 15B) of a gel containing the yeast extracts in the same order and in Figure 15A. To reduce signal intensity, the samples containing the gag recombinant proteins were diluted 50-fold relative to the samples shown in Figure 15A using the yeast extract containing no recombinant protein.

5G2 antibody binds to MDA PCA 2b cells (figure 12B). The cells did not fluoresce in the absence of the antibody (figure 12A). Prostate cell line PC3 was also reactive (figure 12C), but less so than MDA PCA 2b. A transformed fibroblast cell line (NIH3T3) was not reactive with anti-HERV-K-gag antibody (figure 12D).

The gag mRNA structure found in MDA PCA 2b cells begins in the first 5' LTR and splices out the second 5' LTR. Such an arrangement is necessary in order for the RNA to be translationally competent because the second 5' LTR contains many stop codons which, in unspliced mRNA, would prevent gag translation.

### PCAV sequence analysis

The genomic sequence of PCAV from chromosome 22 is given as SEQ ID 1. This sequence extends from the start of the first 5' LTR in the genome to the end of the final fragment of the 3' LTR. It is 12366 bp in total.

Within SEQ ID 1, the first 5' LTR (new) is nucleotides 1-968. This is followed by HERV-K sequence up to nucleotide 1126. Nucleotides 1127-1678 are non-viral, including TG repeats at 1464-1487. The second 5' LTR (old) is from nucleotides 1679-2668. The 3' LTR is fragmented as nucleotides 10520-10838 and 11929-12366. The MER11a insertion is at nucleotides 10839-11834, with its polyA signal located between 11654-11659. The polyA addition site is located between 11736 and 11739, but it is not possible to say precisely where, because these four nucleotides are already As.

Basic coding regions within SEQ ID 1 are:

| **Product** | Gag-pol frag | PCAP6 | Gag | Prt | Pol-Env frag | Env frag |
|---|---|---|---|---|---|---|
| **Start (5')** | 2669 | 2680 | 2813 | 4762 | 8513 | 10244 |
| **End (3')** | 8227 | 2777 | 4960 | 5688 | 9946 | 10463 |

Splice donor (5'SS) sites are located at nucleotides 999-1004, 1076-1081, 2778-2783, 8243-8249, 8372-8378, 8429-8436, 8634-8641, 8701-8708 and 8753-8760. Splice acceptor (3'SS) sites are located at nucleotides 2593-2611, 2680-2699, 8112-8131, 8143-8165 and 10408-10423.

After the first transcribed region, there are three main downstream exons located at nucleotides 2700-2777, 8166-8244 and 10424-11739.

The gag gene (nucleotides 2813-4960 of SEQ ID 1; SEQ ID 57) encodes a 715aa polypeptide (SEQ ID 54).

The protease gene (nucleotides 4762-5688 of SEQ ID 1; SEQ ID 58) is interrupted by three stop codons:

WATIVWKQEEGPASGPPTNWGIPS*TVCSSGFSRTTTPTENTTTSGSQPITTIQQLS
RATAGSTAVDLCSTQMVFLLPGKPPQKIPRGVYGPLPEGRVGL*GRSSLNLKGVQIH
TGVIYSDYKGGIQLVISSTVPRSANPGDRIAQLLLLPYVKIGENKKERTGGFGSTNP
AGKAAYWANQVSEDRPVCTVTIQGKSLKDVDTQADVSVIGIGTASEVYQSAMILHCP
GSDNQESTVQPVITSFIPINLWGRDLLQQWHAEITIPASLYSPRNKKIMTKMG*LPK
KGLGKKEVPIEAEKNQKRKGIGHPF

The four amino acid sequences between stop codons are SEQ IDs 59 to 62.

The pol gene (SEQ ID 86) is also interrupted. Alignment with known pol sequences reveals various fragments of amino acid sequences (SEQ IDs 92 to 97):

ESSKLSIT*LKEQSWLPSLQC*QDFNQSINIVSDSAYVVQATKDIERALIKYIMDDQ
LNPLFNLLQQNVRKRNFPFYITHIRAHTNLPGPLTKANEQADLLVSSAFMEAQELHA
LTHVNAIGLKNKFDITWKQTKNIVQHCTQCQILHLATQEARVNPRGLCPNVLWQMDV
MHVPSFGKLSFVHVTVDTYSHFIWATCQTGESTSHVKRHLLSCFPVMGVPEKVKTDN
GPGYCSKAVQKFLNQWKITHTIGILYNSQGQAIIERTNRTLKAQLVKQKKGKDRSIT
LPRCNLI


*IITLCGNDPDKIT
VPFNKQQVRQAFINSGAWQIGLADFVGIIDNRYPKTKIFQFLKLTTWILPKVTKHKP
LKNALAVFTDGSSNGKVAYTGPKE*

*TKKRKRQEYNTPQMQLNLALYTLNVLNIYRNQTTTSAEQHLTGKRNSPHEGKLIWW
KDNKNKTWEMGKVITWGRGFACVSPGENQLPVWIPTRHLKFYNELTGDAKKSVEMET
PQSTRQVNKMVISEEQKKLPSIKEAELPI

The env gene (nucleotides 9165-9816 of SEQ ID 1; SEQ ID 63) is interrupted by stop codons. The longest uninterrupted sequence encodes amino acid sequence SEQ ID 64. The reading frame +1 to SEQ ID 63 contains several short amino acid sequences (SEQ IDs 65 to 80) between stop codons:

HPELGSLLWPHTTLEFVLEIKL*EQEIVSHIILST*IPV*QFLCKIV*NSLILLVVG
KT*LLNLIPKP*SVKIVECLLALI*LLIGSTVFY*EEQERVCGSLCPWTDHGRLRYP
SIF*RKY*KEF*LDPKDSFLL*WQ*LWASLQSQLLLRLLELLYTPLFKLQNT*MIGK
RIPQNCGILRSK*IKNWQTKLMILDKLSFGWERLMSLEYLFQLRC

Nucleotides 8916-9155 of SEQ ID 1 (SEQ ID 81) are also interrupted to give several short amino acid sequences (SEQ IDs 82 to 85):

VQNNEF*TMIDWVP*GQLYHNCTGQTHSCSQAPSIWPINPAYDGDVTERLDQVYRRL
ESLCPRKWGEKGISSP*PKLVLLLVL

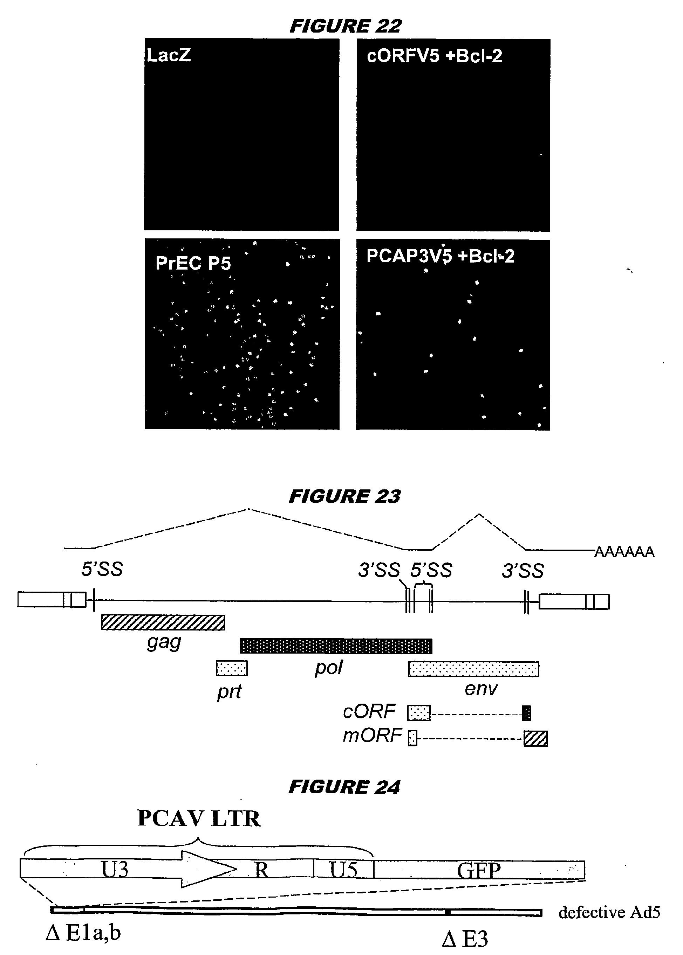

A polypeptide product called 'morf or 'PCAP3' (SEQ ID 87) is roughly equivalent to the 'cORF' product previously seen for HERV-Ks. Its coding sequence begins at nucleotide 8183 of SEQ ID 1, with splicing occurring after nucleotide 8244 and joining to nucleotide 10424. The splice junction forms a AGT serine codon within SEQ ID 88 (Figure 23):

ATGAACTCACTGGAGATGCAAAGAAAAGTGTGGAGATGGAGACACCCCAATCGACTCGCCAGgtaaacaaa 8253
M N S L E M Q R K V W R W R H P N R L A r *

...cctgttctgtctgttgttagTCTACAGGTGTATCCAGCAGCTCCAAAGAGACAGCAACCAGCAAGAATGGGCCATAG 10480
L Q V Y P A A P K R Q Q P A R M G H S

TGACGATGGTGGTTTTGTCAAAAAGAAAAGGGGGGATATGTAAGGAAAAGAGAGATCAGACTTTCACTGTGTCTATGTA 10560
D D G G F V K K K R G G Y V R K R E I R L S L C L C R

GAAAAGGAAGACATAAGAAACTCCATTTTGATCTGTACTAA 10601
K G R H K K L H F D L Y *

Further details about PCAP3 are given below.

### Unique DNA sequence within PCAV gag

PCAV gag contains a 48 nucleotide sequence (SEQ ID 53) which is not found in the closely-related HERV-Ks on chromosomes 3, 6 and 16. The 48mer encodes 16me SEQ ID 110, which is not found in new or in other old HERV-Ks. The top 5 hits in BLAST analysis of a 99mer (3614 to 3712 from SEQ ID 1) comprising SEQ ID 53 shows:

Query: 1      agcacggcaccataccagcaacccacagcgatggcgtctaattcaccagcaacacaggac 60
               ||||||||||||||||||||||||||||||||||||||||||||||||||||||||||||
Sbjct: 125279 agcacggcaccataccagcaacccacagcgatggcgtctaattcaccagcaacacaggac 125338

Query: 61     gcggcgctgtatcctcagccgcccactgtgagacttaat 99
               |||||||||||||||||||||||||||||||||||||||
Sbjct: 125339 gcggcgctgtatcctcagccgcccactgtgagacttaat 125377

>NT_015360S4.5     Genomic Viewer
Homo sapiens chromosome 16 working draft sequence segment
Length = 244218
 Score = 75.8 bits (38), Expect = 3e-12
 Identities = 83/98 (84%)
 Strand = Plus / Plus

Query: 2     gcacggcaccataccagcaacccacagcgatggcgtctaattcaccagcaacacaggacg 61
              |||||||| | ||| |||||||||  ||| ||| || |||| |  ||||| |||||| ||
Sbjct: 15122 gcacggcatcgtacaagcaacccatggcggtggtgtttaatacgtcagcaccacagggcg 15181

Query: 62    cggcgctgtatcctcagccgcccactgtgagacttaat 99
              ||||||||| |||||||||||||||| |||||||||||
Sbjct: 15182 cggcgctgtgtcctcagccgcccactatgagacttaat 15219

>NT_005863S5.5     Genomic Viewer
Homo sapiens chromosome 3 working draft sequence segment
Length = 278948
 Score = 60.0 bits (30), Expect = 2e-07
 Identities = 30/30 (100%)
 Strand = Plus / Plus

Query: 70     tatcctcagccgcccactgtgagacttaat 99
               ||||||||||||||||||||||||||||||

Sbjct: 116212 tatcctcagccgcccactgtgagacttaat 116241

>NT_023409S14.5     Genomic Viewer
Homo sapiens chromosome 6 working draft sequence segment
Length = 238047
 Score = 52.0 bits (26), Expect = 5e-05
 Identities = 26/26 (100%)
 Strand = Plus / Minus

Query: 1     agcacggcaccataccagcaacccac 26
              ||||||||||||||||||||||||||
Sbjct: 63402 agcacggcaccataccagcaacccac 63377

>NT_007592S47.5     Genomic Viewer
Homo sapiens chromosome 6 working draft sequence segment
Length = 250001
 Score = 50.1 bits (25), Expect = 2e-04
 Identities = 28/29 (96%)
 Strand = Plus / Minus

Query: 71    atcctcagccgcccactgtgagacttaat 99
              ||||||||||| |||||||||||||||||
Sbjct: 81143 atcctcagccgtccactgtgagacttaat 81115

### Epitopes within PCAV gag

An alignment of the N-teimini of various HERV-Ks is shown below:

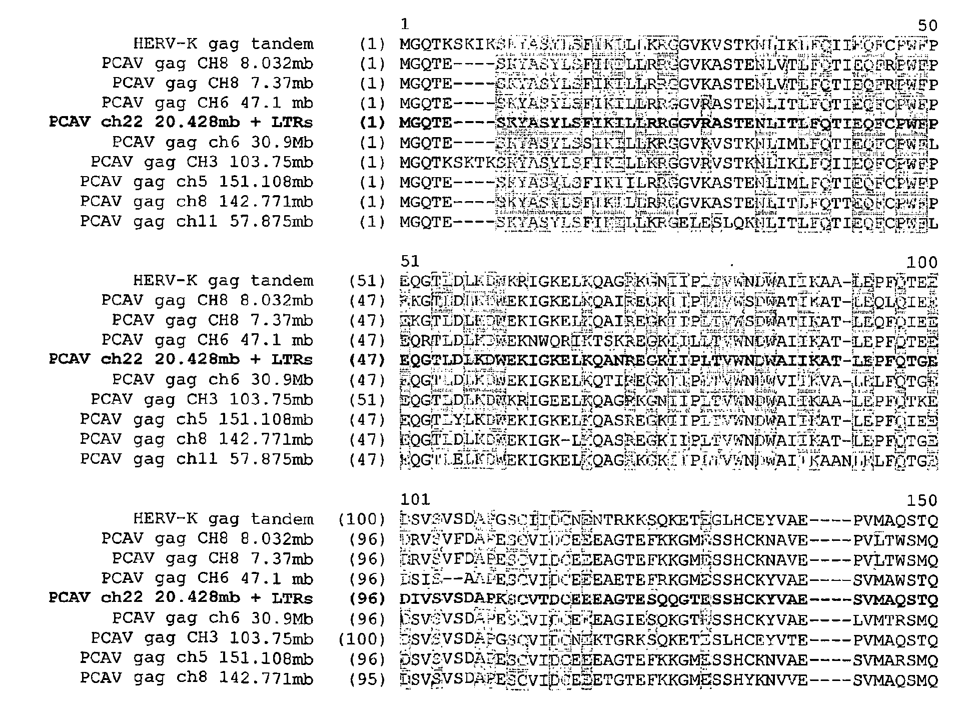

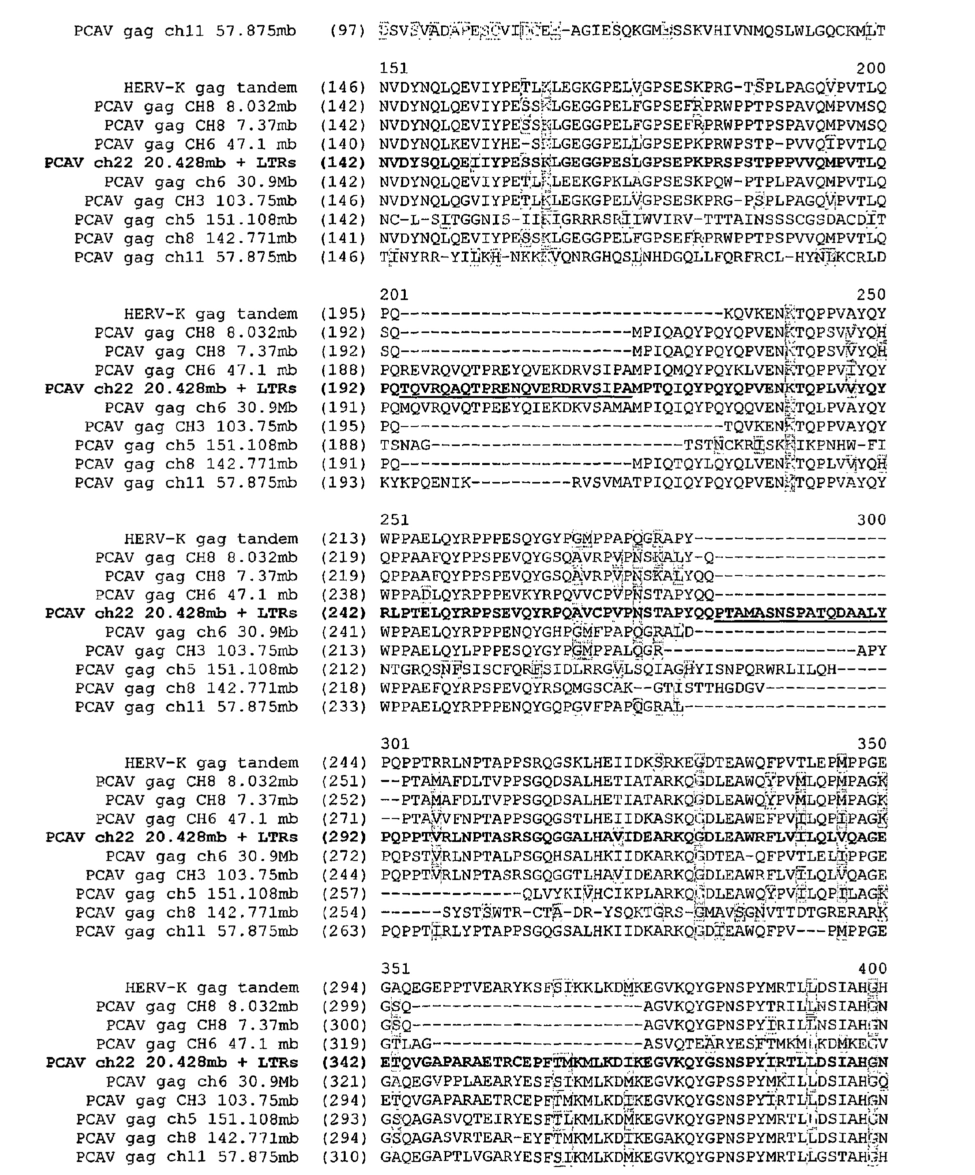

Two regions are particularly useful for generating PCAV-specific detection reagents. The first is from amino acid 203 to 225 in the alignment (SEQ ID 55; encoded by SEQ ID 111). Although this region is present in two other HERV-Ks on chromosome 6, those two viruses are in the old HERV-K group. Background ("ubiquitous") expression of new HERV-Ks is seen in many tissues (*e*.*g*. Figure 10), but not of old HERV-Ks. Detection of SEQ ID 55 therefore distinguishes over background expression of new HERV-Ks and can be used to detect PCAV expression.

The second region is found from amino acids 284-300 (SEQ ID 56; encoded by SEQ ID 112), as this sequence is unique to PCAV. SEQ ID 110 (SEQ ID 53) is a single amino acid truncation fragment of SEQ ID 56.

TBLASTN analysis of SEQ ID 110 against the human genome sequence reveals 100% matches in clones KB208E9 and KB1572G7 at chromosome 22q11.2 but nowhere else. BLASTP analysis fails to identify any matches.

BLASTN analysis of SEQ ID 53 against the human genome sequence reveals a 100% match at nucleotides 3180761 to 3180808 of the Homo sapiens chromosome 22 working draft sequence, and no further hits.

The top five BLASTP hits using SEQ ID 110 against the non-redundant GenBank CDS database are shown below:

>gi|21230944|ref|NP_636861.1|   (NC_003902) conserved hypothetical protein
{Xanthomonas campestris  pv. campestris str. ATCC 33913}
          Length = 515

 Score = 27.8 bits (58), Expect =    12
 Identities = 10/16 (62%), Positives = 12/16 (74%), Gaps = 2/16 (12%)

Query: 1   TAMASNSPATQ--DAA 14
            T MAS++ ATQ  DAA
Sbjct: 483 TGMASDASATQEDDAA 498


>gi|12852148|dbj|BAB29293.1|   (AK014354) data source:SPTR, source key:Q92524,
           evidence:ISS~homolog to 26S PROTEASE REGULATORY SUBUNIT
           S10B (PROTEASOME SUBUNIT P42)~putative {Mus musculus}
          Length = 389

 Score = 27.4 bits (57), Expect =    16
 Identities = 9/13 (69%), Positives = 10/13 (76%)

Query: 3   MASNSPATQDAAL 15
            MA+NSP T D AL
Sbjct: 277 MATNSPDTLDPAL 289


>gi|7105525|gb|AAF35993.1|AC005836_5   (AC005836) 26S Protease Regulatory Subunit
{Leishmania major}
          Length = 396

 Score = 26.9 bits (56), Expect =    22
 Identities = 9/13 (69%), Positives = 10/13 (76%)

Query: 3   MASNSPATQDAAL 15
            MA+N P T DAAL
Sbjct: 283 MATNRPDTLDAAL 295

>gi|15233182|ref|NP_191727.1|   (NM_116033) putative protein {Arabidopsis thaliana}
          Length = 658

 Score = 26.1 bits (54), Expect =    39
 Identities = 8/9 (88%), Positives = 8/9 (88%)

Query: 1  TAMASNSPA 9
          TAMAS SPA
Sbjct: 5  TAMASTSPA 13


>gi|21243749|ref|NP_643331.1|   (NC_003919) hypothetical protein {Xanthomonas
axonopodis pv. Citri str. 306}
          Length = 206

 Score = 25.7 bits (53), Expect =    52
 Identities = 8/12 (66%), Positives = 10/12 (82%)

Query: 2   AMASNSPATQDA 13
           AMA+ SPAT +A
Sbjct: 189 AMAATSPATPNA 200

SEQ ID 110 is therefore unique to PCAV.

### Prediction of cDNA sequences

On the basis of splice donor and acceptor sites, SEQ IDs 99 to 109 were constructed. SEQ ID 109 begins in the second 5' LTR.

SEQ IDs 99 to 108 align to SEQ ID 10 as follows:

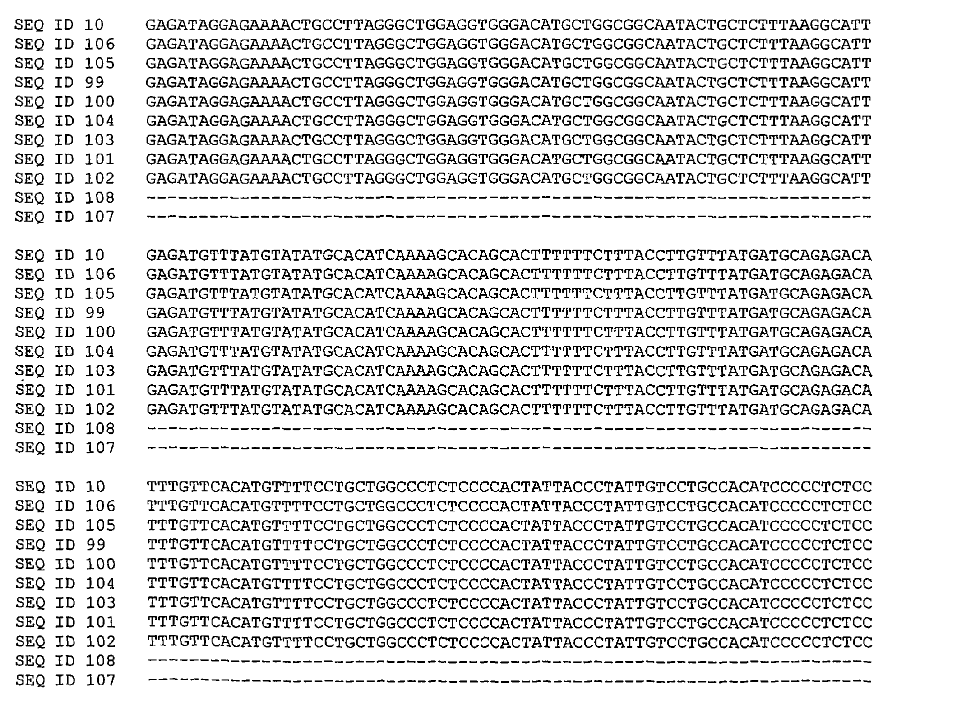

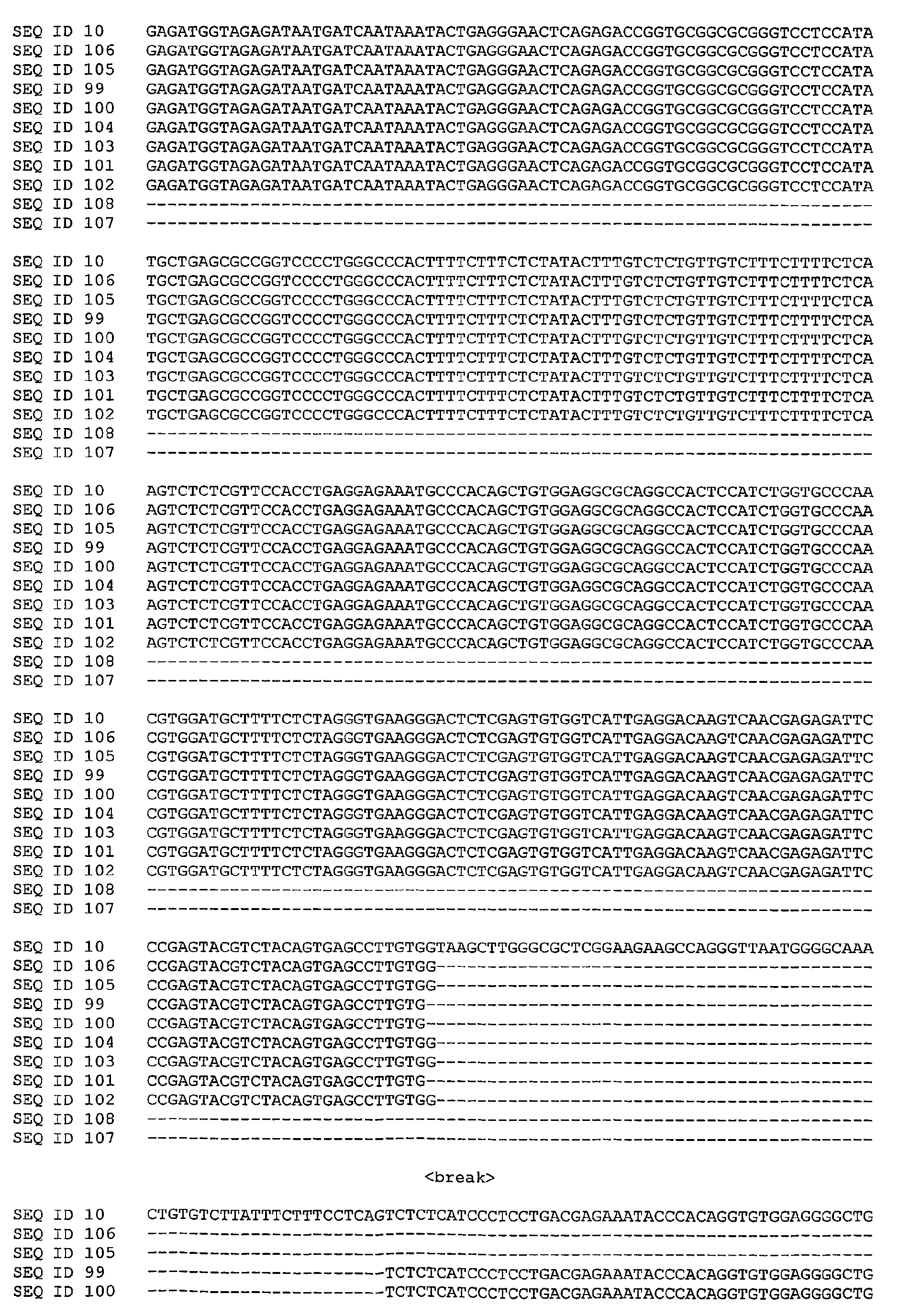

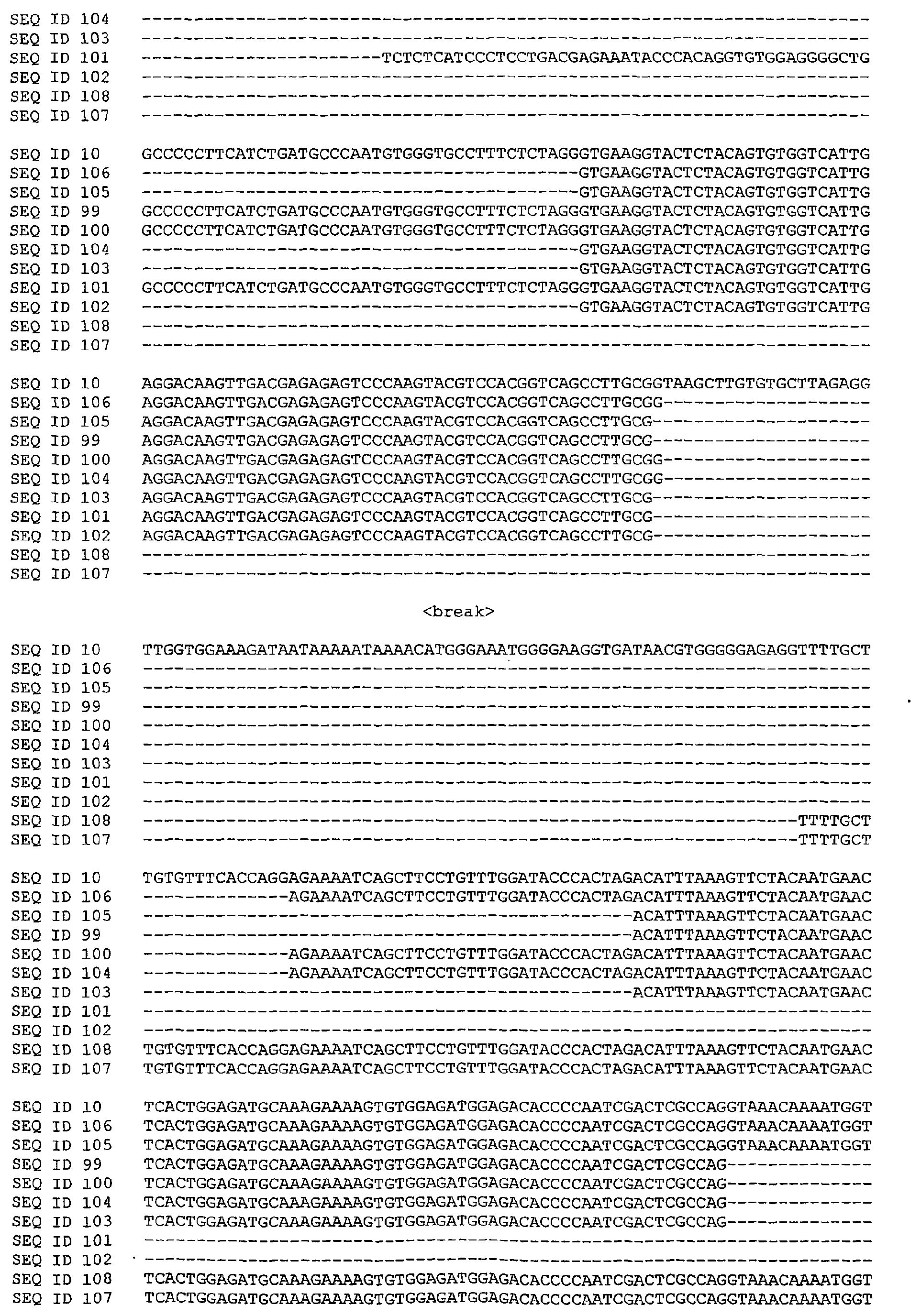

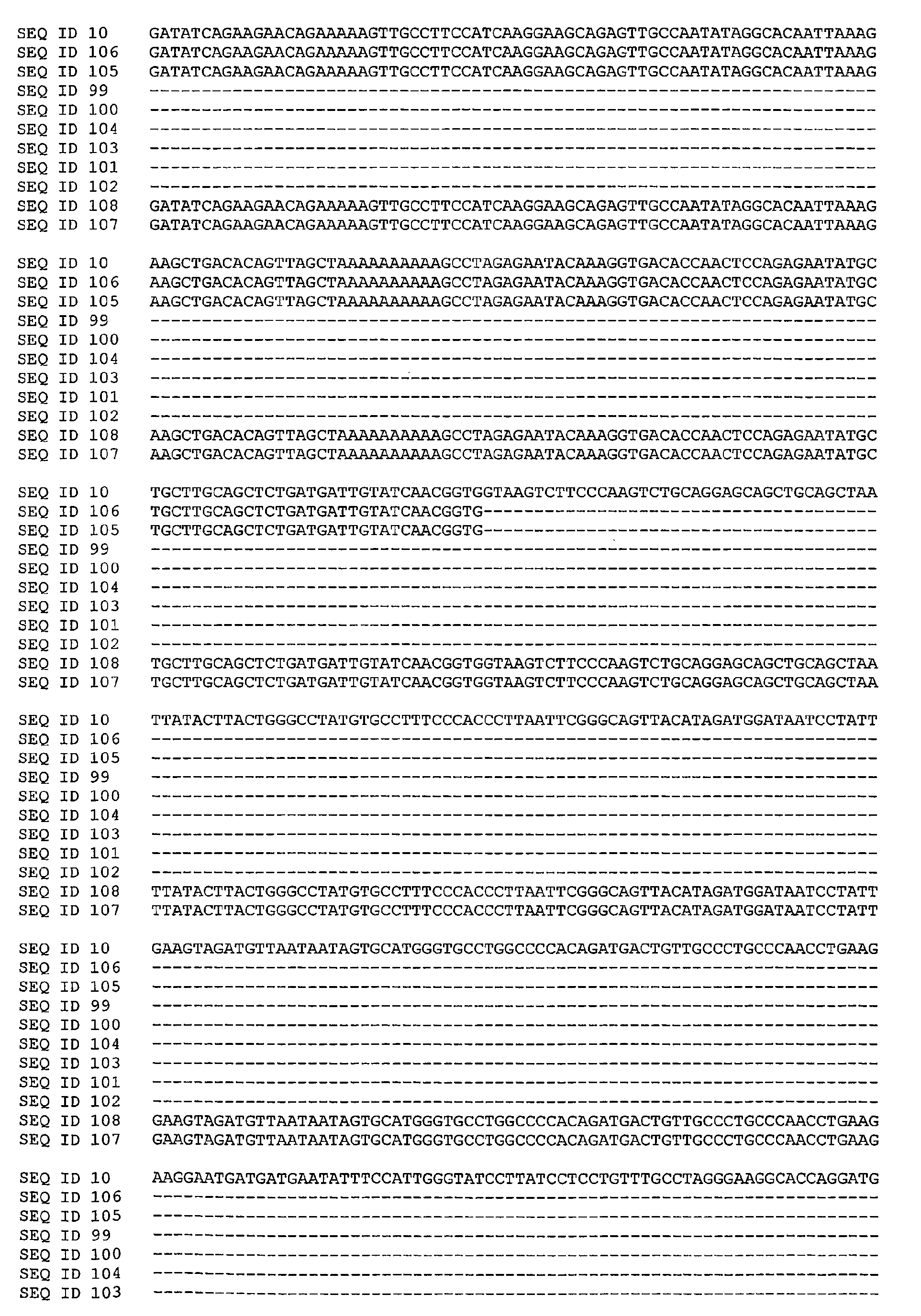

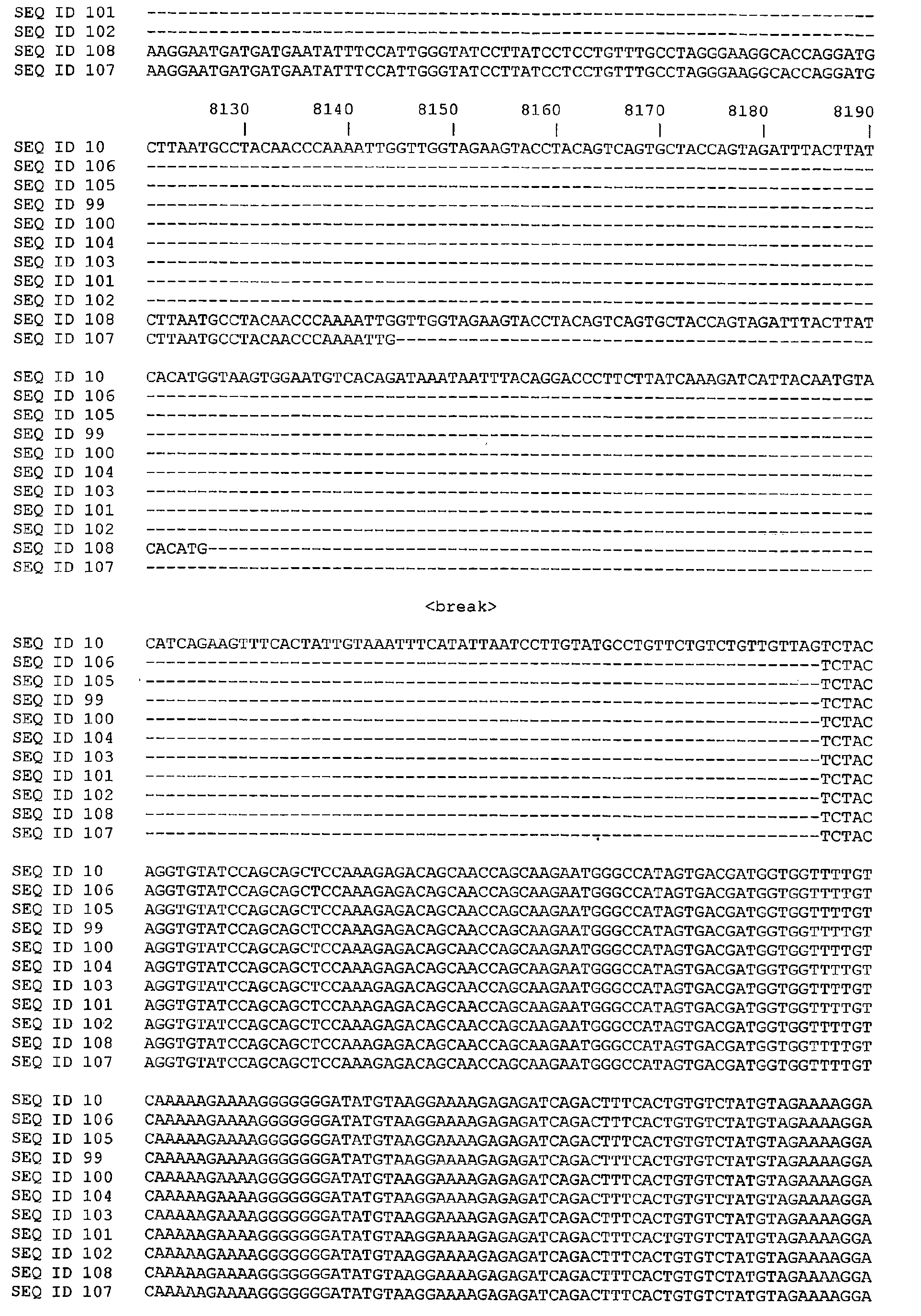

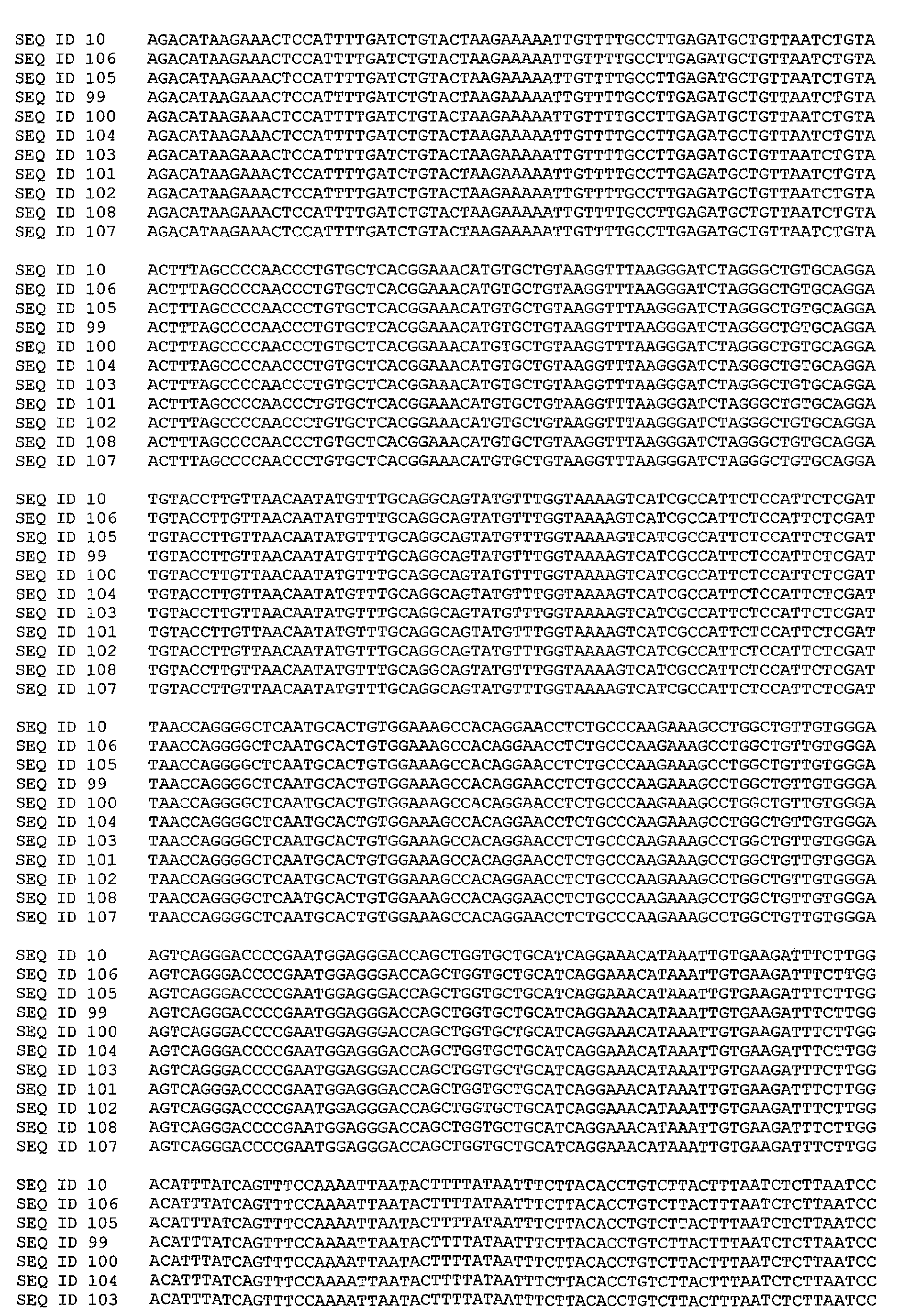

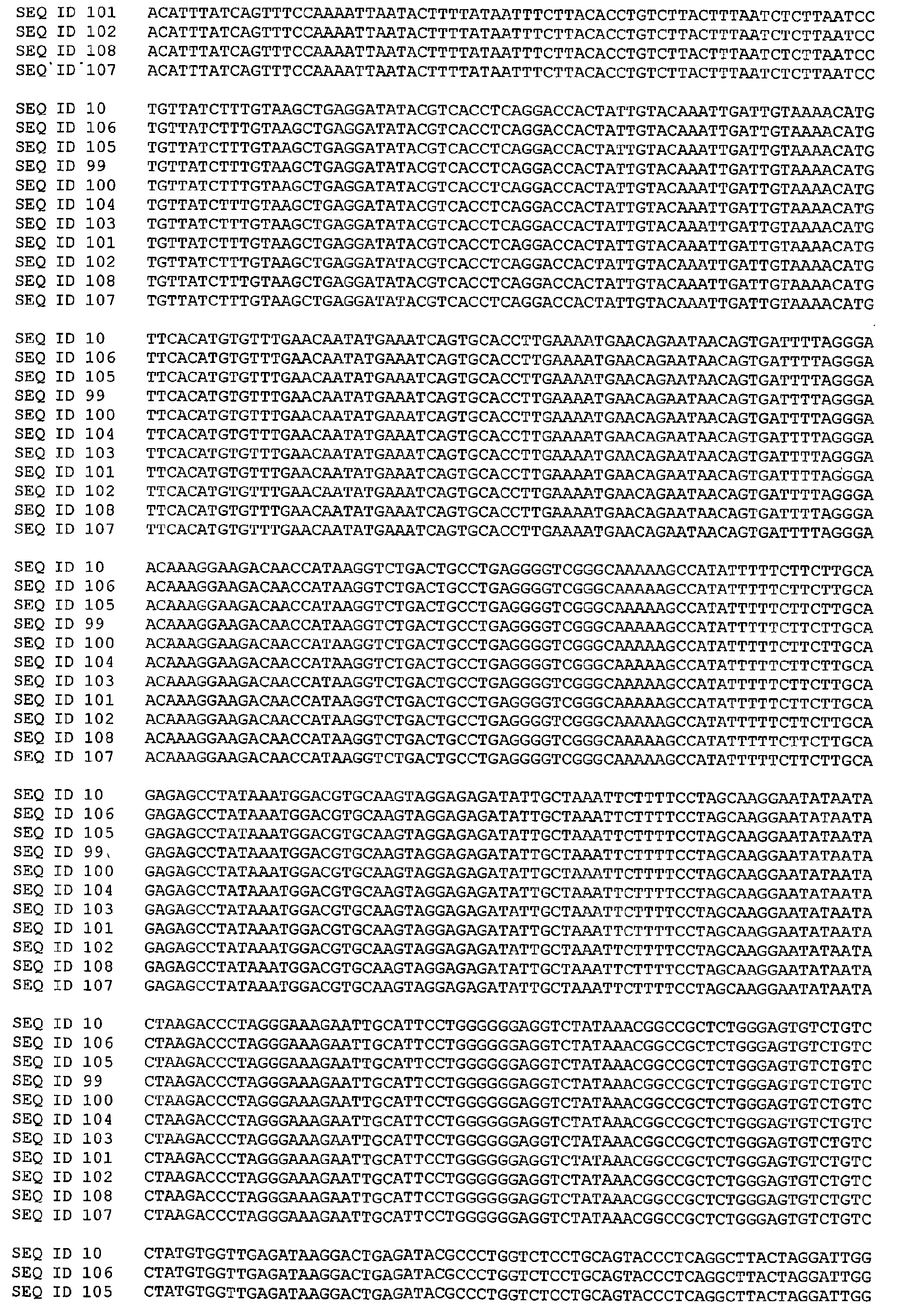

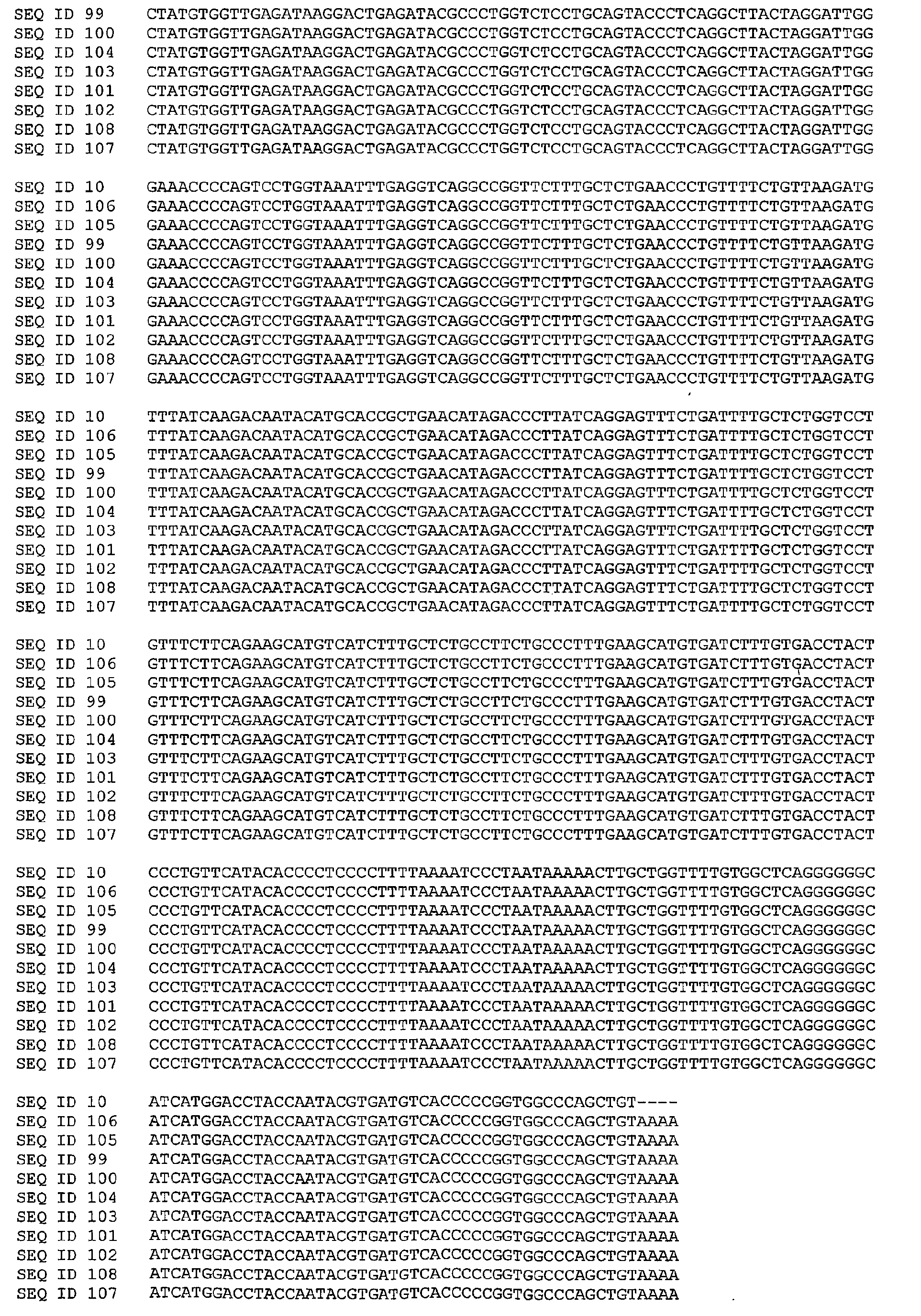

### THE TRANSCRIPTION START SITE OF PCA V

By homology to other retroviruses, the 5' end of PCAV-mRNA (*i*.*e*. the transcription start site within the PCAV genome) should fall 30 bases downstream of the canonical TATA sequence, at nucleotide 559 in SEQ ID 1.

However, empirical work suggests that the 5' end of PCAV-mRNA is further downstream.

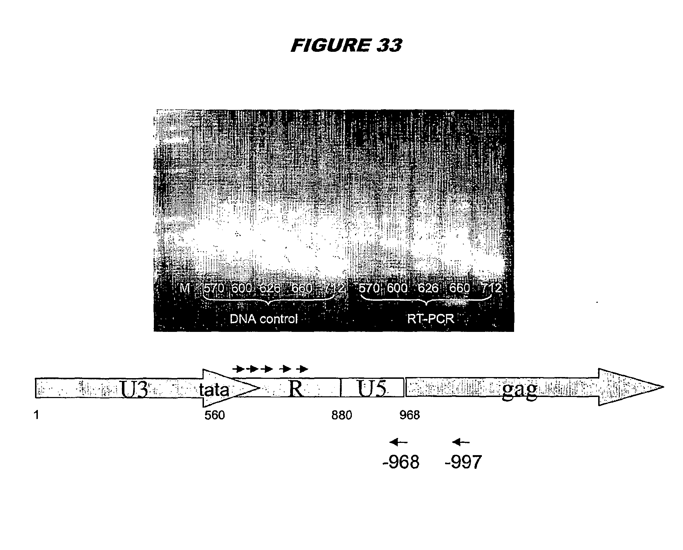

Figure 33 shows the results of a RT-PCR scanning assay used to map the 5' end. cDNA of the 5' LTR was prepared by priming total Tera1 RNA with an antisense oligonucleotide spanning 997 to 972 in the proviral genome (SEQ ID 1202). This cDNA was then divided and run in PCR analyses with an antisense primer from 968 to 950 (SEQ ID 1203) combined with a sense primer from a set of primers designed to cover the likely 5' ends: 1) 571 <SEQ ID 1204>, 2) 600 <SEQ ID 1205>, 3) 626 <SEQ ID 1206>, 4) 660 <SEQ ID 1207>, 5) 712 <SEQ ID 1208>. Duplicate PCR reactions on 1 µg genomic HeLa DNA were used as a positive control, and these reactions showed all primer pairs were effective. The reactions primed with cDNA showed a marked difference between primers 600 and 626, suggesting that the 5' end lies near position 626 in the proviral genome.

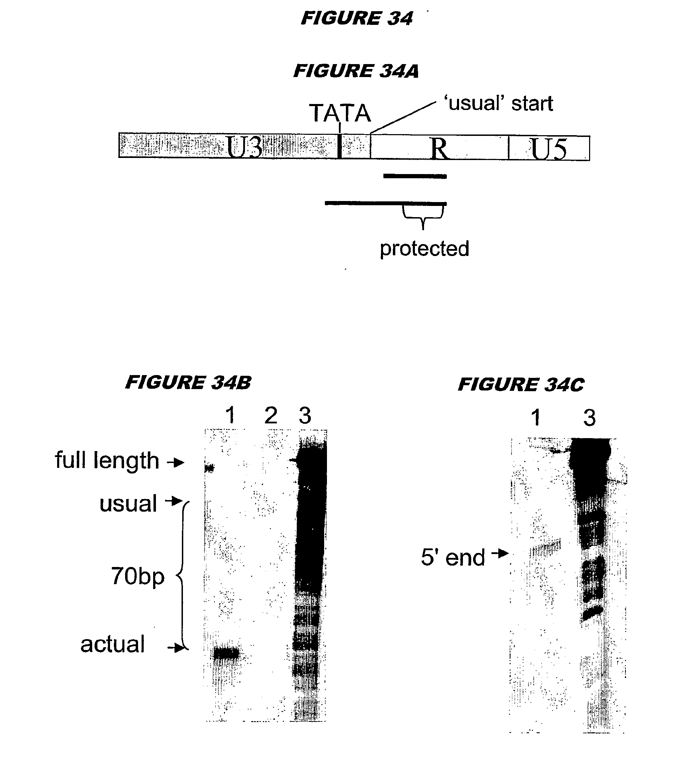

This result was confirmed using RNase protection assays (Figure 34). Labeled antisense RNA probes covering bases (34B) 509-735 and (34C) 600-735 in the proviral genome were hybridized to total RNA from Teral cells and digested with RNase under standard conditions. After processing and detection by urea-containing PAGE, both probes gave 100 base products. These two results agree and show that 5' end of HERV-K RNA is around base 635 in the proviral genome *i*.*e*. around 100bp downstream of the TATA signal, rather than the 30bp which is usual for TATA-dependent genes.

### PCAP3

Within the final exon in the env region of PCAV, reading frames 1 and 2 encode env and cORF, respectively (Figure 23). SEQ ID 87 is PCAP3, which shares the same 5' region and start codon as env, but in which a splicing event removes env-coding sequences and shifts to a reading frame +2 relative to that of env (SEQ IDs 88 & 1191):

ATGAACTCACTGGAGATGCAAAGAAAAGTGTGGAGATGGAGACACCCCAATCGACTCGCCAGgtaaacaaa 8253
M N S L E M Q R K V W R W R H P N R L A

...cctgttctgtctgttgttagTCTACAGGTGTATCCAGCAGCTCCAAAGAGACAGCAACCAGCAAGAATGGGCCATAG 10480
L Q V Y P A A P K R Q Q P A R M G H S

TGACGATGGTGGTTTTGTCAAAAAGAAAAGGGGGGATATGTAAGGAAAAGAGAGATCAGACTTTCACTGTGTCTATGTA 10560
D D G G F V K K K R G G Y V R K R E I R L S L C L C R

GAAAAGGAAGACATAAGAAACTCCATTTTGATCTGTACTAA 10601
K G R H K K L H F D L Y *

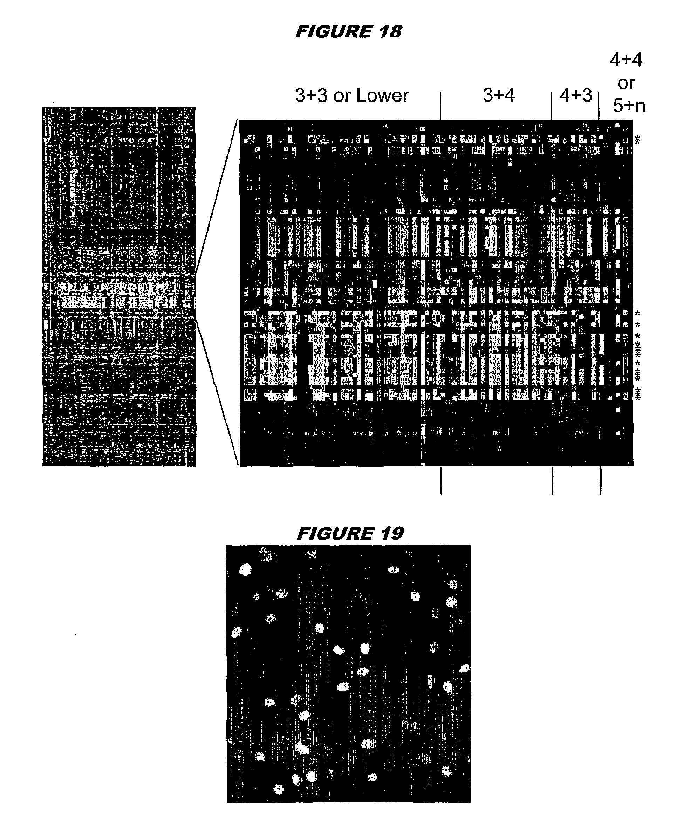

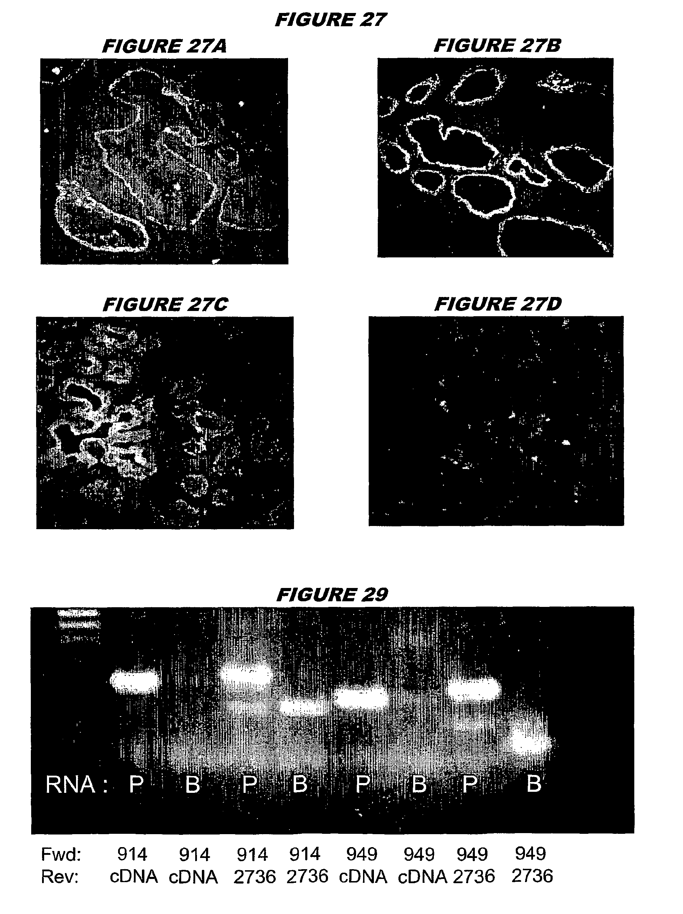

The majority of the coding sequence is thus located after the splice, within the exon which contains the 3' LTR. Although the +2 reading frame has no known function in HERV-K, cDNA prepared from prostate cancer cell line MDA Pca-2b included these transcripts, as did prostate cancer mRNA. For example, spot 34058 (see above) encodes PCAP3 and was up-regulated more than 2-fold in 79% of patient samples and more than 5-fold in 53%. These figures support the view that PCAP3 is involved in many prostate cancers. Furthermore, the figures do not reflect the whole relationship between cancer and PCAP3 expression - if patients are grouped according to Gleason grades, grade 3 tumors show high up-regulation of PCAP3 whereas more developed grade 4 tumors seem to show PCAP3 suppression. Figure 18 shows microarray analysis of prostate cancer employing 6000 random ESTs from a normalized prostate library. RNA levels prepared from laser-captured, micro-dissected tumor is compared to peri-tumor normal tissue RNA. The sequences tagged with asterisks in Figure 18 are up-regulated and are all from a single 12kb site in chromosome 22. These sequences span all portions of PCAV. Relative PCAV expression is very high in grade 3 tumors, with many of the patients having tumor/normal ratios in the 10 to 50 fold range. In Gleason grade 4 and above, however, the ratios return to 1 and in some cases the virus expression is suppressed. A similar pattern is seen with gag expression (Figure 27), suggestion that PCAV expression is involved in the early stages of prostate cancer.

PCAP3 is similar to the cORF protein, and the two ORFs share a start codon, but two small deletions in PCAV introduce both a frameshift and an 'old virus' 5' splice site (splice acceptor), thereby permitting the PCAP3-specific splice event. Inspection of various aligned HERV-K genomes gives further evidence that PCAP3 is a mutated form of an original protein. The protein is thus unlikely to be functioning in its original capacity, and oncogenic activity could arise through retention of a functional domain. The coding exon common to env, cORF and PCAP3 contains a RNA-binding domain that also functions as a nuclear localization signal (NLS).

To study the subcellular localization of PCAP3, in order to better understand its role, an adenovirus expressing PCAP3 with a C-terminal V5 tag (SEQ ID 1189) was used to infect primary prostate epithelial cells. The protein was relatively stable and was labeled in the nucleoplasm by anti-V5 (Figure 19). The concentration of this small protein in this cellular location shows that it is specifically interacting with something within the nucleus.

A functional expression assay was also designed. The first component of the assay is an adenovirus vector with a PCAV LTR (SEQ ID 1190) driving GFP expression (Figure 24). A variety of human cell lines were infected with this virus and fluorescence was measured either by fluorescent microscopy or by FACS. As a positive control, a vector was used in which GFP expression was driven by the EF-a promoter, which should be active in all eukaryotic cells.

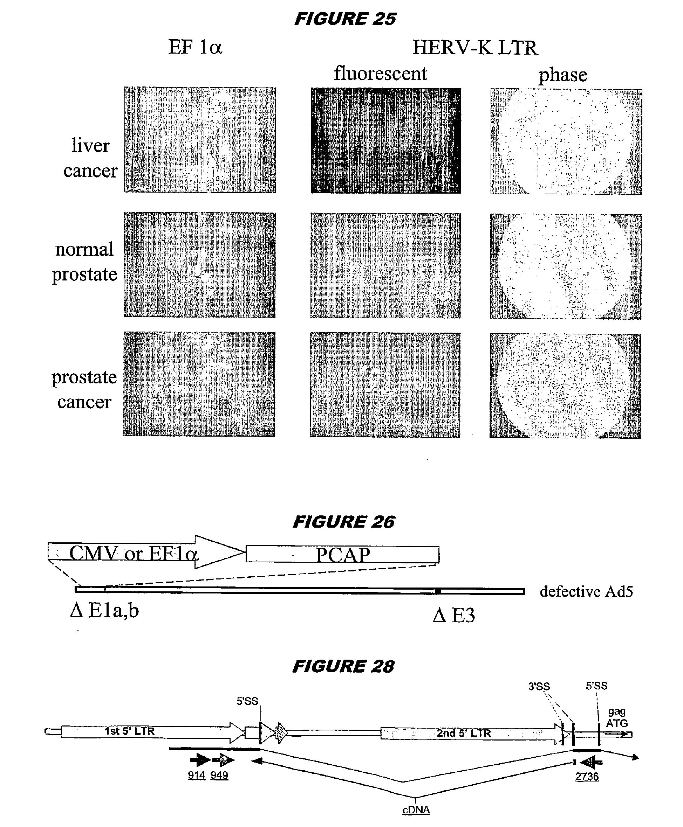

GFP expression was minimal in ovarian, colon and liver cancer cells. It was also minimal in 293 cells, an immortalized kidney cell line, and in primary prostate epithelium cells. GFP was easily detected in various prostate cancer cell lines (PC3, LNCaP, MDA2B PCA, DU145). Representative data are shown in figure 25. The GFP expression pattern exactly matches genomics results from patient samples. These data indicate that expression driven from a PCAV-mRNA LTR is a marker for prostate cancer.

As GFP expression from the LTR appeared to be silent in primary prostate cells, but active in prostate cancer tissue, PCAP3 was tested for its ability to activate expression in primary prostate cells. The coding sequence was inserted into an expression cassette and incorporated into an adenovirus vector (Figure 26). The vector was co-infected with the GFP vector into primary prostate epithelial cells, and PCAP3 weakly activated GFP expression.

In a separate experiment, high passage PrECs (approaching senescence) were co-infected with an adenovirus vector expressing GFP from an old-type HERV-K LTR ('MDALTR': SEQ ID 1196), and a second vector expressing PCAP3 at moi of about 20. After 3 days, the fluorescent intensity was measured by FACs and activation by PCAP3 was seen. In a similar experiment with LTR60, however, there was no activation.

### PCAP3 AND SENESCENCE

Prostate cancer is believed to arise in the luminal epithelial layer, but normal luminal epithelial cells are capable of very few cell divisions. In contrast, NIH3T3 and RWPE1 cells (see Figures 11 & 12) are immortal. Because PCAV seems to be involved in early stages of cancer, the effects of PCAP3 on primary prostate epithelial cells (PrEC), which normally senesce rapidly, were tested.

Primary human epithelial cells have a very limited division potential. After a certain number of divisions the cells will enter senescence. Senescence is distinct from quiescence (immortal or pre-senescent cells enter quiescence when a positive growth signal is withdrawn, or when an inhibitory signal such as cell-cell contact is received, but can be induced to divide again by adding growth factors or by re-plating the cells at lower density) and is a permanent arrest in division, although senescent cells can live for many months without dividing if growth medium is regularly renewed.

Certain genes, particularly viral oncogenes (*e*.*g*. SV40 T-antigen) force cells to ignore senescence signals. T-antigen stimulates cells to continue division up to a further expansion barrier termed 'replicative crisis'. Two processes occur in crisis: cells continue to divide, but cells die in parallel at a very high rate from accumulated genetic damage. When cell death exceeds division then virtually all cells die in a short period. The rare cells which grow out after crisis have become immortal and yield cell lines. Cell lines typically have obvious genetic rearrangements: they are frequently close to tetraploid, there are frequent non-reciprocal chromosomal translocations, and many chromosomes have deletions and amplifications of multiple loci {169, 170, 171}.

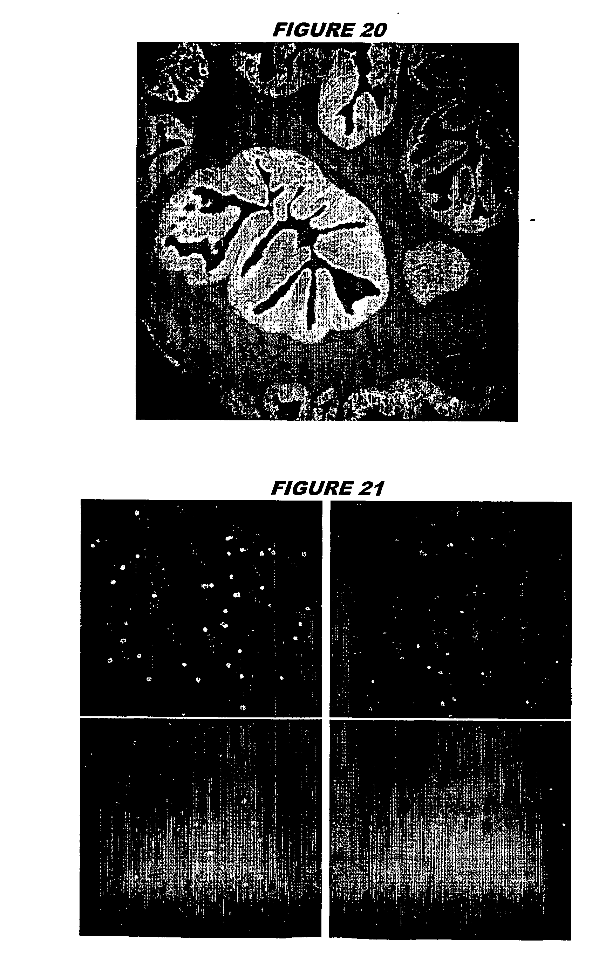

Gene products that lead to crisis are particularly interesting because prostate cancers exhibit high genomic instability, which could be caused by post-senescence replication. Current theory holds that prostate cancer arises from lesions termed prostatic intraepithelial neoplasia (PIN) {172}. Genetic analyses of PIN show that many of the genetic rearrangements characteristic of prostate cancer have already occurred at this stage {173}. PIN cells were thus tested for PCAV expression to determine if the virus could play a role in the earliest stages of prostate cancer. PCAV gag was found to be abundantly expressed (Figure 20), indicating that PCAV expression is high at the time when the genetic changes associated with prostate cancer occur. As PCAP3 was seen to be expressed in prostate cancer, its role was investigated by seeing if it is capable of inducing cell division in PrEC after senescence.

Initial attempts to select drug-resistant PrECs after transfection with PCAP expression plasmids failed. Analysis of PrEC after infection with adenovirus vectors expressing either GFP or PCAP3 revealed abundant cell death on day 4 post-infection in the PCAP3 cells. A dose-dependent increase in terminal deoxytransferase end labeling (TUNEL), to mark nuclei with nicked DNA, confirmed that the cells were undergoing apoptosis (Figure 21). This apoptosis may explain the failure to isolate drug-resistant PrECs, and is consistent with engagement of cell division machinery by PCAP3, as an unbalanced growth signal is an inducer of apoptosis.

These results suggested that apoptosis would have to be blocked before the effect of PCAP3 expression in PrECs could be assessed. Plasmids encoding PCAP3 plus a neomycin marker were thus co-transfected with an expression plasmid encoding bcl-2 (anti-apoptosis) and lacZ (marker). As controls, cells were transfected with plasmids expressing neomycin and either lacZ, bcl-2, bcl-X_{L}, or PCAP3. After two weeks under selection, the lacZ, bcl-2 and bcl-XL dishes all had numerous resistant cells that grew to fill in a fraction of the dish. When these cell were split they failed to divide further, but were viable and resembled senescent parental cells. In contrast, the cells which expressed PCAP3 and bcl-2 yielded some colonies made up of small cells which divided to fill the initial plate and continued to divide when split.

In parallel to the above drug selections, the growth potential of cells was assessed. The parental PrECs went through seven population doublings before reaching senescence. In contrast, drug-resistant cells co-transfected with an anti-apoptotic gene plus PCAP3 expanded well beyond the senescence point before ceasing to grow, going through sixteen doublings. After rapid growth for around two weeks, expansion of the cells slowed and finally ceased. Concomitantly, the number of floating and dead cells increased and the appearance of the cells changed - they no longer had the regular "cobblestone" appearance of epithelial cells, but instead had several morphologies, and there were many multinucleate cells. Cells died two weeks later, while the cells transfected with lacZ or lacZ+bcl-2 were still alive one month later.

Neither senescent cells nor cells approaching crisis expand in number. One difference between them, however, is that cells approaching crisis are dividing and dying at an appreciable rate, and so cell division can distinguish between the two states. After labeling with bromo-deoxyuridine, 30% of pre-senescent PrECs were labeled, as were 10% of PrEC transfected with PCAP3 + bcl-2, but none of the senescent lacZ or cORF + bcl-2 controls were labeled (Fig. 22).

These results show that PCAP3 is capable of inducing growth in prostate epithelial cells, and this growth could be an underlying cause of prostate cancer.

### PCAV DETECTION BY PCR

Primer pairs were tested to determine those which produced the expected PCAV product on prostate samples (P) and little or no product on breast sample (B). The primers are shown on the map of the 5' LTRs of PCAV in Figure 28. Forward primers were '914' (SEQ ID 1192) or '949' (SEQ ID 1193); reverse primers were '2736' (SEQ ID 1194) or 'cDNA' (SEQ ID 1195). The cDNA primer spans the splice junction. Each reaction was run for 30 cycles on dT-primed cDNA prepared from total RNA extracted from either MCF7 (B) or MDA PCA 2b (P) cells.

Results are shown in Figure 29. The primers clearly show preferential amplification in the prostate cells, and the primer bridging the splice junction ('cDNA') is highly specific.

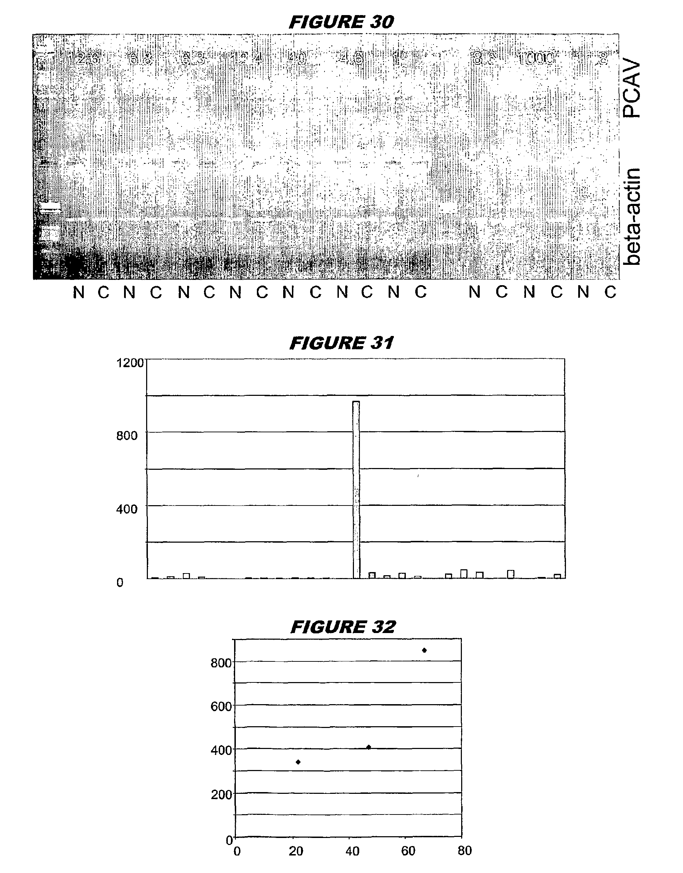

Semi-quantitative RT-PCR experiments were also performed. Amplified RNA from LCM-derived prostate tissue from 10 patients was reverse transcribed using the 2736 primer, followed by PCR amplification either with the '914' and 'cDNA' primer pairs (28 cycles), or with standard primers for human β-actin (25 cycles). Results are shown in Figure 30. Matched samples of normal (N) or cancer (C) were amplified. The signal ratio in cancer tissue compared to normal tissue for each pair is shown above the PCAV PCR products.

Primers '914' and 'cDNA' were also tested in quantitative PCR against dT-primed cDNA from a variety of tissues. As shown in Figure 31, only prostate tissue from a 47 year old patient gave a significant signal.

RT-PCR was also performed on prostate tissue from patients of various ages. Expression levels were compared to gusB (β-glucuronidase). Results were as follows:

| **Age** | **PCAV RT-PCR** | **GusB RT-PCR** | **Normalized PCAV** | **Normalized GusB** |
|---|---|---|---|---|
| 22 | 546 | 1105 | 1.60 | 340 |
| 47 | 430 | 729 | 1.06 | 406 |
| 67 | 848 | 689 | 1 | 848 |

| | | | | |
|---|---|---|---|---|
| The normalized PCAV figures are also shown in Figure 32. | | | | |

The above description of preferred embodiments of the invention has been presented by way of illustration and example for purposes of clarity and understanding. It is not intended to be exhaustive or to limit the invention to the precise forms disclosed. It will be readily apparent to those of ordinary skill in the art in light of the teachings of this invention that many changes and modifications may be made thereto without departing from the spirit of the invention. It is intended that the scope of the invention be defined by the appended claims and their equivalents.

All patents, applications and references cited herein are incorporated by reference in their entirety.

### SEQUENCE LISTING INDEX

| **SEQ ID** | **DESCRIPTION** |
|---|---|
| 1 | PCAV, from the beginning of its first 5' LTR to the end of its fragmented 3' LTR |
| 2 | Fragment of SEQ ID 1, from predicted transcription start site (559) to conserved splice donor site (1075) |
| 3 | Fragment of SEQ ID 1, following a splice acceptor site within second 5' LTR (2611-2620) |
| 4 | Fragment of SEQ ID 1, following a splice acceptor site downstream of second 5' LTR (2700-2709) |
| 5 | SEQ ID 2 + SEQ ID 3 |
| 6 | SEQ ID 2 + SEQ ID 4 |
| 7 | Fragment of SEQ ID 1: 5' end of 3' LTR (10520-10838) |
| 8 | Fragment of SEQ ID 1: MER11a insertion within 3' LTR, up to polyA site (10839-11736) |
| 9 | SEQ ID 7 + SEQ ID 8 |
| 10 | Fragment of SEQ ID 1, from transcription start site to poly-A signal |
| 11 | Four 3' nucleotides of SEQ ID 2 + four 5' nucleotides of SEQ ID 3 |
| 12 | Four 3' nucleotides of SEQ ID 2 + four 5' nucleotides of SEQ ID 4 |
| 13 | Four 3' nucleotides of SEQ ID 7 + four 5' nucleotides of SEQ ID 8 |
| 14 | 27378 |
| 15 | 34058 |
| 16 | 26254 |
| 17 | Contig AP000345 |
| 18 | Contig AP000346 |
| 19 | cDNA sequence SP MDA#6 x SP6 rev |
| 20-22 | RACE primers |
| 23 | mRNA form of SEQ ID 10 |
| 24 | mRNA form of SEQ ID 5 |
| 25 | mRNA form of SEQ ID 6 |
| 26 | mRNA form of SEQ ID 2 |
| 27 | mRNA form of SEQ ID 3 |
| 28 | mRNA form of SEQ ID 4 |
| 29 | mRNA form of SEQ ID 9 |
| 30 | mRNA form of SEQ ID 7 |
| 31 | mRNA form of SEQ 1D 8 |
| 32 | The alu interruption of env (9938-10244 of SEQ ID 1) |
| 33 | The 10 nucleotides upstream of SEQ ID 32 in SEQ ID 1 |
| 34 | The 10 nucleotides downstream of SEQ ID 32 in SEQ ID 1 |
| 35 | First 10 nucleotides of SEQ ID 32 |
| 36 | SEQ ID 33 + SEQ ID 35 |
| 37 | The 100 nucleotides upstream of SEQ ID 32 in SEQ ID 1 |
| 38 | SEQ ID 37 + SEQ ID 32 |
| 39 | Four 3' nucleotides of SEQ ID 37 + four 5' nucleotides of SEQ ID 32 |
| 40 | The 100 nucleotides downstream of SEQ ID 32 in SEQ ID 1 |
| 41 | Last 10 nucleotides of SEQ ID 32 |
| 42 | SEQ ID 41 + SEQ ID 40 |
| 43 | SEQ ID 32 + SEQ ID 40 |
| 44 | Four 3' nucleotides of SEQ ID 32 + four 5' nucleotides of SEQ ID 40 |
| 45 | Ten 3' nucleotides of SEQ ID 32 + ten 5' nucleotides of SEQ ID 40 |
| 46 | Fragment of SEQ ID 1, following a splice acceptor site within second 5' LTR (2611-2710) |
| 47 | SEQ ID 2 + SEQ ID 46 |
| 48 | Fragment of SEQ ID 1, following a splice acceptor site downstream of second 5' LTR (2700-2799) |
| 49 | SEQ ID 2 + SEQ ID 48 |
| 50 | Ten 3' nucleotides of SEQ ID 2 + SEQ ID 3 |
| 51 | Ten 3' nucleotides of SEQ ID 2 + SEQ ID 4 |
| 52 | Ten 3' nucleotides of SEQ ID 7 + ten 5' nucleotides of SEQ ID 8 |
| 53 | Gag nucleotide sequence unique to PCAV |
| 54 | PCAV gag |
| 55 | Gag fragment of SEQ ID 54 |
| 56 | Gag fragment of SEQ ID 54 |
| 57 | Gag (encodes SEQ ID 54) |
| 58 | Prt |
| 59-62 | Prt amino acid fragments |
| 63 | Env |
| 64-80 | Env amino acid fragments |
| 81 | Env |
| 82-85 | Env amino acid fragments |
| 86 | Pol |
| 87 | PCAP3 amino acid sequence |
| 88 | PCAP3 gene (spliced) |
| 89 | MDARU3#1xT7rev |
| 90 | MDARU3#2xSP6REV |
| 91 | MDARU3#4xSP6rev |
| 92-97 | Pol amino acid fragment |
| 98 | Variant of SEQ ID 87 |
| 99-109 | Sequences of spliced cDNAs |
| 110 | Amino acids encoded by SEQ ID 53 |
| 111 | Nucleotides encoding SEQ ID 55 |
| 112 | Nucleotides encoding SEQ ID 56 |
| 113-119 | Hybridizing sequences with homology to chromosome 22 |
| 120-599 | 25mer PCAV fragments |
| 600-1184 | 25mer PCAV fragments with good predicted Tm values |
| 1185 | "New" gag construct |
| 1186 | "New" gag protein |
| 1187 | "Hybrid" gag construct |
| 1188 | "Hybrid" gag protein |
| 1189 | V5 tag |
| 1190 | HML-2 LTR |
| 1191 | cDNA sequence encoding PCAP3 |
| 1192-95 | PCAV-specific primers |
| 1196 | MDALTR |
| 1197 | SEQ ID 23 excluding its 77 5' nucleotides |
| 1198 | SEQ ID 23 excluding its 100 5' nucleotides |
| 1199 | SEQ ID 24 excluding its 77 5' nucleotides |
| 1200 | SEQ ID 25 excluding its 77 5' nucleotides |
| 1201 | SEQ ID 26 excluding its 77 5' nucleotides |
| 1202-08 | Oligonucleotides used during RT-PCR mapping of transcription start site |

### REFERENCES (the contents of which are hereby incorporated in full by reference)

{1} International patent application WO02/46477 (PCT/US01/47824. filed December 7, 2001).
{2} US patent application 10/016,604 (filed December 7, 2001).
{3} Reus et al. (2001) J. Virol. 75:8917-8926.
{4} Dunham et al. (1999) Nature 402:489-495.
{5} Prediger (2001) Methods Mol Biol160:49-63.
{6} Bustin (2000) J. Mol. Endocrinol. 25:169-193.
{7} Gene Cloning and Analysis by RT-PCR (eds. Siebert et al.) ISBN: 1881299147.
{8} RT-PCR Protocols (ed. O'Connell) ISBN: 0896038750.
{9} The PCR Technique: RT-PCR (ed. Siebert) ISBN: 1881299139.
{10} Thaker (1999) Methods Mol Biol 115:379-402.
{11} Seiden & Sklar (1996) Important Adv Oncol 191-204.
{12} Hagen-Mann & Mann (1995) Exp Clin Endocrinol Diabetes 103:150-155.
{13} Clementi et al. (1993) PCR Methods Appl 2:191-196.
{14} Robbins et al. (1997) Clin Lab Sci. 10(5):265-71.
{15} de la Taille (1999) Prog Urol 9:1084-1089.
{16} Ylikoski et al. (1999) Clin Chem 45(9):1397-1407.
{17} Yao et al. (1996) Cancer Treat Res 88:77-91.
{18} Ylikoski et al. (2001) Biotechniques 30:832-840
{19} Shirahata & Pegg (1986) J. Biol. Chem. 261(29):13833-7.
{20} RNA Methodologies (Farrell, 1998) (Academic Press; ISBN 0-12-249695-7).
{21} Sambrook et al. (1989) Molecular Cloning: A Laboratory Manual. NY, Cold Spring Harbor Laboratory
{22} Yang et al. (1999) Proc Natl Acad Sci U S A 96(23):13404-8
{23} Short protocols in molecular biology (4th edition, 1999) Ausubel et al. eds. ISBN 0-471-32938-X.
{24} US patent 5,707,829
{25} Fille et al. (1997) Biotechniques 23:34-36.
{26} EP-B-0509612
{27} EP-B-0505012
{28} Current Protocols in Molecular Biology (F.M. Ausubel et al. eds., 1987) Supplement 30.
{29} International patent application WO00/73801
{30} International patent application WO01/51633
{31} International patent application WO01/73032
{32} US patent application 20020022248.
{33} International patent application WO01/57270.
{34} International patent application WO01/75067.
{35} International patent application WO01/57182.
{36} International patent application WO01/57277.
{37} International patent application WO01/57274.
{38} International patent application WO01/57275.
{39} International patent application WO01/57276.
{40} International patent application WO01/57278.
{41} International patent application WO01/57272.
{42} International patent application WO01/42467.
{43} European patent application EP-A-1074617.
{44} Mayer et al. (1999) Nat. Genet. 21 (3), 257-258
{45} Löwer et al. (1996) Proc. Natl. Acad. Sci USA 93:5177
{46} Berkhout et al. (1999) J. Virol. 73:2365-2375.
{47} Löwer et al. (1995) J. Virol. 69:141-149.
{48} Magin et al. (1999) J. Virol. 73:9496-9507.
{49} Magin et al. (2000) Virology 274:11-16.
{50} Boese et al. (2001) FEBS Lett 493(2-3):117-21.
{51} Mueller- Lantzsch et al. AIDS Research and Human Retroviruses 9:343-350 (1993)
{52} Hashido et al. (1992) Biochem. Biophys. Res. Comm. 187:1241-1248.
{53} Vogetseder et al. (1995) Exp Clin Immunogenet. 12:96-102.
{54} Sauter et al. (1995) J. Virol. 69:414-421.
{55} Geysen et al. (1984) PNAS USA 81:3998-4002.
{56} Carter (1994) Methods Mol Biol 36:207-23.
{57} Jameson, BA et al. 1988, CABIOS 4(1):181-186.
{58} Raddrizzani & Hammer (2000) Brief Bioinform 1(2):179-89.
{59} De Lalla et al. (1999) J. Immunol. 163:1725-29.
{60} Brusic et al. (1998) Bioinformatics 14(2):121-30
{61} Meister et al. (1995) Vaccine 13(6):581-91.
{62} Roberts et al. (1996) AIDS Res Hum Retroviruses 12(7):593-610.
{63} Maksyutov & Zagrebelnaya (1993) Comput Appl Biosci 9(3):291-7.
{64} Feller & de la Cruz (1991) Nature 349(6311):720-1.
{65} Hopp (1993) Peptide Research 6:183-190.
{66} Welling et al. (1985) FEBS Lett. 188:215-218.
{67} Davenport et al. (1995) Immunogenetics 42:392-297.
{68} Go et al. (1980) Int. J. Peptide Protein Res. 15:211
{69} Querol et al. (1996) Prot. Eng. 9:265
{70} Olsen & Thomsen (1991) J. Gen. Microbiol. 137:579
{71} Clarke et al. (1993) Biochemistry 32:4322
{72} Wakarchuk et al. (1994) Protein Eng. 7:1379
{73} Toma et al. (1991) Biochemistry 30:97
{74} Haezerbrouck et al. (1993) Protein Eng. 6:643
{75} Masul et al. (1994) Appl. Env. Microbiol. (1994) 60:3579
{76} US patent 4,959,314
{77} Smith & Waterman (1981) Adv. Appl. Math. 2: 482-489.
{78} Breedveld (2000) Lancet 355(9205):735-740.
{79} Gorman & Clark (1990) Semin. Immunol. 2:457-466
{80} Jones et al. (1986) Nature 321:522-525.
{81} Morrison et al. (1984) Proc. Natl. Acad. Sci, US.A., 81:6851-6855.
{82} Morrison & Oi (1988) Adv. Immunol., 44:65-92.
{83} Verhoeyer et al. (1988) Science 239:1534-1536.
{84} Padlan (1991) Molec. Immun. 28:489-498.
{85} Padlan (1994) Molec, Immunol. 31(3):169-217.
{86} Kettleborough et al. (1991) Protein Eng. 4(7):773-83
{87} Chothia et al.(1987) J. Mol. Biol. 196:901-917.
{88} Kabat *et al*. U.S. Dept. of Health and Human Services NIH Publication No. 91-3242 (1991)
{89} WO 98/24893
{90} WO 91/10741
{91} WO 96/30498
{92} WO 94/02602
{93} US Patent 5,939,598.
{94} WO 96/33735
{95} Gennaro (2000) Remington: The Science and Practice of Pharmacy. 20th edition, ISBN: 0683306472.
{96} WO 93/14778
{97} Findeis et al. (1993) Trends Biotechnol. 11:202
{98} Chiou et al. (1994) Gene Therapeutics: Methods And Applications Of Direct Gene Transfer. ed. Wolff
{99} Wu et al. (1988), J. Biol. Chem. 263:621
{100} Wu et al. (1994) J. Biol. Chem. 269:542
{101} Zenke et al. (1998) Proc. Natl. Acad. Sci. (USA) 87:3655
{102} Wu et al. (1991) J. Biol. Chem. 266:338.
{103} Jolly (1994) Cancer Gene Therapy 1:51.
{104} Kimura (1994) Human Gene Therapy 5:845
{105} Connelly (1995) Human Gene Therapy 1:185
{106} Kaplitt (1994) Nature Genetics 6:148
{107} WO 90/07936
{108} WO 94/03622
{109} WO 93/25698
{110} WO 93/25234
{111} US patent 5,219,740
{112} WO 93/11230
{113} WO 93/10218
{114} US patent 4,777,127
{115} GB Patent No. 2,200,651
{116} EP-A- 0 345 242
{117} WO 91/02805
{118} WO 94/12649
{119} WO 93/03769
{120} WO 93/19191
{121} WO 94/28938
{122} WO 95/11984
{123} WO 95/00655
{124} Curie (1992) Hum. Gene Ther. 3:147
{125} Wu, (1989) J. Biol. Chem. 264:16985
{126} US patent 5,814,482
{127} WO 95/07994
{128} WO 96/17072
{129} WO 95/30763
{130} WO 97/42338
{131} WO 90/11092
{132} US patent 5,580,859
{133} US patent 5,422,120
{134} WO 95/13796
{135} WO 94/23697
{136} WO 91/14445
{137} EP 0524968
{138} Philip (1994) Mol. Cell Biol. 14:2411
{139} Woffendin(1994) Proc. Natl. Acad. Sci. USA 91:11581
{140} US patent 5,206,152
{141} WO 92/11033
{142} US patent 5,149,655
{143} WO90/14837
{144} Vaccine Design - the subunit and adjuvant approach (1995) eds. Powell & Newman. ASIN: 030644867X
{145} WO00/07621
{146} GB-2220221
{147} EP-A-0689454
{148} EP-A-0835318
{149} EP-A-0735898
{150} EP-A-0761231
{151} WO99/52549
{152} WO01/21207
{153} WO01/21152
{154} WO00/62800
{155} WO00/23105
{156} WO99/11241
{157} WO98/57659
{158} WO93/13202.
{159} McSharry (1999) Antiviral Res 43(1):1-21.
{160} Weissman (1987) Mol Biol. Med. 4(3):133-143
{161} Patanjali et al. (1991) Proc. Natl. Acad. Sci. USA 88: 1943-1947
{162} Simone et al. (2000) Am J Patho/. 156(2):445-52.
{163} Claverie (1996) Meth. Enzymol. 266:212-227.
{164} Chapter 36 (page 267ff) of Automated DNA Sequencing and Analysis Techniques (eds. Adams et al.) ISBN: 0127170103.
{165} Claverie et al. (1993) Comput. Chem. 17:191
{166} Altschul et al. (1990), J. Mol. Biol. 215:403-410.
{167} Pearson & Lipman (1988) PNAS USA, 85:2444.
{168} Luo et al. (1999) Nature Med 5:117-122.
{169} Sedivy (1998) Proc Natl Acad Sci USA 95:9078-9081.
{170} Hahn et al. (2002) Mol Cell Biol. 22(7):2111-2123.
{171} Hahn et al. (1999) Nature 400(6743):464-468.
{172} De Marzo et al. (1998) J Urol. 160:2381-2392.
{173} Sakr & Partin (2001) Urology 57(4 Suppl 1):115-120.

### SEQUENCE LISTING

**SEQ ID 1** 
TGTGGGGAAAAGAAAGAGAGATCAGACTGTTACTGTGTCTATGTAGAAAGAAATAGACATAAGAGACTCCATTTTGTTCTGTACTAAGA
AAAATTCTTCTGCTTTGAGATGCTGTTAATCTGTAACCCTAGCCCCAACCCTGTGCTCACAGAAACAGGTGCTGTGTTGACTCAAGGTT
TAATGGATTCAGGGCTGTGCAGGATGTGCTTTGTTAAACAAATGCTTGAAGGCAGCAAGCTTGTTAAGAGTCATCACCACTCCCTAATC
TCAAGTAAGCAGGGACACAAACACTGCGGAAGGCCGCAGGGACCTCTGCCTAGGAAAGCCAGGTGTTGTCCAAGGTTTCTCCCCATGTG
ACAGTCTGAAATATGGCCTCTTGGGAAGGGAAAGACCTGACTGTCCCCTGGCCCGACACCCGTAAAGGGTCTGTGCTGAGGATTAGTAA
AAGAGGAAGGAAGGCCTCTTTGCAGTTGAGATAAGAGGAAGGCATCTGTCTCCTGCTCATCCCTGGGCAATGGAATGTCTTGGTGTAAA
GCCTGATTGTATATGCCATCTACTGAGATAGGAGAAAACTGCCTTAGGGCTGGAGGTGGGACATGCTGGCGGCAATACTGCTCTTTAAG
GCATTGAGATGTTTATGTATATGCACATCAAAAGCACAGCACTTTTTCTTTACCTTGTTTATGATGCAGAGACATTTGTTCACATGTT
TTCCTGCTGGCCCTCTCCCCACTATTACCCTATTGTCCTGCCACATCCCCCTCTCCGAGATGGTAGAGATAATGATCAATAAATACTGA
GGGAACTCAGAGACCGGTGCGGCGCGGGTCCTCCATATGCTGAGCGCCGGTCCCCTGGGCCCACTTTTCTTTCTCTATACTTTGTCTCT
GTTGTCTTTCTTTTCTCAAGTCTCTCGTTCCACCTGAGGAGAAATGCCCACAGCTGTGGAGGCGCAGGCCACTCCATCTGGTGCCCAAC
GTGGATGCTTTTCTCTAGGGTGAAGGGACTCTCGAGTGTGGTCATTGAGGACAAGTCAACGAGAGATTCCCGAGTACGTCTACAGTGAG
CCTTGTGGTAAGCTTGGGCGCTCGGAAGAAGCCAGGGTTAATGGGGCAAACTAAAAGTAAAGTCTCTCATTCCACCTGATGAGAAACAC
CCAGAGGTGTGGAGGGGCAGGCCACCCCTTCAGGGTAGGGTCCCCTCCATGCAGACCATAGAGCACAGGTGTGCCCCAAAGAGGAGCAG
AGAGAAGGAGGGAGAGGGCCCACGAGAGACTTGGAAATGAATGGCAGGATTTTAGGCGCTGGACTTGGGTTCGGGGCACCTGGCCTTTC
CTTGTGTATTTCTCCTACTGTCTGCCTAACTATTTAATACAATAAAAGAAAACCAGCCCCTGGTTCTTGTGGTGTTTCCACCCTCCCGG
GTCCCCGCTGGCTGCCTGGCTTCCTCCCGCAGCTCCTGCTGTGTGTGTATGTGTGTGTGTGCACATCTGTGGGGCGTATGTGTGTTC
GTCTTTGTAATTGAGGCTGCAGAGTGGAGAGAGCAGGGGTTTTCTCTGGGGACCCAGAGAGAAGGAGGCGTTTTCACCACAGCCGAACA
GGGCAGGACCCCAGCACCCGGGACCCAGCGGGACTTTGCCAAGGGGATGGACCTGGCTGGCCACGCGGCTGTTTGTGTAGGGAAAAGA
AAGAGAGATCACACTGTTACTGTGTCTATGTAGAAAAGGAAGACATAAACTCCATTTTGAGCTGTACTAAGAAAAATTATTTTGCCTTG
ACCTGCTGTTAACCTGTAACTGTAGCCCCAACCCTGTGCTCAAAGAAACATGTGCTGTATGGAATCAAGGTTTAAGGGATCAAGGGCTG
TACAGGATGTGCCTTGTTAACAATGTGTTTACAGGCAGTATGCTTGGTAAAAGTCATCGCCATTCTCCATTCTCCATTAATCAGGGGCA
CGATGCACTGCGGAAAGCCACAGGGACCTCTGCCCGAGAAAGCCTGGGTATTGTCCAAGGCTTCCCCCCACTGAGACAGCCTGAGATAC
GGCCTCGTGGGAAGGGAAAGACCTGACCGTCCCCCAGCCCGACACCCGTAAAGGGTCTGTGCTGAGGAGGATTAGTAAAAGGGGAAGGC
CTCTTGCAGTTGAGATAAGAGGAAGGCCTCCGTCTCCTGCATGTCCTTGGGAATGGAATGTCTTGGTGTAAAACCCGATAGTACATTCC
TTCTATTCTGAGAGAAGAAAACCACCCTGTGGCTGGAGGTGAGATATGCTAGCGGCAATGCTGCTCTGTTACTCTTTGCTACACTGAGA
TGTTTGGGTGGAGAGAAGCATAAATCTGGCCTATGTGCACATCTGGGCACAGAACCTCCCCTTGAACTTGTGACACAGATTCCTTTGTT
CACATGTTTTCCTGCTGACCTTCTCCCCACTATCGCCCTGTTCTCCCACCGCATTCCCCTTGCTGAGATAGTGAAAATAGTAATCTGTA
GATACCAAGGGAACTCAGAGACCATGGCCGGTGCACATCCTCCGTACGCTGAGCGCTGGTCCCCTGGGCCCATTGTTCTTTCTCTATAC
TTTGTCTCTGTGTCTTATTTCTTTCCTCAGTCTCTCATCCCTCCTGACGAGAAATACCCACAGGTGTGGAGGGGCTGGCCCCCTTCATC
TGATGCCCAATGTGGGTGCCTTTCTCTAGGGTGAAGGTACTCTACAGTGTGGTCATTGAGGACAAGTTGACGAGAGAGTCCCAAGTACG
TCCACGGTCAGCCTTGCGGTAAGCTTGTGTGCTTAGAGGAACCCAGGGTAACGATGGGGCAAACTGAAAGTAAATATGCCTCTTATCTC
AGCTTTATTAAAATTCTTTTAAGAAGAGGGGGAGTTAGAGCTTCTACAGAAAATCTAATTACGCTATTTCAAACAATAGAACAATTCTG
CCCATGGTTTCCAGAACAGGGAACTTTAGATCTAAAAGATTGGGAAAAAATTGGCAAAGAATTAAAACAAGCAAATAGGGAAGGTAAAA
TCATCCCACTTACAGTATGGAATGATTGGGCCATTATTAAAGCAACTTTAGAACCATTTCAAACAGGAGAAGATATTGTTTCAGTTTCT
GATGCCCCTAAAAGCTGTGTAACAGATTGTGAAGAAGAGGCAGGGACAGAATCCCAGCAAGGAACGGAAAGTTCACATTGTAAATATGT
AGCAGAGTCTGTAATGGCTCAGTCAACGCAAAATGTTGACTACAGTCAATTACAGGAGATAATATACCCTGAATCATCAAAATTGGGGG
AAGGAGGTCCAGAATCATTGGGGCCATCAGAGCCTAAACCACGATCGCCATCAACTCCTCCTCCCGTGGTTCAGATGCCTGTAACATTA
CAACCTCAAACGCAGGTTAGACAAGCACAAACCCCAAGAGAAAATCAAGTAGAAAGGGACAGAGTCTCTATCCCGGCAATGCCAACTCA
GATACAGTATCCACAATATCAGCCGGTAGAAAATAAGACCCAACCGCTGGTAGTTTATCAATACCGGCTGCCAACCGAGCTTCAGTATC
GGCCTCCTTCAGAGGTTCAATACAGACCTCAAGCGGTGTGTCCTGTGCCAAATAGCACGGCACCATACCAGCAACCCACAGCGATGGCG
TCTAATTCACCAGCAACACAGGACGCGGCGCTGTATCCTCAGCCGCCCACTGTGAGACTTAATCCTACAGCATCACGTAGTGGACAGGG
TGGTGCACTGCATGCAGTCATTGATGAAGCCAGAAAACAGGGCGATCTTGAGGCATGGCGGTTCCTGGTAATTTTACAACTGGTACAGG
CCGGGGAAGAGACTCAAGTAGGAGCGCCTGCCCGAGCTGAGACTAGATGTGAACCTTTCACCATGAAAATGTTAAAAGATATAAAGGAA
GGAGTTAAACAATATGGATCCAACTCCCCTTATATAAGAACATTATTAGATTCCATTGCTCATGGAAATAGACTTACTCCTTATGACTG
GGAAATTTTGGCCAAATCTTCCCTTTCATCCTCTCAGTATCTACAGTTTAAAACCTGGTGGATTGATGGAGTACAAGAACAGGTACGAA
AAAATCAGGCTACTAAGCCCACTGTTAATATAGACGCAGACCAATTGTTAGGAACAGGTCCAAATTGGAGCACCATTAACCAACAATCA
GTGATGCAGAATGAGGCTATTGAACAAGTAAGGGCTATTTGCCTCAGGGCCTGGGGAAAAATTCAGGACCCAGGAACAGCTTTCCCTAT
TAATTCAATTAGACAAGGCTCTAAAGAGCCATATCCTGACTTTGTGGCAAGATTACAAGATGCTGCTCAAAAGTCTATTACAGATGACA
ATGCCCGAAAAGTTATTGTAGAATTAATGGCCTATGAAAATGCAAATCCAGAATGTCAGTCGGCCATAAAGCCATTAAAAGGAAAAGTT
 
CCAGCAGGAGTTGATGTAATTACAGAATATGTGAAGGCTTGTGATGGGATTGGAGGAGCTATGCATAAGGCAATGCTAATGGCTCAAGC
AATGAGGGGGCTCACTCTAGGAGGACAAGTTAGAACATTTGGGAAAAAATGTTATAATTGTGGTCAAATCGGTCATCTGAAAAGGAGTT
GCCCAGGCTTAAATAAACAGAATATAATAAATCAAGCTATTACAGCAAAAAATAAAAAGCCATCTGGCCTGTGTCCAAAATGTGGAAAA
GCAAAACATTGGGCCAATCAATGTCATTCTAAATTTGATAAAGATGGGCAACCATTGTCTGGAAACAGGAAGAGGGGCCAGCCTCAGGC
CCCCCAACAAACTGGGGCATTCCCAGTTAAACTGTTTGTTCCTCAGGGTTTTCAAGGACAACAACCCCTACAGAAAATACCACCACTTC
AGGGAGTCAGCCAATTACAACAATCCAACAGCTGTCCCGCGCCACAGCAGGCAGCACCGCAGTAGATTTATGTTCCACCCAAATGGTCT
TTTTACTCCCTGGAAAGCCCCCACAAAAGATTCCTAGAGGGGTATATGGCCCGCTGCCAGAAGGGAGGGTAGGCCTTTGAGGGAGATCA
AGTCTAAATTTGAAGGGAGTCCAAATTCATACTGGGGTAATTTATTCAGATTATAAAGGGGGAATTCAGTTAGTGATCAGCTCCACTGT
TCCCCGGAGTGCCAATCCAGGTGATAGAATTGCTCAATTACTGCTTTTGCCTTATGTTAAAATTGGGGAAAACAAAAAGGAAAGAACAG
GAGGGTTTGGAAGTACCAACCCTGCAGGAAAAGCTGCTTATTGGGCTAATCAGGTCTCAGAGGATAGACCCGTGTGTACAGTCACTATT
CAGGGAAAGAGTTTGAAGGATTAGTGGATACCCAGGCTGATGTTTCTGTCATCGGCATAGGTACTGCCTCAGAAGTGTATCAAAGTGCC
ATGATTTTACATTGTCCAGGATCTGATAATCAAGAAAGTACGGTTCAGCCTGTGATCACTTCATTCCAATCAATTTATGGGGCCGAGAC
TTGTTACAACAATGGCATGCAGAGATTACTATCCCAGCCTCCCTATACAGCCCCAGGAATAAAAAAATCATGACTAAAATGGGATAGCT
CCCTAAAAAGGGACTAGGAAAGAAGTCCCAATTGAGGCTGAAAAAAATCAAAAAAGAAAAGGAATAGGGCATCCTTTTTAGGAGCGGTC
ACTGTAGAGCCTCCAAAACCCATTCCATTAACTTGGGGGAAAAAAAAACAACTGTATGGTAAATCAGCAGCGCTTCCAAAACAAAAACT
GGAGGCTTTACATTTATTAGCAAAGAAACAATTAGAAAAAGGACATTGAGCCTTCATTTTCGCCTTGGAATTCTGTTTGTAATTCAGAA
AAAATCCGGCAGATGGCGTATAATGCCGTAATTCAACCCATGGGGGCTCTCCCACCCCGGTTGCCCTCTCCAGCCATGGTCCCCTTTAA
TTATAATTGATCTGAAGGATTGCTTTTTTACCATTCCTCTGGCAAAACAGGATTTTGAAAAATTTGCTTTTACCACACCAGCCTAAATA
ATAAAGAACCAGCCACCAGGTTTCAGTGGAAAGTATTGCCTCAGGGAATGCTTAATAGTTCAACTATTTGTCAGCTCAAGCTCTGCAAC
CAGTTAGAGACAAGTTTTCAGACTGTTACATCGTTCACTATGTTGATATTTTGTGTGCTGCAGAAACGAGAGACAAATTAATTGACCGT
TACACATTTCTGCAGACAGAGGTTGCCAACGCGGGACTGACAATAACATCTGATAAGATTCAAACCTCTACTCCTTTCCGTTACTTGGG
AATGCAGGTAGAGGAAAGGAAAATTAAACCACAAAAAATAGAAATAAGAAAAGACACATTAAAAGCATTAAATGAGTTTCAAAAGTTGC
TAGGAGATACTAATTGGATTTGGAGATATTAATTGGATTTGGCCAACTCTAGGCATTCCTACTTATGCCATGTCAAATTTGTTCTCTTT
CTTAAGAGGGGACTCGGAATTAAATAGTGAAAGAACGTTAACTCCAGAGGCAACTAAAGAAATTAAATTAATTGAAGAAAAAAATTCGGT
CAGCACAAGTAAATAGAATAGATCACTTGGCCCCACTCCAAATTTTGATTTTTGCTACTGCACATTCCCTAACAGGCATCATTGTTCAA
AATACAGATCTTGTGGAGTGGTCCTTCCTTCCTCACAGTACAATTAAGACTTTTACATTGTACTTGGATCAAATGGCTACATTAATTGG
TCAGGGAAGATTATGAATAATAACATTGTGTGGAAATGACCCAGATAAAATCACTGTTCCTTTCAACAAGCAACAGGTTAGACAAGCCT
TTATCAATTCTGGTGCATGGCAGATTGGTCTTGCCGATTTTGTGGGAATTATTGACAATCGTTACCCCAAAACAAAAATCTTCCAGTTT
TTAAAATTGACTACTTGGATTTACCTAAAGTTACCAAACATAAGCCTTTAAAAAATGCTCTGGCAGTGTTTACTGATGGTTCCAGCAA
TGGAAAAGTGGCTTACACCGGGCCAAAAGAATGAGTCATCAAAACTCAGTATCACTTGACTCAAAGAGCAGAGTTGGTTGCCGTCATTA
CAGTGTTAACAAGATTTTAATCAGTCTATTAACATTGTATCAGATTCTGCATATGTAGTACAGGCTACAAAGGATATTGAGAGAGCCCT
AATCAAATACATTATGGATGATCAGTTAAACCCGCTGTTTAATTTGTTACAACAAAATGTAAGAAAAAGAAATTTCCCATTTTATATTA
CTCATATTCGAGCACACACTAATTTACCAGGGCCTTTAACTAAAGCAAATGAACAAGCTGACTTGCTAGTATCATCTGCATTCATGGAA
GCACAAGAACTTCATGCCTTGACTCATGTAAATGCAATAGGATTAAAAAATAAATTTGATATCACATGGAAACAGACAAAAAATATTGT
ACAACATTGCACCCAGTGTCAGATTCTACACCTGGCCACTCAGGAGGCAAGAGTTAATCCCAGAGGTCTATGTCCTAATGTGTTATGGC
AAATGGATGTCATGCACGTACCTTCATTTGGAAAATTGTCATTTGTCCATGTGACAGTTGATACTTATTCACATTTCATATGGGCAACC
TGCCAGACAGGAGAAAGTACTTCCCATGTTAAAAGACATTTATTATCTTGTTTTCCTGTCATGGGAGTTCCAGAAAAAGTTAAAACAGA
CAATGGGCCAGGTTACTGTAGTAAAGCAGTTCAAAAATTCTTAAATCAGTGGAAAATTACACATACAATAGGAATTCTCTATAATTCCC
AAGGACAGGCCATAATTGAAAGAACTAATAGAACACTCAAAGCTCAATTGGTTAAACAAAAAAAAGGAAAAGACAGGAGTATAACACTC
CCCAGATGCAACTTAATCTAGCACTCTATACTTTAAATGTTTTAAACATTTATAGAAATCAGACCACTACCTCTGCAGAACAACATCTT
ACTGGTAAAAGGAACAGCCCACATGAAGGAAAACTGATTTGGTGGAAAGATAATAAAAATAAAACATGGGAAATGGGGAAGGTGATAAC
GTGGGGGAGAGGTTTTGCTTGTGTTTCACCAGGAGAAAATCAGCTTCCTGTTTGGATACCCACTAGACATTTAAAGTTCTACAATGAAC
TCACTGGAGATGCAAAGAAAAGTGTGGAGATGGAGACACCCCAATCGACTCGCCAGGTAAACAAAATGGTGATATCAGAAGAACAGAAA
AAGTTGCCTTCCATCAAGGAAGCAGAGTTGCCAATATAGGCACAATTAAAGAAGCTGACACAGTTAGCTAAAAAAAAAAGCCTAGAGAA
TACAAAGGTGACACCAACTCCAGAGAATATGCTGCTTGCAGCTCTGATGATTGTATCAACGGTGGTAAGTCTTCCCAAGTCTGCAGGAG
CAGCTGCAGCTAATTATACTTACTGGGCCTATGTGCCTTTCCCACCCTTAATTCGGGCAGTTACATAGATGGATAATCCTATTGAAGTA
GATGTTAATAATAGTGCATGGGTGCCTGGCCCCACAGATGACTGTTGCCCTGCCCAACCTGAAGAAGGAATGATGATGAATATTTCCAT
TGGGTATCCTTATCCTCCTGTTTGCCTAGGGAAGGCACCAGGATGCTTAATGCCTACAACCCAAAATTGGTTGGTAGAAGTACCTACAG
TCAGTGCTACCAGTAGATTTACTTATCACATGGTAAGTGGAATGTCACAGATAAATAATTTACAGGACCCTTCTTATCAAAGATCATTA
CAATGTAGGCCTAAGGGGAAGGCTTGCCCCAAGGAAATTCCCAAAGAATCAAAAAGCCCAGAAGTCTTAGTCTGCGGAGAATGTGTGGC
TGATACTGCAGTGTAGTACAAAACAATGAATTTTGAACTATGATAGACTGGGTCCCTTGAGGCCAATTATATCATAACTGTACAGGCCA
GACTCATTCATGTTCACAGGCCCCATCCATCTGGCCCATTAATCCAGCCTATGACGGTGATGTAACTGAAAGGCTGGACCAGGTTTATA
GAAGGTTAGAATCACTCTGTCCAAGGAAATGGGGTGAAAAGGGAATTTCATCACCTTGACCAAAGTTAGTCCTGTTACTGGTCCTGAAC
 
ATCCAGAATTAGGAAGCTTACTGTGGCCTCACACCACATTAGAATTTGTTCTGGAAATCAAGCTATAGGAACAAGAGATCGTAAGTCAT
ATTATACTATCAACCTAAATTCCAGTCTGACAATTCCTTTGCAAAATTGTGTAAAACTCCCTTATATTGCTAGTTGTAGGAAAAACATA
GTTATTAAACCTGATTCCCAAACCATAATCTGTGAAAATTGTGGAATGTTTACTTGCATTGATTTGACTTTAATTGGCAGCACCGTAT
TCTACTAGGAAGAGCAAGAGAGGGTGTGTGGATCCTTGTGTCCATGGACCGACCATGGGAGGCTTCGCTATCCATCCATATTTTAACGG
AAGTATTAAAAGGAATTCTAACTAGATCCAAAAGATTCATTTTTACTTTGATGGCAGTGATTATGGGCCTCATTGCAGTCACAGCTACT
GCTGCGGCTGCTGGAATTGCTTTACACTCCTCTGTTCAAACTGCAGAATACGTAAATGATTGGCAAAAGAATTCCTCAAAATTGTGGAA
TTCTCAGATCCAAATAGATCAAAAATTGGCAAACCAAATTAATGATCTTAGACAAACTGTCATTTGGATGGGAGAGGCTCATGAGCTTG
GAATATCTTTTTCAGTTACGATGTGACTGGAATACATCAGATTTTTGTGTTACACCACAAGCCTATAATGAGTCTGAGCATCACTGGGA
CATGGTTAGATGCCATCTGCAAGGAGGAGAAGATAATCTTACTTTAGACATTTCAAAATTAAAAGAATTTTTTTTTCTTTGAGACAGA
GTCTCGCTCTGTCGCCCAGGCTGGAGTGCAGTGGCGTGATCTCAGCTCACTGCAAGTTCCGCCTCCTGGGTTTACACCATTCTCCTGCC
TCAGCCTCCCAAGTAGTTGGGACTACAGGAGCCCACCACCATGCCTGGCTAATTTTTTTGGGTTTTAATAGAGATGGAGTTTCACCG
TGTTAGCCAGGATGGTCTCGATCTCCTGACCTTGTGATCTGCCCACCTTGGCCTCCCAAAGTGCTGGGATTACAGTCGTGAGCCACCGT
GCCCAGCCAAGAAAAAATTTTGAGGCATCAAAGCCCATTTAAATTGGTGCCAGGAACGGAGACAATCGTGAAAGCTGCTGATAGCC
TCACAAATCTTAAGCCAGTCACTTGGGTTAAAAGCATCAGAAGTTTCACTATTGTAAATTTCATATTAATCCTTGTATGCCTGTTCTGT
CTGTTGTTAGTCTACAGGTGTATCCAGCAGCTCCAAAGAGACAGCAACCAGCAAGAATGGGCCATAGTGACGATGGTGGTTTTGTCAAA
AAGAAAAGGGGGGGATATGTAAGGAAAAGAGAGATCAGACTTTCACTGTGTCTATGTAGAAAAGGAAGACATAAGAAACTCCATTTTGA
TCTGTACTAAGAAAAATTGTTTTGCCTTGAGATGCTGTTAATCTGTAACTTTAGCCCCAACCCTGTGCTCACGGAAACATGTGCTGTAA
GGTTTAAGGGATCTAGGGCTGTGCAGGATGTACCTTGTTAACAATATGTTTGCAGGCAGTATGTTTGGTAAAAGTCATCGCCATTCTCC
ATTCTCGATTAACCAGGGGCTCAATGCACTGTGGAAAGCCACAGGAACCTCTGCCCAAGAAAGCCTGGCTGTTGTGGGAAGTCAGGGAC
CCCGAATGGAGGGACCAGCTGGTGCTGCATCAGGAAACATAAATTGTGAAGATTTCTTGGACATTTATCAGTTTCCAAAATTAATACTT
TTATAATTTCTTACACCTGTCTTACTTTAATCTCTTAATCCTGTTATCTTTGTAAGCTGAGGATATACGTCACCTCAGGACCACTATTG
TACAAATTGATTGTAAAACATGTTCACATGTGTTTGAACAATATGAAATCAGTGCACCTTGAAAATGAACAGAATAACAGTGATTTTAG
GGAACAAAGGAAGACAACCATAAGGTCTGACTGCCTGAGGGGTCGGGCAAAAAGCCATATTTTTCTTCTTGCAGAGAGCCTATAAATGG
ACGTGCAAGTAGGAGAGATATTGCTAAATTCTTTTCCTAGCAAGGAATATAATACTAAGACCCTAGGGAAAGAATTGCATTCCTGGGGG
GAGGTCTATAAACGGCCGCTCTGGGAGTGTCTGTCCTATGTGGTTGAGATAAGGACTGAGATACGCCCTGGTCTCCTGCAGTACCCTCA
GGCTTACTAGGATTGGAAACCCCAGTCCTGGTAAATTTGAGGTCAGGCCGGTTCTTTGCTCTGAACCCTGTTTTCTGTTAAGATGTTT
ATCAAGACAATACATGCACCGCTGAACATAGACCCTTATCAGGAGTTTCTGATTTTGCTCTGGTCCTGTTTCTTCAGAAGCATGTCATC
TTTGCTCTGCCTTCTGCCCTTTGAAGCATGTGATCTTTGTGACCTACTCCCTGTTCATACACCCCTCCCCTTTTAAAATCCCTAATAAA
AACTTGCTGGTTTTGTGGCTCAGGGGGGCATCATGGACCTACCAATACGTGATGTCACCCCCGGTGGCCCAGCTGTAAAATTCCTTTCT
TTATACTCTTATTTCTCAGACCAGCTGACACTTAGGGAAAATAGAAAGAACCTATGTTGAAATATTGGAGGCGGGTTCCCCCGATACCT
GGGTATTGTCCAAGGTTTCCTTTGCTGAGGAGGATTAGTAAAAGGAATGCCTCCATCTCCTGCATGTCCCTGGAACAGAATGTTCCCA
CCAACCACCCTGTGGCTGGAGGCGGGATATGCTGGCAGCAATGCTGCTCTATTACTCTTTGCTACACTGAGATGTTTGGGTGGAGAGAA
GCATAAATCTGGCCTATGTGCACATCTGGGCACAGCACCTTCCTTTGAACTTATTTGTGACACAGATTCCTTTGCTCACGTTTTCCTGT
TGACTTTCTCACCACTCACCCTATTCTCCTGTGGCATTCGCCTTGCGGAGATAGTGAAAATAGTAATAAATACTGAGGGAACTCAGACT
GAGGGAACTCAGACTGGGCAGACCGGGGCCAGTGTGGGTCCTCCATATGCTGAGCGCCGGTTCCCTGGGCCCACTGTTCTTTCTCTATA
CTTTGTCTCTGTGCCTTATTTCTCAGTCTCTCATTCCACCTGATGAGAAATACCCACAGGTGTGGAGGGGCTGGCCCCCTTCA
**SEQ ID 2** 
GAGATAGGAGAAAACTGCCTTAGGGCTGGAGGTGGACATGCTGGCGGCAATACTGCTCTTTAAGGCATTGAGATGTTTATGTATATGC
ACATCAAAAGCACAGCACTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTG
**SEQ ID 3**
   TCTCTCATCC
**SEQ ID 4**
   GGTGAAGGTA
**SEQ ID 5** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTCTTTACCTTGTTTATGATGCAGAGACATTTGTTCACATGTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
 
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCC
**SEQ ID 6** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTCTTTACCTTGTTATGATGCAGAGACATTTGTTCACATGTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTA
**SEQ ID 7** 
TGTAAGGAAAAGAGAGATCAGACTTTCACTGTGTCTATGTAGAAAAGGAAGACATAAGAAACTCCATTTTGATCTGTACTAAGAAAAAT
TGTTTTGCCTTGAGATGCTGTTAATCTGTAACTTTAGCCCCAACCCTGTGCTCACGGAAACATGTGCTGTAAGGTTTAAGGGATCTAGG
GCTGTGCAGGATGTACCTTGTTAACAATATGTTTGCAGGCAGTATGTTTGGTAAAAGTCATCGCCATTCTCCATTCTCGATTAACCAGG
GGCTCAATGCACTGTGGAAAGCCACAGGAACCTCTGCCCAAGAAAGCCTGGC
**SEQ ID 8** 
TGTTGTGGGAAGTCAGGGACCCCGAATGGAGGGACCAGCTGGTGCTGCATCAGGAAACATAAATTGTGAAGATTTCTTGGACATTTATC
AGTTTCCAAAATTAATACTTTTATAATTTCTTACACCTGTCTTACTTTAATCTCTTAATCCTGTTATCTTTGTAAGCTGAGGATATACG
TCACCTCAGGACCACTATTGTACAAATTGATTGTAAAACATGTTCACATGTGTTTGAACAATATGAAATCAGTGCACCTTGAAAATGAA
CAGAATAACAGTGATTTAGGGAACAAAGGAAGACAACCATAAGGTCTGACTGCCTGAGGGGTCGGCAAAAAGCCATATTTTTCTTCT
TGCAGAGAGCCTATAAATGGACGTGCAAGTAGGAGAGATATTGCTAAATTCTTTTCCTAGCAAGGAATATAATACTAAGACCCTAGGGA
AAGAATTGCATTCCTGGGGGGAGGTCTATAAACGGCCGCTCTGGGAGTGTCTGTCCTATGTGGTTGAGATAAGGACTGAGATACGCCCT
GGTCTCCTGCAGTACCCTCAGGCTTACTAGGATTGGGAAACCCCAGTCCTGGTAAATTTGAGGTCAGGCCGGTTCTTTGCTCTGAACCC
TGTTTTCTGTTAAGATGTTTATCAAGACAATACATGCACCGCTGAACATAGACCCTTATCAGGAGTTTCTGATTTTGCTCTGGTCCTGT
TTCTTCAGAAGCATGTCATCTTTGCTCTGCCTTCTGCCCTTTGAAGCATGTGATCTTTGTGACCTACTCCCTGTTCATACACCCCTCCC
CTTTTAAAATCCCTAATAAAAACTTGCTGGTTTTGTGGCTCAGGGGGCATCATGGACCTACCAATACGTGATGTCACCCCCGGTGGCC
CAGCTGT
**SEQ ID 9** 
TGTAAGGAAAAGAGAGATCAGACTTTCACTGTGTCTATGTAGAAAAGGAAGACATAAGAAACTCCATTTTGATCTGTACTAAGAAAAAT
TGTTTTGCCTTGAGATGCTGTTAATCTGTAACTTTAGCCCCAACCCTGTGCTCACGGAAACATGTGCTGTAAGGTTTAAGGGATCTAGG
GCTGTGCAGGATGTACCTTGTTAACAATATGTTTGCAGGCAGTATGTTTGGTAAAAGTCATCGCCATTCTCCATTCTCGATTAACCAGG
GGCTCAATGCACTGTGGAAAGCCACAGGAACCTCTGCCCAAGAAAGCCTGGCTGTTGTGGGAAGTCAGGGACCCCGAATGGAGGGACCA
GCTGGTGCTGCATCAGGAAACATAAATTGTGAAGATTTCTTGGACATTTATCAGTTTCCAAAATTAATACTTTTATAATTTCTTACACC
TGTCTTACTTTAATCTCTTAATCCTGTTATCTTTGTAAGCTGAGGATATACGTCACCTCAGGACCACTATTGTACAAATTGATTGTAAA
ACATGTTCACATGTGTTTGAACAATATGAAATCAGTGCACCTTGAAAATGAACAGAATAACAGTGATTTAGGGAACAAAGGAAGACAA
CCATAAGGTCTGACTGCCTGAGGGGTCGGCAAAAAGCCATATTTTTCTTCTTGCAGAGAGCCTATAAATGGACGTGCAAGTAGGAGAG
ATATTGCTAAATTCTTTTCCTAGCAAGGAATATAATACTAAGACCCTAGGGAAAGAATTGCATTCCTGGGGGGAGGTCTATAAACGGCC
GCTCTGGGAGTGTCTGTCCTATGTGGTTGAGATAAGGACTGAGATACGCCCTGGTCTCCTGCAGTACCCTCAGGCTTACTAGGATTGGG
AAACCCCAGTCCTGGTAAATTTGAGGTCAGGCCGGTTCTTTGCTCTGAACCCTGTTTTCTGTTAAGATGTTTATCAAGACAATACATGC
ACCGCTGAACATAGACCCTTATCAGGAGTTTCTGATTTTGCTCTGGTCCTGTTTCTTCAGAAGCATGTCATCTTTGCTCTGCCTTCTGC
CCTTTGAAGCATGTGATCTTTGTGACCTACTCCCTGTTCATACACCCCTCCCCTTTTAAAATCCCTAATAAAAACTTGCTGGTTTTGTG
GCTCAGGGGGGCATCATGGACCTACCAATACGTGATGTCACCCCCGGTGGCCCAGCTGT
**SEQ ID 10** 
GAGATAGGAGAAAACTGCCTTAGGGCTGGAGGTGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGTAAGCTTGGGCGCTCG
GAAGAAGCCAGGGTTAATGGGGCAAACTAAAAGTAAAGTCTCTCATTCCACCTGATGAGAAACACCCAGAGGTGTGGAGGGGCAGGCCA
CCCCTTCAGGGTAGGGTCCCCTCCATGCAGACCATAGAGCACAGGTGTGCCCCAAAGAGGAGCAGAGAGAAGGAGGGAGAGGGCCCACG
AGAGACTTGGAAATGAATGGCAGGATTTTAGGCGCTGGACTTGGGTTCGGGGCACCTGGCCTTTCCTTGTGTATTTCTCCTACTGTCTG
CCTAACTATTTAATACAATAAAAGAAAACCAGCCCCTGGTTC[?][?][?]TGGTGTTTCCACCCTCCCGGGTCCCCGCTGGCTGCCTGGCTTCC
 
TCCCGCAGCTCCTGCTGTGTGTGTATGTGTGTGTGTGTGCACATCTGTGGGGCGTATGTGTGTTCGTCTTTGTAATTGAGGCTGCAGAG
TGGAGAGAGCAGGGGTTTCTCTGGGACCCAGAGAGAAGGAGGCGTTTCACCACAGCCGAACAGGGCAGGACCCCAGCACCCGGGAC
CCAGCGGGACTTTGCCAAGGGGATGGACCTGGCTGGGCCACGCGGCTGTTTGTGTAGGGAAAAGAAAGAGAGATCACACTGTTACTGTG
TCTATGTAGAAAAGGAAGACATAAACTCCATTTTGAGCTGTACTAAGAAAAATTATTTTGCCTTGACCTGCTGTTAACCTGTAACTGTA
GCCCCAACCCTGTGCTCAAAGAAACATGTGCTGTATGGAATCAAGGTTTAAGGGATCAAGGGCTGTACAGGATGTGCCTTGTTAACAAT
GTGTTTACAGGCAGTATGCTTGGTAAAAGTCATCGCCATTCTCCATTCTCCATTAATCAGGGGCACGATGCACTGCGGAAAGCCACAGG
GACCTCTGCCCGAGAAAGCCTGGGTATTGTCCAAGGCTTCCCCCCACTGAGACAGCCTGAGATACGGCCTCGTGGGAAGGGAAAGACCT
GACCGTCCCCCAGCCCGACACCCGTAAAGGGTCTGTGCTGAGGAGGATTAGTAAAAGGGGAAGGCCTCTTGCAGTTGAGATAAGAGGAA
GGCCTCCGTCTCCTGCATGTCCTTGGGAATGGAATGTCTTGGTGTAAAACCCGATAGTACATTCCTTCTATTCTGAGAGAAGAAAACCA
CCCTGTGGCTGGAGGTGAGATATGCTAGCGGCAATGCTGCTCTGTTACTCTTTGCTACACTGAGATGTTTGGGTGGAGAGAAGCATAAA
TCTGGCCTATGTGCACATCTGGGCACAGAACCTCCCCTTGAACTTGTGACACAGATTCCTTTGTTCACATGTTTCCTGCTGACCTTCT
CCCCACTATCGCCCTGTTCTCCCACCGCATTCCCCTTGCTGAGATAGTGAAAATAGTAATCTGTAGATACCAAGGGAACTCAGAGACCA
TGGCCGGTGCACATCCTCCGTACGCTGAGCGCTGGTCCCCTGGGCCCATTGTTCTTTCTCTATACTTTGTCTCTGTGTCTTATTTCTTT
CCTCAGTCTCTCATCCCTCCTGACGAGAAATACCCACAGGTGTGGAGGGGCTGGCCCCCTTCATCTGATGCCCAATGTGGGTGCCTTTC
TCTAGGGTGAAGGTACTCTACAGTGTGGTCATTGAGGACAAGTTGACGAGAGAGTCCCAAGTACGTCCACGGTCAGCCTTGCGGTAAGC
TTGTGTGCTTAGAGGAACCCAGGGTAACGATGGGGCAAACTGAAAGTAAATATGCCTCTTATCTCAGCTTTATTAAAATTCTTTTAAGA
AGAGGGGGAGTTAGAGCTTCTACAGAAAATCTAATTACGCTATTTCAAACAATAGAACAATTCTGCCCATGGTTTCCAGAACAGGGAAC
TTTAGATCTAAAAGATTGGAAAAAAATTGGCAAAGAATTAAAACAAGCAAATAGGGAAGGTAAAATCATCCCACTTACAGTATGGAATG
ATTGGGCCATTATTAAAGCAACTTTAGAACCATTTCAAACAGGAGAAGATATTGTTTCAGTTTCTGATGCCCCTAAAAGCTGTGTAACA
GATTGTGAAGAAGAGGCAGGGACAGAATCCCAGCAAGGAACGGAAAGTTCACATTGTAAATATGTAGCAGAGTCTGTAATGGCTCAGTC
AACGCAAAATGTTGACTACAGTCAATTACAGGAGATAATATACCCTGAATCATCAAAATTGGGGGAAGGAGGTCCAGAATCATTGGGGC
CATCAGAGCCTAAACCACGATCGCCATCAACTCCTCCTCCCGTGGTTCAGATGCCTGTAACATTACAACCTCAAACGCAGGTTAGACAA
GCACAAACCCCAAGAGAAAATCAAGTAGAAAGGGACAGAGTCTCTATCCCGGCAATGCCAACTCAGATACAGTATCCACAATATCAGCC
GGTAGAAAATAAGACCCAACCGCTGGTAGTTTATCAATACCGGCTGCCAACCGAGCTTCAGTATCGGCCTCCTTCAGAGGTTCAATACA
GACCTCAAGCGGTGTGTCCTGTGCCAAATAGCACGGCACCATACCAGCAACCCACAGCGATGGCGTCTAATTCACCAGCAACACAGGAC
GCGGCGCTGTATCCTCAGCCGCCCACTGTGAGACTTAATCCTACAGCATCACGTAGTGGACAGGGTGGTGCACTGCATGCAGTCATTGA
TGAAGCCAGAAAACAGGGCGATCTTGAGGCATGGCGGTTCCTGGTAATTTTACAACTGGTACAGGCCGGGGAAGAGACTCAAGTAGGAG
CGCCTGCCCGAGCTGAGACTAGATGTGAACCTTTCACCATGAAAATGTTAAAAGATATAAAGGAAGGAGTTAAACAATATGGATCCAAC
TCCCCTTATATAAGAACATTATTAGATTCCATTGCTCATGGAAATAGACTTACTCCTTATGACTGGGAAATTTTGGCCAAATCTTCCCT
TTCATCCTCTCAGTATCTACAGTTTAAAACCTGGTGGATTGATGGAGTACAAGAACAGGTACGAAAAAATCAGGCTACTAAGCCCACTG
TTAATATAGACGCAGACCAATTGTTAGGAACAGGTCCAAATTGGAGCACCATTAACCAACAATCAGTGATGCAGAATGAGGCTATTGAA
CAAGTAAGGGCTATTTGCCTCAGGGCCTGGGGAAAAATTCAGGACCCAGGAACAGCTTTCCCTATTAATTCAATTAGACAAGGCTCTAA
AGAGCCATATCCTGACTTTGTGGCAAGATTACAAGATGCTGCTCAAAAGTCTATTACAGATGACAATGCCCGAAAAGTTATTGTAGAAT
TAATGGCCTATGAAAATGCAAATCCAGAATGTCAGTCGGCCATAAAGCCATTAAAAGGAAAAGTTCCAGCAGGAGTTGATGTAATTACA
GAATATGTGAAGGCTTGTGATGGGATTGGAGGAGCTATGCATAAGGCAATGCTAATGGCTCAAGCAATGAGGGGGCTCACTCTAGGAGG
ACAAGTTAGAACATTTGGGAAAAAATGTTATAATTGTGGTCAAATCGGTCATCTGAAAAGGAGTTGCCCAGGCTTAAATAAACAGAATA
TAATAAATCAAGCTATTACAGCAAAAAATAAAAAGCCATCTGGCCTGTGTCCAAAATGTGGAAAAGCAAAACATTGGGCCAATCAATGT
CATTCTAAATTTGATAAAGATGGGCAACCATTGTCTGGAAACAGGAAGAGGGGCCAGCCTCAGGCCCCCCAACAAACTGGGGCATTCCC
AGTTAAACTGTTTGTTCCTCAGGGTTTTCAAGGACAACAACCCCTACAGAAAATACCACCACTTCAGGGAGTCAGCCAATTACAACAAT
CCAACAGCTGTCCCGCGCCACAGCAGGCAGCACCGCAGTAGATTTATGTTCCACCCAAATGGTCTTTTTACTCCCTGGAAAGCCCCCAC
AAAAGATTCCTAGAGGGGTATATGGCCCGCTGCCAGAAGGGAGGGTAGGCCTTTGAGGGAGATCAAGTCTAAATTTGAAGGGAGTCCAA
ATTCATACTGGGGTAATTTATTCAGATTATAAAGGGGAATTCAGTTAGTGATCAGCTCCACTGTTCCCCGGAGTGCCAATCCAGGTGA
TAGAATTGCTCAATTACTGCTTTTGCCTTATGTTAAAATTGGGGAAAACAAAAAGGAAAGAACAGGAGGGTTTGGAAGTACCAACCCTG
CAGGAAAAGCTGCTTATTGGGCTAATCAGGTCTCAGAGGATAGACCCGTGTGTACAGTCACTATTCAGGGAAAGAGTTTGAAGGATTAG
TGGATACCCAGGCTGATGTTTCTGTCATCGGCATAGGTACTGCCTCAGAAGTGTATCAAAGTGCCATGATTTTACATTGTCCAGGATCT
GATAATCAAGAAAGTACGGTTCAGCCTGTGATCACTTCATTCCAATCAATTTATGGGGCCGAGACTTGTTACAACAATGGCATGCAGAG
ATTACTATCCCAGCCTCCCTATACAGCCCCAGGAATAAAAAAATCATGACTAAAATGGGATAGCTCCCTAAAAAGGGACTAGGAAAGAA
GTCCCAATTGAGGCTGAAAAAAATCAAAAAAGAAAAGGAATAGGGCATCCTTTTTAGGAGCGGTCACTGTAGAGCCTCCAAAACCCATT
CCATTAACTTGGGGGAAAAAAAAACAACTGTATGGTAAATCAGCAGCGCTTCCAAAACAAAAACTGGAGGCTTTACATTTATTAGCAAA
GAAACAATTAGAAAAAGGACATTGAGCCTTCATTTTCGCCTTGGAATTCTGTTTGTAATTCAGAAAAAATCCGGCAGATGGCGTATAAT
GCCGTAATTCAACCCATGGGGGCTCTCCCACCCCGGTTGCCCTCTCCAGCCATGGTCCCCTTTAATTATAATTGATCTGAAGGATTGCT
TTTTTACCATTCCTCTGGCAAAACAGGATTTTGAAAAATTTGCTTTTACCACACCAGCCTAAATAATAAAGAACCAGCCACCAGGTTTC
AGTGGAAAGTATTGCCTCAGGGAATGCTTAATAGTTCAACTATTTGTCAGCTCAAGCTCTGCAACCAGTTAGAGACAAGTTTTCAGACT
 
GTTACATCGTTCACTATGTTGATATTTGTGTGCTGCAGAAACGAGAGACAAATTAATTGACCGTTACACATTTCTGCAGACAGAGGTT
GCCAACGCGGGACTGACAATAACATCTGATAAGATTCAAACCTCTACTCCTTTCCGTTACTTGGGAATGCAGGTAGAGGAAAGGAAAAT
TAAACCACAAAAAATAGAAATAAGAAAAGACACATTAAAAGCATTAAATGAGTTTCAAAAGTTGCTAGGAGATACTAATTGGATTTGGA
GATATTAATTGGATTTGGCCAACTCTAGGCATTCCTACTTATGCCATGTCAAATTTGTTCTCTTTCTTAAGAGGGGACTCGGAATTAAA
TAGTGAAAGAACGTTAACTCCAGAGGCAACTAAAGAAATTAAATTAATTGAAGAAAAAATTCGGTCAGCACAAGTAAATAGAATAGATC
ACTTGGCCCCACTCCAAATTTTGATTTTTGCTACTGCACATTCCCTAACAGGCATCATTGTTCAAAATACAGATCTTGTGGAGTGGTCC
TTCCTTCCTCACAGTACAATTAAGACTTTTACATTGTACTTGGATCAAATGGCTACATTAATTGGTCAGGGAAGATTATGAATAATAAC
ATTGTGTGGAAATGACCCAGATAAAATCACTGTTCCTTTCAACAAGCAACAGGTTAGACAAGCCTTTATCAATTCTGGTGCATGGCAGA
TTGGTCTTGCCGATTTTGTGGGAATTATTGACAATCGTTACCCCAAAACAAAAATCTTCCAGTTTTTAAAATTGACTACTTGGATTTTA
CCTAAAGTTACCAAACATAAGCCTTTAAAAAATGCTCTGGCAGTGTTTACTGATGGTTCCAGCAATGGAAAAGTGGCTTACACCGGGCC
AAAAGAATGAGTCATCAAAACTCAGTATCACTTGACTCAAAGAGCAGAGTTGGTTGCCGTCATTACAGTGTTAACAAGATTTTAATCAG
TCTATTAACATTGTATCAGATTCTGCATATGTAGTACAGGCTACAAAGGATATTGAGAGAGCCCTAATCAAATACATTATGGATGATCA
GTTAAACCCGCTGTTTAATTTGTTACAACAAAATGTAAGAAAAAGAAATTTCCCATTTTATATTACTCATATTCGAGCACACACTAATT
TACCAGGGCCTTTAACTAAAGCAAATGAACAAGCTGACTTGCTAGTATCATCTGCATTCATGGAAGCACAAGAACTTCATGCCTTGACT
CATGTAAATGCAATAGGATTAAAAAATAAATTTGATATCACATGGAAACAGACAAAAAATATTGTACAACATTGCACCCAGTGTCAGAT
TCTACACCTGGCCACTCAGGAGGCAAGAGTTAATCCCAGAGGTCTATGTCCTAATGTGTTATGGCAAATGGATGTCATGCACGTACCTT
CATTTGGAAAATTGTCATTTGTCCATGTGACAGTTGATACTTATTCACATTTCATATGGGCAACCTGCCAGACAGGAGAAAGTACTTCC
CATGTTAAAAGACATTTATTATCTTGTTTTCCTGTCATGGGAGTTCCAGAAAAAGTTAAAACAGACAATGGGCCAGGTTACTGTAGTAA
AGCAGTTCAAAAATTCTTAAATCAGTGGAAAATTACACATACAATAGGAATTCTCTATAATTCCCAAGGACAGGCCATAATTGAAAGAA
CTAATAGAACACTCAAAGCTCAATTGGTTAAACAAAAAAAAGGAAAAGACAGGAGTATAACACTCCCCAGATGCAACTTAATCTAGCAC
TCTATACTTTAAATGTTTTAAACATTTATAGAAATCAGACCACTACCTCTGCAGAACAACATCTTACTGGTAAAAGGAACAGCCCACAT
GAAGGAAAACTGATTTGGTGGAAAGATAATAAAAATAAAACATGGGAAATGGGGAAGGTGATAACGTGGGGGAGAGGTTTTGCTTGTGT
TTCACCAGGAGAAAATCAGCTTCCTGTTTGGATACCCACTAGACATTTAAAGTTCTACAATGAACTCACTGGAGATGCAAAGAAAAGTG
TGGAGATGGAGACACCCCAATCGACTCGCCAGGTAAACAAAATGGTGATATCAGAAGAACAGAAAAAGTTGCCTTCCATCAAGGAAGCA
GAGTTGCCAATATAGGCACAATTAAAGAAGCTGACACAGTTAGCTAAAAAAAAAAGCCTAGAGAATACAAAGGTGACACCAACTCCAGA
GAATATGCTGCTTGCAGCTCTGATGATTGTATCAACGGTGGTAAGTCTTCCCAAGTCTGCAGGAGCAGCTGCAGCTAATTATACTTACT
GGGCCTATGTGCCTTTCCCACCCTTAATTCGGGCAGTTACATAGATGGATAATCCTATTGAAGTAGATGTTAATAATAGTGCATGGGTG
CCTGGCCCCACAGATGACTGTTGCCCTGCCCAACCTGAAGAAGGAATGATGATGAATATTTCCATTGGGTATCCTTATCCTCCTGTTTG
CCTAGGGAAGGCACCAGGATGCTTAATGCCTACAACCCAAAATTGGTTGGTAGAAGTACCTACAGTCAGTGCTACCAGTAGATTTACTT
ATCACATGGTAAGTGGAATGTCACAGATAAATAATTTACAGGACCCTTCTTATCAAAGATCATTACAATGTAGGCCTAAGGGGAAGGCT
TGCCCCAAGGAAATTCCCAAAGAATCAAAAAGCCCAGAAGTCTTAGTCTGCGGAGAATGTGTGGCTGATACTGCAGTGTAGTACAAAAC
AATGAATTTTGAACTATGATAGACTGGGTCCCTTGAGGCCAATTATATCATAACTGTACAGGCCAGACTCATTCATGTTCACAGGCCCC
ATCCATCTGGCCCATTAATCCAGCCTATGACGGTGATGTAACTGAAAGGCTGGACCAGGTTTATAGAAGGTTAGAATCACTCTGTCCAA
GGAAATGGGGTGAAAAGGGAATTTCATCACCTTGACCAAAGTTAGTCCTGTTACTGGTCCTGAACATCCAGAATTAGGAAGCTTACTGT
GGCCTCACACCACATTAGAATTTGTTCTGGAAATCAAGCTATAGGAACAAGAGATCGTAAGTCATATTATACTATCAACCTAAATTCCA
GTCTGACAATTCCTTTGCAAAATTGTGTAAAACTCCCTTATATTGCTAGTTGTAGGAAAAACATAGTTATTAAACCTGATTCCCAAACC
ATAATCTGTGAAAATTGTGGAATGTTTACTTGCATTGATTTGACTTTTAATTGGCAGCACCGTATTCTACTAGGAAGAGCAAGAGAGGG
TGTGTGGATCCTTGTGTCCATGGACCGACCATGGGAGGCTTCGCTATCCATCCATATTTTAACGGAAGTATTAAAAGGAATTCTAACTA
GATCCAAAAGATTCATTTTTACTTTGATGGCAGTGATTATGGGCCTCATTGCAGTCACAGCTACTGCTGCGGCTGCTGGAATTGCTTTA
CACTCCTCTGTTCAAACTGCAGAATACGTAAATGATTGGCAAAAGAATTCCTCAAAATTGTGGAATTCTCAGATCCAAATAGATCAAAA
ATTGGCAAACCAAATTAATGATCTTAGACAAACTGTCATTTGGATGGGAGAGGCTCATGAGCTTGGAATATCTTTTTCAGTTACGATGT
GACTGGAATACATCAGATTTTTGTGTTACACCACAAGCCTATAATGAGTCTGAGCATCACTGGGACATGGTTAGATGCCATCTGCAAGG
AGGAGAAGATAATCTTACTTTAGACATTTCAAAATTAAAAGAATTTTTTTTTCTTTGAGACAGAGTCTCGCTCTGTCGCCCAGGCTGG
AGTGCAGTGGCGTGATCTCAGCTCACTGCAAGTTCCGCCTCCTGGGTTTACACCATTCTCCTGCCTCAGCCTCCCAAGTAGTTGGGACT
ACAGGAGCCCACCACCATGCCTGGCTAATTTTTTTTGGGTTTTAATAGAGATGGAGTTTCACCGTGTTAGCCAGGATGGTCTCGATCT
CCTGACCTTGTGATCTGCCCACCTTGGCCTCCCAAAGTGCTGGGATTACAGTCGTGAGCCACCGTGCCCAGCCAAGAAAAAATTTTTGA
GGCATCAAAAGCCCATTTAAATTTGGTGCCAGGAACGGAGACAATCGTGAAAGCTGCTGATAGCCTCACAAATCTTAAGCCAGTCACTT
GGGTTAAAAGCATCAGAAGTTTCACTATTGTAAATTTCATATTAATCCTTGTATGCCTGTTCTGTCTGTTGTTAGTCTACAGGTGTATC
CAGCAGCTCCAAAGAGACAGCAACCAGCAAGAATGGGCCATAGTGACGATGGTGGTTTTGTCAAAAAGAAAAGGGGGGGATATGTAAGG
AAAAGAGAGATCAGACTTTCACTGTGTCTATGTAGAAAAGGAAGACATAAGAAACTCCATTTGATCTGTACTAAGAAAAATTGTTTTG
CCTTGAGATGCTGTTAATCTGTAACTTTAGCCCCAACCCTGTGCTCACGGAAACATGTGCTGTAAGGTTTAAGGGATCTAGGGCTGTGC
AGGATGTACCTTGTTAACAATATGTTTGCAGGCAGTATGTTTGGTAAAAGTCATCGCCATTCTCCATTCTCGATTAACCAGGGGCTCAA
TGCACTGTGGAAAGCCACAGGAACCTCTGCCCAAGAAAGCCTGGCTGTTGTGGGAAGTCAGGGACCCCGAATGGAGGGACCAGCTGGTG
 
CTGCATCAGGAAACATAAATTGTGAAGATTTCTTGGACATTTATCAGTTTCCAAAATTAATACTTTTATAATTTCTTACACCTGTCTTA
CTTTAATCTCTTAATCCTGTTATCTTTGTAAGCTGAGGATATACGTCACCTCAGGACCACTATTGTACAAATTGATTGTAAAACATGTT
CACATGTGTTTGAACAATATGAAATCAGTGCACCTTGAAAATGAACAGAATAACAGTGATTTTAGGGAACAAAGGAAGACAACCATAAG
GTCTGACTGCCTGAGGGGTCGGGCAAAAAGCCATATTTTTCTTCTTGCAGAGAGCCTATAAATGGACGTGCAAGTAGGAGAGATATTGC
TAAATTCTTTTCCTAGCAAGGAATATAATACTAAGACCCTAGGGAAAGAATTGCATTCCTGGGGGGAGGTCTATAAACGGCCGCTCTGG
GAGTGTCTGTCCTATGTGGTTGAGATAAGGACTGAGATACGCCCTGGTCTCCTGCAGTACCCTCAGGCTTACTAGGATTGGGAAACCCC
AGTCCTGGTAAATTTGAGGTCAGGCCGGTTCTTTGCTCTGAACCCTGTTTTCTGTTAAGATGTTTATCAAGACAATACATGCACCGCTG
AACATAGACCCTTATCAGGAGTTTCTGATTTTGCTCTGGTCCTGTTTCTTCAGAAGCATGTCATCTTTGCTCTGCCTTCTGCCCTTTGA
AGCATGTGATCTTTGTGACCTACTCCCTGTTCATACACCCCTCCCCTTTTAAAATCCCTAATAAAAACTTGCTGGTTTTGTGGCTCAGG
GGGCATCATGGACCTACCAATACGTGATGTCACCCCCGGTGGCCCAGCTGT
**SEQ ID 11**
   TAGGTCTC
**SEQ ID 12**
   TAGGGGTG
**SEQ ID 13**
   TGGCTGTT
**SEQ ID 14** 
AACGTGGATGCTTTTCTCTAGGGTGAAGGACTCTCGAGTGTGGTCATTGAGGACAAGTCAACGAGAGATTCCCGAGTACGTCTACAGT
GAGCCTTGTGGGTGAAGGTACTCTACAGTGTGGTCATTGAGGACAAGTTGACGAGAGAGTCCCAAGTACGTCCACGGTCAGCCTTGCGA
CATTTTAAAGTTCTACAATGAACTCA
**SEQ ID 15** 
ACAGAAGGGTACATGAAGGAAAACTGATTTGGTGGAAAGATAATAAAAATAAAACATGGGAAATGGGGAAGGTGATAACGTGGGGGAGA
GGTTTTGCTTGTGTTTCACCAGGAGAAAATCAGCTTCCTGTTTGGATACCCACTAGACATTTAAAGTTCTACAATGAACTCACTGGAGA
TGCAAAGAAAAGTGTGGAGATGGAGACACCCCAATCGACTCGCCAGTCTACAGGTGTATCCAGCAGCTCCAAAGAGACAGCAACCAGCA
AGAATGGGCCATAGTGACGATGGTGGTTTTGTCAAAAAGAAAAGGGGGGATATGTAAGGAAAAGAGAGATCAGACTTTCACTGTGTCT
ATGTAGAAAAGGAAGACATAAGAAACTCCATTTTGATCTGTACTAAGAAAAATTGTTTTGCCTTGAGATGCTGTTAATCTGTAACTTTA
GCCCCAGCCCTGTGCTCACGGAAACATGTGCTGTAAGGTTTAAGGGATCTAGGGCTGT
**SEQ ID 16** 
AGTCATCAAAACTCAGTATCACTTGACTCAAAGAGCAGAGTTGGTTGCCGTCATTACAGTGTTAACAAGATTTTAATCAGTCTATTAAC
ATTGTATCAGATTCTGCATATGTAGTACAGGCTACAAAGGATATTGAGAGAGCCCTAATCAAATACATTATGGATGATCAGTTAAACCC
GCTGTTTAATTTGTTACAACAAAATGTAAGAAAAAGAAATTTCCCATTTTATATTACTCATATTCGAGCACACACTAATTTACCAGGGC
CTTTAACTAAAGCAAATGAACAAGCTGACTCGCTAGTATCATCTGCATTCATGGAAGCACAAGACCTTCATGCCTTGACTCATGTAAAT
GCAATAGGATTAAAAAATAAATTTAATATCACATGGAAACAGACAAAAAATATTGTACAACATTGCACCCAGTGTCAGATTCTACACCT
GGCCACTCAGGAGGCAAGAGTTAATCCCAGAGGTCTATGTCCTAATGTGTTATGGCAAATGGATGTCATGCACGTACCTTCATTTGGAA
AATTGTCATTTGTCCATGTGACAGTTGATACTTATTCACATTTCATATGGGCAACCTGCCAGACAGGAGAAAGTACTTCCCATGTTAAG
AGACATTTATTATC
**SEQ ID 17** 
AGTCATCAAAACTCAGTATCACTTGACTCAAAGAGCAGAGTTGGTTGCCGTCATTACAGTGTTAACAAGATTTTAATCAGTCTATTAAC
ATTGTATCAGATTCTGCATATGTAGTACAGGCTACAAAGGATATTGAGAGAGCCCTAATCAAATACATTATGGATGATCAGTTAAACCC
GCTGTTTAATTTGTTACAACAAAATGTAAGAAAAAGAAATTTCCCATTTTATATTACTCATATTCGAGCACACACTAATTTACCAGGGC
CTTTAACTAAAGCAAATGAACAAGCTGACTTGCTAGTATCATCTGCATTCATGGAAGCACAAGAACTTCATGCCTTGACTCATGTAAAT
GCAATAGGATTAAAAAATAAATTTGATATCACATGGAAACAGACAAAAAATATTGTACAACATTGCACCCAGTGTCAGATTCTACACCT
GGCCACTCAGGAGGCAAGAGTTAATCCCAGAGGTCTATGTCCTAATGTGTTATGGCAAATGGATGTCATGCACGTACCTTCATTTGGAA
AATTGTCATTTGTCCATGTGACAGTTGATACTTATTCACATTTCATATGGGCAACCTGCCAGACAGGAGAAAGTACTTCCCATGTTAAA
AGACATTTATTATCTTGTTTTCCTGTC
**SEQ ID 18** 
AGTCATCAAAACTCAGTATCACTTGACTCAAAGAGCAGAGTTGGTTGCCGTCATTACAGTGTTAACAAGATTTTAATCAGTCTATTAAC
ATTGTATCAGATTCTGCATATGTAGTACAGGCTACAAAGGATATTGAGAGAGCCCTAATCAAATACATTATGGATGATCAGTTAAACCC
GCTGTTTAATTTGTTACAACAAAATGTAAGAAAAAGAAATTTCCCATTTTATATTACTCATATTCGAGCACACACTAATTTACCAGGGC
CTTTAACTAAAGCAAATGAACAAGCTGACTTGCTAGTATCATCTGCATTCATGGAAGCACAAGAACTTCATGCCTTGACTCATGTAAAT
GCAATAGGATTAAAAAATAAATTTGATATCACATGGAAACAGACAAAAAATATTGTACAACATTGCACCCAGTGTCAGATTCTACACCT
 
GGCCACTCAGGAGGCAAGAGTTAATCCCAGAGGTCTATGTCCTAATGTGTTATGGCAAATGGATGTCATGCACGTACCTTCATTTGGAA
AATTGTCATTTGTCCATGTGACAGTTGATACTTATTCACATTTCATATGGGCAACCTGCCAGACAGGAGAAAGTACTTCCCATGTTAAA
AGACATTTATTATCTTGTTTTCCTGTC
**SEQ ID 19** 
AGATCTGATCATCTGGTGCCCAACGTGGAGGCTTTTCTCTAGGGTGAAGGACTCTCGAGTGTGGTCATTGAGGACAAGTCAACGAGAG
ATTCCCGAGTACGTCTACAGTGAGCCTTGTGGGTGAAGGTACTCTACAGTGTGGTCATTGAGGACAAGTTGACGAGAGAGTCCCAAGTA
CGTCCACGGTCAGCCTTGCGAC
**SEQ ID 20**
   AACTGGAAGAATTCGCGGCCGCATTTTTTTTTTTTTTTTTTTTTTTTTTTTTT
**SEQ ID 21**
   CAGGTGTAYCCARCAGCTCC
**SEQ ID 22**
   AACTGGAAGAATTCGCGGCCGCA
**SEQ ID 23** 
GAGAUAGGAGAAAACUGCCUUAGGGCUGGAGGUGGACAUGCUGGCGGCAAUACUGCUCUUUAAGGCAUUGAGAUGUUUAUGUAUAUGC
ACAUCAAAAGCACAGCACUUUUUCUUUACCUUGUUUAUGAUGCAGAGACAUUUGUUCACAUGUUUCCUGCUGGCCCUCUCCCCACUA
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUGGUAAGCUUGGGCGCUCG
GAAGAAGCCAGGGUUAAUGGGGCAAACUAAAAGUAAAGUCUCUCAUUCCACCUGAUGAGAAACACCCAGAGGUGUGGAGGGGCAGGCCA
CCCCUUCAGGGUAGGGUCCCCUCCAUGCAGACCAUAGAGCACAGGUGUGCCCCAAAGAGGAGCAGAGAGAAGGAGGGAGAGGGCCCACG
AGAGACUUGGAAAUGAAUGGCAGGAUUUUAGGCGCUGGACUUGGGUUCGGGGCACCUGGCCUUUCCUUGUGUAUUUCUCCUACUGUCUG
CCUAACUAUUUAAUACAAUAAAAGAAAACCAGCCCCUGGUUCUUGUGGUGUUUCCACCCUCCCGGGUCCCCGCUGGCUGCCUGGCUUCC
UCCCGCAGCUCCUGCUGUGUGUGUAUGUGUGUGUGUGCACAUCUGUGGGGCGUAUGUGUGUUCGUCUUUGUAAUUGAGGCUGCAGAG
UGGAGAGAGCAGGGGUUUUCUCUGGGGACCCAGAGAGAAGGAGGCGUUUUCACCACAGCCGAACAGGGCAGGACCCCAGCACCCGGGAC
CCAGCGGGACUUUGCCAAGGGGAUGGACCUGGCUGGGCCACGCGGCUGUUUGUGUAGGGAAAAGAAAGAGAGAUCACACUGUUACUGUG
UCUAUGUAGAAAAGGAAGACAUAAACUCCAUUUUGAGCUGUACUAAGAAAAAUUAUUUUGCCUUGACCUGCUGUUAACCUGUAACUGUA
GCCCCAACCCUGUGCUCAAAGAAACAUGUGCUGUAUGGAAUCAAGGUUUAAGGGAUCAAGGGCUGUACAGGAUGUGCCUUGUUAACAAU
GUGUUUACAGGCAGUAUGCUUGGUAAAAGUCAUCGCCAUUCUCCAUUCUCCAUUAAUCAGGGGCACGAUGCACUGCGGAAAGCCACAGG
GACCUCUGCCCGAGAAAGCCUGGGUAUUGUCCAAGGCUUCCCCCCACUGAGACAGCCUGAGAUACGGCCUCGUGGGAAGGGAAAGACCU
GACCGUCCCCCAGCCCGACACCCGUAAAGGGUCUGUGCUGAGGAGGAUUAGUAAAAGGGGAAGGCCUCUUGCAGUUGAGAUAAGAGGAA
GGCCUCCGUCUCCUGCAUGUCCUUGGGAAUGGAAUGUCUUGGUGUAAAACCCGAUAGUACAUUCCUUCUAUUCUGAGAGAAGAAAACCA
CCCUGUGGCUGGAGGUGAGAUAUGCUAGCGGCAAUGCUGCUCUGUUACUCUUUGCUACACUGAGAUGUUUGGGUGGAGAGAAGCAUAAA
UCUGGCCUAUGUGCACAUCUGGGCACAGAACCUCCCCUUGAACUUGUGACACAGAUUCCUUUGUUCACAUGUUUUCCUGCUGACCUUCU
CCCCACUAUCGCCCUGUUCUCCCACCGCAUUCCCCUUGCUGAGAUAGUGAAAAUAGUAAUCUGUAGAUACCAAGGGAACUCAGAGACCA
UGGCCGGUGCACAUCCUCCGUACGCUGAGCGCUGGUCCCCUGGGCCCAUUGUUCUUUCUCUAUACUUUGUCUCUGUGUCUUAUUUCUUU
CCUCAGUCUCUCAUCCCUCCUGACGAGAAAUACCCACAGGUGUGGAGGGGCUGGCCCCCUUCAUCUGAUGCCCAAUGUGGGUGCCUUUC
UCUAGGGUGAAGGUACUCUACAGUGUGGUCAUUGAGGACAAGUUGACGAGAGAGUCCCAAGUACGUCCACGGUCAGCCUUGCGGUAAGC
UUGUGUGCUUAGAGGAACCCAGGGUAACGAUGGGGCAAACUGAAAGUAAAUAUGCCUCUUAUCUCAGCUUUAUUAAAAUUCUUUUAAGA
AGAGGGGGAGUUAGAGCUUCUACAGAAAAUCUAAUUACGCUAUUUCAAACAAUAGAACAAUUCUGCCCAUGGUUUCCAGAACAGGGAAC
UUUAGAUCUAAAAGAUUGGGAAAAAAUUGGCAAAGAAUUAAAACAAGCAAAUAGGGAAGGUAAAAUCAUCCCACUUACAGUAUGGAAUG
AUUGGGCCAUUAUUAAAGCAACUUUAGAACCAUUUCAAACAGGAGAAGAUAUUGUUUCAGUUUCUGAUGCCCCUAAAAGCUGUGUAACA
GAUUGUGAAGAAGAGGCAGGGACAGAAUCCCAGCAAGGAACGGAAAGUUCACAUUGUAAAUAUGUAGCAGAGUCUGUAAUGGCUCAGUC
AACGCAAAAUGUUGACUACAGUCAAUUACAGGAGAUAAUAUACCCUGAAUCAUCAAAAUUGGGGAAGGAGGUCCAGAAUCAUUGGGGC
CAUCAGAGCCUAAACCACGAUCGCCAUCAACUCCUCCUCCCGUGGUUCAGAUGCCUGUAACAUUACAACCUCAAACGCAGGUUAGACAA
GCACAAACCCCAAGAGAAAAUCAAGUAGAAAGGGACAGAGUCUCUAUCCCGGCAAUGCCAACUCAGAUACAGUAUCCACAAUAUCAGCC
GGUAGAAAAUAAGACCCAACCGCUGGUAGUUUAUCAAUACCGGCUGCCAACCGAGCUUCAGUAUCGGCCUCCUUCAGAGGUUCAAUACA
GACCUCAAGCGGUGUGUCCUGUGCCAAAUAGCACGGCACCAUACCAGCAACCCACAGCGAUGGCGUCUAAUUCACCAGCAACACAGGAC
GCGGCGCUGUAUCCUCAGCCGCCCACUGUGAGACUUAAUCCUACAGCAUCACGUAGUGGACAGGGUGGUGCACUGCAUGCAGUCAUUGA
UGAAGCCAGAAAACAGGGCGAUCUUGAGGCAUGGCGGUUCCUGGUAAUUUUACAACUGGUACAGGCCGGGGAAGAGACUCAAGUAGGAG
CGCCUGCCCGAGCUGAGACUAGAUGUGAACCUUUCACCAUGAAAAUGUUAAAAGAUAUAAAGGAAGGAGUUAAACAAUAUGGAUCCAAC
 
UCCCCUUAUAUAAGAACAUUAUUAGAUUCCAUUGCUCAUGGAAAUAGACUUACUCCUUAUGACUGGGAAAUUUUGGCCAAAUCUUCCCU
UUCAUCCUCUCAGUAUCUACAGUUUAAAACCUGGUGGAUUGAUGGAGUACAAGAACAGGUACGAAAAAUCAGGCUACUAAGCCCACUG
UUAAUAUAGACGCAGACCAAUUGUUAGGAACAGGUCCAAAUUGGAGCACCAUUAACCAACAAUCAGUGAUGCAGAAUGAGGCUAUUGAA
CAAGUAAGGGCUAUUUGCCUCAGGGCCUGGGGAAAAAUUCAGGACCCAGGAACAGCUUUCCCUAUUAAUUCAAUUAGACAAGGCUCUAA
AGAGCCAUAUCCUGACUUUGUGGCAAGAUUACAAGAUGCUGCUCAAAAGUCUAUUACAGAUGACAAUGCCCGAAAAGUUAUUGUAGAAU
UAAUGGCCUAUGAAAAUGCAAAUCCAGAAUGUCAGUCGGCCAUAAAGCCAUUAAAAGGAAAAGUUCCAGCAGGAGUUGAUGUAAUUACA
GAAUAUGUGAAGGCUUGUGAUGGGAUUGGAGGAGCUAUGCAUAAGGCAAUGCUAAUGGCUCAAGCAAUGAGGGGGCUCACUCUAGGAGG
ACAAGUUAGAACAUUUGGGAAAAAAUGUUAUAAUUGUGGUCAAAUCGGUCAUCUGAAAAGGAGUUGCCCAGGCUUAAAUAAACAGAAUA
UAAUAAAUCAAGCUAUUACAGCAAAAAAUAAAAAGCCAUCUGGCCUGUGUCCAAAAUGUGGAAAAGCAAAACAUUGGGCCAAUCAAUGU
CAUUCUAAAUUUGAUAAAGAUGGGCAACCAUUGUCUGGAAACAGGAAGAGGGGCCAGCCUCAGGCCCCCCAACAAACUGGGGCAUUCCC
AGUUAAACUGUUUGUUCCUCAGGGUUUUCAAGGACAACAACCCCUACAGAAAAUACCACCACUUCAGGGAGUCAGCCAAUUACAACAAU
CCAACAGCUGUCCCGCGCCACAGCAGGCAGCACCGCAGUAGAUUUAUGUUCCACCCAAAUGGUCUUUUUACUCCCUGGAAAGCCCCCAC
AAAAGAUUCCUAGAGGGGUAUAUGGCCCGCUGCCAGAAGGGAGGGUAGGCCUUUGAGGGAGAUCAAGUCUAAAUUUGAAGGGAGUCCAA
AUUCAUACUGGGGUAAUUUAUUCAGAUUAUAAAGGGGGAAUUCAGUUAGUGAUCAGCUCCACUGUUCCCCGGAGUGCCAAUCCAGGUGA
UAGAAUUGCUCAAUUACUGCUUUUGCCUUAUGUUAAAAUUGGGGAAAACAAAAAGGAAAGAACAGGAGGGUUUGGAAGUACCAACCCUG
CAGGAAAAGCUGCUUAUUGGGCUAAUCAGGUCUCAGAGGAUAGACCCGUGUGUACAGUCACUAUUCAGGGAAAGAGUUUGAAGGAUUAG
UGGAUACCCAGGCUGAUGUUUCUGUCAUCGGCAUAGGUACUGCCUCAGAAGUGUAUCAAAGUGCCAUGAUUUUACAUUGUCCAGGAUCU
GAUAAUCAAGAAAGUACGGUUCAGCCUGUGAUCACUUCAUUCCAAUCAAUUUAUGGGGCCGAGACUUGUUACAACAAUGGCAUGCAGAG
AUUACUAUCCCAGCCUCCCUAUACAGCCCCAGGAAUAAAAAAAUCAUGACUAAAAUGGGAUAGCUCCCUAAAAAGGGACUAGGAAAGAA
GUCCCAAUUGAGGCUGAAAAAAAUCAAAAAGAAAAGGAAUAGGGCAUCCUUUUUAGGAGCGGUCACUGUAGAGCCUCCAAAACCCAUU
CCAUUAACUUGGGGGAAAAAAAAACAACUGUAUGGUAAAUCAGCAGCGCUUCCAAAACAAAAACUGGAGGCUUUACAUUUAUUAGCAAA
GAAACAAUUAGAAAAAGGACAUUGAGCCUUCAUUUUCGCCUUGGAAUUCUGUUUGUAAUUCAGAAAAAAUCCGGCAGAUGGCGUAUAAU
GCCGUAAUUCAACCCAUGGGGGCUCUCCCACCCCGGUUGCCCUCUCCAGCCAUGGUCCCCUUUAAUUAUAAUUGAUCUGAAGGAUUGCU
UUUUUACCAUUCCUCUGGCAAAACAGGAUUUUGAAAAAUUUGCUUUUACCACACCAGCCUAAAUAAUAAAGAACCAGCCACCAGGUUUC
AGUGGAAAGUAUUGCCUCAGGGAAUGCUUAAUAGUUCAACUAUUUGUCAGCUCAAGCUCUGCAACCAGUUAGAGACAAGUUUUCAGACU
GUUACAUCGUUCACUAUGUUGAUAUUUUGUGUGCUGCAGAAACGAGAGACAAAUUAAUUGACCGUUACACAUUUCUGCAGACAGAGGUU
GCCAACGCGGGACUGACAAUAACAUCUGAUAAGAUUCAAACCUCUACUCCUUUCCGUUACUUGGGAAUGCAGGUAGAGGAAAGGAAAAU
UAAACCACAAAAAAUAGAAAUAAGAAAAGACACAUUAAAAGCAUUAAAUGAGUUUCAAAAGUUGCUAGGAGAUACUAAUUGGAUUUGGA
GAUAUUAAUUGGAUUUGGCCAACUCUAGGCAUUCCUACUUAUGCCAUGUCAAAUUUGUUCUCUUUCUUAAGAGGGGACUCGGAAUUAAA
UAGUGAAAGAACGUUAACUCCAGAGGCAACUAAAGAAAUUAAAUUAAUUGAAGAAAAAAUUCGGUCAGCACAAGUAAAUAGAAUAGAUC
ACUUGGCCCCACUCCAAAUUUUGAUUUUUGCUACUGCACAUUCCCUAACAGGCAUCAUUGUUCAAAAUACAGAUCUUGUGGAGUGGUCC
UUCCUUCCUCACAGUACAAUUAAGACUUUUACAUUGUACUUGGAUCAAAUGGCUACAUUAAUUGGUCAGGGAAGAUUAUGAAUAAUAAC
AUUGUGUGGAAAUGACCCAGAUAAAAUCACUGUUCCUUUCAACAAGCAACAGGUUAGACAAGCCUUUAUCAAUUCUGGUGCAUGGCAGA
UUGGUCUUGCCGAUUUUGUGGGAAUUAUUGACAAUCGUUACCCCAAAACAAAAAUCUUCCAGUUUUAAAAUUGACUACUUGGAUUUUA
CCUAAAGUUACCAAACAUAAGCCUUUAAAAAAUGCUCUGGCAGUGUUUACUGAUGGUUCCAGCAAUGGAAAAGUGGCUUACACCGGGCC
AAAAGAAUGAGUCAUCAAAACUCAGUAUCACUUGACUCAAAGAGCAGAGUUGGUUGCCGUCAUUACAGUGUUAACAAGAUUUUAAUCAG
UCUAUUAACAUUGUAUCAGAUUCUGCAUAUGUAGUACAGGCUACAAAGGAUAUUGAGAGAGCCCUAAUCAAAUACAUUAUGGAUGAUCA
GUUAAACCCGCUGUUUAAUUUGUUACAACAAAAUGUAAGAAAAAGAAAUUUCCCAUUUUAUAUUACUCAUAUUCGAGCACACACUAAUU
UACCAGGGCCUUUAACUAAAGCAAAUGAACAAGCUGACUUGCUAGUAUCAUCUGCAUUCAUGGAAGCACAAGAACUUCAUGCCUUGACU
CAUGUAAAUGCAAUAGGAUUAAAAAAUAAAUUUGAUAUCACAUGGAAACAGACAAAAAAUAUUGUACAACAUUGCACCCAGUGUCAGAU
UCUACACCUGGCCACUCAGGAGGCAAGAGUUAAUCCCAGAGGUCUAUGUCCUAAUGUGUUAUGGCAAAUGGAUGUCAUGCACGUACCUU
CAUUUGGAAAAUUGUCAUUUGUCCAUGUGACAGUUGAUACUUAUUCACAUUUCAUAUGGGCAACCUGCCAGACAGGAGAAAGUACUUCC
CAUGUUAAAAGACAUUUAUUAUCUUGUUUCCUGUCAUGGGAGUUCCAGAAAAAGUUAAAACAGACAAUGGGCCAGGUUACUGUAGUAA
AGCAGUUCAAAAAUUCUUAAAUCAGUGGAAAAUUACACAUACAAUAGGAAUUCUCUAUAAUUCCCAAGGACAGGCCAUAAUUGAAAGAA
CUAAUAGAACACUCAAAGCUCAAUUGGUUAAACAAAAAAAAGGAAAAGACAGGAGUAUAACACUCCCCAGAUGCAACUUAAUCUAGCAC
UCUAUACUUUAAAUGUUUUAAACAUUUAUAGAAAUCAGACCACUACCUCUGCAGAACAACAUCUUACUGGUAAAAGGAACAGCCCACAU
GAAGGAAAACUGAUUUGGUGGAAAGAUAAUAAAAAUAAAACAUGGGAAAUGGGGAAGGUGAUAACGUGGGGGAGAGGUUUUGCUUGUGU
UUCACCAGGAGAAAAUCAGCUUCCUGUUUGGAUACCCACUAGACAUUUAAAGUUCUACAAUGAACUCACUGGAGAUGCAAAGAAAAGUG
UGGAGAUGGAGACACCCCAAUCGACUCGCCAGGUAAACAAAAUGGUGAUAUCAGAAGAACAGAAAAAGUUGCCUUCCAUCAAGGAAGCA
GAGUUGCCAAUAUAGGCACAAUUAAAGAAGCUGACACAGUUAGCUAAAAAAAAAAGCCUAGAGAAUACAAAGGUGACACCAACUCCAGA
GAAUAUGCUGCUUGCAGCUCUGAUGAUUGUAUCAACGGUGGUAAGUCUUCCCAAGUCUGCAGGAGCAGCUGCAGCUAAUUAUACUUACU
GGGCCUAUGUGCCUUUCCCACCCUUAAUUCGGGCAGUUACAUAGAUGGAUAAUCCUAUUGAAGUAGAUGUUAAUAAUAGUGCAUGGGUG
CCUGGCCCCACAGAUGACUGUUGCCCUGCCCAACCUGAAGAAGGAAUGAUGAUGAAUAUUUCCAUUGGGUAUCCUUAUCCUCCUGUUUG
 
CCUAGGGAAGGCACCAGGAUGCUUAAUGCCUACAACCCAAAAUUGGUUGGUAGAAGUACCUACAGUCAGUGCUACCAGUAGAUUUACUU
AUCACAUGGUAAGUGGAAUGUCACAGAUAAAUAAUUUACAGGACCCUUCUUAUCAAAGAUCAUUACAAUGUAGGCCUAAGGGAAGGCU
UGCCCCAAGGAAAUUCCCAAAGAAUCAAAAAGCCCAGAAGUCUUAGUCUGCGGAGAAUGUGUGGCUGAUACUGCAGUGUAGUACAAAAC
AAUGAAUUUUGAACUAUGAUAGACUGGGUCCCUUGAGGCCAAUUAUAUCAUAACUGUACAGGCCAGACUCAUUCAUGUUCACAGGCCCC
AUCCAUCUGGCCCAUUAAUCCAGCCUAUGACGGUGAUGUAACUGAAAGGCUGGACCAGGUUUAUAGAAGGUUAGAAUCACUCUGUCCAA
GGAAAUGGGGUGAAAAGGGAAUUUCAUCACCUUGACCAAAGUUAGUCCUGUUACUGGUCCUGAACAUCCAGAAUUAGGAAGCUUACUGU
GGCCUCACACCACAUUAGAAUUUGUUCUGGAAAUCAAGCUAUAGGAACAAGAGAUCGUAAGUCAUAUUAUACUAUCAACCUAAAUUCCA
GUCUGACAAUUCCUUUGCAAAAUUGUGUAAAACUCCCUUAUAUUGCUAGUUGUAGGAAAAACAUAGUUAUUAAACCUGAUUCCCAAACC
AUAAUCUGUGAAAAUUGUGGAAUGUUUACUUGCAUUGAUUUGACUUUUAAUUGGCAGCACCGUAUUCUACUAGGAAGAGCAAGAGAGGG
UGUGUGGAUCCUUGUGUCCAUGGACCGACCAUGGGAGGCUUCGCUAUCCAUCCAUAUUUAACGGAAGUAUUAAAAGGAAUUCUAACUA
GAUCCAAAAGAUUCAUUUUUACUUUGAUGGCAGUGAUUAUGGGCCUCAUUGCAGUCACAGCUACUGCUGCGGCUGCUGGAAUUGCUUUA
CACUCCUCUGUUCAAACUGCAGAAUACGUAAAUGAUUGGCAAAAGAAUUCCUCAAAAUUGUGGAAUUCUCAGAUCCAAAUAGAUCAAAA
AUUGGCAAACCAAAUUAAUGAUCUUAGACAAACUGUCAUUUGGAUGGGAGAGGCUCAUGAGCUUGGAAUAUCUUUUUCAGUUACGAUGU
GACUGGAAUACAUCAGAUUUUUGUGUUACACCACAAGCCUAUAAUGAGUCUGAGCAUCACUGGGACAUGGUUAGAUGCCAUCUGCAAGG
AGGAGAAGAUAAUCUUACUUUAGACAUUUCAAAAUUAAAAGAAUUUUUUUUUCUUUGAGACAGAGUCUCGCUCUGUCGCCCAGGCUGG
AGUGCAGUGGCGUGAUCUCAGCUCACUGCAAGUUCCGCCUCCUGGGUUUACACCAUUCUCCUGCCUCAGCCUCCCAAGUAGUUGGGACU
ACAGGAGCCCACCACCAUGCCUGGCUAAUUUUUUUUGGGUUUUUAAUAGAGAUGGAGUUUCACCGUGUUAGCCAGGAUGGUCUCGAUCU
CCUGACCUUGUGAUCUGCCCACCUUGGCCUCCCAAAGUGCUGGGAUUACAGUCGUGAGCCACCGUGCCCAGCCAAGAAAAAAUUUUUGA
GGCAUCAAAAGCCCAUUUAAAUUUGGUGCCAGGAACGGAGACAAUCGUGAAAGCUGCUGAUAGCCUCACAAAUCUUAAGCCAGUCACUU
GGGUUAAAAGCAUCAGAAGUUUCACUAUUGUAAAUUUCAUAUUAAUCCUUGUAUGCCUGUUCUGUCUGUUGUUAGUCUACAGGUGUAUC
CAGCAGCUCCAAAGAGACAGCAACCAGCAAGAAUGGGCCAUAGUGACGAUGGUGGUUUUGUCAAAAAGAAAAGGGGGGAUAUGUAAGG
AAAAGAGAGAUCAGACUUUCACUGUGUCUAUGUAGAAAAGGAAGACAUAAGAAACUCCAUUUUGAUCUGUACUAAGAAAAAUUGUUUUG
CCUUGAGAUGCUGUUAAUCUGUAACUUUAGCCCCAACCCUGUGCUCACGGAAACAUGUGCUGUAAGGUUUAAGGGAUCUAGGGCUGUGC
AGGAUGUACCUUGUUAACAAUAUGUUUGCAGGCAGUAUGUUUGGUAAAAGUCAUCGCCAUUCUCCAUUCUCGAUUAACCAGGGGCUCAA
UGCACUGUGGAAAGCCACAGGAACCUCUGCCCAAGAAAGCCUGGCUGUUGUGGGAAGUCAGGGACCCCGAAUGGAGGGACCAGCUGGUG
CUGCAUCAGGAAACAUAAAUUGUGAAGAUUUCUUGGACAUUUAUCAGUUUCCAAAAUUAAUACUUUUAUAAUUUCUUACACCUGUCUUA
CUUUAAUCUCUUAAUCCUGUUAUCUUUGUAAGCUGAGGAUAUACGUCACCUCAGGACCACUAUUGUACAAAUUGAUUGUAAAACAUGUU
CACAUGUGUUUGAACAAUAUGAAAUCAGUGCACCUUGAAAAUGAACAGAAUAACAGUGAUUUUAGGGAACAAAGGAAGACAACCAUAAG
GUCUGACUGCCUGAGGGGUCGGGCAAAAAGCCAUAUUUUUCUUCUUGCAGAGAGCCUAUAAAUGGACGUGCAAGUAGGAGAGAUAUUGC
UAAAUUCUUUUCCUAGCAAGGAAUAUAAUACUAAGACCCUAGGGAAAGAAUUGCAUUCCUGGGGGGAGGUCUAUAAACGGCCGCUCUGG
GAGUGUCUGUCCUAUGUGGUUGAGAUAAGGACUGAGAUACGCCCUGGUCUCCUGCAGUACCCUCAGGCUUACUAGGAUUGGAAACCCC
AGUCCUGGUAAAUUUGAGGUCAGGCCGGUUCUUUGCUCUGAACCCUGUUUUCUGUUAAGAUGUUUAUCAAGACAAUACAUGCACCGCUG
AACAUAGACCCUUAUCAGGAGUUUCUGAUUUUGCUCUGGUCCUGUUUCUUCAGAAGCAUGUCAUCUUUGCUCUGCCUUCUGCCCUUUGA
AGCAUGUGAUCUUUGUGACCUACUCCCUGUUCAUACACCCCUCCCCUUUUAAAAUCCCUAAUAAAAACUUGCUGGUUUUGUGGCUCAGG
GGGGCAUCAUGGACCUACCAAUACGUGAUGUCACCCCCGGUGGCCCAGCUGU
**SEQ ID 24** 
GAGAUAGGAGAAAACUGCCUUAGGGCUGGAGGUGGGACAUGCUGGCGGCAAUACUGCUCUUUAAGGCAUUGAGAUGUUUAUGUAUAUGC
ACAUCAAAAGCACAGCACUUUUUCUUUACCUUGUUUAUGAUGCAGAGACAUUUGUUCACAUGUUUCCUGCUGGCCCUCUCCCCACUA
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUGUCUCUCAUCC
**SEQ ID 25** 
GAGAUAGGAGAAAACUGCCUUAGGGCUGGAGGUGGGACAUGCUGGCGGCAAUACUGCUCUUUAAGGCAUUGAGAUGUUUAUGUAUAUGC
ACAUCAAAAGCACAGCACUUUUUUCUUUACCUUGUUUAUGAUGCAGAGACAUUUGUUCACAUGUUUUCCUGCUGGCCCUCUCCCCACUA
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUGGGUGAAGGUA
**SEQ ID 26** 
GAGAUAGGAGAAAACUGCCUUAGGGCUGGAGGUGGACAUGCUGGCGGCAAUACUGCUCUUUAAGGCAUUGAGAUGUUUAUGUAUAUGC
ACAUCAAAAGCACAGCACUUUUUUCUUUACCUUGUUUAUGAUGCAGAGACAUUUGUUCACAUGUUUUCCUGCUGGCCCUCUCCCCACUA
 
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUG
**SEQ ID 27**
   UCUCUCAUCC
**SEQ ID 28**
   GGUGAAGGUA
**SEQ ID 29** 
UGUAAGGAAAAGAGAGAUCAGACUUUCACUGUGUCUAUGUAGAAAAGGAAGACAUAAGAAACUCCAUUUUGAUCUGUACUAAGAAAAAU
UGUUUUGCCUUGAGAUGCUGUUAAUCUGUAACUUUAGCCCCAACCCUGUGCUCACGGAAACAUGUGCUGUAAGGUUUAAGGGAUCUAGG
GCUGUGCAGGAUGUACCUUGUUAACAAUAUGUUUGCAGGCAGUAUGUUUGGUAAAAGUCAUCGCCAUUCUCCAUUCUCGAUUAACCAGG
GGCUCAAUGCACUGUGGAAAGCCACAGGAACCUCUGCCCAAGAAAGCCUGGCUGUUGUGGGAAGUCAGGGACCCCGAAUGGAGGGACCA
GCUGGUGCUGCAUCAGGAAACAUAAAUUGUGAAGAUUUCUUGGACAUUUAUCAGUUCCAAAAUUAAUACUUUUAUAAUUUCUUACACC
UGUCUUACUUUAAUCUCUUAAUCCUGUUAUCUUUGUAAGCUGAGGAUAUACGUCACCUCAGGACCACUAUUGUACAAAUUGAUUGUAAA
ACAUGUUCACAUGUGUUUGAACAAUAUGAAAUCAGUGCACCUUGAAAAUGAACAGAAUAACAGUGAUUUUAGGGAACAAAGGAAGACAA
CCAUAAGGUCUGACUGCCUGAGGGGUCGGGCAAAAAGCCAUAUUUUUCUUCUUGCAGAGAGCCUAUAAAUGGACGUGCAAGUAGGAGAG
AUAUUGCUAAAUUCUUUUCCUAGCAAGGAAUAUAAUACUAAGACCCUAGGGAAAGAAUUGCAUUCCUGGGGGGAGGUCUAUAAACGGCC
GCUCUGGGAGUGUCUGUCCUAUGUGGUUGAGAUAAGGACUGAGAUACGCCCUGGUCUCCUGCAGUACCCUCAGGCUUACUAGGAUUGGG
AAACCCCAGUCCUGGUAAAUUUGAGGUCAGGCCGGUUCUUUGCUCUGAACCCUGUUUUCUGUUAAGAUGUUUAUCAAGACAAUACAUGC
ACCGCUGAACAUAGACCCUUAUCAGGAGUUUCUGAUUUUGCUCUGGUCCUGUUUCUUCAGAAGCAUGUCAUCUUUGCUCUGCCUUCUGC
CCUUUGAAGCAUGUGAUCUUUGUGACCUACUCCCUGUUCAUACACCCCUCCCCUUUUAAAAUCCCUAAUAAAAACUUGCUGGUUUUGUG
GCUCAGGGGGGCAUCAUGGACCUACCAAUACGUGAUGUCACCCCCGGUGGCCCAGCUGU
**SEQ ID 30** 
UGUAAGGAAAAGAGAGAUCAGACUUUCACUGUGUCUAUGUAGAAAAGGAAGACAUAAGAAACUCCAUUUUGAUCUGUACUAAGAAAAAU
UGUUUUGCCUUGAGAUGCUGUUAAUCUGUAACUUUAGCCCCAACCCUGUGCUCACGGAAACAUGUGCUGUAAGGUUUAAGGGAUCUAGG
GCUGUGCAGGAUGUACCUUGUUAACAAUAUGUUUGCAGGCAGUAUGUUUGGUAAAAGUCAUCGCCAUUCUCCAUUCUCGAUUAACCAGG
GGCUCAAUGCACUGUGGAAAGCCACAGGAACCUCUGCCCAAGAAAGCCUGGC
**SEQ ID 31** 
UGUUGUGGGAAGUCAGGGACCCCGAAUGGAGGGACCAGCUGGUGCUGCAUCAGGAAACAUAAAUUGUGAAGAUUUCUUGGACAUUUAUC
AGUUUCCAAAAUUAAUACUUUUAUAAUUUCUUACACCUGUCUUACUUUAAUCUCUUAAUCCUGUUAUCUUUGUAAGCUGAGGAUAUACG
UCACCUCAGGACCACUAUUGUACAAAUUGAUUGUAAAACAUGUUCACAUGUGUUUGAACAAUAUGAAAUCAGUGCACCUUGAAAAUGAA
CAGAAUAACAGUGAUUUUAGGGAACAAAGGAAGACAACCAUAAGGUCUGACUGCCUGAGGGGUCGGGCAAAAAGCCAUAUUUUUCUUCU
UGCAGAGAGCCUAUAAAUGGACGUGCAAGUAGGAGAGAUAUUGCUAAAUUCUUUUCCUAGCAAGGAAUAUAAUACUAAGACCCUAGGGA
AAGAAUUGCAUUCCUGGGGGGAGGUCUAUAAACGGCCGCUCUGGGAGUGUCUGUCCUAUGUGGUUGAGAUAAGGACUGAGAUACGCCCU
GGUCUCCUGCAGUACCCUCAGGCUUACUAGGAUUGGGAAACCCCAGUCCUGGUAAAUUUGAGGUCAGGCCGGUUCUUUGCUCUGAACCC
UGUUUUCUGUUAAGAUGUUUAUCAAGACAAUACAUGCACCGCUGAACAUAGACCCUUAUCAGGAGUUUCUGAUUUUGCUCUGGUCCUGU
UUCUUCAGAAGCAUGUCAUCUUUGCUCUGCCUUCUGCCCUUUGAAGCAUGUGAUCUUUGUGACCUACUCCCUGUUCAUACACCCCUCCC
CUUUUAAAAUCCCUAAUAAAAACUUGCUGGUUUUGUGGCUCAGGGGGCAUCAUGGACCUACCAAUACGUGAUGUCACCCCCGGUGGCC
CAGCUGU
**SEQ ID 32** 
TTAAAAGAATTTTTTTTTCTTTGAGACAGAGTCTCGCTCTGTCGCCCAGGCTGGAGTGCAGTGGCGTGATCTCAGCTCACTGCAAGTT
CCGCCTCCTGGGTTTACACCATTCTCCTGCCTCAGCCTCCCAAGTAGTTGGGACTACAGGAGCCCACCACCATGCCTGGCTAATTTTTT
TTGGGTTTTTAATAGAGATGGAGTTTCACCGTGTTAGCCAGGATGGTCTCGATCTCCTGACCTTGTGATCTGCCCACCTTGGCCTCCCA
AAGTGCTGGGATTACAGTCGTGAGCCACCGTGCCCAGCCA
**SEQ ID 33**
   CATTTCAAAA
**SEQ ID 34**
   AGAAAAAATT
**SEQ ID 35**
   TTAAAAGAAT

**SEQ ID 36**
   CATTTCAAAATTAAAAGAAT
**SEQ ID 37** 
TGTTACACCACAAGCCTATAATGAGTCTGAGCATCACTGGGACATGGTTAGATGCCATCTGCAAGGAGGAGAAGATAATCTTACTTTAG
ACATTTCAAAA
**SEQ ID 38** 
TGTTACACCACAAGCCTATAATGAGTCTGAGCATCACTGGGACATGGTTAGATGCCATCTGCAAGGAGGAGAAGATAATCTTACTTTAG
ACATTTCAAAATTAAAAGAATTTTTTTTTCTTTGAGACAGAGTCTCGCTCTGTCGCCCAGGCTGGAGTGCAGTGGCGTGATCTCAGCT
CACTGCAAGTTCCGCCTCCTGGGTTTACACCATTCTCCTGCCTCAGCCTCCCAAGTAGTTGGGACTACAGGAGCCCACCACCATGCCTG
GCTAATTTTTTTTGGGTTTTAATAGAGATGGAGTTTCACCGTGTTAGCCAGGATGGTCTCGATCTCCTGACCTTGTGATCTGCCCACC
TTGGCCTCCCAAAGTGCTGGGATTACAGTCGTGAGCCACCGTGCCCAGCCA
**SEQ ID 39**
   AAAATTAA
**SEQ ID 40** 
AGAAAAAATTTTTGAGGCATCAAAAGCCCATTTAAATTTGGTGCCAGGAACGGAGACAATCGTGAAAGCTGCTGATAGCCTCACAAATC
TTAAGCCAGTC
**SEQ ID 41**
   TGCCCAGCCA
**SEQ ID 42** 
TGCCCAGCCAAGAAAAAATTTTTGAGGCATCAAAAGCCCATTTAAATTTGGTGCCAGGAACGGAGACAATCGTGAAAGCTGCTGATAGC
CTCACAAATCTTAAGCCAGTC
**SEQ ID 43** 
TTAAAAGAATTTTTTTTTCTTTGAGACAGAGTCTCGCTCTGTCGCCCAGGCTGGAGTGCAGTGGCGTGATCTCAGCTCACTGCAAGTT
CCGCCTCCTGGGTTTACACCATTCTCCTGCCTCAGCCTCCCAAGTAGTTGGGACTACAGGAGCCCACCACCATGCCTGGCTAATTTTTT
TTGGGTTTTTAATAGAGATGGAGTTTCACCGTGTTAGCCAGGATGGTCTCGATCTCCTGACCTTGTGATCTGCCCACCTTGGCCTCCCA
AAGTGCTGGGATTACAGTCGTGAGCCACCGTGCCCAGCCAAGAAAAAATTTTTGAGGCATCAAAAGCCCATTTAAATTTGGTGCCAGGA
ACGGAGACAATCGTGAAAGCTGCTGATAGCCTCACAAATCTTAAGCCAGTC
**SEQ ID 44**
   GCCAAGAA
**SEQ ID 45**
   TGCCCAGCCAAGAAAAAATT
**SEQ ID 46** 
TCTCTCATCCCTCCTGACGAGAAATACCCACAGGTGTGGAGGGGCTGGCCCCCTTCATCTGATGCCCAATGTGGGTGCCTTTCTCTAGG
GTGAAGGTACT
**SEQ ID 47** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACT
**SEQ ID 48** 
GGTGAAGGTACTCTACAGTGTGGTCATTGAGGACAAGTTGACGAGAGAGTCCCAAGTACGTCCACGGTCAGCCTTGCGGTAAGCTTGTG
TGCTTAGAGGA
**SEQ ID 49** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
 
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGGTAAGCTTGTGTGCTTAGAGGA
**SEQ ID 50**
   GAGCCTTGTGTCTCTCATCC
**SEQ ID 51**
   GAGCCTTGTGGGTGAAGGTA
**SEQ ID 52**
   AAAGCCTGGCTGTTGTGGGA
**SEQ ID 53**
   ACAGCGATGGCGTCTAATTCACCAGCAACACAGGACGCGGCGCTGTAT
**SEQ ID 54** 
MGQTESKYASYLSFIKILLRRGGVRASTENLITLFQTIEQFCPWFPEQGTLDLKDWEKIGKELKQANREGKIIPLTVWNDWAIIKATLE
PFQTGEDIVSVSDAPKSCVTDCEEEAGTESQQGTESSHCKYVAESVMAQSTQNVDYSQLQEIIYPESSKLGEGGPESLGPSEPKPRSPS
TPPPVVQMPVTLQPQTQVRQAQTPRENQVERDRVSIPAMPTQIQYPQYQPVENKTQPLVVYQYRLPTELQYRPPSEVQYRPQAVCPVPN
STAPYQQPTAMASNSPATQDAALYPQPPTVRLNPTASRSGQGGALHAVIDEARKQGDLEAWRFLVILQLVQAGEETQVGAPARAETRCE
PFTMKMLKDIKEGVKQYGSNSPYIRTLLDSIAHGNRLTPYDWEILAKSSLSSSQYLQFKTWWIDGVQEQVRKNQATKPTVNIDADQLLG
TGPNWSTINQQSVMQNEAIEQVRAICLRAWGKIQDPGTAFPINSIRQGSKEPYPDFVARLQDAAQKSITDDNARKVIVELMAYENANPE
CQSAIKPLKGKVPAGVDVITEYVKACDGIGGAMHKAMLMAQAMRGLTLGGQVRTFGKKCYNCGQIGHLKRSCPGLNKQNIINQAITAKN
KKPSGLCPKCGKAKHWANQCHSKFDKDGQPLSGNRKRGQPQAPQQTGAFPVKLFVPQGFQGQQPLQKIPPLQGVSQLQQSNSCPAPQQA
APQ
**SEQ ID 55**
   TQVRQAQTPRENQVERDRVSIPA
**SEQ ID 56**
   PTAMASNSPATQDAALY
**SEQ ID 57** 
ATGGGGCAAACTGAAAGTAAATATGCCTCTTATCTCAGCTTTATTAAAATTCTTTTAAGAAGAGGGGGAGTTAGAGCTTCTACAGAAAA
TCTAATTACGCTATTTCAAACAATAGAACAATTCTGCCCATGGTTTCCAGAACAGGGAACTTTAGATCTAAAAGATTGGAAAAAATTG
GCAAAGAATTAAAACAAGCAAATAGGGAAGGTAAAATCATCCCACTTACAGTATGGAATGATTGGGCCATTATTAAAGCAACTTTAGAA
CCATTTCAAACAGGAGAAGATATTGTTTCAGTTTCTGATGCCCCTAAAAGCTGTGTAACAGATTGTGAAGAAGAGGCAGGGACAGAATC
CCAGCAAGGAACGGAAAGTTCACATTGTAAATATGTAGCAGAGTCTGTAATGGCTCAGTCAACGCAAAATGTTGACTACAGTCAATTAC
AGGAGATAATATACCCTGAATCATCAAAATTGGGGGAAGGAGGTCCAGAATCATTGGGGCCATCAGAGCCTAAACCACGATCGCCATCA
ACTCCTCCTCCCGTGGTTCAGATGCCTGTAACATTACAACCTCAAACGCAGGTTAGACAAGCACAAACCCCAAGAGAAAATCAAGTAGA
AAGGGACAGAGTCTCTATCCCGGCAATGCCAACTCAGATACAGTATCCACAATATCAGCCGGTAGAAAATAAGACCCAACCGCTGGTAG
TTTATCAATACCGGCTGCCAACCGAGCTTCAGTATCGGCCTCCTTCAGAGGTTCAATACAGACCTCAAGCGGTGTGTCCTGTGCCAAAT
AGCACGGCACCATACCAGCAACCCACAGCGATGGCGTCTAATTCACCAGCAACACAGGACGCGGCGCTGTATCCTCAGCCGCCCACTGT
GAGACTTAATCCTACAGCATCACGTAGTGGACAGGGTGGTGCACTGCATGCAGTCATTGATGAAGCCAGAAAACAGGGCGATCTTGAGG
CATGGCGGTTCCTGGTAATTTTACAACTGGTACAGGCCGGGGAAGAGACTCAAGTAGGAGCGCCTGCCCGAGCTGAGACTAGATGTGAA
CCTTTCACCATGAAAATGTTAAAAGATATAAAGGAAGGAGTTAAACAATATGGATCCAACTCCCCTTATATAAGAACATTATTAGATTC
CATTGCTCATGGAAATAGACTTACTCCTTATGACTGGGAAATTTTGGCCAAATCTTCCCTTTCATCCTCTCAGTATCTACAGTTTAAAA
CCTGGTGGATTGATGGAGTACAAGAACAGGTACGAAAAAATCAGGCTACTAAGCCCACTGTTAATATAGACGCAGACCAATTGTTAGGA
ACAGGTCCAAATTGGAGCACCATTAACCAACAATCAGTGATGCAGAATGAGGCTATTGAACAAGTAAGGGCTATTTGCCTCAGGGCCTG
GGGAAAAATTCAGGACCCAGGAACAGCTTTCCCTATTAATTCAATTAGACAAGGCTCTAAAGAGCCATATCCTGACTTTGTGGCAAGAT
TACAAGATGCTGCTCAAAAGTCTATTACAGATGACAATGCCCGAAAAGTTATTGTAGAATTAATGGCCTATGAAAATGCAAATCCAGAA
TGTCAGTCGGCCATAAAGCCATTAAAAGGAAAAGTTCCAGCAGGAGTTGATGTAATTACAGAATATGTGAAGGCTTGTGATGGGATTGG
AGGAGCTATGCATAAGGCAATGCTAATGGCTCAAGCAATGAGGGGGCTCACTCTAGGAGGACAAGTTAGAACATTTGGGAAAAAATGTT
ATAATTGTGGTCAAATCGGTCATCTGAAAAGGAGTTGCCCAGGCTTAAATAAACAGAATATAATAAATCAAGCTATTACAGCAAAAAAT
AAAAAGCCATCTGGCCTGTGTCCAAAATGTGGAAAAGCAAAACATTGGGCCAATCAATGTCATTCTAAATTTGATAAAGATGGCAACC
ATTGTCTGGAAACAGGAAGAGGGGCCAGCCTCAGGCCCCCCAACAAACTGGGGCATTCCCAGTTAAACTGTTTGTTCCTCAGGGTTTTC
AAGGACAACAACCCCTACAGAAAATACCACCACTTCAGGGAGTCAGCCAATTACAACAATCCAACAGCTGTCCCGCGCCACAGCAGGCA
GCACCGCAG
**SEQ ID 58** 
TGGGCAACCATTGTCTGGAAACAGGAAGAGGGGCCAGCCTCAGGCCCCCAACAAACTGGGGCATTCCCAGTTAAACTGTTTGTTCCTC
AGGGTTTTCAAGGACAACAACCCCTACAGAAAATACCACCACTTCAGGGAGTCAGCCAATTACAACAATCCAACAGCTGTCCCGCGCCA
CAGCAGGCAGCACCGCAGTAGATTTATGTTCCACCCAAATGGTCTTTTTACTCCCTGGAAAGCCCCCACAAAAGATTCCTAGAGGGGTA
TATGGCCCGCTGCCAGAAGGGAGGGTAGGCCTTTGAGGGAGATCAAGTCTAAATTTGAAGGGAGTCCAAATTCATACTGGGGTAATTTA
TTCAGATTATAAAGGGGGAATTCAGTTAGTGATCAGCTCCACTGTTCCCCGGAGTGCCAATCCAGGTGATAGAATTGCTCAATTACTGC
TTTTGCCTTATGTTAAAATTGGGGAAAACAAAAAGGAAAGAACAGGAGGGTTTGGAAGTACCAACCCTGCAGGAAAAGCTGCTTATTGG
GCTAATCAGGTCTCAGAGGATAGACCCGTGTGTACAGTCACTATTCAGGGAAAGAGTTTGAAGGATTAGTGGATACCCAGGCTGATGTT
TCTGTCATCGGCATAGGTACTGCCTCAGAAGTGTATCAAAGTGCCATGATTTTACATTGTCCAGGATCTGATAATCAAGAAAGTACGGT
TCAGCCTGTGATCACTTCATTCCAATCAATTTATGGGGCCGAGACTTGTTACAACAATGGCATGCAGAGATTACTATCCCAGCCTCCCT
ATACAGCCCCCAGGAATAAAAAAATCATGACTAAAATGGGATAGCTCCCTAAAAAGGGACTAGGAAAGAAGTCCCAATTGAGGCTGAAAA
AAATCAAAAAAGAAAAGGAATAGGGCATCCTTTTTAG
**SEQ ID 59**
   WATIVWKQEEGPASGPPTNWGIPS
**SEQ ID 60**
   TVCSSGFSRTTTPTENTTTSGSQPITTIQQLSRATAGSTAVDLCSTQMVFLLPGKPPQKIPRGVYGPLPEGRVGL
**SEQ ID 61** 
GRSSLNLKGVQIHTGVIYSDYKGGIQLVISSTVPRSANPGDRIAQLLLLPYVKIGENKKERTGGFGSTNPAGKAAYWANQVSEDRPVCT
VTIQGKSLKDVDTQADVSVIGIGTASEVYQSAMILHCPGSDNQESTVQPVITSFIPINLWGRDLLQQWHAEITIPASLYSPRNKKIMTK
MG
**SEQ ID 62**
   LPKKGLGKKEVPIEAEKNQKRKGIGHPF
**SEQ ID 63** 
ACATCCAGAATTAGGAAGCTTACTGTGGCCTCACACCACATTAGAATTTGTTCTGGAAATCAAGCTATAGGAACAAGAGATCGTAAGTC
ATATTATACTATCAACCTAAATTCCAGTCTGACAATTCCTTTGCAAAATTGTGTAAAACTCCCTTATATTGCTAGTTGTAGGAAAAACA
TAGTTATTAAACCTGATTCCCAAACCATAATCTGTGAAAATTGTGGAATGTTTACTTGCATTGATTTGACTTTTAATTGGCAGCACCGT
ATTCTACTAGGAAGAGCAAGAGAGGGTGTGTGGATCCTTGTGTCCATGGACCGACCATGGGAGGCTTCGCTATCCATCCATATTTTAAC
GGAAGTATTAAAAGGAATTCTAACTAGATCCAAAAGATTCATTTTTACTTTGATGGCAGTGATTATGGGCCTCATTGCAGTCACAGCTA
CTGCTGCGGCTGCTGGAATTGCTTTACACTCCTCTGTTCAAACTGCAGAATACGTAAATGATTGGCAAAAGAATTCCTCAAAATTGTGG
AATTCTCAGATCCAAATAGATCAAAAATTGGCAAACCAAATTAATGATCTTAGACAAACTGTCATTTGGATGGGAGAGGCTCATGAGCT
TGGAATATCTTTTTCAGTTACGATGTGA
**SEQ ID 64** 
TSRIRKLTVASHHIRICSGNQAIGTRDRKSYYTINLNSSLTIPLQNCVKLPYIASCRKNIVIKPDSQTIICENCGMFTCIDLTFNWQHR
ILLGRAREGVWILVSMDRPWEASLSIHILTEVLKGILTRSKRFIFTLMAVIMGLIAVTATAAAAGIALHSSVQTAEYVNDWQKNSSKLW
NSQIQIDQKLANQINDLRQTVIWMGEAHELGISFSVTM
**SEQ ID 65**
   HPELGSLLWPHTTLEFVLEIKL
**SEQ ID 66**
   EQEIVSHIILST
**SEQ ID 67**
   IPV
**SEQ ID 68**
   QFLCKIV
**SEQ ID 69**
   NSLILLWGKT
**SEQ ID 70**
   LLNLIPKP
**SEQ ID 71**
   SVKIVECLLALI

**SEQ ID 72**
   LLIGSTVFY
**SEQ ID 73**
   EEQERVCGSLCPWTDHGRLRYPSIF
**SEQ ID 74**
   RKY
**SEQ ID 75**
   KEF
**SEQ ID 76**
   LDPKDSFLL
**SEQ ID 77**
   WQ
**SEQ ID 78**
   LWASLQSQLLLRLLELLYTPLFKLQNT
**SEQ ID 79**
   MIGKRIPQNCGILRSK
**SEQ ID 80**
   IKNWQTKLMILDKLSFGWERLMSLEYLFQLRC
**SEQ ID 81** 
GTACAAAACAATGAATTTTGAACTATGATAGACTGGGTCCCTTGAGGCCAATTATATCATAACTGTACAGGCCAGACTCATTCATGTTC
ACAGGCCCCATCCATCTGGCCCATTAATCCAGCCTATGACGGTGATGTAACTGAAAGGCTGGACCAGGTTTATAGAAGGTTAGAATCAC
TCTGTCCAAGGAAATGGGGTGAAAAGGGAATTTCATCACCTTGACCAAAGTTAGTCCTGTTACTGGTCCTG
**SEQ ID 82**
   VQNNEF
**SEQ ID 83**
   TMIDWVP
**SEQ ID 84**
   GQLYHNCTGQTHSCSQAPSIWPINPAYDGDVTERLDQVYRRLESLCPRKWGEKGISSP
**SEQ ID 85**
   PKLVLLLVL
**SEQ ID 86** 
ATGTCAAATTTGTTCTCTTTCTTAAGAGGGGACTCGGAATTAAATAGTGAAAGAACGTTAACTCCAGAGGCAACTAAAGAAATTAAATT
AATTGAAGAAAAAATTCGGTCAGCACAAGTAAATAGAATAGATCACTTGGCCCCACTCCAAATTTTGATTTTGCTACTGCACATTCCC
TAACAGGCATCATTGTTCAAAATACAGATCTTGTGGAGTGGTCCTTCCTTCCTCACAGTACAATTAAGACTTTTACATTGTACTTGGAT
CAAATGGCTACATTAATTGGTCAGGGAAGATTATGAATAATAACATTGTGTGGAAATGACCCAGATAAAATCACTGTTCCTTTCAACAA
GCAACAGGTTAGACAAGCCTTTATCAATTCTGGTGCATGGCAGATTGGTCTTGCCGATTTTGTGGGAATTATTGACAATCGTTACCCCA
AAACAAAAATCTTCCAGTTTTTAAAATTGACTACTTGGATTTTACCTAAAGTTACCAAACATAAGCCTTTAAAAAATGCTCTGGCAGTG
TTTACTGATGGTTCCAGCAATGGAAAAGTGGCTTACACCGGGCCAAAAGAATGAGTCATCAAAACTCAGTATCACTTGACTCAAAGAGC
AGAGTTGGTTGCCGTCATTACAGTGTTAACAAGATTTTAATCAGTCTATTAACATTGTATCAGATTCTGCATATGTAGTACAGGCTACA
AAGGATATTGAGAGAGCCCTAATCAAATACATTATGGATGATCAGTTAAACCCGCTGTTTAATTTGTTACAACAAAATGTAAGAAAAAG
AAATTTCCCATTTTATATTACTCATATTCGAGCACACACTAATTTACCAGGGCCTTTAACTAAAGCAAATGAACAAGCTGACTTGCTAG
TATCATCTGCATTCATGGAAGCACAAGAACTTCATGCCTTGACTCATGTAAATGCAATAGGATTAAAAAATAAATTTGATATCACATGG
AAACAGACAAAAAATATTGTACAACATTGCACCCAGTGTCAGATTCTACACCTGGCCACTCAGGAGGCAAGAGTTAATCCCAGAGGTCT
ATGTCCTAATGTGTTATGGCAAATGGATGTCATGCACGTACCTTCATTTGGAAAATTGTCATTTGTCCATGTGACAGTTGATACTTATT
CACATTTCATATGGGCAACCTGCCAGACAGGAGAAAGTACTTCCCATGTTAAAAGACATTTATTATCTTGTTTTCCTGTCATGGGAGTT
CCAGAAAAAGTTAAAACAGACAATGGGCCAGGTTACTGTAGTAAAGCAGTTCAAAAATTCTTAAATCAGTGGAAAATTACACATACAAT
AGGAATTCTCTATAATTCCCAAGGACAGGCCATAATTGAAAGAACTAATAGAACACTCAAAGCTCAATTGGTTAAACAAAAAAAAGGAA
AAGACAGGAGTATAACACTCCCCAGATGCAACTTAATCTAGCACTCTATACTTTAAATGTTTTAAACATTTATAGAAATCAGACCACTA
CCTCTGCAGAACAACATCTTACTGGTAAAAGGAACAGCCCACATGAAGGAAAACTGATTTGGTGGAAAGATAATAAAAATAAAACATGG
GAAATGGGGAAGGTGATAACGTGGGGGAGAGGTTTTGCTTGTGTTTCACCAGGAGAAAATCAGCTTCCTGTTTGGATACCCACTAGACA
 
TTTAAAGTTCTACAATGAACTCACTGGAGATGCAAAGAAAAGTGTGGAGATGGAGACACCCCAATCGACTCGCCAGGTAAACAAAATGG
TGATATCAGAAGAACAGAAAAAGTTGCCTTCCATCAAGGAAGCAGAGTTGCCAATATAG
**SEQ ID 87**
   MNSLEMQRKVWRWRHPNRLASLQVYPAAPKRQQPARMGHSDDGGFVKKKRGGYVRKREIRLSLCLCRKGRHKKLHFDLY
**SEQ ID 88** 
ATGAACTCACTGGAGATGCAAAGAAAAGTGTGGAGATGGAGACACCCCAATCGACTCGCCAGTCTACAGGTGTATCCAGCAGCTCCAAA
GAGACAGCAACCAGCAAGAATGGGCCATAGTGACGATGGTGGTTTTGTCAAAAAGAAAAGGGGGGGATATGTAAGGAAAAGAGAGATCA
GACTTTCACTGTGTCTATGTAGAAAAGGAAGACATAAGAAACTCCATTTTGATCTGTAC
**SEQ ID 89** 
GGCCCACTATTGTACAAATTGATTGTAAAACATGTTCACATGTGTTGAACAATATNAAATCAGGGCCCNTTGAAAATGAACAGAATAAC
AGTGATTTTAGGGAACAAAGGAAGNCANCCATAAGGTCTGCCTGCCTGAGGGGTCGGCAAAAACCCATATTTTTNTTNTTGCAGAGAG
CCTATAAATGGACGTGCAAGTAGGANAGANATTGCTAAATTCTTTTCCTAGCAAGGAATATAATACTAAGNCCCTAGGGAAAGAATTGC
ATTCCTGGGGGGAGGTNTATAAACGGCCGCTNTGGGAGTGTCTGTCCTATGTGGTTGANANAAGGACTGANANACGCCCTGGTCGCCTG
CAGTACCCTCAGGCTTACTAGGATTGGGAAACCCCAGTCCTGGTAAATTTGAGGTCAGGCCGGTTNTTTGCTNTGAACCCTGTTTTNTG
TTAANATGTTTATCAAGACAATACGTGCNCCGCTGAACATAGACCCTTATCAGGAGTTTNTGATTTTGCTCTGGTCCTGTTTNTTCANA
AGCATGTCATCTTTGCTCTGCCTTNTGCCCTTTGAAGCATGTGATCTTTGTGACCTACTCCCTGTTCATACACCCCTCCCCTTTTAAAA
TCCCTAATAAAAACTTGCTGGTTTTGTGGCTCAGGGGGGCATCATGGACCTACCAATACGTGATGTCACCCCCGGTGGCCCAGCTGTAA
AAAAAAAAAAA
**SEQ ID 90** 
CNNGNNCCAAAATTTTGATTTTGNAAAAAAAAATTTTTCCCCCATTGTGGTTTTGGNCCCAAATAATNAAAAACCCGGNGGCCCCNTTT
GAAAAAATGGNCNCANAAATTNCCCGGGTNTTTTTTAGGGAACAAANGGGAAGCCCCCCCAATAAGGGTNTNNCCTCCCNTGAGGGGG
TGGGGGAAAAAAACCCAATTTTTTTTTTTTTGCNNNAGAGCCTTAAAAATGGCGGTGCAAGTAGNAAAAANATTGNTAAATTNTTTTC
CCAGCAAGGAANATAATTNTAAGCCCCTAGGGAAAAAATTGCNTTNTTGGGGGGAGGTTTATAAACGNCCNCTCTGGGAGTGTNTGTCN
TATGTGGTTGANANAAGGATTNAAANACNCCNTGGTCGCCTGNAGNACCCTCNGGCTTATTAGGATTGGGAAACCCCAGTCNTGGTAAA
TTTNAGGTCNGGCNGGTTTTTNNTTTNANCCCTGTTTTTGTTAANATNTTTATCAANACAANANGNGCCCCGNTGAANANANACCCT
TATCAGGAGTTTNTGATTTTGCTCGGGTCCTGTTTNTTCAAAAGCATGTCATTTTTGNTTTGCCTTNTGCCCTTTNAAGNATGTGATNT
TTGTGACCTACTCCCTGTTCANACNCCCCNCCCCTTTTAAAATCCCTAATAAAAACTTGCTGGTTTTGTGGCTCAGGGGGGCATCATGG
ACCTACCAANACGTGATGTCNCCCCCGGTGGCCCAGCTGTAAAAAAAAAAAAA
**SEQ ID 91** 
ANCCCANTTTTTGGTNNCAAAATTTGAATTGTAAAAAACAATGGTTNCCACCATTGTTGTTTTGGANCCAAATATTGNAAANTCCAGNG
GCNCCCTTNGAAAAATGGAANCANGAAATAANCCAGGTGNTTTTTAGGGGGAACCAAAAGGAAAGNCCACCCCATAAAGGGTNTTGAC
TTGCCTTGAGGGGGTCGGGGGCAAAAAAAGCCAATATTTTTNTTTNTTTGCAGAGAGCCTATAAATGGACGTGCAAAGTAGGAAAAATA
TTGCTAAATTCTTTTCCTAGCAAGGAATATAATATTAAGNCCCTAGGAAAGAATTGCATTCCTGGGGGGAGGTCTATAAACGGCCGCTC
TGGGAGTGTCTGTCCTATGTGGTTAAGANAAGGATTGAGANACGCCCCTGGTCGCCTGCAGTACCCTCAGGCTTANTAGGATTGGGAAA
CCCCAGTCCTGGTAAATTTGAGGTCAGGCCGGTTNTTTGCTTTGAACCCTGTTTTNTGTTAANATGTTTATCAAGACAANACGTGCNCC
GCTGAACANANACCCTTATCAGGAGTTTCTGATTTGCTCTGGTCCTGTTTNTTCANAAGCATGTCATCTTTGCTNTGCCTTCTGCCCT
TTGAAGCATGTGATNTTTGTGACCTACTCCCTGTTCATACACCCCTCCCCTTTTAAAATCCCTAATAAAAACTTGCTGGTTTTGTGGCT
CAGGGGGGCATCATGGACCTACCAATACGTGATGTCACCCCCGGTGGCCCAGCTGTAAAAAAAAAAAA
**SEQ ID 92**
   ESSKLSIT
**SEQ ID 93**
   LKEQSWLPSLQC
**SEQ ID 94** 
QDFNQSINIVSDSAYVVQATKDIERALIKYIMDDQLNPLFNLLQQNVRKRNFPFYITHIRAHTNLPGPLTKANEQADLLVSSAFMEAQE
LHALTHVNAIGLKNKFDITWKQTKNIVQHCTQCQILHLATQEARVNPRGLCPNVLWQMDVMHVPSFGKLSFVHVTVDTYSHFIWATCQT
GESTSHVKRHLLSCFPVMGVPEKVKTDNGPGYCSKAVQKFLNQWKITHTIGILYNSQGQAIIERTNRTLKAQLVKQKKGKDRSITLPRC
NLI
**SEQ ID 95**
**SEQ ID 96** 
IITLCGNDPDKITVPFNKQQVRQAFINSGAWQIGLADFVGIIDNRYPKTKIFQFLKLTTWILPKVTKHKPLKNALAVFTDGSSNGKVAY
TGPKE
**SEQ ID 97** 
TKKRKRQEYNTPQMQLNLALYTLNVLNIYRNQTTTSAEQHLTGKRNSPHEGKLIWWKDNKNKTWEMGKVITWGRGFACVSPGENQLPVW
IPTRHLKFYNELTGDAKKSVEMETPQSTRQVNKMVISEEQKKLPSIKEAELPI
 **SEQ ID 98**
   MNSLEMQRKVWRWRHPNRLASLQVYPAAPKRQQPARMGHSDDGGFVKKKRGGYVRKREIRLSLCLCRKGRHKKLHFVLY
**SEQ ID 99** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACTCTACAG
TGTGGTCATTGAGGACAAGTTGACGAGAGAGTCCCAAGTACGTCCACGGTCAGCCTTGCGACATTTAAAGTTCTACAATGAACTCACTG
GAGATGCAAAGAAAAGTGTGGAGATGGAGACACCCCAATCGACTCGCCAGTCTACAGGTGTATCCAGCAGCTCCAAAGAGACAGCAACC
AGCAAGAATGGGCCATAGTGACGATGGTGGTTTGTCAAAAAGAAAAGGGGGGGATATGTAAGGAAAAGAGAGATCAGACTTTCACTGT
GTCTATGTAGAAAAGGAAGACATAAGAAACTCCATTTTGATCTGTACTAAGAAAAATTGTTTTGCCTTGAGATGCTGTTAATCTGTAAC
TTTAGCCCCAACCCTGTGCTCACGGAAACATGTGCTGTAAGGTTTAAGGGATCTAGGGCTGTGCAGGATGTACCTTGTTAACAATATGT
TTGCAGGCAGTATGTTTGGTAAAAGTCATCGCCATTCTCCATTCTCGATTAACCAGGGGCTCAATGCACTGTGGAAAGCCACAGGAACC
TCTGCCCAAGAAAGCCTGGCTGTTGTGGGAAGTCAGGGACCCCGAATGGAGGGACCAGCTGGTGCTGCATCAGGAAACATAAATTGTGA
AGATTTCTTGGACATTTATCAGTTTCCAAAATTAATACTTTTATAATTTCTTACACCTGTCTTACTTTAATCTCTTAATCCTGTTATCT
TTGTAAGCTGAGGATATACGTCACCTCAGGACCACTATTGTACAAATTGATTGTAAAACATGTTCACATGTGTTTGAACAATATGAAAT
CAGTGCACCTTGAAAATGAACAGAATAACAGTGATTTTAGGGAACAAAGGAAGACAACCATAAGGTCTGACTGCCTGAGGGGTCGGGCA
AAAAGCCATATTTTTCTTCTTGCAGAGAGCCTATAAATGGACGTGCAAGTAGGAGAGATATTGCTAAATTCTTTTCCTAGCAAGGAATA
TAATACTAAGACCCTAGGGAAAGAATTGCATTCCTGGGGGGAGGTCTATAAACGGCCGCTCTGGGAGTGTCTGTCCTATGTGGTTGAGA
TAAGGACTGAGATACGCCCTGGTCTCCTGCAGTACCCTCAGGCTTACTAGGATTGGGAAACCCCAGTCCTGGTAAATTTGAGGTCAGGC
CGGTTCTTTGCTCTGAACCCTGTTTTCTGTTAAGATGTTTATCAAGACAATACATGCACCGCTGAACATAGACCCTTATCAGGAGTTTC
TGATTTTGCTCTGGTCCTGTTTCTTCAGAAGCATGTCATCTTTGCTCTGCCTTCTGCCCTTTGAAGCATGTGATCTTTGTGACCTACTC
CCTGTTCATACACCCCTCCCCTTTTAAAATCCCTAATAAAAACTTGCTGGTTTTGTGGCTCAGGGGGGCATCATGGACCTACCAATACG
TGATGTCACCCCCGGTGGCCCAGCTGTAAAA
**SEQ ID 100** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACTCTACAG
TGTGGTCATTGAGGACAAGTTGACGAGAGAGTCCCAAGTACGTCCACGGTCAGCCTTGCGGAGAAAATCAGCTTCCTGTTTGGATACCC
ACTAGACATTTAAAGTTCTACAATGAACTCACTGGAGATGCAAAGAAAAGTGTGGAGATGGAGACACCCCAATCGACTCGCCAGTCTAC
AGGTGTATCCAGCAGCTCCAAAGAGACAGCAACCAGCAAGAATGGGCCATAGTGACGATGGTGGTTTTGTCAAAAAGAAAAGGGGGGGA
TATGTAAGGAAAAGAGAGATCAGACTTTCACTGTGTCTATGTAGAAAAGGAAGACATAAGAAACTCCATTTTGATCTGTACTAAGAAAA
ATTGTTTTGCCTTGAGATGCTGTTAATCTGTAACTTTAGCCCCAACCCTGTGCTCACGGAAACATGTGCTGTAAGGTTTAAGGATCTA
GGGCTGTGCAGGATGTACCTTGTTAACAATATGTTTGCAGGCAGTATGTTTGGTAAAAGTCATCGCCATTCTCCATTCTCGATTAACCA
GGGGCTCAATGCACTGTGGAAAGCCACAGGAACCTCTGCCCAAGAAAGCCTGGCTGTTGTGGGAAGTCAGGGACCCCGAATGGAGGGAC
CAGCTGGTGCTGCATCAGGAAACATAAATTGTGAAGATTTCTTGGACATTTATCAGTTTCCAAAATTAATACTTTTATAATTTCTTACA
CCTGTCTTACTTTAATCTCTTAATCCTGTTATCTTTGTAAGCTGAGGATATACGTCACCTCAGGACCACTATTGTACAAATTGATTGTA
AAACATGTTCACATGTGTTTGAACAATATGAAATCAGTGCACCTTGAAAATGAACAGAATAACAGTGATTTAGGGAACAAAGGAAGAC
AACCATAAGGTCTGACTGCCTGAGGGGTCGGGCAAAAAGCCATATTTTTCTTCTTGCAGAGAGCCTATAAATGGACGTGCAAGTAGGAG
 
AGATATTGCTAAATTCTTTTCCTAGCAAGGAATATAATACTAAGACCCTAGGGAAAGAATTGCATTCCTGGGGGGAGGTCTATAAACGG
CCGCTCTGGGAGTGTCTGTCCTATGTGGTTGAGATAAGGACTGAGATACGCCCTGGTCTCCTGCAGTACCCTCAGGCTTACTAGGATTG
GGAAACCCCAGTCCTGGTAAATTTGAGGTCAGGCCGGTTCTTTGCTCTGAACCCTGTTTTCTGTTAAGATGTTTATCAAGACAATACAT
GCACCGCTGAACATAGACCCTTATCAGGAGTTTCTGATTTTGCTCTGGTCCTGTTTCTTCAGAAGCATGTCATCTTTGCTCTGCCTTCT
GCCCTTTGAAGCATGTGATCTTTGTGACCTACTCCCTGTTCATACACCCCTCCCCTTTTAAAATCCCTAATAAAAACTTGCTGGTTTTG
TGGCTCAGGGGGGCATCATGGACCTACCAATACGTGATGTCACCCCCGGTGGCCCAGCTGTAAAA
**SEQ ID 101** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTCTTTACCTTGTTTATGATGCAGAGACATTTGTTCACATGTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACTCTACAG
TGTGGTCATTGAGGACAAGTTGACGAGAGAGTCCCAAGTACGTCCACGGTCAGCCTTGCGTCTACAGGTGTATCCAGCAGCTCCAAAGA
GACAGCAACCAGCAAGAATGGGCCATAGTGACGATGGTGGTTTTGTCAAAAAGAAAAGGGGGGGATATGTAAGGAAAAGAGAGATCAGA
CTTTCACTGTGTCTATGTAGAAAAGGAAGACATAAGAAACTCCATTTTGATCTGTACTAAGAAAAATTGTTTTGCCTTGAGATGCTGTT
AATCTGTAACTTTAGCCCCAACCCTGTGCTCACGGAAACATGTGCTGTAAGGTTTAAGGGATCTAGGGCTGTGCAGGATGTACCTTGTT
AACAATATGTTTGCAGGCAGTATGTTTGGTAAAAGTCATCGCCATTCTCCATTCTCGATTAACCAGGGGCTCAATGCACTGTGGAAAGC
CACAGGAACCTCTGCCCAAGAAAGCCTGGCTGTTGTGGAAGTCAGGGACCCCGAATGGAGGGACCAGCTGGTGCTGCATCAGGAAACA
TAAATTGTGAAGATTTCTTGGACATTTATCAGTTTCCAAAATTAATACTTTTATAATTTCTTACACCTGTCTTACTTTAATCTCTTAAT
CCTGTTATCTTTGTAAGCTGAGGATATACGTCACCTCAGGACCACTATTGTACAAATTGATTGTAAAACATGTTCACATGTGTTTGAAC
AATATGAAATCAGTGCACCTTGAAAATGAACAGAATAACAGTGATTTAGGGAACAAAGGAAGACAACCATAAGGTCTGACTGCCTGAG
GGGTCGGGCAAAAAGCCATATTTTTCTTCTTGCAGAGAGCCTATAAATGGACGTGCAAGTAGGAGAGATATTGCTAAATTCTTTTCCTA
GCAAGGAATATAATACTAAGACCCTAGGGAAAGAATTGCATTCCTGGGGGGAGGTCTATAAACGGCCGCTCTGGGAGTGTCTGTCCTAT
GTGGTTGAGATAAGGACTGAGATACGCCCTGGTCTCCTGCAGTACCCTCAGGCTTACTAGGATTGGGAAACCCCAGTCCTGGTAAATTT
GAGGTCAGGCCGGTTCTTTGCTCTGAACCCTGTTTTCTGTTAAGATGTTTATCAAGACAATACATGCACCGCTGAACATAGACCCTTAT
CAGGAGTTTCTGATTTTGCTCTGGTCCTGTTTCTTCAGAAGCATGTCATCTTTGCTCTGCCTTCTGCCCTTTGAAGCATGTGATCTTTG
TGACCTACTCCCTGTTCATACACCCCTCCCCTTTTAAAATCCCTAATAAAAACTTGCTGGTTTTGTGGCTCAGGGGGGCATCATGGACC
TACCAATACGTGATGTCACCCCCGGTGGCCCAGCTGTAAAA
**SEQ ID 102** 
GAGATAGGAGAAAACTGCCTTAGGGCTGGAGGTGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGTCTACAGGTGTATCCAGCAGCTCCAAAG
AGACAGCAACCAGCAAGAATGGGCCATAGTGACGATGGTGGTTTTGTCAAAAAGAAAAGGGGGGGATATGTAAGGAAAAGAGAGATCAG
ACTTTCACTGTGTCTATGTAGAAAAGGAAGACATAAGAAACTCCATTTTGATCTGTACTAAGAAAAATTGTTTTGCCTTGAGATGCTGT
TAATCTGTAACTTTAGCCCCAACCCTGTGCTCACGGAAACATGTGCTGTAAGGTTTAAGGGATCTAGGGCTGTGCAGGATGTACCTTGT
TAACAATATGTTTGCAGGCAGTATGTTTGGTAAAAGTCATCGCCATTCTCCATTCTCGATTAACCAGGGGCTCAATGCACTGTGGAAAG
CCACAGGAACCTCTGCCCAAGAAAGCCTGGCTGTTGTGGGAAGTCAGGGACCCCGAATGGAGGGACCAGCTGGTGCTGCATCAGGAAAC
ATAAATTGTGAAGATTTCTTGGACATTTATCAGTTTCCAAAATTAATACTTTTATAATTTCTTACACCTGTCTTACTTTAATCTCTTAA
TCCTGTTATCTTTGTAAGCTGAGGATATACGTCACCTCAGGACCACTATTGTACAAATTGATTGTAAAACATGTTCACATGTGTTTGAA
CAATATGAAATCAGTGCACCTTGAAAATGAACAGAATAACAGTGATTTAGGGAACAAAGGAAGACAACCATAAGGTCTGACTGCCTGA
GGGGTCGGGCAAAAAGCCATATTTTTCTTCTTGCAGAGAGCCTATAAATGGACGTGCAAGTAGGAGAGATATTGCTAAATTCTTTTCCT
AGCAAGGAATATAATACTAAGACCCTAGGGAAAGAATTGCATTCCTGGGGGGAGGTCTATAAACGGCCGCTCTGGGAGTGTCTGTCCTA
TGTGGTTGAGATAAGGACTGAGATACGCCCTGGTCTCCTGCAGTACCCTCAGGCTTACTAGGATTGGGAAACCCCAGTCCTGGTAAATT
TGAGGTCAGGCCGGTTCTTTGCTCTGAACCCTGTTTTCTGTTAAGATGTTTATCAAGACAATACATGCACCGCTGAACATAGACCCTTA
TCAGGAGTTTCTGATTTTGCTCTGGTCCTGTTTCTTCAGAAGCATGTCATCTTTGCTCTGCCTTCTGCCCTTTGAAGCATGTGATCTTT
GTGACCTACTCCCTGTTCATACACCCCTCCCCTTTTAAAATCCCTAATAAAAACTTGCTGGTTTTGTGGCTCAGGGGGGCATCATGGAC
CTACCAATACGTGATGTCACCCCCGGTGGCCCAGCTGTAAA
18/72
**SEQ ID 103** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGACATTTAAAGTTCTACAATGAACTCACT
GGAGATGCAAAGAAAAGTGTGGAGATGGAGACACCCCAATCGACTCGCCAGTCTACAGGTGTATCCAGCAGCTCCAAAGAGACAGCAAC
CAGCAAGAATGGGCCATAGTGACGATGGTGGTTTTGTCAAAAAGAAAAGGGGGGGATATGTAAGGAAAAGAGAGATCAGACTTTCACTG
TGTCTATGTAGAAAAGGAAGACATAAGAAACTCCATTTTGATCTGTACTAAGAAAAATTGTTTTGCCTTGAGATGCTGTTAATCTGTAA
CTTTAGCCCCAACCCTGTGCTCACGGAAACATGTGCTGTAAGGTTTAAGGGATCTAGGGCTGTGCAGGATGTACCTTGTTAACAATATG
TTTGCAGGCAGTATGTTTGGTAAAAGTCATCGCCATTCTCCATTCTCGATTAACCAGGGGCTCAATGCACTGTGGAAAGCCACAGGAAC
CTCTGCCCAAGAAAGCCTGGCTGTTGTGGGAAGTCAGGGACCCCGAATGGAGGGACCAGCTGGTGCTGCATCAGGAAACATAAATTGTG
AAGATTTCTTGGACATTTATCAGTTTCCAAAATTAATACTTTTATAATTTCTTACACCTGTCTTACTTTAATCTCTTAATCCTGTTATC
TTTGTAAGCTGAGGATATACGTCACCTCAGGACCACTATTGTACAAATTGATTGTAAAACATGTTCACATGTGTTTGAACAATATGAAA
TCAGTGCACCTTGAAAATGAACAGAATAACAGTGATTTTAGGGAACAAAGGAAGACAACCATAAGGTCTGACTGCCTGAGGGGTCGGGC
AAAAAGCCATATTTTTCTTCTTGCAGAGAGCCTATAAATGGACGTGCAAGTAGGAGAGATATTGCTAAATTCTTTTCCTAGCAAGGAAT
ATAATACTAAGACCCTAGGGAAAGAATTGCATTCCTGGGGGGAGGTCTATAAACGGCCGCTCTGGGAGTGTCTGTCCTATGTGGTTGAG
ATAAGGACTGAGATACGCCCTGGTCTCCTGCAGTACCCTCAGGCTTACTAGGATTGGGAAACCCCAGTCCTGGTAAATTTGAGGTCAGG
CCGGTTCTTTGCTCTGAACCCTGTTTTCTGTTAAGATGTTTATCAAGACAATACATGCACCGCTGAACATAGACCCTTATCAGGAGTTT
CTGATTTTGCTCTGGTCCTGTTTCTTCAGAAGCATGTCATCTTTGCTCTGCCTTCTGCCCTTTGAAGCATGTGATCTTTGTGACCTACT
CCCTGTTCATACACCCCTCCCCTTTTAAAATCCCTAATAAAAACTTGCTGGTTTTGTGGCTCAGGGGGGCATCATGGACCTACCAATAC
GTGATGTCACCCCCGGTGGCCCAGCTGTAAAA
**SEQ ID 104** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGGAGAAAATCAGCTTCCTGTTTGGATACC
CACTAGACATTTAAAGTTCTACAATGAACTCACTGGAGATGCAAAGAAAAGTGTGGAGATGGAGACACCCCAATCGACTCGCCAGTCTA
CAGGTGTATCCAGCAGCTCCAAAGAGACAGCAACCAGCAAGAATGGCCATAGTGACGATGGTGGTTTTGTCAAAAAGAAAAGGGGGGG
ATATGTAAGGAAAAGAGAGATCAGACTTTCACTGTGTCTATGTAGAAAAGGAAGACATAAGAAACTCCATTTTGATCTGTACTAAGAAA
AATTGTTTTGCCTTGAGATGCTGTTAATCTGTAACTTTAGCCCCAACCCTGTGCTCACGGAAACATGTGCTGTAAGGTTTAAGGGATCT
AGGGCTGTGCAGGATGTACCTTGTTAACAATATGTTTGCAGGCAGTATGTTTGGTAAAAGTCATCGCCATTCTCCATTCTCGATTAACC
AGGGGCTCAATGCACTGTGGAAAGCCACAGGAACCTCTGCCCAAGAAAGCCTGGCTGTTGTGGGAAGTCAGGGACCCCGAATGGAGGGA
CCAGCTGGTGCTGCATCAGGAAACATAAATTGTGAAGATTTCTTGGACATTTATCAGTTTCCAAAATTAATACTTTTATAATTTCTTAC
ACCTGTCTTACTTTAATCTCTTAATCCTGTTATCTTTGTAAGCTGAGGATATACGTCACCTCAGGACCACTATTGTACAAATTGATTGT
AAAACATGTTCACATGTGTTTGAACAATATGAAATCAGTGCACCTTGAAAATGAACAGAATAACAGTGATTTAGGGAACAAAGGAAGA
CAACCATAAGGTCTGACTGCCTGAGGGGTCGGGCAAAAAGCCATATTTTTCTTCTTGCAGAGAGCCTATAAATGGACGTGCAAGTAGGA
GAGATATTGCTAAATTCTTTTCCTAGCAAGGAATATAATACTAAGACCCTAGGGAAAGAATTGCATTCCTGGGGGGAGGTCTATAAACG
GCCGCTCTGGGAGTGTCTGTCCTATGTGGTTGAGATAAGGACTGAGATACGCCCTGGTCTCCTGCAGTACCCTCAGGCTTACTAGGATT
GGGAAACCCCAGTCCTGGTAAATTTGAGGTCAGGCCGGTTCTTTGCTCTGAACCCTGTTTTCTGTTAAGATGTTTATCAAGACAATACA
TGCACCGCTGAACATAGACCCTTATCAGGAGTTTCTGATTTTGCTCTGGTCCTGTTTCTTCAGAAGCATGTCATCTTTGCTCTGCCTTC
TGCCCTTTGAAGCATGTGATCTTTGTGACCTACTCCCTGTTCATACACCCCCTCCCCTTTTAAAATCCCTAATAAAAACTTGCTGGTTTT
GTGGCTCAGGGGGGCATCATGGACCTACCAATACGTGATGTCACCCCCGGTGGCCCAGCTGTAAAA
**SEQ ID 105** 
GAGATAGGAGAAAACTGCCTTAGGGCTGGAGGTGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCAC[?][?][?][?][?]TTTCTCTATACTTTGTCTCTGTTGTCTTTCTTTTCTCAAGTCTC
 
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGACATTTAAAGTTCTACAATGAACTCACT
GGAGATGCAAAGAAAAGTGTGGAGATGGAGACACCCCAATCGACTCGCCAGGTAAACAAAATGGTGATATCAGAAGAACAGAAAAAGTT
GCCTTCCATCAAGGAAGCAGAGTTGCCAATATAGGCACAATTAAAGAAGCTGACACAGTTAGCTAAAAAAAAAAGCCTAGAGAATACAA
AGGTGACACCAACTCCAGAGAATATGCTGCTTGCAGCTCTGATGATTGTATCAACGGTGTCTACAGGTGTATCCAGCAGCTCCAAAGAG
ACAGCAACCAGCAAGAATGGCCATAGTGACGATGGTGGTTTTGTCAAAAAGAAAAGGGGGGATATGTAAGGAAAAGAGAGATCAGAC
TTTCACTGTGTCTATGTAGAAAAGGAAGACATAAGAAACTCCATTTTGATCTGTACTAAGAAAAATTGTTTTGCCTTGAGATGCTGTTA
ATCTGTAACTTTAGCCCCAACCCTGTGCTCACGGAAACATGTGCTGTAAGGTTTAAGGGATCTAGGGCTGTGCAGGATGTACCTTGTTA
ACAATATGTTTGCAGGCAGTATGTTTGGTAAAAGTCATCGCCATTCTCCATTCTCGATTAACCAGGGGCTCAATGCACTGTGGAAAGCC
ACAGGAACCTCTGCCCAAGAAAGCCTGGCTGTTGTGGGAAGTCAGGGACCCCGAATGGAGGGACCAGCTGGTGCTGCATCAGGAAACAT
AAATTGTGAAGATTTCTTGGACATTTATCAGTTTCCAAAATTAATACTTTTATAATTTCTTACACCTGTCTTACTTTAATCTCTTAATC
CTGTTATCTTTGTAAGCTGAGGATATACGTCACCTCAGGACCACTATTGTACAAATTGATTGTAAAACATGTTCACATGTGTTTGAACA
ATATGAAATCAGTGCACCTTGAAAATGAACAGAATAACAGTGATTTAGGGAACAAAGGAAGACAACCATAAGGTCTGACTGCCTGAGG
GGTCGGGCAAAAAGCCATATTTTTCTTCTTGCAGAGAGCCTATAAATGGACGTGCAAGTAGGAGAGATATTGCTAAATTCTTTTCCTAG
CAAGGAATATAATACTAAGACCCTAGGGAAAGAATTGCATTCCTGGGGGGAGGTCTATAAACGGCCGCTCTGGGAGTGTCTGTCCTATG
TGGTTGAGATAAGGACTGAGATACGCCCTGGTCTCCTGCAGTACCCTCAGGCTTACTAGGATTGGGAAACCCCAGTCCTGGTAAATTTG
AGGTCAGGCCGGTTCTTTGCTCTGAACCCTGTTTTCTGTTAAGATGTTTATCAAGACAATACATGCACCGCTGAACATAGACCCTTATC
AGGAGTTTCTGATTTTGCTCTGGTCCTGTTTCTTCAGAAGCATGTCATCTTTGCTCTGCCTTCTGCCCTTTGAAGCATGTGATCTTTGT
GACCTACTCCCTGTTCATACACCCCTCCCCTTTTAAAATCCCTAATAAAAACTTGCTGGTTTTGTGGCTCAGGGGGGCATCATGGACCT
ACCAATACGTGATGTCACCCCCGGTGGCCCAGCTGTAAAA
**SEQ ID 106** 
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGGAGAAAATCAGCTTCCTGTTTGGATACC
CACTAGACATTTAAAGTTCTACAATGAACTCACTGGAGATGCAAAGAAAAGTGTGGAGATGGAGACACCCCAATCGACTCGCCAGGTAA
ACAAAATGGTGATATCAGAAGAACAGAAAAAGTTGCCTTCCATCAAGGAAGCAGAGTTGCCAATATAGGCACAATTAAAGAAGCTGACA
CAGTTAGCTAAAAAAAAAAGCCTAGAGAATACAAAGGTGACACCAACTCCAGAGAATATGCTGCTTGCAGCTCTGATGATTGTATCAAC
GGTGTCTACAGGTGTATCCAGCAGCTCCAAAGAGACAGCAACCAGCAAGAATGGGCCATAGTGACGATGGTGGTTTTGTCAAAAAGAAA
AGGGGGGGATATGTAAGGAAAAGAGAGATCAGACTTTCACTGTGTCTATGTAGAAAAGGAAGACATAAGAAACTCCATTTTGATCTGTA
CTAAGAAAAATTGTTTTGCCTTGAGATGCTGTTAATCTGTAACTTTAGCCCCAACCCTGTGCTCACGGAAACATGTGCTGTAAGGTTTA
AGGGATCTAGGGCTGTGCAGGATGTACCTTGTTAACAATATGTTTGCAGGCAGTATGTTTGGTAAAAGTCATCGCCATTCTCCATTCTC
GATTAACCAGGGGCTCAATGCACTGTGGAAAGCCACAGGAACCTCTGCCCAAGAAAGCCTGGCTGTTGTGGAAGTCAGGGACCCCGAA
TGGAGGGACCAGCTGGTGCTGCATCAGGAAACATAAATTGTGAAGATTTCTTGGACATTTATCAGTTTCCAAAATTAATACTTTTATAA
TTTCTTACACCTGTCTTACTTTAATCTCTTAATCCTGTTATCTTTGTAAGCTGAGGATATACGTCACCTCAGGACCACTATTGTACAAA
TTGATTGTAAAACATGTTCACATGTGTTTGAACAATATGAAATCAGTGCACCTTGAAAATGAACAGAATAACAGTGATTTTAGGGAACA
AAGGAAGACAACCATAAGGTCTGACTGCCTGAGGGGTCGGGCAAAAAGCCATATTTTTCTTCTTGCAGAGAGCCTATAAATGGACGTGC
AAGTAGGAGAGATATTGCTAAATTCTTTTCCTAGCAAGGAATATAATACTAAGACCCTAGGGAAAGAATTGCATTCCTGGGGGGAGGTC
TATAAACGGCCGCTCTGGGAGTGTCTGTCCTATGTGGTTGAGATAAGGACTGAGATACGCCCTGGTCTCCTGCAGTACCCTCAGGCTTA
CTAGGATTGGGAAACCCCAGTCCTGGTAAATTTGAGGTCAGGCCGGTTCTTTGCTCTGAACCCTGTTTTCTGTTAAGATGTTTATCAAG
ACAATACATGCACCGCTGAACATAGACCCTTATCAGGAGTTTCTGATTTTGCTCTGGTCCTGTTTCTTCAGAAGCATGTCATCTTTGCT
CTGCCTTCTGCCCTTTGAAGCATGTGATCTTTGTGACCTACTCCCTGTTCATACACCCCTCCCCTTTTAAAATCCCTAATAAAAACTTG
CTGGTTTTGTGGCTCAGGGGGCATCATGGACCTACCAATACGTGATGTCACCCCCGGTGGCCCAGCTGTAAAA
**SEQ ID 107** 
TTTTGCTTGTGTTTCACCAGGAGAAAATCAGCTTCCTGTTTGGATACCCACTAGACATTTAAAGTTCTACAATGAACTCACTGGAGATG
CAAAGAAAAGTGTGGAGATGGAGACACCCCAATCGACTCGCCAGGTAAACAAAATGGTGATATCAGAAGAACAGAAAAAGTTGCCTTCC
ATCAAGGAAGCAGAGTTGCCAATATAGGCACAATTAAAGAAGCTGACACAGTTAGCTAAAAAAAAAAGCCTAGAGAATACAAAGGTGAC
ACCAACTCCAGAGAATATGCTGCTTGCAGCTCTGATGATTGTATCAACGGTGGTAAGTCTTCCCAAGTCTGCAGGAGCAGCTGCAGCTA
ATTATACTTACTGGGCCTATGTGCCTTTCCCACCCTTAATT[?][?][?][?][?]CAGTTACATAGATGGATAATCCTATTGAAGTAGATGTTAATAAT
 
AGTGCATGGGTGCCTGGCCCCACAGATGACTGTTGCCCTGCCCAACCTGAAGAAGGAATGATGATGAATATTTCCATTGGGTATCCTTA
TCCTCCTGTTTGCCTAGGGAAGGCACCAGGATGCTTAATGCCTACAACCCAAAATTGTCTACAGGTGTATCCAGCAGCTCCAAAGAGAC
AGCAACCAGCAAGAATGGGCCATAGTGACGATGGTGGTTTTGTCAAAAAGAAAAGGGGGGGATATGTAAGGAAAAGAGAGATCAGACTT
TCACTGTGTCTATGTAGAAAAGGAAGACATAAGAAACTCCATTTTGATCTGTACTAAGAAAAATTGTTTTGCCTTGAGATGCTGTTAAT
CTGTAACTTTAGCCCCAACCCTGTGCTCACGGAAACATGTGCTGTAAGGTTTAAGGGATCTAGGGCTGTGCAGGATGTACCTTGTTAAC
AATATGTTTGCAGGCAGTATGTTTGGTAAAAGTCATCGCCATTCTCCATTCTCGATTAACCAGGGGCTCAATGCACTGTGGAAAGCCAC
AGGAACCTCTGCCCAAGAAAGCCTGGCTGTTGTGGGAAGTCAGGGACCCCGAATGGAGGGACCAGCTGGTGCTGCATCAGGAAACATAA
ATTGTGAAGATTTCTTGGACATTTATCAGTTTCCAAAATTAATACTTTTATAATTTCTTACACCTGTCTTACTTTAATCTCTTAATCCT
GTTATCTTTGTAAGCTGAGGATATACGTCACCTCAGGACCACTATTGTACAAATTGATTGTAAAACATGTTCACATGTGTTTGAACAAT
ATGAAATCAGTGCACCTTGAAAATGAACAGAATAACAGTGATTTTAGGGAACAAAGGAAGACAACCATAAGGTCTGACTGCCTGAGGGG
TCGGGCAAAAAGCCATATTTTTCTTCTTGCAGAGAGCCTATAAATGGACGTGCAAGTAGGAGAGATATTGCTAAATTCTTTTCCTAGCA
AGGAATATAATACTAAGACCCTAGGGAAAGAATTGCATTCCTGGGGGGAGGTCTATAAACGGCCGCTCTGGGAGTGTCTGTCCTATGTG
GTTGAGATAAGGACTGAGATACGCCCTGGTCTCCTGCAGTACCCTCAGGCTTACTAGGATTGGGAAACCCCAGTCCTGGTAAATTTGAG
GTCAGGCCGGTTCTTTGCTCTGAACCCTGTTTTCTGTTAAGATGTTTATCAAGACAATACATGCACCGCTGAACATAGACCCTTATCAG
GAGTTTCTGATTTTGCTCTGGTCCTGTTTCTTCAGAAGCATGTCATCTTTGCTCTGCCTTCTGCCCTTTGAAGCATGTGATCTTTGTGA
CCTACTCCCTGTTCATACACCCCTCCCCTTTTAAAATCCCTAATAAAAACTTGCTGGTTTTGTGGCTCAGGGGGGCATCATGGACCTAC
CAATACGTGATGTCACCCCCGGTGGCCCAGCTGTAAAA
**SEQ ID 108** 
TTTTGCTTGTGTTTCACCAGGAGAAAATCAGCTTCCTGTTTGGATACCCACTAGACATTTAAAGTTCTACAATGAACTCACTGGAGATG
CAAAGAAAAGTGTGGAGATGGAGACACCCCAATCGACTCGCCAGGTAAACAAAATGGTGATATCAGAAGAACAGAAAAAGTTGCCTTCC
ATCAAGGAAGCAGAGTTGCCAATATAGGCACAATTAAAGAAGCTGACACAGTTAGCTAAAAAAAAAAGCCTAGAGAATACAAAGGTGAC
ACCAACTCCAGAGAATATGCTGCTTGCAGCTCTGATGATTGTATCAACGGTGGTAAGTCTTCCCAAGTCTGCAGGAGCAGCTGCAGCTA
ATTATACTTACTGGGCCTATGTGCCTTTCCCACCCTTAATTCGGGCAGTTACATAGATGGATAATCCTATTGAAGTAGATGTTAATAAT
AGTGCATGGGTGCCTGGCCCCACAGATGACTGTTGCCCTGCCCAACCTGAAGAAGGAATGATGATGAATATTTCCATTGGGTATCCTTA
TCCTCCTGTTTGCCTAGGGAAGGCACCAGGATGCTTAATGCCTACAACCCAAAATTGGTTGGTAGAAGTACCTACAGTCAGTGCTACCA
GTAGATTTACTTATCACATGTCTACAGGTGTATCCAGCAGCTCCAAAGAGACAGCAACCAGCAAGAATGGCCATAGTGACGATGGTGG
TTTTGTCAAAAAGAAAAGGGGGGGATATGTAAGGAAAAGAGAGATCAGACTTTCACTGTGTCTATGTAGAAAAGGAAGACATAAGAAAC
TCCATTTTGATCTGTACTAAGAAAAAATTGTTTTGCCTTGAGATGCTGTTAATCTGTAACTTTAGCCCCAACCCTGTGCTCACGGAAACA
TGTGCTGTAAGGTTTAAGGGATCTAGGGCTGTGCAGGATGTACCTTGTTAACAATATGTTTGCAGGCAGTATGTTTGGTAAAAGTCATC
GCCATTCTCCATTCTCGATTAACCAGGGGCTCAATGCACTGTGGAAAGCCACAGGAACCTCTGCCCAAGAAAGCCTGGCTGTTGTGGGA
AGTCAGGGACCCCGAATGGAGGGACCAGCTGGTGCTGCATCAGGAAACATAAATTGTGAAGATTTCTTGGACATTTATCAGTTTCCAAA
ATTAATACTTTTATAATTTCTTACACCTGTCTTACTTTAATCTCTTAATCCTGTTATCTTTGTAAGCTGAGGATATACGTCACCTCAGG
ACCACTATTGTACAAATTGATTGTAAAACATGTTCACATGTGTTTGAACAATATGAAATCAGTGCACCTTGAAAATGAACAGAATAACA
GTGATTTTAGGGAACAAAGGAAGACAACCATAAGGTCTGACTGCCTGAGGGGTCGGGCAAAAAGCCATATTTTTCTTCTTGCAGAGAGC
CTATAAATGGACGTGCAAGTAGGAGAGATATTGCTAAATTCTTTTCCTAGCAAGGAATATAATACTAAGACCCTAGGGAAAGAATTGCA
TTCCTGGGGGGAGGTCTATAAACGGCCGCTCTGGGAGTGTCTGTCCTATGTGGTTGAGATAAGGACTGAGATACGCCCTGGTCTCCTGC
AGTACCCTCAGGCTTACTAGGATTGGGAAACCCCAGTCCTGGTAAATTTGAGGTCAGGCCGGTTCTTTGCTCTGAACCCTGTTTTCTGT
TAAGATGTTTATCAAGACAATACATGCACCGCTGAACATAGACCCTTATCAGGAGTTTCTGATTTTGCTCTGGTCCTGTTTCTTCAGAA
GCATGTCATCTTTGCTCTGCCTTCTGCCCTTTGAAGCATGTGATCTTTGTGACCTACTCCCTGTTCATACACCCCTCCCCTTTTAAAAT
CCCTAATAAAAACTTGCTGGTTTTGTGGCTCAGGGGGGCATCATGGACCTACCAATACGTGATGTCACCCCCGGTGGCCCAGCTGTAAA
A
**SEQ ID 109**

GAGAAGAAAACCACCCTGTGGCTGGAGGTGAGATATGCTAGCGGCAATGCTGCTCTGTTACTCTTTGCTACACTGAGATGTTTGGGTGG
AGAGAAGCATAAATCTGGCCTATGTGCACATCTGGGCACAGAACCTCCCCTTGAACTTGTGACACAGATTCCTTTGTTCACATGTTTTC
CTGCTGACCTTCTCCCCACTATCGCCCTGTTCTCCCACCGCATTCCCCTTGCTGAGATAGTGAAAATAGTAATCTGTAGATACCAAGGG
AACTCAGAGACCATGGCCGGTGCACATCCTCCGTACGCTGAGCGCTGGTCCCCTGGGCCCATTGTTCTTTCTCTATACTTTGTCTCTGT
GTCTTATTTCTTTCCTCAGTCTCTCATCCCTCCTGACGAGAAATACCCACAGGTGTGGAGGGGCTGGCCCCCTTCATCTGATGCCCAAT
GTGGGTGCCTTTCTCTAGGGTGAAGGTACTCTACAGTGTGGTCATTGAGGACAAGTTGACGAGAGAGTCCCAAGTACGTCCACGGTCAG
CCTTGCGACATTTAAAGTTCTACAATGAACTCACTGGAGATGCAAAGAAAAGTGTGGAGATGGAGACACCCCAATCGACTCGCCAGTCT
ACAGGTGTATCCAGCAGCTCCAAAGAGACAGCAACCAGCAAGAATGGGCCATAGTGACGATGGTGGTTTTGTCAAAAAGAAAAGGGGGG
GATATGTAAGGAAAAGAGAGATCAGACTTTCACTGTGTCTATGTAGAAAAGGAAGACATAAGAAACTCCATTTTGATCTGTACTAAGAA
AAATTGTTTTGCCTTGAGATGCTGTTAATCTGTAACTTTAGCCCCAACCCTGTGCTCACGGAAACATGTGCTGTAAGGTTTAAGGATC
TAGGGCTGTGCAGGATGTACCTTGTTAACAATATGTTTGC[?][?][?][?]AGTATGTTTGGTAAAAGTCATCGCCATTCTCCATTCTCGATTAAC

CAGGGGCTCAATGCACTGTGGAAAGCCACAGGAACCTCTGCCCAAGAAAGCCTGGCTGTTGTGGGAAGTCAGGGACCCCGAATGGAGGG
ACCAGCTGGTGCTGCATCAGGAAACATAAATTGTGAAGATTTCTTGGACATTTATCAGTTTCCAAAATTAATACTTTTATAATTTCTTA
CACCTGTCTTACTTTAATCTCTTAATCCTGTTATCTTTGTAAGCTGAGGATATACGTCACCTCAGGACCACTATTGTACAAATTGATTG
TAAAACATGTTCACATGTGTTTGAACAATATGAAATCAGTGCACCTTGAAAATGAACAGAATAACAGTGATTTTAGGGAACAAAGGAAG
ACAACCATAAGGTCTGACTGCCTGAGGGGTCGGCAAAAAGCCATATTTTTCTTCTTGCAGAGAGCCTATAAATGGACGTGCAAGTAGG
AGAGATATTGCTAAATTCTTTTCCTAGCAAGGAATATAATACTAAGACCCTAGGGAAAGAATTGCATTCCTGGGGGGAGGTCTATAAAC
GGCCGCTCTGGGAGTGTCTGTCCTATGTGGTTGAGATAAGGACTGAGATACGCCCTGGTCTCCTGCAGTACCCTCAGGCTTACTAGGAT
TGGGAAACCCCAGTCCTGGTAAATTTGAGGTCAGGCCGGTTCTTTGCTCTGAACCCTGTTTTCTGTTAAGATGTTTATCAAGACAATAC
ATGCACCGCTGAACATAGACCCTTATCAGGAGTTTCTGATTTTGCTCTGGTCCTGTTTCTTCAGAAGCATGTCATCTTTGCTCTGCCTT
CTGCCCTTTGAAGCATGTGATCTTTGTGACCTACTCCCTGTTCATACACCCCTCCCCTTTTAAAATCCCTAATAAAAACTTGCTGGTTT
TGTGGCTCAGGGGGGCATCATGGACCTACCAATACGTGATGTCACCCCCGGTGGCCCAGCTGTAAAA

**SEQ ID 110**
   TAMASNSPATQDAALY
**SEQ ID 111**
   ACGCAGGTTAGACAAGCACAAACCCCAAGAGAAAATCAAGTAGAAAGGGACAGAGTCTCTATCCCGGCA
**SEQ ID 112**
   CCCACAGCGATGGCGTCTAATTCACCAGCAACACAGGACGCGGCGCTGTAT
**SEQ ID 113** 
NCGGCCTNCGGCTGCGNNTANTCGACAGNANGGNGGGTAGGCCTTATTTAGGGAGATCAAGTCTAAATTTGAAGGGAGTCCAAATTCA
TACTGGGGTAATTTATTCAGATTATAAAGGGGGAATTCAGTTAGTGTCAGCTCCACTGTTCCCCGGAGTGCCAATCCAGGTGATAGAAT
TGCTCAATTACTGCTTTTGCCTTATGTTAAAATTGGGGAAAACAAAACGGAAAGAACAGGAGGGTTTGGAAGTACCAACCCTGCAGGAA
AAGCTGCTTATTGGGCTAATCAGGTCTCAGAAGATAGACCCGTGTGTACAGTCACTATTCAGGGAAAGAGTTTGAAGGATTAGTGGATA
CCCAGGCTGATTCTATCATCGGCATAGGTACCGCCTCAGAAGTGTATCAAAGTGCCATGATTTTACATTGTCTAGGATCTGATAATCAA
GAAAGTACGGTTCAGCCTGTGATCACTTCATTCCAATCAATTTATGGGGCCGAGACTTGTTACAACAATGGCATGCAGAGATTACTATC
CCAGCCTCCCTATACAGCCCCAGGAATCAAAAAATCATGACTAAAATGGGATAGCTCCCTAAAAAGGGACTAGGAAAGAAAGAAGTCCC
AATTGAGGCTGAAAAAATCAAAAAAGAAANGAATAGGGCATCCTTTTTAGGAGCGTCACTGTANAGCCTCCAAACCCATTCATTAACTT
GGGAAAAAAAACTGNNTGGTAAATCANCANCCGCTTCCAAAAAAAAAAAAAAAAAAAACCCNNNNCCN
**SEQ ID 114** 
ATCTTTACCCTGTATAAACATCTTTCTCTTCCCAGTATTTCTAAGCATGTGACAATGAATATGCAAAGGAAGCGCAGCAGTCCACCAGG
TGTGGGATATGTGTGGCACAATTCAAGACAATGATTAAACCTCCACTTGATGTTGCAAAAGAGATTTTGAAAAATTTGCTTTCACCACA
CCAGCCTAAATAATAAAGAACCAGCCACCAGGTTTCAGTGGAAAGTATTGCCTCAGGGAATGCTTAATAGTTCAACTATTTGTCAGCTC
AAGCTCTGCAACCAGTTAGAGACAAGTTTTCAGACTGTTACATCGTTCACTATGTTGATATTTTGTGTGCTGCAGAAACGAGAGACAAA
TTAATTGACCGTTACACATTTCTGCAGACAGAGGTTGCCAACGCGGGACTGACAATAACATCTGATAAGATTCAAGCCTCTACTCCTTT
CCGTTACTTGGGAATGCAGGTAGAGGAAAGGAAAATTAAACCACAAAAAAATAGAAATAAGAAAAGACACATTAAAAGCATTAAATGAG
TTTCAAAAGTTGCTAGGAGATACTAATTGGATTTGGAGATATTAATTGGATTTGGCCAACTCTAGGCATTCCTACTTATGCCATGTCAA
ATTTGTTCTCTTTCTTAAGAGGGGACTCGGAATTAAATAGTGAAAGAACGTTAACTCCAGAGGCAACTAAAGAAATTAAATTAATTGAA
GAAAAAATTCGGTCAGCACAAGTAAATAGAATAGATCACTTGGCCCCACTCCAAATTTTGATTTTTACTACTGCACATTCCCTAACAGG
CATCATTGTTCAAAACACAGATCTTGTGGAGTGGTCCTTCCTTCCTCACAGTACAATTAAGACTTTTACATTGTACTTGGATCAAATGG
CTACATTAATTGGTCAGGGAAGATTATGAATAATAACATTGTGTGGAAATGACCCAGATAAAATCACTGTTCCTTTCAACAAGCAACAG
GTTAGACAAGCCTTTATCAATTCTGGTGCATGGCAGATTGGTCTTGCCGATTTTGTGGGAATTATTGACAATCGTTACC
**SEQ ID 115** 
CCAAAAGAATGAGTCATCAAAACTCAGTATCACTTGACTCAAAGAGCAGAGTTGGTTGCCGTCATTACAGTGTTAACAAGATTTTAATC
AGTCTATTAACATTGTATCAGATTCTGCATATGTAGTACAGGCTACAAAGGATATTGAGAGAGCCCTAATCAAATACATTATGGATGAT
CAGTTAAACCCGCTGTTTAATTTGTTACAACAAAATGTAAGAAAAAGAAATTTCCCATTTTATATTACTCATATTCGAGCACACACTAA
TTTACCAGGGCCTTTAACTAAAGCAAATGAACAAGCTGACTCGCTAGTATCATCTGCATTCATGGAAGCACAAGACCTTCATGCCTTGA
CTCATGTAAATGCAATAGGATTAAAAAATAAATTTAATATCACATGGAAACAGACAAAAAATATTGTACAACATTGCACCCAGTGTCAG
ATTCTACACCTGGCCACTCAGGAGGCAAGAGTTAATCCCAGAGGTCTATGTCCTAATGTGTTATGGCAAATGGATGTCATGCACGTACC
TTCATTTGGAAAATTGTCATTTGTCCATGTGACAGTTGATACTTATTCACATTTCATATGGGCAACCTGCCAGACAGGAGAAAGTACTT
CCCATGTTAAGAGACATTTATTATCTTGTTTTCCTGTCATGGGAGTTCCAGAAAAAGTTAAAACAGACAATGGGCCAGGTTACTGTAGT
AAAGCAGTTCAAAAATTCTTAAATCAGTGGAAAATTACACATACAATAGGAATTCTCTATAATTCCCAAGGACAGGCCATAATTGAAAG
AACTAATAGAACACTCAAAGCTCAATTGGTTAAACAAAAAA
**SEQ ID 116** 
CCTTACGGCCGGGAAGGGCCGGAAAGGGAGTCAAGCAGGAGCGTCTGTCCGAACGGAGGCTAGGTAAGAATATTTCACCATGAAAATGT
TAAAAGACATAAAGGAAGGAGCTAAACAATATGGACCCAACTCTCCTTATATGAGAACGTTATTAGATTCCATTGCTCATGGAAATAGA
CTTATTCCTTATGATTGGGAAATTTTACCTAAATCTTCCCTTTCACCCTCTCAGTATCTACAGTTTAAAACCTGGTGGATTGATGGAGT
ACAAGAACAGGTACGGAAAAATCAGGCTACTTATCCTGTTGTTAATATAGATGCAGACCAATTGCTAGGAACACGTCCAAATTGGAGCA
CTATTAACCAACAATCAGTAATGCAAAATGAGGCTATTGAACAACTAGGGGCTATTTGCCTCAGGGCCTGGGAAAAGATTCAGGACCCA
GGAACCAGTTAGAGACAGTTTTCAGACTGTTATATCATTCATTATGTTGATGATATTTTGTGTGCTGCAGAAACAAGAGACAAATTAAT
TGACTTTTACATGTTTCTGCAGACAGAGGTTGCAAACACAGGCCTGACAATAGCATCTGATAAGATTCAGACCTCCACTCCTTTTAATT
ATTTGGGAATGCAGGTAGAGGAAAGAAAAATTAAACCA
**SEQ ID 117** 
CTGAAAAAAATCAAAAAAGAAAAGGAATAGGGCATCCTTTTTAGGAGCGGTCACTGTAGAGCCTCCAAAACCCATTCCATTAACTTGGG
GGAAAAAAAAACAACTGTATGGTAAATCAGCAGCGCTTCCAAAACAAAAACTGGAGGCTTTACATTTATTAGCAAAGAAACAATTAGAA
AAAGGACATTGAGCCTTCATTTCGCCTTGGAATTCTGTTTGTAATTCAGAAAAAATCCGGCAGATGGCGTATAATGCCGTAATTCAAC
CCATGGGGGCTCTCCCACCCCGGTTGCCCTCTCCAGCCATGGTCCCCTTTAATTATAATTGATCTGAAGGATTGCTTTTTTACCATTCC
TCTGGCAAAACAGGATTTTGAGAAATTTGCTTTTACCACACCAGCCTAAATAATAAAGAACCAGCCACCAGGTTTCAGTGGAAAGTATT
GCCTCAGGGAATGCTTAATAGTTCAACTATTTGTCAGCTCAAGCTCTGCAACCAGTTAGAGACAAGTTTTCAGACTGTTACATCGTTCA
CTATGTTGATATTTTGTGTGCTGCAGAAACGAGAGACAAATTAATTGACCGTTACACATTTCTGCAGACAGAGGTTGCCAACGCGGGAC
TGACAATAACATCTGATAAGATTCAAACCTCTACTCCTTTCCGTTACTTGGGAATGCAGGTAGAGGAAAGGAAAATTAAACCACAAAA
**SEQ ID 118** 
ACAACAATGGCATGCAGAGATTACTATCCCAGCCTCCCTATACAGCCCCAGGAATCAAAAAATCATGACTAAAATGGGATAGCTCCCTA
AAAAGGGACTAGGAAAGAAAGAAGTCCCAATTGAGGCTGAAAAAAATTAAAAAAGAAAAGGAATAGGGCATCCTTTTTAGGAGCGGTCA
CTGTAGAGCCTCCAAAACCCATTCCATTAACTTGGGAAAAAAAAAACTGTATGGTAAATCAGCAGCCGCTTCCAAAACAAAAGCTGGAG
GCCTTACACTTATTAGCAAAGAAACCATTAGAAAAAGGACATTGAGCCTTCATTTTCGCCTTGGAATTCTGTTTGTGATTCAGAAAAAA
TCCGGCAGATGGCGTATGCTAACTGAGCCATTAATGCCGTAATTCAACCCATGGGGGCTCTCCCACCCCGGTTGCCCTCTCCAGCCATG
GTCCCCTTTAATTATAATTGATCTGAAGGATTGCTTTTTACCATTCCTCTGGCAAAACAGGATTTTGAAAAATTTGCTTTTACCACAC
CAGCCTAAATAATAAAGAACCAGCCACCAGGTTTCAGTGGAAAGTATTGCCTCAGGGAATGCTTAATAGTTCAACTATTTGTCAGCTCA
AGCTCTGCAACCAGTTAGAGACAAGTTTTCAGACTGTTACATCGTTCACTATGTTGATATTTTGTGTGCTGCAGAAACGAGAGACAAAT
TAATTGACCGTTACACATTTCTGCAGACAGAGGTTGCCAACGCGGGGCTGACAATAACATCTGATAAGATTCAAACCTCTACTCCTTTC
CGTTACTTGGGAATGCAGGTAGAGGAAAGGAAAATTA
**SEQ ID 119** 
CATTAGAAAAAGGACATTGAGCCTTCATTTTCGCCTTGGAATTCTGTTTGTAATTCAGAAAAAATCCGGCAGATGGCGTATGCTAACTG
AGCCATTAATGCCGTAATTCAACCCATGGGGGCTCTCCCACCCCGGTTGCCCTCTCCAGCCATGGTCCCCTTTAATTATAATTGATCTG
AAGGATTGCTTTTTTACCATTCCTCTGGCAAAACAGGATTTTGAAAAATTTGCTTTTACCACACCAGCCTAAATAATAAAGAACCAGCC
ACCAGGTTTCAGTGGAAAGTATTGCCTCAGGGAATGCTTAATAGTTCAACTATTTGTCAGCTCAAGCTCTGCAACCAGTTAGAGACAAG
TTTTCAGACTGTTACATCGTTCACTATGTTGATATTTTGTGTGCTGCAGAAACGAGAGACAAATTAATTGACCGTTACACATTTCTGCA
GACAGAGGTTGCCAACGCGGGACTGACAATAACATCTGATAAGATTCAAACCTCTACTCCTTTCCGTTACTTGGGAATGCAGGTAGAGG
AAAGGAAAATTAAACCACAAAAAATAGAAATAAGAAAAGACACATTAAAAGCATTAAATGAGTTTCAAAAGTTGCTAGGAGATACTAAT
TGGATTTGGAGATATTAATTGGATTTGGCCAACTCTAGGCATTCCTACTTATGCCATGTCAAATTTGTACTCTTTCTTAAGAGGGGACT
CGGAATTAAATAGTGAAAGAACGTTAACTCCAGAGGCAACTAAAGAAAAA
**SEQ ID 120**
   ACTGAGATAGGAGAAAACTGCCTTA
**SEQ ID 121**
   GATAGGAGAAAACTGCCTTAGGGCT
**SEQ ID 122**
   GAGAAAACTGCCTTAGGGCTGGAGG
**SEQ ID 123**
   AACTGCCTTAGGGCTGGAGGTGGGA
**SEQ ID 124**
   CCTTAGGGCTGGAGGTGGGACATGC
**SEQ ID 125**
   GGGCTGGAGGTGGGACATGCTGGCG
**SEQ ID 126**
   GGAGGTGGGACATGCTGGCGGCAAT
**SEQ ID 127**
   TGGGACATGCTGGCGGCAATACTGC
**SEQ ID 128**
   CATGCTGGCGGCAATACTGCTCTTT
**SEQ ID 129**
   TGGCGGCAATACTGCTCTTTAAGGC
**SEQ ID 130**
   GCAATACTGCTCTTTAAGGCATTGA
**SEQ ID 131**
   ACTGCTCTTTAAGGCATTGAGATGT
**SEQ ID 132**
   TCTTTAAGGCATTGAGATGTTTATG
**SEQ ID 133**
   AAGGCATTGAGATGTTTATGTATAT
**SEQ ID 134**
   ATTGAGATGTTTATGTATATGCACA
**SEQ ID 135**
   GATGTTTATGTATATGCACATCAAA
**SEQ ID 136**
   TTATGTATATGCACATCAAAAGCAC
**SEQ ID 137**
   TATATGCACATCAAAAGCACAGCAC
**SEQ ID 138**
   GCACATCAAAAGCACAGCACTTTTT
**SEQ ID 139**
   TCAAAAGCACAGCACTTTTTTCTTT
**SEQ ID 140**
   AGCACAGCACTTTTTTCTTTACCTT
**SEQ ID 141**
   AGCACTTTTTTCTTTACCTTGTTTA
**SEQ ID 142**
   TTTTTTCTTTACCTTGTTTATGATG
**SEQ ID 143**
   TCTTTACCTTGTTTATGATGCAGAG
**SEQ ID 144**
   ACCTTGTTTATGATGCAGAGACATT
**SEQ ID 145**
   GTTTATGATGCAGAGACATTTGTTC
**SEQ ID 146**
   TGATGCAGAGACATTTGTTCACATG
**SEQ ID 147**
   CAGAGACATTTGTTCACATGTTTTC
**SEQ ID 148**
   ACATTTGTTCACATGTTTTCCTGCT
**SEQ ID 149**
   TGTTCACATGTTTTCCTGCTGGCCC
**SEQ ID 150**
   ACATGTTTTCCTGCTGGCCCTCTCC
**SEQ ID 151**
   TTTTCCTGCTGGCCCTCTCCCCACT

**SEQ ID 152**
   CTGCTGGCCCTCTCCCCACTATTAC
**SEQ ID 153**
   GGCCCTCTCCCCACTATTACCCTAT
**SEQ ID 154**
   TCTCCCCACTATTACCCTATTGTCC
**SEQ ID 155**
   CCACTATTACCCTATTGTCCTGCCA
**SEQ ID 156**
   ATTACCCTATTGTCCTGCCACATCC
**SEQ ID 157**
   CCTATTGTCCTGCCACATCCCCCTC
**SEQ ID 158**
   TGTCCTGCCACATCCCCCTCTCCGA
**SEQ ID 159**
   TGCCACATCCCCCTCTCCGAGATGG
**SEQ ID 160**
   CATCCCCCTCTCCGAGATGGTAGAG
**SEQ ID 161**
   CCCTCTCCGAGATGGTAGAGATAAT
**SEQ ID 162**
   TCCGAGATGGTAGAGATAATGATCA
**SEQ ID 163**
   GATGGTAGAGATAATGATCAATAAA
**SEQ ID 164**
   TAGAGATAATGATCAATAAATACTG
**SEQ ID 165**
   ATAATGATCAATAAATACTGAGGGA
**SEQ ID 166**
   GATCAATAAATACTGAGGGAACTCA
**SEQ ID 167**
   ATAAATACTGAGGGAACTCAGAGAC
**SEQ ID 168**
   TACTGAGGGAACTCAGAGACCGGTG
**SEQ ID 169**
   AGGGAACTCAGAGACCGGTGCGGCG
**SEQ ID 170**
   ACTCAGAGACCGGTGCGGCGCGGGT
**SEQ ID 171**
   GAGACCGGTGCGGCGCGGGTCCTCC
**SEQ ID 172**
   CGGTGCGGCGCGGGTCCTCCATATG
**SEQ ID 173**
   CGGCGCGGGTCCTCCATATGCTGAG
**SEQ ID 174**
   CGGGTCCTCCATATGCTGAGCGCCG
**SEQ ID 175**
   CCTCCATATGCTGAGCGCCGGTCCC
**SEQ ID 176**
   ATATGCTGAGCGCCGGTCCCCTGGG
**SEQ ID 177**
   CTGAGCGCCGGTCCCCTGGGCCCAC
**SEQ ID 178**
   CGCCGGTCCCCTGGGCCCACTTTTC
**SEQ ID 179**
   GTCCCCTGGGCCCACTTTTCTTTCT
**SEQ ID 180**
   CTGGGCCCACTTTTCTTTCTCTATA
**SEQ ID 181**
   CCCACTTTTCTTTCTCTATACTTTG
**SEQ ID 182**
   TTTTCTTTCTCTATACTTTGTCTCT
**SEQ ID 183**
   TTTCTCTATACTTTGTCTCTGTTGT
**SEQ ID 184**
   CTATACTTTGTCTCTGTTGTCTTTC
**SEQ ID 185**
   CTTTGTCTCTGTTGTCTTTCTTTTC
**SEQ ID 186**
   TCTCTGTTGTCTTTCTTTTCTCAAG
**SEQ ID 187**
   GTTGTCTTTCTTTTCTCAAGTCTCT
**SEQ ID 188**
   CTTTCTTTTCTCAAGTCTCTCGTTC
**SEQ ID 189**
   TTTTCTCAAGTCTCTCGTTCCACCT
**SEQ ID 190**
   TCAAGTCTCTCGTTCCACCTGAGGA
**SEQ ID 191**
   TCTCTCGTTCCACCTGAGGAGAAAT
**SEQ ID 192**
   CGTTCCACCTGAGGAGAAATGCCCA
**SEQ ID 193**
   CACCTGAGGAGAAATGCCCACAGCT
**SEQ ID 194**
   GAGGAGAAATGCCCACAGCTGTGGA
**SEQ ID 195**
   GAAATGCCCACAGCTGTGGAGGCGC
**SEQ ID 196**
   GCCCACAGCTGTGGAGGCGCAGGCC
**SEQ ID 197**
   CAGCTGTGGAGGCGCAGGCCACTCC
**SEQ ID 198**
   GTGGAGGCGCAGGCCACTCCATCTG
**SEQ ID 199**
   GGCGCAGGCCACTCCATCTGGTGCC
**SEQ ID 200**
   AGGCCACTCCATCTGGTGCCCAACG
**SEQ ID 201**
   ACTCCATCTGGTGCCCAACGTGGAT
**SEQ ID 202**
   ATCTGGTGCCCAACGTGGATGCTTT
**SEQ ID 203**
   GTGCCCAACGTGGATGCTTTTCTCT

**SEQ ID 204**
   CAACGTGGATGCTTTTCTCTAGGGT
**SEQ ID 205**
   TGGATGCTTTTCTCTAGGGTGAAGG
**SEQ ID 206**
   GCTTTTCTCTAGGGTGAAGGGACTC
**SEQ ID 207**
   TCTCTAGGGTGAAGGGACTCTCGAG
**SEQ ID 208**
   AGGGTGAAGGGACTCTCGAGTGTGG
**SEQ ID 209**
   GAAGGGACTCTCGAGTGTGGTCATT
**SEQ ID 210**
   GACTCTCGAGTGTGGTCATTGAGGA
**SEQ ID 211**
   TCGAGTGTGGTCATTGAGGACAAGT
**SEQ ID 212**
   TGTGGTCATTGAGGACAAGTCAACG
**SEQ ID 213**
   TCATTGAGGACAAGTCAACGAGAGA
**SEQ ID 214**
   GAGGACAAGTCAACGAGAGATTCCC
**SEQ ID 215**
   CAAGTCAACGAGAGATTCCCGAGTA
**SEQ ID 216**
   CAACGAGAGATTCCCGAGTACGTCT
**SEQ ID 217**
   AGAGATTCCCGAGTACGTCTACAGT
**SEQ ID 218**
   TTCCCGAGTACGTCTACAGTGAGCC
**SEQ ID 219**
   GAGTACGTCTACAGTGAGCCTTGTG
**SEQ ID 220**
   gagaaaatcagcttcctgtttggat
**SEQ ID 221**
   aatcagcttcctgtttggataccca
**SEQ ID 222**
   gcttcctgtttggatacccactaga
**SEQ ID 223**
   ctgtttggatacccactagacattt
**SEQ ID 224**
   acccactagacatttaaagttctac
**SEQ ID 225**
   ctagacatttaaagttctacaatga
**SEQ ID 226**
   GGTGAAGGTACTCTACAGTGTGGTC
**SEQ ID 227**
   AGGTACTCTACAGTGTGGTCATTGA
**SEQ ID 228**
   CTCTACAGTGTGGTCATTGAGGACA
**SEQ ID 229**
   CAGTGTGGTCATTGAGGACAAGTTG
**SEQ ID 230**
   TGGTCATTGAGGACAAGTTGACGAG
**SEQ ID 231**
   ATTGAGGACAAGTTGACGAGAGAGT
**SEQ ID 232**
   GGACAAGTTGACGAGAGAGTCCCAA
**SEQ ID 233**
   AGTTGACGAGAGAGTCCCAAGTACG
**SEQ ID 234**
   ACGAGAGAGTCCCAAGTACGTCCAC
**SEQ ID 235**
   AGAGTCCCAAGTACGTCCACGGTCA
**SEQ ID 236**
   CCCAAGTACGTCCACGGTCAGCCTT
**SEQ ID 237**
   GTACGTCCACGGTCAGCCTTGCGAC
**SEQ ID 238**
   TCCACGGTCAGCCTTGCGACATTTA
**SEQ ID 239**
   GGTCAGCCTTGCGACATTTAAAGTT
**SEQ ID 240**
   GCCTTGCGACATTTAAAGTTCTACA
**SEQ ID 241**
   GCGACATTTAAAGTTCTACAATGAA
**SEQ ID 242**
   ATTTAAAGTTCTACAATGAACTCAC
**SEQ ID 243**
   AAGTTCTACAATGAACTCACTGGAG
**SEQ ID 244**
   CTACAATGAACTCACTGGAGATGCA
**SEQ ID 245**
   ATGAACTCACTGGAGATGCAAAGAA
**SEQ ID 246**
   CTCACTGGAGATGCAAAGAAAAGTG
**SEQ ID 247**
   TGGAGATGCAAAGAAAAGTGTGGAG
**SEQ ID 248**
   ATGCAAAGAAAAGTGTGGAGATGGA
**SEQ ID 249**
   AAGAAAAGTGTGGAGATGGAGACAC
**SEQ ID 250**
   AAGTGTGGAGATGGAGACACCCCAA
**SEQ ID 251**
   TGGAGATGGAGACACCCCAATCGAC
**SEQ ID 252**
   ATGGAGACACCCCAATCGACTCGCC
**SEQ ID 253**
   GACACCCCAATCGACTCGCCAGTCT
**SEQ ID 254**
   CCCAATCGACTCGCCAGTCTACAGG
**SEQ ID 255**
   TCGACTCGCCAGTCTACAGGTGTAT

**SEQ ID 256**
   TCGCCAGTCTACAGGTGTATCCAGC
**SEQ ID 257**
   AGTCTACAGGTGTATCCAGCAGCTC
**SEQ ID 258**
   ACAGGTGTATCCAGCAGCTCCAAAG
**SEQ ID 259**
   TGTATCCAGCAGCTCCAAAGAGACA
**SEQ ID 260**
   CCAGCAGCTCCAAAGAGACAGCAAC
**SEQ ID 261**
   AGCTCCAAAGAGACAGCAACCAGCA
**SEQ ID 262**
   CAAAGAGACAGCAACCAGCAAGAAT
**SEQ ID 263**
   AGACAGCAACCAGCAAGAATGGGCC
**SEQ ID 264**
   GCAACCAGCAAGAATGGGCCATAGT
**SEQ ID 265**
   CAGCAAGAATGGGCCATAGTGACGA
**SEQ ID 266**
   AGAATGGGCCATAGTGACGATGGTG
**SEQ ID 267**
   GGGCCATAGTGACGATGGTGGTTTT
**SEQ ID 268**
   ATAGTGACGATGGTGGTTTTGTCAA
**SEQ ID 269**
   GACGATGGTGGTTTTGTCAAAAAGA
**SEQ ID 270**
   TGGTGGTTTTGTCAAAAAGAAAAGG
**SEQ ID 271**
   GTTTTGTCAAAAAGAAAAGGGGGGG
**SEQ ID 272**
   GTCAAAAAGAAAAGGGGGGGATATG
**SEQ ID 273**
   AAAGAAAAGGGGGGGATATGTAAGG
**SEQ ID 274**
   AAAGGGGGGGATATGTAAGGAAAAG
**SEQ ID 275**
   GGGGGATATGTAAGGAAAAGAGAGA
**SEQ ID 276**
   ATATGTAAGGAAAAGAGAGATCAGA
**SEQ ID 277**
   TAAGGAAAAGAGAGATCAGACTTTC
**SEQ ID 278**
   AAAAGAGAGATCAGACTTTCACTGT
**SEQ ID 279**
   AGAGATCAGACTTTCACTGTGTCTA
**SEQ ID 280**
   TCAGACTTTCACTGTGTCTATGTAG
**SEQ ID 281**
   CTTTCACTGTGTCTATGTAGAAAAG
**SEQ ID 282**
   ACTGTGTCTATGTAGAAAAGGAAGA
**SEQ ID 283**
   GTCTATGTAGAAAAGGAAGACATAA
**SEQ ID 284**
   TGTAGAAAAGGAAGACATAAGAAAC
**SEQ ID 285**
   AAAAGGAAGACATAAGAAACTCCAT
**SEQ ID 286**
   GAAGACATAAGAAACTCCATTTTGA
**SEQ ID 287**
   CATAAGAAACTCCATTTTGATCTGT
**SEQ ID 288**
   GAAACTCCATTTTGATCTGTACTAA
**SEQ ID 289**
   TCCATTTTGATCTGTACTAAGAAAA
**SEQ ID 290**
   TTTGATCTGTACTAAGAAAAATTGT
**SEQ ID 291**
   TCTGTACTAAGAAAAATTGTTTTGC
**SEQ ID 292**
   ACTAAGAAAAATTGTTTTGCCTTGA
**SEQ ID 293**
   GAAAAATTGTTTTGCCTTGAGATGC
**SEQ ID 294**
   ATTGTTTTGCCTTGAGATGCTGTTA
**SEQ ID 295**
   TTTGCCTTGAGATGCTGTTAATCTG
**SEQ ID 296**
   CTTGAGATGCTGTTAATCTGTAACT
**SEQ ID 297**
   GATGCTGTTAATCTGTAACTTTAGC
**SEQ ID 298**
   TGTTAATCTGTAACTTTAGCCCCAA
**SEQ ID 299**
   ATCTGTAACTTTAGCCCCAACCCTG
**SEQ ID 300**
   TAACTTTAGCCCCAACCCTGTGCTC
**SEQ ID 301**
   TTAGCCCCAACCCTGTGCTCACGGA
**SEQ ID 302**
   CCCAACCCTGTGCTCACGGAAACAT
**SEQ ID 303**
   CCCTGTGCTCACGGAAACATGTGCT
**SEQ ID 304**
   TGCTCACGGAAACATGTGCTGTAAG
**SEQ ID 305**
   ACGGAAACATGTGCTGTAAGGTTTA
**SEQ ID 306**
   AACATGTGCTGTAAGGTTTAAGGGA
**SEQ ID 307**
   GTGCTGTAAGGTTTAAGGGATCTAG

**SEQ ID 308**
   GTAAGGTTTAAGGGATCTAGGGCTG
**SEQ ID 309**
   GTTTAAGGGATCTAGGGCTGTGCAG
**SEQ ID 310**
   AGGGATCTAGGGCTGTGCAGGATGT
**SEQ ID 311**
   TCTAGGGCTGTGCAGGATGTACCTT
**SEQ ID 312**
   GGCTGTGCAGGATGTACCTTGTTAA
**SEQ ID 313**
   TGCAGGATGTACCTTGTTAACAATA
**SEQ ID 314**
   GATGTACCTTGTTAACAATATGTTT
**SEQ ID 315**
   ACCTTGTTAACAATATGTTTGCAGG
**SEQ ID 316**
   GTTAACAATATGTTTGCAGGCAGTA
**SEQ ID 317**
   CAATATGTTTGCAGGCAGTATGTTT
**SEQ ID 318**
   TGTTTGCAGGCAGTATGTTTGGTAA
**SEQ ID 319**
   GCAGGCAGTATGTTTGGTAAAAGTC
**SEQ ID 320**
   CAGTATGTTTGGTAAAAGTCATCGC
**SEQ ID 321**
   TGTTTGGTAAAAGTCATCGCCATTC
**SEQ ID 322**
   GGTAAAAGTCATCGCCATTCTCCAT
**SEQ ID 323**
   AAGTCATCGCCATTCTCCATTCTCG
**SEQ ID 324**
   ATCGCCATTCTCCATTCTCGATTAA
**SEQ ID 325**
   CATTCTCCATTCTCGATTAACCAGG
**SEQ ID 326**
   TCCATTCTCGATTAACCAGGGGCTC
**SEQ ID 327**
   TCTCGATTAACCAGGGGCTCAATGC
**SEQ ID 328**
   ATTAACCAGGGGCTCAATGCACTGT
**SEQ ID 329**
   CCAGGGGCTCAATGCACTGTGGAAA
**SEQ ID 330**
   GGCTCAATGCACTGTGGAAAGCCAC
**SEQ ID 331**
   AATGCACTGTGGAAAGCCACAGGAA
**SEQ ID 332**
   ACTGTGGAAAGCCACAGGAACCTCT
**SEQ ID 333**
   GGAAAGCCACAGGAACCTCTGCCCA
**SEQ ID 334**
   GCCACAGGAACCTCTGCCCAAGAAA
**SEQ ID 335**
   AGGAACCTCTGCCCAAGAAAGCCTG
**SEQ ID 336**
   CCTCTGCCCAAGAAAGCCTGGCTGT
**SEQ ID 337**
   TGTGGGGAAAAGAAAGAGAGATCAG
**SEQ ID 338**
   GGAAAAGAAAGAGAGATCAGACTGT
**SEQ ID 339**
   AGAAAGAGAGATCAGACTGTTACTG
**SEQ ID 340**
   GAGAGATCAGACTGTTACTGTGTCT
**SEQ ID 341**
   ATCAGACTGTTACTGTGTCTATGTA
**SEQ ID 342**
   ACTGTTACTGTGTCTATGTAGAAAG
**SEQ ID 343**
   TACTGTGTCTATGTAGAAAGAAATA
**SEQ ID 344**
   TGTCTATGTAGAAAGAAATAGACAT
**SEQ ID 345**
   ATGTAGAAAGAAATAGACATAAGAG
**SEQ ID 346**
   GAAAGAAATAGACATAAGAGACTCC
**SEQ ID 347**
   AAATAGACATAAGAGACTCCATTTT
**SEQ ID 348**
   GACATAAGAGACTCCATTTTGTTCT
**SEQ ID 349**
   AAGAGACTCCATTTTGTTCTGTACT
**SEQ ID 350**
   ACTCCATTTTGTTCTGTACTAAGAA
**SEQ ID 351**
   ATTTTGTTCTGTACTAAGAAAAATT
**SEQ ID 352**
   GTTCTGTACTAAGAAAAATTCTTCT
**SEQ ID 353**
   GTACTAAGAAAAATTCTTCTGCTTT
**SEQ ID 354**
   AAGAAAAATTCTTCTGCTTTGAGAT
**SEQ ID 355**
   AAATTCTTCTGCTTTGAGATGCTGT
**SEQ ID 356**
   CTTCTGCTTTGAGATGCTGTTAATC
**SEQ ID 357**
   GCTTTGAGATGCTGTTAATCTGTAA
**SEQ ID 358**
   GAGATGCTGTTAATCTGTAACCCTA
**SEQ ID 359**
   GCTGTTAATCTGTAACCCTAGCCCC
**SEQ ID 360**
   TAATCTGTAACCCTAGCCCCAACCC
**SEQ ID 361**
   TGTAACCCTAGCCCCAACCCTGTGC
**SEQ ID 362**
   CCCTAGCCCCAACCCTGTGCTCACA
**SEQ ID 363**
   GCCCCAACCCTGTGCTCACAGAAAC
**SEQ ID 364**
   AACCCTGTGCTCACAGAAACAGGTG
**SEQ ID 365**
   GAAACAGGTGCTGTGTTGACTCAAG
**SEQ ID 366**
   AGGTGCTGTGTTGACTCAAGGTTTA
**SEQ ID 367**
   CTGTGTTGACTCAAGGTTTAATGGA
**SEQ ID 368**
   TTGACTCAAGGTTTAATGGATTCAG
**SEQ ID 369**
   ATGGATTCAGGGCTGTGCAGGATGT
**SEQ ID 370**
   TTCAGGGCTGTGCAGGATGTGCTTT
**SEQ ID 371**
   GGCTGTGCAGGATGTGCTTTGTTAA
**SEQ ID 372**
   TGCAGGATGTGCTTTGTTAAACAAA
**SEQ ID 373**
   GATGTGCTTTGTTAAACAAATGCTT
**SEQ ID 374**
   GCTTTGTTAAACAAATGCTTGAAGG
**SEQ ID 375**
   GTTAAACAAATGCTTGAAGGCAGCA
**SEQ ID 376**
   GTTAAGAGTCATCACCACTCCCTAA
**SEQ ID 377**
   GAGTCATCACCACTCCCTAATCTCA
**SEQ ID 378**
   ATCACCACTCCCTAATCTCAAGTAA
**SEQ ID 379**
   GCAGGGACACAAACACTGCGGAAGG
**SEQ ID 380**
   GACACAAACACTGCGGAAGGCCGCA
**SEQ ID 381**
   AAACACTGCGGAAGGCCGCAGGGAC
**SEQ ID 382**
   CTGCGGAAGGCCGCAGGGACCTCTG
**SEQ ID 383**
   GAAGGCCGCAGGGACCTCTGCCTAG
**SEQ ID 384**
   CCGCAGGGACCTCTGCCTAGGAAAG
**SEQ ID 385**
   GGGACCTCTGCCTAGGAAAGCCAGG
**SEQ ID 386**
   CTCTGCCTAGGAAAGCCAGGTGTTG
**SEQ ID 387**
   CCTAGGAAAGCCAGGTGTTGTCCAA
**SEQ ID 388**
   GAAAGCCAGGTGTTGTCCAAGGTTT
**SEQ ID 389**
   CCAGGTGTTGTCCAAGGTTTCTCCC
**SEQ ID 390**
   TGTTGTCCAAGGTTTCTCCCCATGT
**SEQ ID 391**
   TCCAAGGTTTCTCCCCATGTGACAG
**SEQ ID 392**
   GGTTTCTCCCCATGTGACAGTCTGA
**SEQ ID 393**
   CTCCCCATGTGACAGTCTGAAATAT
**SEQ ID 394**
   CATGTGACAGTCTGAAATATGGCCT
**SEQ ID 395**
   TCTGAAATATGGCCTCTTGGGAAGG
**SEQ ID 396**
   AATATGGCCTCTTGGGAAGGGAAAG
**SEQ ID 397**
   GGCCTCTTGGGAAGGGAAAGACCTG
**SEQ ID 398**
   CTTGGGAAGGGAAAGACCTGACTGT
**SEQ ID 399**
   GAAGGGAAAGACCTGACTGTCCCCT
**SEQ ID 400**
   GGCCCGACACCCGTAAAGGGTCTGT
**SEQ ID 401**
   GACACCCGTAAAGGGTCTGTGCTGA
**SEQ ID 402**
   CCGTAAAGGGTCTGTGCTGAGGATT
**SEQ ID 403**
   GCTGAGGATTAGTAAAAGAGGAAGG
**SEQ ID 404**
   GGATTAGTAAAAGAGGAAGGAAGGC
**SEQ ID 405**
   AGTAAAAGAGGAAGGAAGGCCTCTT
**SEQ ID 406**
   AAGGCCTCTTTGCAGTTGAGATAAG
**SEQ ID 407**
   CTCTTTGCAGTTGAGATAAGAGGAA
**SEQ ID 408**
   TGCAGTTGAGATAAGAGGAAGGCAT
**SEQ ID 409**
   TTGAGATAAGAGGAAGGCATCTGTC
**SEQ ID 410**
   ATAAGAGGAAGGCATCTGTCTCCTG
**SEQ ID 411**
   AGGAAGGCATCTGTCTCCTGCTCAT

**SEQ ID 412**
   GGCATCTGTCTCCTGCTCATCCCTG
**SEQ ID 413**
   CTGTCTCCTGCTCATCCCTGGGCAA
**SEQ ID 414**
   TCCTGCTCATCCCTGGGCAATGGAA
**SEQ ID 415**
   CTCATCCCTGGGCAATGGAATGTCT
**SEQ ID 416**
   TTGTATATGCCATCTACTGAGATAG
**SEQ ID 417**
   CGTCTACAGTGAGCCTTGTGGGTGA
**SEQ ID 418**
   ACAGTGAGCCTTGTGGGTGAAGGTA
**SEQ ID 419**
   GAGCCTTGTGGGTGAAGGTACTCTA
**SEQ ID 420**
   TTGTGGGTGAAGGTACTCTACAGTG
**SEQ ID 421**
   GCCCAAGAAAGCCTGGCTGTTGTGG
**SEQ ID 422**
   AGAAAGCCTGGCTGTTGTGGGAAGT
**SEQ ID 423**
   GCCTGGCTGTTGTGGGAAGTCAGGG
**SEQ ID 424**
   GCTGTTGTGGGAAGTCAGGGACCCC
**SEQ ID 425**
   TGTGGGAAGTCAGGGACCCCGAATG
**SEQ ID 426**
   GAAGTCAGGGACCCCGAATGGAGGG
**SEQ ID 427**
   CAGGGACCCCGAATGGAGGGACCAG
**SEQ ID 428**
   ACCCCGAATGGAGGGACCAGCTGGT
**SEQ ID 429**
   GAATGGAGGGACCAGCTGGTGCTGC
**SEQ ID 430**
   GAGGGACCAGCTGGTGCTGCATCAG
**SEQ ID 431**
   ACCAGCTGGTGCTGCATCAGGAAAC
**SEQ ID 432**
   CTGGTGCTGCATCAGGAAACATAAA
**SEQ ID 433**
   GCTGCATCAGGAAACATAAATTGTG
**SEQ ID 434**
   ATCAGGAAACATAAATTGTGAAGAT
**SEQ ID 435**
   GAAACATAAATTGTGAAGATTTCTT
**SEQ ID 436**
   ATAAATTGTGAAGATTTCTTGGACA
**SEQ ID 437**
   TTGTGAAGATTTCTTGGACATTTAT
**SEQ ID 438**
   AAGATTTCTTGGACATTTATCAGTT
**SEQ ID 439**
   TTCTTGGACATTTATCAGTTTCCAA
**SEQ ID 440**
   GGACATTTATCAGTTTCCAAAATTA
**SEQ ID 441**
   TTTATCAGTTTCCAAAATTAATACT
**SEQ ID 442**
   CAGTTTCCAAAATTAATACTTTTAT
**SEQ ID 443**
   TCCAAAATTAATACTTTTATAATTT
**SEQ ID 444**
   AATTAATACTTTTATAATTTCTTAC
**SEQ ID 445**
   ATACTTTTATAATTTCTTACACCTG
**SEQ ID 446**
   TTTATAATTTCTTACACCTGTCTTA
**SEQ ID 447**
   AATTTCTTACACCTGTCTTACTTTA
**SEQ ID 448**
   CTTACACCTGTCTTACTTTAATCTC
**SEQ ID 449**
   ACCTGTCTTACTTTAATCTCTTAAT
**SEQ ID 450**
   TCTTACTTTAATCTCTTAATCCTGT
**SEQ ID 451**
   CTTTAATCTCTTAATCCTGTTATCT
**SEQ ID 452**
   ATCTCTTAATCCTGTTATCTTTGTA
**SEQ ID 453**
   TTAATCCTGTTATCTTTGTAAGCTG
**SEQ ID 454**
   CCTGTTATCTTTGTAAGCTGAGGAT
**SEQ ID 455**
   TATCTTTGTAAGCTGAGGATATACG
**SEQ ID 456**
   TTGTAAGCTGAGGATATACGTCACC
**SEQ ID 457**
   AGCTGAGGATATACGTCACCTCAGG
**SEQ ID 458**
   AGGATATACGTCACCTCAGGACCAC
**SEQ ID 459**
   ATACGTCACCTCAGGACCACTATTG
**SEQ ID 460**
   TCACCTCAGGACCACTATTGTACAA
**SEQ ID 461**
   TCAGGACCACTATTGTACAAATTGA
**SEQ ID 462**
   ACCACTATTGTACAAATTGATTGTA
**SEQ ID 463**
   TATTGTACAAATTGATTGTAAAACA
**SEQ ID 464**
   TACAAATTGATTGTAAAACATGTTC
**SEQ ID 465**
   ATTGATTGTAAAACATGTTCACATG
**SEQ ID 466**
   TTGTAAAACATGTTCACATGTGTTT
**SEQ ID 467**
   AAACATGTTCACATGTGTTTGAACA
**SEQ ID 468**
   TGTTCACATGTGTTTGAACAATATG
**SEQ ID 469**
   ACATGTGTTTGAACAATATGAAATC
**SEQ ID 470**
   TGTTTGAACAATATGAAATCAGTGC
**SEQ ID 471**
   GAACAATATGAAATCAGTGCACCTT
**SEQ ID 472**
   ATATGAAATCAGTGCACCTTGAAAA
**SEQ ID 473**
   AAATCAGTGCACCTTGAAAATGAAC
**SEQ ID 474**
   AGTGCACCTTGAAAATGAACAGAAT
**SEQ ID 475**
   ACCTTGAAAATGAACAGAATAACAG
**SEQ ID 476**
   GAAAATGAACAGAATAACAGTGATT
**SEQ ID 477**
   TGAACAGAATAACAGTGATTTTAGG
**SEQ ID 478**
   AGAATAACAGTGATTTTAGGGAACA
**SEQ ID 479**
   AACAGTGATTTTAGGGAACAAAGGA
**SEQ ID 480**
   TGATTTTAGGGAACAAAGGAAGACA
**SEQ ID 481**
   TTAGGGAACAAAGGAAGACAACCAT
**SEQ ID 482**
   GAACAAAGGAAGACAACCATAAGGT
**SEQ ID 483**
   AAGGAAGACAACCATAAGGTCTGAC
**SEQ ID 484**
   AGACAACCATAAGGTCTGACTGCCT
**SEQ ID 485**
   ACCATAAGGTCTGACTGCCTGAGGG
**SEQ ID 486**
   AAGGTCTGACTGCCTGAGGGGTCGG
**SEQ ID 487**
   CTGACTGCCTGAGGGGTCGGGCAAA
**SEQ ID 488**
   TGCCTGAGGGGTCGGGCAAAAAGCC
**SEQ ID 489**
   GAGGGGTCGGGCAAAAAGCCATATT

**SEQ ID 490**
   GTCGGGCAAAAAGCCATATTTTTCT
**SEQ ID 491**
   GCAAAAAGCCATATTTTTCTTCTTG
**SEQ ID 492**
   AAGCCATATTTTTCTTCTTGCAGAG
**SEQ ID 493**
   ATATTTTTCTTCTTGCAGAGAGCCT
**SEQ ID 494**
   TTTCTTCTTGCAGAGAGCCTATAAA
**SEQ ID 495**
   TCTTGCAGAGAGCCTATAAATGGAC
**SEQ ID 496**
   CAGAGAGCCTATAAATGGACGTGCA
**SEQ ID 497**
   AGCCTATAAATGGACGTGCAAGTAG
**SEQ ID 498**
   ATAAATGGACGTGCAAGTAGGAGAG
**SEQ ID 499**
   TGGACGTGCAAGTAGGAGAGATATT
**SEQ ID 500**
   GTGCAAGTAGGAGAGATATTGCTAA
**SEQ ID 501**
   AGTAGGAGAGATATTGCTAAATTCT
**SEQ ID 502**
   GAGAGATATTGCTAAATTCTTTTCC
**SEQ ID 503**
   ATATTGCTAAATTCTTTTCCTAGCA
**SEQ ID 504**
   GCTAAATTCTTTTCCTAGCAAGGAA
**SEQ ID 505**
   ATTCTTTTCCTAGCAAGGAATATAA
**SEQ ID 506**
   TTTCCTAGCAAGGAATATAATACTA
**SEQ ID 507**
   TAGCAAGGAATATAATACTAAGACC
**SEQ ID 508**
   AGGAATATAATACTAAGACCCTAGG
**SEQ ID 509**
   TATAATACTAAGACCCTAGGGAAAG
**SEQ ID 510**
   TACTAAGACCCTAGGGAAAGAATTG
**SEQ ID 511**
   AGACCCTAGGGAAAGAATTGCATTC
**SEQ ID 512**
   CTAGGGAAAGAATTGCATTCCTGGG
**SEQ ID 513**
   GAAAGAATTGCATTCCTGGGGGGAG
**SEQ ID 514**
   AATTGCATTCCTGGGGGGAGGTCTA
**SEQ ID 515**
   CATTCCTGGGGGGAGGTCTATAAAC
**SEQ ID 516**
   CTGGGGGGAGGTCTATAAACGGCCG
**SEQ ID 517**
   GGGAGGTCTATAAACGGCCGCTCTG
**SEQ ID 518**
   GTCTATAAACGGCCGCTCTGGGAGT
**SEQ ID 519**
   TAAACGGCCGCTCTGGGAGTGTCTG
**SEQ ID 520**
   GGCCGCTCTGGGAGTGTCTGTCCTA
**SEQ ID 521**
   CTCTGGGAGTGTCTGTCCTATGTGG
**SEQ ID 522**
   GGAGTGTCTGTCCTATGTGGTTGAG
**SEQ ID 523**
   GTCTGTCCTATGTGGTTGAGATAAG
**SEQ ID 524**
   TCCTATGTGGTTGAGATAAGGACTG
**SEQ ID 525**
   TGTGGTTGAGATAAGGACTGAGATA
**SEQ ID 526**
   TTGAGATAAGGACTGAGATACGCCC
**SEQ ID 527**
   ATAAGGACTGAGATACGCCCTGGTC
**SEQ ID 528**
   GACTGAGATACGCCCTGGTCTCCTG
**SEQ ID 529**
   AGATACGCCCTGGTCTCCTGCAGTA
**SEQ ID 530**
   CGCCCTGGTCTCCTGCAGTACCCTC
**SEQ ID 531**
   TGGTCTCCTGCAGTACCCTCAGGCT
**SEQ ID 532**
   TCCTGCAGTACCCTCAGGCTTACTA
**SEQ ID 533**
   CAGTACCCTCAGGCTTACTAGGATT
**SEQ ID 534**
   CCCTCAGGCTTACTAGGATTGGGAA
**SEQ ID 535**
   AGGCTTACTAGGATTGGGAAACCCC
**SEQ ID 536**
   TACTAGGATTGGGAAACCCCAGTCC
**SEQ ID 537**
   GGATTGGGAAACCCCAGTCCTGGTA
**SEQ ID 538**
   GGGAAACCCCAGTCCTGGTAAATTT
**SEQ ID 539**
   ACCCCAGTCCTGGTAAATTTGAGGT
**SEQ ID 540**
   AGTCCTGGTAAATTTGAGGTCAGGC
**SEQ ID 541**
   TGGTAAATTTGAGGTCAGGCCGGTT
**SEQ ID 542**
   AATTTGAGGTCAGGCCGGTTCTTTG
**SEQ ID 543**
   GAGGTCAGGCCGGTTCTTTGCTCTG
**SEQ ID 544**
   CAGGCCGGTTCTTTGCTCTGAACCC
**SEQ ID 545**
   CGGTTCTTTGCTCTGAACCCTGTTT
**SEQ ID 546**
   CTTTGCTCTGAACCCTGTTTTCTGT
**SEQ ID 547**
   CTCTGAACCCTGTTTTCTGTTAAGA
**SEQ ID 548**
   AACCCTGTTTTCTGTTAAGATGTTT
**SEQ ID 549**
   TGTTTTCTGTTAAGATGTTTATCAA
**SEQ ID 550**
   TCTGTTAAGATGTTTATCAAGACAA
**SEQ ID 551**
   TAAGATGTTTATCAAGACAATACAT
**SEQ ID 552**
   TGTTTATCAAGACAATACATGCACC
**SEQ ID 553**
   ATCAAGACAATACATGCACCGCTGA
**SEQ ID 554**
   GACAATACATGCACCGCTGAACATA
**SEQ ID 555**
   TACATGCACCGCTGAACATAGACCC
**SEQ ID 556**
   GCACCGCTGAACATAGACCCTTATC
**SEQ ID 557**
   GCTGAACATAGACCCTTATCAGGAG
**SEQ ID 558**
   ACATAGACCCTTATCAGGAGTTTCT
**SEQ ID 559**
   GACCCTTATCAGGAGTTTCTGATTT
**SEQ ID 560**
   TTATCAGGAGTTTCTGATTTTGCTC
**SEQ ID 561**
   AGGAGTTTCTGATTTTGCTCTGGTC
**SEQ ID 562**
   TTTCTGATTTTGCTCTGGTCCTGTT
**SEQ ID 563**
   GATTTTGCTCTGGTCCTGTTTCTTC
**SEQ ID 564**
   TGCTCTGGTCCTGTTTCTTCAGAAG
**SEQ ID 565**
   TGGTCCTGTTTCTTCAGAAGCATGT
**SEQ ID 566**
   CTGTTTCTTCAGAAGCATGTCATCT
**SEQ ID 567**
   TCTTCAGAAGCATGTCATCTTTGCT

**SEQ ID 568**
   AGAAGCATGTCATCTTTGCTCTGCC
**SEQ ID 569**
   CATGTCATCTTTGCTCTGCCTTCTG
**SEQ ID 570**
   CATCTTTGCTCTGCCTTCTGCCCTT
**SEQ ID 571**
   TTGCTCTGCCTTCTGCCCTTTGAAG
**SEQ ID 572**
   CTGCCTTCTGCCCTTTGAAGCATGT
**SEQ ID 573**
   TTCTGCCCTTTGAAGCATGTGATCT
**SEQ ID 574**
   CCCTTTGAAGCATGTGATCTTTGTG
**SEQ ID 575**
   TGAAGCATGTGATCTTTGTGACCTA
**SEQ ID 576**
   CATGTGATCTTTGTGACCTACTCCC
**SEQ ID 577**
   GATCTTTGTGACCTACTCCCTGTTC
**SEQ ID 578**
   TTGTGACCTACTCCCTGTTCATACA
**SEQ ID 579**
   ACCTACTCCCTGTTCATACACCCCT
**SEQ ID 580**
   CTCCCTGTTCATACACCCCTCCCCT
**SEQ ID 581**
   TGTTCATACACCCCTCCCCTTTTAA
**SEQ ID 582**
   ATACACCCCTCCCCTTTTAAAATCC
**SEQ ID 583**
   CCCCTCCCCTTTTAAAATCCCTAAT
**SEQ ID 584**
   CCCCTTTTAAAATCCCTAATAAAAA
**SEQ ID 585**
   TTTAAAATCCCTAATAAAAACTTGC
**SEQ ID 586**
   AATCCCTAATAAAAACTTGCTGGTT
**SEQ ID 587**
   CTAATAAAAACTTGCTGGTTTTGTG
**SEQ ID 588**
   AAAAACTTGCTGGTTTTGTGGCTCA
**SEQ ID 589**
   CTTGCTGGTTTTGTGGCTCAGGGGG
**SEQ ID 590**
   TGGTTTTGTGGCTCAGGGGGGCATC
**SEQ ID 591**
   TTGTGGCTCAGGGGGGCATCATGGA
**SEQ ID 592**
   GCTCAGGGGGGCATCATGGACCTAC
**SEQ ID 593**
   GGGGGGCATCATGGACCTACCAATA
**SEQ ID 594**
   GCATCATGGACCTACCAATACGTGA
**SEQ ID 595**
   ATGGACCTACCAATACGTGATGTCA
**SEQ ID 596**
   CCTACCAATACGTGATGTCACCCCC
**SEQ ID 597**
   CAATACGTGATGTCACCCCCGGTGG
**SEQ ID 598**
   CGTGATGTCACCCCCGGTGGCCCAG
**SEQ ID 599**
   TGTCACCCCCGGTGGCCCAGCTGTA
**SEQ ID 600**
   TGTGCTCACAGAAACAGGTGCTGTG
**SEQ ID 601**
   TCACAGAAACAGGTGCTGTGTTGAC
**SEQ ID 602**
   TCAAGGTTTAATGGATTCAGGGCTG
**SEQ ID 603**
   GTTTAATGGATTCAGGGCTGTGCAG
**SEQ ID 604**
   ACAAATGCTTGAAGGCAGCAAGCTT
**SEQ ID 605**
   TGCTTGAAGGCAGCAAGCTTGTTAA
**SEQ ID 606**
   GAAGGCAGCAAGCTTGTTAAGAGTC
**SEQ ID 607**
   CAGCAAGCTTGTTAAGAGTCATCAC
**SEQ ID 608**
   AGCTTGTTAAGAGTCATCACCACTC
**SEQ ID 609**
   CACTCCCTAATCTCAAGTAAGCAGG
**SEQ ID 610**
   CCTAATCTCAAGTAAGCAGGGACAC
**SEQ ID 611**
   TCTCAAGTAAGCAGGGACACAAACA
**SEQ ID 612**
   AGTAAGCAGGGACACAAACACTGCG
**SEQ ID 613**
   GACAGTCTGAAATATGGCCTCTTGG
**SEQ ID 614**
   GAAAGACCTGACTGTCCCCTGGCCC
**SEQ ID 615**
   ACCTGACTGTCCCCTGGCCCGACAC
**SEQ ID 616**
   ACTGTCCCCTGGCCCGACACCCGTA
**SEQ ID 617**
   CCCCTGGCCCGACACCCGTAAAGGG
**SEQ ID 618**
   AAGGGTCTGTGCTGAGGATTAGTAA
**SEQ ID 619**
   TCTGTGCTGAGGATTAGTAAAAGAG
**SEQ ID 620**
   AAGAGGAAGGAAGGCCTCTTTGCAG
**SEQ ID 621**
   GAAGGAAGGCCTCTTTGCAGTTGAG
**SEQ ID 622**
   CCCTGGGCAATGGAATGTCTTGGTG
**SEQ ID 623**
   GGCAATGGAATGTCTTGGTGTAAAG
**SEQ ID 624**
   TGGAATGTCTTGGTGTAAAGCCTGA
**SEQ ID 625**
   TGTCTTGGTGTAAAGCCTGATTGTA
**SEQ ID 626**
   TGGTGTAAAGCCTGATTGTATATGC
**SEQ ID 627**
   TAAAGCCTGATTGTATATGCCATCT
**SEQ ID 628**
   CCTGATTGTATATGCCATCTACTGA
**SEQ ID 629**
   TATGCCATCTACTGAGATAGGAGAA
**SEQ ID 630**
   CATCTACTGAGATAGGAGAAAACTG
**SEQ ID 631**
   GGGCTGGAGGTGGGACATGCTGGCG
**SEQ ID 632**
   GGAGGTGGGACATGCTGGCGGCAAT
**SEQ ID 633**
   TGGGACATGCTGGCGGCAATACTGC
**SEQ ID 634**
   CATGCTGGCGGCAATACTGCTCTTT
**SEQ ID 635**
   TGGCGGCAATACTGCTCTTTAAGGC
**SEQ ID 636**
   GCAATACTGCTCTTTAAGGCATTGA
**SEQ ID 637**
   TTATGTATATGCACATCAAAAGCAC
**SEQ ID 638**
   TATATGCACATCAAAAGCACAGCAC
**SEQ ID 639**
   GCACATCAAAAGCACAGCACTTTTT
**SEQ ID 640**
   TCAAAAGCACAGCACTTTTTTCTTT
**SEQ ID 641**
   AGCACAGCACTTTTTTCTTTACCTT
**SEQ ID 642**
   AGCACTTTTTTCTTTACCTTGTTTA
**SEQ ID 643**
   ACCTTGTTTATGATGCAGAGACATT
**SEQ ID 644**
   GTTTATGATGCAGAGACATTTGTTC
**SEQ ID 645**
   TGATGCAGAGACATTTGTTCACATG

**SEQ ID 646**
   ACTCAGAGACCGGTGCGGCGCGGGT
**SEQ ID 647**
   GAGACCGGTGCGGCGCGGGTCCTCC
**SEQ ID 648**
   CGGTGCGGCGCGGGTCCTCCATATG
**SEQ ID 649**
   CTTTGTCTCTGTTGTCTTTCTTTTC
**SEQ ID 650**
   TCTCTGTTGTCTTTCTTTTCTCAAG
**SEQ ID 651**
   TCAAGTCTCTCGTTCCACCTGAGGA
**SEQ ID 652**
   TCTCTCGTTCCACCTGAGGAGAAAT
**SEQ ID 653**
   CGTTCCACCTGAGGAGAAATGCCCA
**SEQ ID 654**
   CACCTGAGGAGAAATGCCCACAGCT
**SEQ ID 655**
   GAGGAGAAATGCCCACAGCTGTGGA
**SEQ ID 656**
   GAAATGCCCACAGCTGTGGAGGCGC
**SEQ ID 657**
   GCCCACAGCTGTGGAGGCGCAGGCC
**SEQ ID 658**
   CAGCTGTGGAGGCGCAGGCCACTCC
**SEQ ID 659**
   GTGGAGGCGCAGGCCACTCCATCTG
**SEQ ID 660**
   GGCGCAGGCCACTCCATCTGGTGCC
**SEQ ID 661**
   AGGCCACTCCATCTGGTGCCCAACG
**SEQ ID 662**
   ACTCCATCTGGTGCCCAACGTGGAT
**SEQ ID 663**
   GCTTTTCTCTAGGGTGAAGGGACTC
**SEQ ID 664**
   TCTCTAGGGTGAAGGGACTCTCGAG
**SEQ ID 665**
   AGGGTGAAGGGACTCTCGAGTGTGG
**SEQ ID 666**
   GAAGGGACTCTCGAGTGTGGTCATT
**SEQ ID 667**
   GACTCTCGAGTGTGGTCATTGAGGA
**SEQ ID 668**
   TGTGGTCATTGAGGACAAGTCAACG
**SEQ ID 669**
   GAGTACGTCTACAGTGAGCCTTGTG
**SEQ ID 670**
   CGTCTACAGTGAGCCTTGTGGTAAG
**SEQ ID 671**
   ACAGTGAGCCTTGTGGTAAGCTTGG
**SEQ ID 672**
   GAGCCTTGTGGTAAGCTTGGGCGCT
**SEQ ID 673**
   TTGTGGTAAGCTTGGGCGCTCGGAA
**SEQ ID 674**
   CTTGGGCGCTCGGAAGAAGCCAGGG
**SEQ ID 675**
   GCGCTCGGAAGAAGCCAGGGTTAAT
**SEQ ID 676**
   CGGAAGAAGCCAGGGTTAATGGGGC
**SEQ ID 677**
GAAGCCAGGGTTAATGGGGCAAACT
   **SEQ ID 678**
   CAGGGTTAATGGGGCAAACTAAAAG
**SEQ ID 679**
   GGGGCAAACTAAAAGTAAAGTCTCT
**SEQ ID 680**
   AAAAGTAAAGTCTCTCATTCCACCT
**SEQ ID 681**
   TAAAGTCTCTCATTCCACCTGATGA
**SEQ ID 682**
   GGGGCAGGCCACCCCTTCAGGGTAG
**SEQ ID 683**
   AGGCCACCCCTTCAGGGTAGGGTCC
**SEQ ID 684**
   ACCCCTTCAGGGTAGGGTCCCCTCC
**SEQ ID 685**
   TTCAGGGTAGGGTCCCCTCCATGCA
**SEQ ID 686**
   GGTAGGGTCCCCTCCATGCAGACCA
**SEQ ID 687**
   GGTCCCCTCCATGCAGACCATAGAG
**SEQ ID 688**
   CCTCCATGCAGACCATAGAGCACAG
**SEQ ID 689**
   ATGCAGACCATAGAGCACAGGTGTG
**SEQ ID 690**
   GACCATAGAGCACAGGTGTGCCCCA
**SEQ ID 691**
   TAGAGCACAGGTGTGCCCCAAAGAG
**SEQ ID 692**
   CACAGGTGTGCCCCAAAGAGGAGCA
**SEQ ID 693**
   GTGTGCCCCAAAGAGGAGCAGAGAG
**SEQ ID 694**
   CCCCAAAGAGGAGCAGAGAGAAGGA
**SEQ ID 695**
   AAGAGGAGCAGAGAGAAGGAGGGAG
**SEQ ID 696**
   GAGCAGAGAGAAGGAGGGAGAGGGC
**SEQ ID 697**
   GAGAGAAGGAGGGAGAGGGCCCACG
**SEQ ID 698**
   AAGGAGGGAGAGGGCCCACGAGAGA
**SEQ ID 699**
   GGGAGAGGGCCCACGAGAGACTTGG
**SEQ ID 700**
   AGGGCCCACGAGAGACTTGGAAATG
**SEQ ID 701**
   CCACGAGAGACTTGGAAATGAATGG
**SEQ ID 702**
   AGAGACTTGGAAATGAATGGCAGGA
**SEQ ID 703**
   CTTGGAAATGAATGGCAGGATTTTA
**SEQ ID 704**
   AAATGAATGGCAGGATTTTAGGCGC
**SEQ ID 705**
   AATGGCAGGATTTTAGGCGCTGGAC
**SEQ ID 706**
   CAGGATTTTAGGCGCTGGACTTGGG
**SEQ ID 707**
   TTTTAGGCGCTGGACTTGGGTTCGG
**SEQ ID 708**
   GGCGCTGGACTTGGGTTCGGGGCAC
**SEQ ID 709**
   TGGACTTGGGTTCGGGGCACCTGGC
**SEQ ID 710**
   TTGGGTTCGGGGCACCTGGCCTTTC
**SEQ ID 711**
   TTCGGGGCACCTGGCCTTTCCTTGT
**SEQ ID 712**
   GGCACCTGGCCTTTCCTTGTGTATT
**SEQ ID 713**
   CTGGCCTTTCCTTGTGTATTTCTCC
**SEQ ID 714**
   CTTTCCTTGTGTATTTCTCCTACTG
**SEQ ID 715**
   CTTGTGTATTTCTCCTACTGTCTGC
**SEQ ID 716**
   GTATTTCTCCTACTGTCTGCCTAAC
**SEQ ID 717**
   TCTCCTACTGTCTGCCTAACTATTT
**SEQ ID 718**
   AATACAATAAAAGAAAACCAGCCCC
**SEQ ID 719**
   AATAAAAGAAAACCAGCCCCTGGTT
**SEQ ID 720**
   AAGAAAACCAGCCCCTGGTTCTTGT
**SEQ ID 721**
   AACCAGCCCCTGGTTCTTGTGGTGT
**SEQ ID 722**
   GCCCCTGGTTCTTGTGGTGTTTCCA
**SEQ ID 723**
   TGGTTCTTGTGGTGTTTCCACCCTC

**SEQ ID 724**
   CTTGTGGTGTTTCCACCCTCCCGGG
**SEQ ID 725**
   GGTGTTTCCACCCTCCCGGGTCCCC
**SEQ ID 726**
   TTCCACCCTCCCGGGTCCCCGCTGG
**SEQ ID 727**
   CCCTCCCGGGTCCCCGCTGGCTGCC
**SEQ ID 728**
   CCGGGTCCCCGCTGGCTGCCTGGCT
**SEQ ID 729**
   TCCCCGCTGGCTGCCTGGCTTCCTC
**SEQ ID 730**
   GCTGGCTGCCTGGCTTCCTCCCGCA
**SEQ ID 731**
   CTGCCTGGCTTCCTCCCGCAGCTCC
**SEQ ID 732**
   TGGCTTCCTCCCGCAGCTCCTGCTG
**SEQ ID 733**
   TCCTCCCGCAGCTCCTGCTGTGTGT
**SEQ ID 734**
   CCGCAGCTCCTGCTGTGTGTGTATG
**SEQ ID 735**
   GCTCCTGCTGTGTGTGTATGTGTGT
**SEQ ID 736**
   TGCTGTGTGTGTATGTGTGTGTGTG
**SEQ ID 737**
   TGTGTGTATGTGTGTGTGTGTGCAC
**SEQ ID 738**
   GTATGTGTGTGTGTGTGCACATCTG
**SEQ ID 739**
   TGTGTGTGTGTGCACATCTGTGGGG
**SEQ ID 740**
   GTGTGTGCACATCTGTGGGGCGTAT
**SEQ ID 741**
   TGCACATCTGTGGGGCGTATGTGTG
**SEQ ID 742**
   ATCTGTGGGGCGTATGTGTGTTCGT
**SEQ ID 743**
   TGGGGCGTATGTGTGTTCGTCTTTG
**SEQ ID 744**
   CGTATGTGTGTTCGTCTTTGTAATT
**SEQ ID 745**
   GTGTGTTCGTCTTTGTAATTGAGGC
**SEQ ID 746**
   TTCGTCTTTGTAATTGAGGCTGCAG
**SEQ ID 747**
   CTTTGTAATTGAGGCTGCAGAGTGG
**SEQ ID 748**
   TAATTGAGGCTGCAGAGTGGAGAGA
**SEQ ID 749**
   GAGGCTGCAGAGTGGAGAGAGCAGG
**SEQ ID 750**
   TGCAGAGTGGAGAGAGCAGGGGTTT
**SEQ ID 751**
   AGTGGAGAGAGCAGGGGTTTTCTCT
**SEQ ID 752**
   AGAGAGCAGGGGTTTTCTCTGGGGA
**SEQ ID 753**
   GCAGGGGTTTTCTCTGGGGACCCAG
**SEQ ID 754**
   GGTTTTCTCTGGGGACCCAGAGAGA
**SEQ ID 755**
   TCTCTGGGGACCCAGAGAGAAGGAG
**SEQ ID 756**
   GGGGACCCAGAGAGAAGGAGGCGTT
**SEQ ID 757**
   CCCAGAGAGAAGGAGGCGTTTTCAC
**SEQ ID 758**
   AGAGAAGGAGGCGTTTTCACCACAG
**SEQ ID 759**
   AGGAGGCGTTTTCACCACAGCCGAA
**SEQ ID 760**
   GCGTTTTCACCACAGCCGAACAGGG
**SEQ ID 761**
   TTCACCACAGCCGAACAGGGCAGGA
**SEQ ID 762**
   CACAGCCGAACAGGGCAGGACCCCA
**SEQ ID 763**
   CCGAACAGGGCAGGACCCCAGCACC
**SEQ ID 764**
   CAGGGCAGGACCCCAGCACCCGGGA
**SEQ ID 765**
   CAGGACCCCAGCACCCGGGACCCAG
**SEQ ID 766**
   CCCCAGCACCCGGGACCCAGCGGGA
**SEQ ID 767**
   GCACCCGGGACCCAGCGGGACTTTG
**SEQ ID 768**
   CGGGACCCAGCGGGACTTTGCCAAG
**SEQ ID 769**
   CCCAGCGGGACTTTGCCAAGGGGAT
**SEQ ID 770**
   CGGGACTTTGCCAAGGGGATGGACC
**SEQ ID 771**
   CTTTGCCAAGGGGATGGACCTGGCT
**SEQ ID 772**
   CCAAGGGGATGGACCTGGCTGGGCC
**SEQ ID 773**
   GGGATGGACCTGGCTGGGCCACGCG
**SEQ ID 774**
   GGACCTGGCTGGGCCACGCGGCTGT
**SEQ ID 775**
   TGGCTGGGCCACGCGGCTGTTTGTG
**SEQ ID 776**
   GGGCCACGCGGCTGTTTGTGTAGGG
**SEQ ID 777**
   ACGCGGCTGTTTGTGTAGGGAAAAG
**SEQ ID 778**
   GTAGAAAAGGAAGACATAAACTCCA
**SEQ ID 779**
   AAAGGAAGACATAAACTCCATTTTG
**SEQ ID 780**
   AAGACATAAACTCCATTTTGAGCTG
**SEQ ID 781**
   ATAAACTCCATTTTGAGCTGTACTA
**SEQ ID 782**
   AGAAAAATTATTTTGCCTTGACCTG
**SEQ ID 783**
   AATTATTTTGCCTTGACCTGCTGTT
**SEQ ID 784**
   TTTTGCCTTGACCTGCTGTTAACCT
**SEQ ID 785**
   CCTTGACCTGCTGTTAACCTGTAAC
**SEQ ID 786**
   ACCTGCTGTTAACCTGTAACTGTAG
**SEQ ID 787**
   CTGTTAACCTGTAACTGTAGCCCCA
**SEQ ID 788**
   AACCTGTAACTGTAGCCCCAACCCT
**SEQ ID 789**
   TGTAGCCCCAACCCTGTGCTCAAAG
**SEQ ID 790**
   TTTAAGGGATCAAGGGCTGTACAGG
**SEQ ID 791**
   GGGATCAAGGGCTGTACAGGATGTG
**SEQ ID 792**
   CAAGGGCTGTACAGGATGTGCCTTG
**SEQ ID 793**
   ATGTGCCTTGTTAACAATGTGTTTA
**SEQ ID 794**
   CCATTCTCCATTAATCAGGGGCACG
**SEQ ID 795**
   CTCCATTAATCAGGGGCACGATGCA
**SEQ ID 796**
   TTAATCAGGGGCACGATGCACTGCG
**SEQ ID 797**
   GCACGATGCACTGCGGAAAGCCACA
**SEQ ID 798**
   CCACAGGGACCTCTGCCCGAGAAAG
**SEQ ID 799**
   GGGACCTCTGCCCGAGAAAGCCTGG
**SEQ ID 800**
   CTCTGCCCGAGAAAGCCTGGGTATT
**SEQ ID 801**
   GAAAGCCTGGGTATTGTCCAAGGCT

**SEQ ID 802**
   CCTGGGTATTGTCCAAGGCTTCCCC
**SEQ ID 803**
   GTATTGTCCAAGGCTTCCCCCCACT
**SEQ ID 804**
   GTCCAAGGCTTCCCCCCACTGAGAC
**SEQ ID 805**
   AGGCTTCCCCCCACTGAGACAGCCT
**SEQ ID 806**
   CCACTGAGACAGCCTGAGATACGGC
**SEQ ID 807**
   AGGAAGGCCTCCGTCTCCTGCATGT
**SEQ ID 808**
   GGCCTCCGTCTCCTGCATGTCCTTG
**SEQ ID 809**
   CCGTCTCCTGCATGTCCTTGGGAAT
**SEQ ID 810**
   TCCTGCATGTCCTTGGGAATGGAAT
**SEQ ID 811**
   CATGTCCTTGGGAATGGAATGTCTT
**SEQ ID 812**
   CCTTGGGAATGGAATGTCTTGGTGT
**SEQ ID 813**
   GGAATGTCTTGGTGTAAAACCCGAT
**SEQ ID 814**
   GTCTTGGTGTAAAACCCGATAGTAC
**SEQ ID 815**
   GGTGTAAAACCCGATAGTACATTCC
**SEQ ID 816**
   AAAACCCGATAGTACATTCCTTCTA
**SEQ ID 817**
   CCGATAGTACATTCCTTCTATTCTG
**SEQ ID 818**
   AGTACATTCCTTCTATTCTGAGAGA
**SEQ ID 819**
   TTCTATTCTGAGAGAAGAAAACCAC
**SEQ ID 820**
   TTCTGAGAGAAGAAAACCACCCTGT
**SEQ ID 821**
   AGAGAAGAAAACCACCCTGTGGCTG
**SEQ ID 822**
   GAGGTGAGATATGCTAGCGGCAATG
**SEQ ID 823**
   GAGATATGCTAGCGGCAATGCTGCT
**SEQ ID 824**
   ATGCTAGCGGCAATGCTGCTCTGTT
**SEQ ID 825**
   TGGCCTATGTGCACATCTGGGCACA
**SEQ ID 826**
   TATGTGCACATCTGGGCACAGAACC
**SEQ ID 827**
   GCACATCTGGGCACAGAACCTCCCC
**SEQ ID 828**
   TCTGGGCACAGAACCTCCCCTTGAA
**SEQ ID 829**
   GCACAGAACCTCCCCTTGAACTTGT
**SEQ ID 830**
   GAACCTCCCCTTGAACTTGTGACAC
**SEQ ID 831**
   TCCCCTTGAACTTGTGACACAGATT
**SEQ ID 832**
   TTGAACTTGTGACACAGATTCCTTT
**SEQ ID 833**
   CTTGTGACACAGATTCCTTTGTTCA
**SEQ ID 834**
   AGATTCCTTTGTTCACATGTTTTCC
**SEQ ID 835**
   CTCCCCACTATCGCCCTGTTCTCCC
**SEQ ID 836**
   CACTATCGCCCTGTTCTCCCACCGC
**SEQ ID 837**
   TCGCCCTGTTCTCCCACCGCATTCC
**SEQ ID 838**
   CTGTTCTCCCACCGCATTCCCCTTG
**SEQ ID 839**
   CTCCCACCGCATTCCCCTTGCTGAG
**SEQ ID 840**
   TAGTAATCTGTAGATACCAAGGGAA
**SEQ ID 841**
   ATCTGTAGATACCAAGGGAACTCAG
**SEQ ID 842**
   TAGATACCAAGGGAACTCAGAGACC
**SEQ ID 843**
   ACCAAGGGAACTCAGAGACCATGGC
**SEQ ID 844**
   GGGAACTCAGAGACCATGGCCGGTG
**SEQ ID 845**
   CTCAGAGACCATGGCCGGTGCACAT
**SEQ ID 846**
   AGACCATGGCCGGTGCACATCCTCC
**SEQ ID 847**
   ATGGCCGGTGCACATCCTCCGTACG
**SEQ ID 848**
   CGGTGCACATCCTCCGTACGCTGAG
**SEQ ID 849**
   CTGAGCGCTGGTCCCCTGGGCCCAT
**SEQ ID 850**
   CGCTGGTCCCCTGGGCCCATTGTTC
**SEQ ID 851**
   CCTCAGTCTCTCATCCCTCCTGACG
**SEQ ID 852**
   GTCTCTCATCCCTCCTGACGAGAAA
**SEQ ID 853**
   TCATCCCTCCTGACGAGAAATACCC
**SEQ ID 854**
   AGGGGCTGGCCCCCTTCATCTGATG
**SEQ ID 855**
   CTGGCCCCCTTCATCTGATGCCCAA
**SEQ ID 856**
   TCATCTGATGCCCAATGTGGGTGCC
**SEQ ID 857**
   TGATGCCCAATGTGGGTGCCTTTCT
**SEQ ID 858**
   TTTCTCTAGGGTGAAGGTACTCTAC
**SEQ ID 859**
   CTAGGGTGAAGGTACTCTACAGTGT
**SEQ ID 860**
   GTGAAGGTACTCTACAGTGTGGTCA
**SEQ ID 861**
   GGTACTCTACAGTGTGGTCATTGAG
**SEQ ID 862**
   TCTACAGTGTGGTCATTGAGGACAA
**SEQ ID 863**
   GACAAGTTGACGAGAGAGTCCCAAG
**SEQ ID 864**
   GTTGACGAGAGAGTCCCAAGTACGT
**SEQ ID 865**
   CGAGAGAGTCCCAAGTACGTCCACG
**SEQ ID 866**
   CTTAGAGGAACCCAGGGTAACGATG
**SEQ ID 867**
   CAAACAGGAGAAGATATTGTTTCAG
**SEQ ID 868**
   AGGAGAAGATATTGTTTCAGTTTCT
**SEQ ID 869**
   GATGCCCCTAAAAGCTGTGTAACAG
**SEQ ID 870**
   CCCTAAAAGCTGTGTAACAGATTGT
**SEQ ID 871**
   GAAGAAGAGGCAGGGACAGAATCCC
**SEQ ID 872**
   AGAGGCAGGGACAGAATCCCAGCAA
**SEQ ID 873**
   CAGGGACAGAATCCCAGCAAGGAAC
**SEQ ID 874**
   ACAGAATCCCAGCAAGGAACGGAAA
**SEQ ID 875**
   ATCCCAGCAAGGAACGGAAAGTTCA
**SEQ ID 876**
   TGACTACAGTCAATTACAGGAGATA
**SEQ ID 877**
   ACAGGAGATAATATACCCTGAATCA
**SEQ ID 878**
   ACCACGATCGCCATCAACTCCTCCT
**SEQ ID 879**
   GATCGCCATCAACTCCTCCTCCCGT

**SEQ ID 880**
   CCATCAACTCCTCCTCCCGTGGTTC
**SEQ ID 881**
   AACTCCTCCTCCCGTGGTTCAGATG
**SEQ ID 882**
   CCTCAAACGCAGGTTAGACAAGCAC
**SEQ ID 883**
   AACGCAGGTTAGACAAGCACAAACC
**SEQ ID 884**
   AGACAAGCACAAACCCCAAGAGAAA
**SEQ ID 885**
   AGCACAAACCCCAAGAGAAAATCAA
**SEQ ID 886**
   CCAAGAGAAAATCAAGTAGAAAGGG
**SEQ ID 887**
   AGAAAATCAAGTAGAAAGGGACAGA
**SEQ ID 888**
   ATCAAGTAGAAAGGGACAGAGTCTC
**SEQ ID 889**
   GTAGAAAGGGACAGAGTCTCTATCC
**SEQ ID 890**
   AAGGGACAGAGTCTCTATCCCGGCA
**SEQ ID 891**
   TATCCCGGCAATGCCAACTCAGATA
**SEQ ID 892**
   CGGCAATGCCAACTCAGATACAGTA
**SEQ ID 893**
   AAAATAAGACCCAACCGCTGGTAGT
**SEQ ID 894**
   AAGACCCAACCGCTGGTAGTTTATC
**SEQ ID 895**
   CGCTGGTAGTTTATCAATACCGGCT
**SEQ ID 896**
   GTAGTTTATCAATACCGGCTGCCAA
**SEQ ID 897**
   TTATCAATACCGGCTGCCAACCGAG
**SEQ ID 898**
   AATACCGGCTGCCAACCGAGCTTCA
**SEQ ID 899**
   CGGCTGCCAACCGAGCTTCAGTATC
**SEQ ID 900**
   GCCAACCGAGCTTCAGTATCGGCCT
**SEQ ID 901**
   CCGAGCTTCAGTATCGGCCTCCTTC
**SEQ ID 902**
   CTTCAGTATCGGCCTCCTTCAGAGG
**SEQ ID 903**
   GTATCGGCCTCCTTCAGAGGTTCAA
**SEQ ID 904**
   GGCCTCCTTCAGAGGTTCAATACAG
**SEQ ID 905**
   CCTTCAGAGGTTCAATACAGACCTC
**SEQ ID 906**
   CACCATACCAGCAACCCACAGCGAT
**SEQ ID 907**
   GCAACCCACAGCGATGGCGTCTAAT
**SEQ ID 908**
   CCACAGCGATGGCGTCTAATTCACC
**SEQ ID 909**
   GCGATGGCGTCTAATTCACCAGCAA
**SEQ ID 910**
   GGCGTCTAATTCACCAGCAACACAG
**SEQ ID 911**
   CTAATTCACCAGCAACACAGGACGC
**SEQ ID 912**
   TCACCAGCAACACAGGACGCGGCGC
**SEQ ID 913**
   AGCAACACAGGACGCGGCGCTGTAT
**SEQ ID 914**
   CACAGGACGCGGCGCTGTATCCTCA
**SEQ ID 915**
   GACGCGGCGCTGTATCCTCAGCCGC
**SEQ ID 916**
   GGCGCTGTATCCTCAGCCGCCCACT
**SEQ ID 917**
   ATCACGTAGTGGACAGGGTGGTGCA
**SEQ ID 918**
   GTAGTGGACAGGGTGGTGCACTGCA
**SEQ ID 919**
   GGACAGGGTGGTGCACTGCATGCAG
**SEQ ID 920**
   GGGTGGTGCACTGCATGCAGTCATT
**SEQ ID 921**
   GTGCACTGCATGCAGTCATTGATGA
**SEQ ID 922**
   AAATGGTCTTTTTACTCCCTGGAAA
**SEQ ID 923**
   GTCTTTTTACTCCCTGGAAAGCCCC
**SEQ ID 924**
   AGGGAGGGTAGGCCTTTGAGGGAGA
**SEQ ID 925**
   GGGTAGGCCTTTGAGGGAGATCAAG
**SEQ ID 926**
   GGCCTTTGAGGGAGATCAAGTCTAA
**SEQ ID 927**
   CAGGAAAAGCTGCTTATTGGGCTAA
**SEQ ID 928**
   AAAGCTGCTTATTGGGCTAATCAGG
**SEQ ID 929**
   TGTTTCTGTCATCGGCATAGGTACT
**SEQ ID 930**
   CTGTCATCGGCATAGGTACTGCCTC
**SEQ ID 931**
   ATCGGCATAGGTACTGCCTCAGAAG
**SEQ ID 932**
   GGTTCAGCCTGTGATCACTTCATTC
**SEQ ID 933**
   AGCCTGTGATCACTTCATTCCAATC
**SEQ ID 934**
   GTGATCACTTCATTCCAATCAATTT
**SEQ ID 935**
   CACTTCATTCCAATCAATTTATGGG
**SEQ ID 936**
   AGGGACTAGGAAAGAAGTCCCAATT
**SEQ ID 937**
   CTAGGAAAGAAGTCCCAATTGAGGC
**SEQ ID 938**
   CCATTCCATTAACTTGGGGGAAAAA
**SEQ ID 939**
   CCATTAACTTGGGGGAAAAAAAAAC
**SEQ ID 940**
   AACTTGGGGGAAAAAAAAACAACTG
**SEQ ID 941**
   GGGGGAAAAAAAAACAACTGTATGG
**SEQ ID 942**
   AAAACAACTGTATGGTAAATCAGCA
**SEQ ID 943**
   AACTGTATGGTAAATCAGCAGCGCT
**SEQ ID 944**
   TATGGTAAATCAGCAGCGCTTCCAA
**SEQ ID 945**
   TAAATCAGCAGCGCTTCCAAAACAA
**SEQ ID 946**
   CAGCAGCGCTTCCAAAACAAAAACT
**SEQ ID 947**
   ATTAGAAAAAGGACATTGAGCCTTC
**SEQ ID 948**
   AAAAAGGACATTGAGCCTTCATTTT
**SEQ ID 949**
   ATTTTCGCCTTGGAATTCTGTTTGT
**SEQ ID 950**
   CGCCTTGGAATTCTGTTTGTAATTC
**SEQ ID 951**
   AAATCCGGCAGATGGCGTATAATGC
**SEQ ID 952**
   CGGCAGATGGCGTATAATGCCGTAA
**SEQ ID 953**
   GATGGCGTATAATGCCGTAATTCAA
**SEQ ID 954**
   TTTGCTTTTACCACACCAGCCTAAA
**SEQ ID 955**
   TTTTACCACACCAGCCTAAATAATA
**SEQ ID 956**
   AATAGTTCAACTATTTGTCAGCTCA
**SEQ ID 957**
   TTCAACTATTTGTCAGCTCAAGCTC
**SEQ ID 958**
   CTATTTGTCAGCTCAAGCTCTGCAA
**SEQ ID 959**
   TTTTCAGACTGTTACATCGTTCACT
**SEQ ID 960**
   AGACTGTTACATCGTTCACTATGTT
**SEQ ID 961**
   CTCTACTCCTTTCCGTTACTTGGGA
**SEQ ID 962**
   TCGGAATTAAATAGTGAAAGAACGT
**SEQ ID 963**
   ATAGTGAAAGAACGTTAACTCCAGA
**SEQ ID 964**
   GAAAGAACGTTAACTCCAGAGGCAA
**SEQ ID 965**
   TTTTGCTACTGCACATTCCCTAACA
**SEQ ID 966**
   CTACTGCACATTCCCTAACAGGCAT
**SEQ ID 967**
   GCACATTCCCTAACAGGCATCATTG
**SEQ ID 968**
   TTCCCTAACAGGCATCATTGTTCAA
**SEQ ID 969**
   ATTATTGACAATCGTTACCCCAAAA
**SEQ ID 970**
   TGACAATCGTTACCCCAAAACAAAA
**SEQ ID 971**
   ATCGTTACCCCAAAACAAAAATCTT
**SEQ ID 972**
   ACCTAAAGTTACCAAACATAAGCCT
**SEQ ID 973**
   ACATAAGCCTTTAAAAAATGCTCTG
**SEQ ID 974**
   AGCCTTTAAAAAATGCTCTGGCAGT
**SEQ ID 975**
   GGGCCAAAAGAATGAGTCATCAAAA
**SEQ ID 976**
   AAAAGAATGAGTCATCAAAACTCAG
**SEQ ID 977**
   AATGAGTCATCAAAACTCAGTATCA
**SEQ ID 978**
   GTCATCAAAACTCAGTATCACTTGA
**SEQ ID 979**
   CAAAACTCAGTATCACTTGACTCAA
**SEQ ID 980**
   CTCAGTATCACTTGACTCAAAGAGC
**SEQ ID 981**
   TATCACTTGACTCAAAGAGCAGAGT
**SEQ ID 982**
   CTTGACTCAAAGAGCAGAGTTGGTT
**SEQ ID 983**
   TGGTTGCCGTCATTACAGTGTTAAC
**SEQ ID 984**
   GCCGTCATTACAGTGTTAACAAGAT
**SEQ ID 985**
   CAGGCTACAAAGGATATTGAGAGAG
**SEQ ID 986**
   TACAAAGGATATTGAGAGAGCCCTA
**SEQ ID 987**
   AGGATATTGAGAGAGCCCTAATCAA
**SEQ ID 988**
   ATTGAGAGAGCCCTAATCAAATACA
**SEQ ID 989**
   TTATGGATGATCAGTTAAACCCGCT
**SEQ ID 990**
   TCAGTTAAACCCGCTGTTTAATTTG
**SEQ ID 991**
   TAAACCCGCTGTTTAATTTGTTACA
**SEQ ID 992**
   TTGACTCATGTAAATGCAATAGGAT
**SEQ ID 993**
   CATTGCACCCAGTGTCAGATTCTAC
**SEQ ID 994**
   AGTGTCAGATTCTACACCTGGCCAC
**SEQ ID 995**
   CAGATTCTACACCTGGCCACTCAGG
**SEQ ID 996**
   TCTACACCTGGCCACTCAGGAGGCA
**SEQ ID 997**
   ACCTGGCCACTCAGGAGGCAAGAGT
**SEQ ID 998**
   GCCACTCAGGAGGCAAGAGTTAATC
**SEQ ID 999**
   ATGGCAAATGGATGTCATGCACGTA
**SEQ ID 1000**
   AAATGGATGTCATGCACGTACCTTC
**SEQ ID 1001**
   GATGTCATGCACGTACCTTCATTTG
**SEQ ID 1002**
   CATGCACGTACCTTCATTTGGAAAA
**SEQ ID 1003**
   GTTCCAGAAAAAGTTAAAACAGACA
**SEQ ID 1004**
   AGAAAAAGTTAAAACAGACAATGGG
**SEQ ID 1005**
   AAGTTAAAACAGACAATGGGCCAGG
**SEQ ID 1006**
   AAAACAGACAATGGGCCAGGTTACT
**SEQ ID 1007**
   AGACAATGGGCCAGGTTACTGTAGT
**SEQ ID 1008**
   CCAGGTTACTGTAGTAAAGCAGTTC
**SEQ ID 1009**
   AAACAAAAAAAAGGAAAAGACAGGA
**SEQ ID 1010**
   AAGGAAAAGACAGGAGTATAACACT
**SEQ ID 1011**
   AAAGACAGGAGTATAACACTCCCCA
**SEQ ID 1012**
   CACTACCTCTGCAGAACAACATCTT
**SEQ ID 1013**
   AAAATAAAACATGGGAAATGGGGAA
**SEQ ID 1014**
   AAAACATGGGAAATGGGGAAGGTGA
**SEQ ID 1015**
   TAAAGTTCTACAATGAACTCACTGG
**SEQ ID 1016**
   TTCTACAATGAACTCACTGGAGATG
**SEQ ID 1017**
   CAATGAACTCACTGGAGATGCAAAG
**SEQ ID 1018**
   AACTCACTGGAGATGCAAAGAAAAG
**SEQ ID 1019**
   ACTGGAGATGCAAAGAAAAGTGTGG
**SEQ ID 1020**
   AGATGCAAAGAAAAGTGTGGAGATG
**SEQ ID 1021**
   CAAAGAAAAGTGTGGAGATGGAGAC
**SEQ ID 1022**
   AAAAGTGTGGAGATGGAGACACCCC
**SEQ ID 1023**
   TGTGGAGATGGAGACACCCCAATCG
**SEQ ID 1024**
   AGATGGAGACACCCCAATCGACTCG
**SEQ ID 1025**
   GAGACACCCCAATCGACTCGCCAGG
**SEQ ID 1026**
   ACCCCAATCGACTCGCCAGGTAAAC
**SEQ ID 1027**
   AATCGACTCGCCAGGTAAACAAAAT
**SEQ ID 1028**
   GGTGATATCAGAAGAACAGAAAAAG
**SEQ ID 1029**
   TATCAGAAGAACAGAAAAAGTTGCC
**SEQ ID 1030**
   GAAGAACAGAAAAAGTTGCCTTCCA
**SEQ ID 1031**
   ACAGAAAAAGTTGCCTTCCATCAAG
**SEQ ID 1032**
   AAAAGTTGCCTTCCATCAAGGAAGC
**SEQ ID 1033**
   TTGCCTTCCATCAAGGAAGCAGAGT
**SEQ ID 1034**
   TTCCATCAAGGAAGCAGAGTTGCCA
**SEQ ID 1035**
   TCAAGGAAGCAGAGTTGCCAATATA
**SEQ ID 1036**
   GAAGCAGAGTTGCCAATATAGGCAC
**SEQ ID 1037**
   AGAGTTGCCAATATAGGCACAATTA
**SEQ ID 1038**
   TGCCAATATAGGCACAATTAAAGAA
**SEQ ID 1039**
   ATATAGGCACAATTAAAGAAGCTGA
**SEQ ID 1040**
   CACAGTTAGCTAAAAAAAAAAGCCT
**SEQ ID 1041**
   AAAAAAGCCTAGAGAATACAAAGGT
**SEQ ID 1042**
   AGCCTAGAGAATACAAAGGTGACAC
**SEQ ID 1043**
   AGAGAATACAAAGGTGACACCAACT
**SEQ ID 1044**
   ATACAAAGGTGACACCAACTCCAGA
**SEQ ID 1045**
   AAGGTGACACCAACTCCAGAGAATA
**SEQ ID 1046**
   GACACCAACTCCAGAGAATATGCTG
**SEQ ID 1047**
   GAATATGCTGCTTGCAGCTCTGATG
**SEQ ID 1048**
   ATCAACGGTGGTAAGTCTTCCCAAG
**SEQ ID 1049**
   CGGTGGTAAGTCTTCCCAAGTCTGC
**SEQ ID 1050**
   GTAAGTCTTCCCAAGTCTGCAGGAG
**SEQ ID 1051**
   TCTTCCCAAGTCTGCAGGAGCAGCT
**SEQ ID 1052**
   CCTTAATTCGGGCAGTTACATAGAT
**SEQ ID 1053**
   ATTCGGGCAGTTACATAGATGGATA
**SEQ ID 1054**
   GGCAGTTACATAGATGGATAATCCT
**SEQ ID 1055**
   TAGTGCATGGGTGCCTGGCCCCACA
**SEQ ID 1056**
   CATGGGTGCCTGGCCCCACAGATGA
**SEQ ID 1057**
   GTGCCTGGCCCCACAGATGACTGTT
**SEQ ID 1058**
   TGGCCCCACAGATGACTGTTGCCCT
**SEQ ID 1059**
   GCCCAACCTGAAGAAGGAATGATGA
**SEQ ID 1060**
   CATTGGGTATCCTTATCCTCCTGTT
**SEQ ID 1061**
   GGTATCCTTATCCTCCTGTTTGCCT

**SEQ ID 1062**
   CCTTATCCTCCTGTTTGCCTAGGGA
**SEQ ID 1063**
   CCTACAGTCAGTGCTACCAGTAGAT
**SEQ ID 1064**
   AGTCAGTGCTACCAGTAGATTTACT
**SEQ ID 1065**
   CATGGTAAGTGGAATGTCACAGATA
**SEQ ID 1066**
   TCATTACAATGTAGGCCTAAGGGGA
**SEQ ID 1067**
   ACAATGTAGGCCTAAGGGGAAGGCT
**SEQ ID 1068**
   GTAGGCCTAAGGGGAAGGCTTGCCC
**SEQ ID 1069**
   CAAAAAGCCCAGAAGTCTTAGTCTG
**SEQ ID 1070**
   AGCCCAGAAGTCTTAGTCTGCGGAG
**SEQ ID 1071**
   AGAAGTCTTAGTCTGCGGAGAATGT
**SEQ ID 1072**
   TCTTAGTCTGCGGAGAATGTGTGGC
**SEQ ID 1073**
   GTCTGCGGAGAATGTGTGGCTGATA
**SEQ ID 1074**
   GTGGCTGATACTGCAGTGTAGTACA
**SEQ ID 1075**
   TGATACTGCAGTGTAGTACAAAACA
**SEQ ID 1076**
   CTGCAGTGTAGTACAAAACAATGAA
**SEQ ID 1077**
   TTTTGAACTATGATAGACTGGGTCC
**SEQ ID 1078**
   AACTATGATAGACTGGGTCCCTTGA
**SEQ ID 1079**
   TGATAGACTGGGTCCCTTGAGGCCA
**SEQ ID 1080**
   GGTCCCTTGAGGCCAATTATATCAT
**SEQ ID 1081**
   CTTGAGGCCAATTATATCATAACTG
**SEQ ID 1082**
   ATTATATCATAACTGTACAGGCCAG
**SEQ ID 1083**
   ATCATAACTGTACAGGCCAGACTCA
**SEQ ID 1084**
   AACTGTACAGGCCAGACTCATTCAT
**SEQ ID 1085**
   TACAGGCCAGACTCATTCATGTTCA
**SEQ ID 1086**
   TGGCCCATTAATCCAGCCTATGACG
**SEQ ID 1087**
   CATTAATCCAGCCTATGACGGTGAT
**SEQ ID 1088**
   ATCCAGCCTATGACGGTGATGTAAC
**SEQ ID 1089**
   GCCTATGACGGTGATGTAACTGAAA
**SEQ ID 1090**
   TGACGGTGATGTAACTGAAAGGCTG
**SEQ ID 1091**
   ATAGAAGGTTAGAATCACTCTGTCC
**SEQ ID 1092**
   AGGTTAGAATCACTCTGTCCAAGGA
**SEQ ID 1093**
   AGAATCACTCTGTCCAAGGAAATGG
**SEQ ID 1094**
   CACTCTGTCCAAGGAAATGGGGTGA
**SEQ ID 1095**
   TGTCCAAGGAAATGGGGTGAAAAGG
**SEQ ID 1096**
   TCATCACCTTGACCAAAGTTAGTCC
**SEQ ID 1097**
   ACCTTGACCAAAGTTAGTCCTGTTA
**SEQ ID 1098**
   GACCAAAGTTAGTCCTGTTACTGGT
**SEQ ID 1099**
   CCTGAACATCCAGAATTAGGAAGCT
**SEQ ID 1100**
   ACATCCAGAATTAGGAAGCTTACTG
**SEQ ID 1101**
   CAGAATTAGGAAGCTTACTGTGGCC
**SEQ ID 1102**
   TTAGGAAGCTTACTGTGGCCTCACA
**SEQ ID 1103**
   TTCTGGAAATCAAGCTATAGGAACA
**SEQ ID 1104**
   TCCTTTGCAAAATTGTGTAAAACTC
**SEQ ID 1105**
   TGCAAAATTGTGTAAAACTCCCTTA
**SEQ ID 1106**
   AACTCCCTTATATTGCTAGTTGTAG
**SEQ ID 1107**
   GTTATTAAACCTGATTCCCAAACCA
**SEQ ID 1108**
   TAAACCTGATTCCCAAACCATAATC
**SEQ ID 1109**
   CTGATTCCCAAACCATAATCTGTGA
**SEQ ID 1110**
   TCCCAAACCATAATCTGTGAAAATT
**SEQ ID 1111**
   AACCATAATCTGTGAAAATTGTGGA
**SEQ ID 1112**
   TAATCTGTGAAAATTGTGGAATGTT
**SEQ ID 1113**
   TGTGAAAATTGTGGAATGTTTACTT
**SEQ ID 1114**
   AAATTGTGGAATGTTTACTTGCATT
**SEQ ID 1115**
   GTGGAATGTTTACTTGCATTGATTT
**SEQ ID 1116**
   GCACCGTATTCTACTAGGAAGAGCA
**SEQ ID 1117**
   GTATTCTACTAGGAAGAGCAAGAGA
**SEQ ID 1118**
   CTACTAGGAAGAGCAAGAGAGGGTG
**SEQ ID 1119**
   AGGAAGAGCAAGAGAGGGTGTGTGG
**SEQ ID 1120**
   ATCCTTGTGTCCATGGACCGACCAT
**SEQ ID 1121**
   GACCGACCATGGGAGGCTTCGCTAT
**SEQ ID 1122**
   ACCATGGGAGGCTTCGCTATCCATC
**SEQ ID 1123**
   GGGAGGCTTCGCTATCCATCCATAT
**SEQ ID 1124**
   GCTTCGCTATCCATCCATATTTTAA
**SEQ ID 1125**
   GCTATCCATCCATATTTTAACGGAA
**SEQ ID 1126**
   TTGATGGCAGTGATTATGGGCCTCA
**SEQ ID 1127**
   GGCAGTGATTATGGGCCTCATTGCA
**SEQ ID 1128**
   CAGAATACGTAAATGATTGGCAAAA
**SEQ ID 1129**
   TACGTAAATGATTGGCAAAAGAATT
**SEQ ID 1130**
   TCATTTGGATGGGAGAGGCTCATGA
**SEQ ID 1131**
   TGGATGGGAGAGGCTCATGAGCTTG
**SEQ ID 1132**
   GGGAGAGGCTCATGAGCTTGGAATA
**SEQ ID 1133**
   TCTTTTTCAGTTACGATGTGACTGG
**SEQ ID 1134**
   TTCAGTTACGATGTGACTGGAATAC
**SEQ ID 1135**
   TTACGATGTGACTGGAATACATCAG
**SEQ ID 1136**
   ATCAGATTTTTGTGTTACACCACAA
**SEQ ID 1137**
   ATTTTTGTGTTACACCACAAGCCTA
**SEQ ID 1138**
   TGTGTTACACCACAAGCCTATAATG
**SEQ ID 1139**
   ATGGTTAGATGCCATCTGCAAGGAG
**SEQ ID 1140**
   TAGATGCCATCTGCAAGGAGGAGAA
**SEQ ID 1141**
   GCCATCTGCAAGGAGGAGAAGATAA
**SEQ ID 1142**
   CTGCAAGGAGGAGAAGATAATCTTA
**SEQ ID 1143**
   TAAATTTGGTGCCAGGAACGGAGAC
**SEQ ID 1144**
   TTGGTGCCAGGAACGGAGACAATCG
**SEQ ID 1145**
   GCCAGGAACGGAGACAATCGTGAAA
**SEQ ID 1146**
   GAACGGAGACAATCGTGAAAGCTGC
**SEQ ID 1147**
   GAGACAATCGTGAAAGCTGCTGATA
**SEQ ID 1148**
   GCCTCACAAATCTTAAGCCAGTCAC
**SEQ ID 1149**
   ACAAATCTTAAGCCAGTCACTTGGG
**SEQ ID 1150**
   TCTTAAGCCAGTCACTTGGGTTAAA
**SEQ ID 1151**
   AGCCAGTCACTTGGGTTAAAAGCAT
**SEQ ID 1152**
   TTGGGTTAAAAGCATCAGAAGTTTC
**SEQ ID 1153**
   AGCATCAGAAGTTTCACTATTGTAA
**SEQ ID 1154**
   AGCTCCAAAGAGACAGCAACCAGCA
**SEQ ID 1155**
   CAAAGAGACAGCAACCAGCAAGAAT
**SEQ ID 1156**
   AGACAGCAACCAGCAAGAATGGGCC
**SEQ ID 1157**
   GCAACCAGCAAGAATGGGCCATAGT
**SEQ ID 1158**
   CAGCAAGAATGGGCCATAGTGACGA
**SEQ ID 1159**
   AGAATGGGCCATAGTGACGATGGTG
**SEQ ID 1160**
   GGGCCATAGTGACGATGGTGGTTTT
**SEQ ID 1161**
   ATAGTGACGATGGTGGTTTTGTCAA
**SEQ ID 1162**
   GACGATGGTGGTTTTGTCAAAAAGA
**SEQ ID 1163**
   GTCAAAAAGAAAAGGGGGGGATATG
**SEQ ID 1164**
   AAAGAAAAGGGGGGGATATGTAAGG
**SEQ ID 1165**
   AAAGGGGGGGATATGTAAGGAAAAG
**SEQ ID 1166**
   TAAGGAAAAGAGAGATCAGACTTTC
**SEQ ID 1167**
   AAAAGAGAGATCAGACTTTCACTGT
**SEQ ID 1168**
   CTTTCACTGTGTCTATGTAGAAAAG
**SEQ ID 1169**
   ACTAAGAAAAATTGTTTTGCCTTGA
**SEQ ID 1170**
   TGCTCACGGAAACATGTGCTGTAAG
**SEQ ID 1171**
   ACGGAAACATGTGCTGTAAGGTTTA
**SEQ ID 1172**
   AACATGTGCTGTAAGGTTTAAGGGA
**SEQ ID 1173**
   GTGCTGTAAGGTTTAAGGGATCTAG
**SEQ ID 1174**
   TGCAGGATGTACCTTGTTAACAATA
**SEQ ID 1175**
   ACCTTGTTAACAATATGTTTGCAGG
**SEQ ID 1176**
   CAATATGTTTGCAGGCAGTATGTTT
**SEQ ID 1177**
   TGTTTGCAGGCAGTATGTTTGGTAA
**SEQ ID 1178**
   GCAGGCAGTATGTTTGGTAAAAGTC
**SEQ ID 1179**
   ATTAACCAGGGGCTCAATGCACTGT
**SEQ ID 1180**
   GGCTCAATGCACTGTGGAAAGCCAC
**SEQ ID 1181**
   AATGCACTGTGGAAAGCCACAGGAA
**SEQ ID 1182**
   GGAAAGCCACAGGAACCTCTGCCCA
**SEQ ID 1183**
   GCCACAGGAACCTCTGCCCAAGAAA
**SEQ ID 1184**
   AGGAACCTCTGCCCAAGAAAGCCTG
**SEQ ID 1185** 
AGCTTACAAAACAAAATGGGGCAAACTGAAAGTAAATATGCCTCTTATCTCAGCTTTATTAAAATTCTTTTAAAAAGAGGGGGAGTTAG
AGTATCTACAAAAAATCTAATCAAGCTATTTCAAATAATAGAACAATTTTGCCCATGGTTTCCAGAACAAGGAACTTTAGATCTAAAAG
ATTGGAAAAGAATTGGCGAGGAACTAAAACAAGCAGGTAGAAAGGGTAATATCATTCCACTTACAGTATGGAATGATTGGGCCATTATT
AAAGCAGCTTTAGAACCATTTCAAACAAAAGAAGATAGCGTTTCAGTTTCTGATGCCCCTGGAAGCTGTGTAATAGATTGTAATGAAAA
GACAGGGAGAAAATCCCAGAAAGAAACAGAAAGTTTACATTGCGAATATGTAACAGAGCCAGTAATGGCTCAGTCAACGCAAAATGTTG
ACTATAATCAATTACAGGGGGTGATATATCCTGAAACGTTAAAATTAGAAGGAAAAGGTCCAGAATTAGTGGGGCCATCAGAGTCTAAA
CCACGAGGGCCAAGTCCTCTTCCAGCAGGTCAGGTGCCCGTAACATTACAACCTCAAACGCAGGTTAAAGAAAATAAGACCCAACCGCC
AGTAGCTTATCAATACTGGCCGCCGGCTGAACTTCAGTATCTGCCACCCCCAGAAAGTCAGTATGGATATCCAGGAATGCCCCCAGCAC
TACAGGGCAGGGCGCCATATCCTCAGCCGCCCACTGTGAGACTTAATCCTACAGCATCACGTAGTGGACAAGGTGGTACACTGCACGCA
GTCATTGATGAAGCCAGAAAACAGGGAGATCTTGAGGCATGGCGGTTCCTGGTAATTTTACAACTGGTACAGGCCGGGGAAGAGACTCA
AGTAGGAGCGCCTGCCCGAGCTGAGACTAGATGTGAACCTTTCACCATGAAAATGTTAAAAGATATAAAGGAAGGAGTTAAACAATATG
GATCCAACTCCCCTTATATAAGAACATTATTAGATTCCATTGCTCATGGAAATAGACTTACTCCTTATGACTGGGAAAGTTTGGCCAAA
TCTTCCCTTTCATCCTCTCAGTATCTACAGTTTAAAACCT[?][?][?][?]GATTGATGGAGTACAAGAACAGGTACGAAAAAATCAGGCTACTAA
 
GCCCACTGTTAATATAGACGCAGACCAATTGTTAGGAACAGGTCCAAATTGGAGCACCATTAACCAACAATCAGTGATGCAGAATGAGG
CTATTGAACAAGTAAGGGCTATTTGCCTCAGGGCCTGGGGAAAAATTCAGGACCCAGGAACAGCTTTCCCTATTAATTCAATTAGACAA
GGCTCTAAAGAGCCATATCCTGACTTTGTGGCAAGATTACAAGATGCTGCTCAAAAGTCTATTACAGATGACAATGCCCGAAAAGTTAT
TGTAGAATTAATGGCCTATGAAAATGCAAATCCAGAATGTCAGTCGGCCATAAAGCCATTAAAAGGAAAAGTTCCAGCAGGAGTTGATG
TAATTACAGAATATGTGAAGGCTTGTGATGGGATTGGAGGAGCTATGCATAAGGCAATGCTAATGGCTCAAGCAATGAGGGGGCTCACT
CTAGGAGGACAAGTTAGAACATTTGGGAAAAAATGTTATAATTGTGGTCAAATCGGTCATCTGAAAAGGAGTTGCCCAGTCTTAAATAA
ACAGAATATAATAAATCAAGCTATTACAGCAAAAAATAAAAAGCCATCTGGCCTGTGTCCAAAATGTGGAAAAGGAAAACATTGGGCCA
ATCAATGTCATTCTAAATTTGATAAGGATGGGCAACCATTGTCGGGAAACAGGAAGAGGGGCCAGCCTCAGGCCCCCCAACAAACTGGG
GCATTCCCAGTTCAACTGTTTGTTCCTCAGGGTTTCAAGGACAACAACCCCTACAGAAAATACCACCACTTCAGGGAGTCAGCCAATT
ACAACAATCCAACAGCTGTCCCGCGCCACAGCAGGCAGCACCGCAGTAGTAAGTCGAC
**SEQ ID 1186** 
MGQTESKYASYLSFIKILLKRGGVRVSTKNLIKLFQIIEQFCPWFPEQGTLDLKDWKRIGEELKQAGRKGNIIPLTVWNDWAIIKAALE
PFQTKEDSVSVSDAPGSCVIDCNEKTGRKSQKETESLHCEYVTEPVMAQSTQNVDYNQLQGVIYPETLKLEGKGPELVGPSESKPRGPS
PLPAGQVPVTLQPQTQVKENKTQPPVAYQYWPPAELQYLPPPESQYGYPGMPPALQGRAPYPQPPTVRLNPTASRSGQGGTLHAVIDEA
RKQGDLEAWRFLVILQLVQAGEETQVGAPARAETRCEPFTMKMLKDIKEGVKQYGSNSPYIRTLLDSIAHGNRLTPYDWESLAKSSLSS
SQYLQFKTWWIDGVQEQVRKNQATKPTVNIDADQLLGTGPNWSTINQQSVMQNEAIEQVRAICLRAWGKIQDPGTAFPINSIRQGSKEP
YPDFVARLQDAAQKSITDDNARKVIVELMAYENANPECQSAIKPLKGKVPAGVDVITEYVKACDGIGGAMHKAMLMAQAMRGLTLGGQV
RTFGKKCYNCGQIGHLKRSCPVLNKQNIINQAITAKNKKPSGLCPKCGKGKHWANQCHSKFDKDGQPLSGNRKRGQPQAPQQTGAFPVQ
LFVPQGFQGQQPLQKIPPLQGVSQLQQSNSCPAPQQAAPQ
**SEQ ID 1187** 
AGCTTACAAAACAAAATGGGGCAAACTGAAAGTAAATATGCCTCTTATCTCAGCTTTATTAAAATTCTTTTAAGAAGAGGGGGAGTTAG
AGCTTCTACAGAAAATCTAATTACGCTATTTCAAACAATAGAACAATTCTGCCCATGGTTTCCAGAACAGGGAACTTTAGATCTAAAAG
ATTGGGAAAAAATTGGCAAAGAATTAAAACAAGCAAATAGGGAAGGTAAAATCATCCCACTTACAGTATGGAATGATTGGGCCATTATT
AAAGCAACTTTAGAACCATTTCAAACAGGAGAAGATATTGTTTCAGTTTCTGATGCCCCTAAAAGCTGTGTAACAGATTGTGAAGAAGA
GGCAGGGACAGAATCCCAGCAAGGAACGGAAAGTTCACATTGTAAATATGTAGCAGAGTCTGTAATGGCTCAGTCAACGCAAAATGTTG
ACTACAGTCAATTACAGGAGATAATATACCCTGAATCATCAAAATTGGGGGAAGGAGGTCCAGAATCATTGGGGCCATCAGAGCCTAAA
CCACGATCGCCATCAACTCCTCCTCCCGTGGTTCAGATGCCTGTAACATTACAACCTCAAACGCAGGTTAGACAAGCACAAACCCCAAG
AGAAAATCAAGTAGAAAGGGACAGAGTCTCTATCCCGGCAATGCCAACTCAGATACAGTATCCACAATATCAGCCGGTAGAAAATAAGA
CCCAACCGCTGGTAGTTTATCAATACCGGCTGCCAACCGAGCTTCAGTATCGGCCTCCTTCAGAGGTTCAATACAGACCTCAAGCGGTG
TGTCCTGTGCCAAATAGCACGGCACCATACCAGCAACCCACAGCGATGGCGTCTAATTCACCAGCAACACAGGACGCGGCGCTGTATCC
TCAGCCGCCCACTGTGAGACTTAATCCTACAGCATCACGTAGTGGACAGGGTGGTGCACTGCATGCAGTCATTGATGAAGCCAGAAAAC
AGGGCGATCTTGAGGCATGGCGGTTCCTGGTAATTTTACAACTGGTACAGGCCGGGGAAGAGACTCAAGTAGGAGCGCCTGCCCGAGCT
GAGACTAGATGTGAACCTTTCACCATGAAAATGTTAAAAGATATAAAGGAAGGAGTTAAACAATATGGATCCAACTCCCCTTATATAAG
AACATTATTAGATTCCATTGCTCATGGAAATAGACTTACTCCTTATGACTGGGAAATTTTGGCCAAATCTTCCCTTTCATCCTCTCAGT
ATCTACAGTTTAAAACCTGGTGGATTGATGGAGTACAAGAACAGGTACGAAAAAATCAGGCTACTAAGCCCACTGTTAATATAGACGCA
GACCAATTGTTAGGAACAGGTCCAAATTGGAGCACCATTAACCAACAATCAGTGATGCAGAATGAGGCTATTGAACAAGTAAGGGCTAT
TTGCCTCAGGGCCTGGGGAAAAATTCAGGACCCAGGAACAGCTTTCCCTATTAATTCAATTAGACAAGGCTCTAAAGAGCCATATCCTG
ACTTTGTGGCAAGATTACAAGATGCTGCTCAAAAGTCTATTACAGATGACAATGCCCGAAAAGTTATTGTAGAATTAATGGCCTATGAA
AATGCAAATCCAGAATGTCAGTCGGCCATAAAGCCATTAAAAGGAAAAGTTCCAGCAGGAGTTGATGTAATTACAGAATATGTGAAGGC
TTGTGATGGGATTGGAGGAGCTATGCATAAGGCAATGCTAATGGCTCAAGCAATGAGGGGGCTCACTCTAGGAGGACAAGTTAGAACAT
TTGGGAAAAAATGTTATAATTGTGGTCAAATCGGTCATCTGAAAAGGAGTTGCCCAGTCTTAAATAAACAGAATATAATAAATCAAGCT
ATTACAGCAAAAAATAAAAAGCCATCTGGCCTGTGTCCAAAATGTGGAAAAGGAAAACATTGGGCCAATCAATGTCATTCTAAATTTGA
TAAGGATGGGCAACCATTGTCGGGAAACAGGAAGAGGGGCCAGCCTCAGGCCCCCCAACAAACTGGGGCATTCCCAGTTCAACTGTTTG
TTCCTCAGGGTTTTCAAGGACAACAACCCCTACAGAAAATACCACCACTTCAGGGAGTCAGCCAATTACAACAATCCAACAGCTGTCCC
GCGCCACAGCAGGCAGCACCGCAGTAGTAAGTCGAC
**SEQ ID 1188** 
MGQTESKYASYLSFIKILLRRGGVRASTENLITLFQTIEQFCPWFPEQGTLDLKDWEKIGKELKQANREGKIIPLTVWNDWAIIKATLE
PFQTGEDIVSVSDAPKSCVTDCEEEAGTESQQGTESSHCKYVAESVMAQSTQNVDYSQLQEIIYPESSKLGEGGPESLGPSEPKPRSPS
TPPPVVQMPVTLQPQTQVRQAQTPRENQVERDRVSIPAMPTQIQYPQYQPVENKTQPLVVYQYRLPTELQYRPPSEVQYRPQAVCPVPN
STAPYQQPTAMASNSPATQDAALYPQPPTVRLNPTASRSGQGGALHAVIDEARKQGDLEAWRFLVILQLVQAGEETQVGAPARAETRCE
PFTMKMLKDIKEGVKQYGSNSPYIRTLLDSIAHGNRLTPYDWEILAKSSLSSSQYLQFKTWWIDGVQEQVRKNQATKPTVNIDADQLLG
TGPNWSTINQQSVMQNEAIEQVRAICLRAWGKIQDPGTAFPINSIRQGSKEPYPDFVARLQDAAQKSITDDNARKVIVELMAYENANPE
 
CQSAIKPLKGKVPAGVDVITEYVKACDGIGGAMHKAMLMAQAMRGLTLGGQVRTFGKKCYNCGQIGHLKRSCPVLNKQNIINQAITAKN
KKPSGLCPKCGKGKHWANQCHSKFDKDGQPLSGNRKRGQPQAPQQTGAFPVQLFVPQGFQGQQPLQKIPPLQGVSQLQQSNSCPAPQQA
APQ
**SEQ ID 1189**
   GKPIPNPLLGLDST
**SEQ ID 1190** 
TGTGGGGAAAAGCAAGAGAGATCAGATTGTCACTGTATCTGTGTAGAAAGAAGTAGACATGGGAGACTCCATTTTGTTATGTACTAAGA
AAAATTCTTCTGCCTTGAGATTCTGTGACCTTACCCCCAACCCCGTGCTCTCTGAAACATGTGCTGTGTCAAACTCAGGGTTAAATGGA
TTAAGGGCGGTGCAGGATGTGCTTTGTTAAACAGATGCTTGAAGGCAGCATGCTCCTTAAGAGTCATCACCACTCCCTAATCTCAAGTA
CCCAGGGACACAAACACTGCGGAAGGCCGCAGGGACCTCTGCCTAGGAAAGCCAGGTATTGTCCAAGGTTTCTCCCCATGTGATAGTCT
GAAATATGGCCTCGTGGGAAGGGAAAGACCTGACCGTCCCCCAGCCCGACACCCGTAAAGGGTCTGTGCTGAGGAGGATTAGTAAAAGA
GGAAGGCATGCCTCTTGCAGTTGAGACAAGAGGAAGGCATCTGTCTCCTGCCCGTCCCTGGGCAATGGAATGTCTCGGTATAAAACCGG
ATTGTACGTTCCATCTACTGAGATAGGGAAAAACCGCCTTAGGGCTGGAGGTGGGACCTGCGGGCAGCAATACTGCTTTTTAAAGCATT
GAGATGTTTATGTGTATGCATATCTAAAAGCACAGCACTTAATCCTTTACCTTGTCTATGATGCAAAGATCTTTGTTCACGTGTTTGTC
TGCTGACCCTCTCCCCACTATTGTCTTGTGACCCTGACACATCCCCCTCTCGGAGAAACACCCACGAATGACCAATAAATACTAAAGGG
AACTCAGAGGCTGGCGGGATCCTCCATATGCTGAACGCTGGTTCCCCGGGCCCCCTTATTTCTTTCTCTACACTTTGTCTCTGTGTCTT
TTTCTTTCCTAAGTCTCTCGTTCCACCTTACGAGAAACACCCACAGGTGTGGAGGGGCAACCCACCCCTACA
**SEQ ID 1191** 
GGGTGAAGGTACTCTACAGTGTGGTCATTGAGGACAAGTTGACGAGAGAGTCCCAAGTACGTCCACGGTCAGCCTTGCGACATTTAAAG
TTCTACAATGAACTCACTGGAGATGCAAAGAAAAGTGTGGAGATGGAGACACCCCAATCGACTCGCCAGTCTACAGGTGTATCCAGCAG
CTCCAAAGAGACAGCAACCAGCAAGAATGGGCCATAGTGACGATGGTGGTTTGTCAAAAAGAAAAGGGGGGGATATGTAAGGAAAAGA
GAGATCAGACTTTCACTGTGTCTATGTAGAAAAGGAAGACATAAGAAACTCCATTTTGTTCTGTACTAAGAAAAATTGTTTTGCCTTGA
GATGCTGT
**SEQ ID 1192**
   CTCGTTCCACCTGAGGAGAAATGCC
**SEQ ID 1193**
   GAGGCGCAGGCCACTCCATC
**SEQ ID 1194**
   CTTGTCCTCAATGACCACACTGTAGAG
**SEQ ID 1195**
   AGAGTACCTTCACCCACAAGGCTC
**SEQ ID 1196** 
TGTAGGGAAAAGAAAGAGAGATCACACTGTTACTGTGTCTATGTAGAAAAAGGAAGACATAAGAAACTCCATTTTGATCTGTACTAAGA
AAAATTCTTCTGCTTTGAAATGCTATTAATCTGTAACCCTAGCCCCAACCCTGTGCTCACAGAAACATGCGCTGTATTGACTCAAGGTT
AATGGATTTAGGGCTGTGCAGGATGTGCTTTGTTAACAATGTGTTTGAAGGCAGTATGCTTGGTAAAGGTCATCGCCATTCTCCAGTCT
TGAGTACCCAGGGACACAATGCACTGTGGAAAGCCATGGGGACCTCTGCCCAAGAAAGCCTGGGTGTTGTCCAGGCTTCCCCACACTGA
GACAGCCTGAGATGTGGCCTCGTTGGAAGGGAAAGACCTTACATTATAGTCCCCCAGCCGGACACCCATAAAAGGTCTGTGCTGAGGAG
GATTACTGAAAGAGGAAGGCCTCTTTGCAGTTAAGAGGAAAGCATCTGTCTCATGATCCCCTGGGAATGGAATGTCTTGGTGTAAAACC
TGATCGTACATTCTATTTACTGAGATAGGAGAAAACCGCCCTATGGCTGGAGGTGAGACATGCTGGTGGCAATACCGATCTTTACTGCA
CGGCAATACTGATCTTTACTGCACTGAGATGTTTATGTAAAGTTAAACATAAATCTAGCCTACGTGCACATTCAGGCATAGCACCTTTC
CTTAAACTTATTTATGACACAGAGTCTTTTGTTCACGTGTTTTCCTGTTGACCCTCTCTCCACCATTACCCTATAGTCCTGCCACATCC
CCCTCACTGAGATAGTAGAGATAATGATCAATAAATACTGAGGGAATTCAGAAACCAGTGCCGGTGCAGGTCCTCACTTGCTGAGTGCC
GGTCCCCTGGGCCCACTTTTCTTCCTCTATGCTTTACCTCTGTGTCTTATTTCTTTTCTCAGTCTCTCGTCTCCACCTTGCGAGAAATA
CCCACAGGTGTGGAGGGGCTGGCCCCCTTCA
**SEQ ID 1197** 
UUAUGUAUAUGCACAUCAAAAGCACAGCACUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUGGUAAG
CUUGGGCGCUCGGAAGAAGCCAGGGUUAAUGGGGCAAACUAAAAGUAAAGUCUCUCAUUCCACCUGAUGAGAAACACCCAGAGGUGUGG
 
AGGGGCAGGCCACCCCUUCAGGGUAGGGUCCCCUCCAUGCAGACCAUAGAGCACAGGUGUGCCCCAAAGAGGAGCAGAGAGAAGGAGGG
AGAGGGCCCACGAGAGACUUGGAAAUGAAUGGCAGGAUUUUAGGCGCUGGACUUGGGUUCGGGGCACCUGGCCUUUCCUUGUGUAUUUC
UCCUACUGUCUGCCUAACUAUUUAAUACAAUAAAAGAAAACCAGCCCCUGGUUCUUGUGGUGUUUCCACCCUCCCGGGUCCCCGCUGGC
UGCCUGGCUUCCUCCCGCAGCUCCUGCUGUGUGUGUAUGUGUGUGUGUGUGCACAUCUGUGGGGCGUAUGUGUGUUCGUCUUUGUAAUU
GAGGCUGCAGAGUGGAGAGAGCAGGGGUUUUCUCUGGGGACCCAGAGAGAAGGAGGCGUUUUCACCACAGCCGAACAGGGCAGGACCCC
AGCACCCGGGACCCAGCGGGACUUUGCCAAGGGGAUGGACCUGGCUGGGCCACGCGGCUGUUUGUGUAGGGAAAAGAAAGAGAGAUCAC
ACUGUUACUGUGUCUAUGUAGAAAAGGAAGACAUAAACUCCAUUUUGAGCUGUACUAAGAAAAAUUAUUUUGCCUUGACCUGCUGUUAA
CCUGUAACUGUAGCCCCAACCCUGUGCUCAAAGAAACAUGUGCUGUAUGGAAUCAAGGUUUAAGGGAUCAAGGGCUGUACAGGAUGUGC
CUUGUUAACAAUGUGUUUACAGGCAGUAUGCUUGGUAAAAGUCAUCGCCAUUCUCCAUUCUCCAUUAAUCAGGGGCACGAUGCACUGCG
GAAAGCCACAGGGACCUCUGCCCGAGAAAGCCUGGGUAUUGUCCAAGGCUUCCCCCCACUGAGACAGCCUGAGAUACGGCCUCGUGGGA
AGGGAAAGACCUGACCGUCCCCCAGCCCGACACCCGUAAAGGGUCUGUGCUGAGGAGGAUUAGUAAAAGGGGAAGGCCUCUUGCAGUUG
AGAUAAGAGGAAGGCCUCCGUCUCCUGCAUGUCCUUGGGAAUGGAAUGUCUUGGUGUAAAACCCGAUAGUACAUUCCUUCUAUUCUGAG
AGAAGAAAACCACCCUGUGGCUGGAGGUGAGAUAUGCUAGCGGCAAUGCUGCUCUGUUACUCUUUGCUACACUGAGAUGUUUGGGUGGA
GAGAAGCAUAAAUCUGGCCUAUGUGCACAUCUGGGCACAGAACCUCCCCUUGAACUUGUGACACAGAUUCCUUUGUUCACAUGUUUUCC
UGCUGACCUUCUCCCCACUAUCGCCCUGUUCUCCCACCGCAUUCCCCUUGCUGAGAUAGUGAAAAUAGUAAUCUGUAGAUACCAAGGGA
ACUCAGAGACCAUGGCCGGUGCACAUCCUCCGUACGCUGAGCGCUGGUCCCCUGGGCCCAUUGUUCUUUCUCUAUACUUUGUCUCUGUG
UCUUAUUUCUUUCCUCAGUCUCUCAUCCCUCCUGACGAGAAAUACCCACAGGUGUGGAGGGGCUGGCCCCCUUCAUCUGAUGCCCAAUG
UGGGUGCCUUUCUCUAGGGUGAAGGUACUCUACAGUGUGGUCAUUGAGGACAAGUUGACGAGAGAGUCCCAAGUACGUCCACGGUCAGC
CUUGCGGUAAGCUUGUGUGCUUAGAGGAACCCAGGGUAACGAUGGGGCAAACUGAAAGUAAAUAUGCCUCUUAUCUCAGCUUUAUUAAA
AUUCUUUUAAGAAGAGGGGGAGUUAGAGCUUCUACAGAAAAUCUAAUUACGCUAUUUCAAACAAUAGAACAAUUCUGCCCAUGGUUUCC
AGAACAGGGAACUUUAGAUCUAAAAGAUUGGGAAAAAAUUGGCAAAGAAUUAAAACAAGCAAAUAGGGAAGGUAAAAUCAUCCCACUUA
CAGUAUGGAAUGAUUGGGCCAUUAUUAAAGCAACUUUAGAACCAUUUCAAACAGGAGAAGAUAUUGUUUCAGUUUCUGAUGCCCCUAAA
AGCUGUGUAACAGAUUGUGAAGAAGAGGCAGGGACAGAAUCCCAGCAAGGAACGGAAAGUUCACAUUGUAAAUAUGUAGCAGAGUCUGU
AAUGGCUCAGUCAACGCAAAAUGUUGACUACAGUCAAUUACAGGAGAUAAUAUACCCUGAAUCAUCAAAAUUGGGGGAAGGAGGUCCAG
AAUCAUUGGGGCCAUCAGAGCCUAAACCACGAUCGCCAUCAACUCCUCCUCCCGUGGUUCAGAUGCCUGUAACAUUACAACCUCAAACG
CAGGUUAGACAAGCACAAACCCCAAGAGAAAAUCAAGUAGAAAGGGACAGAGUCUCUAUCCCGGCAAUGCCAACUCAGAUACAGUAUCC
ACAAUAUCAGCCGGUAGAAAAUAAGACCCAACCGCUGGUAGUUUAUCAAUACCGGCUGCCAACCGAGCUUCAGUAUCGGCCUCCUUCAG
AGGUUCAAUACAGACCUCAAGCGGUGUGUCCUGUGCCAAAUAGCACGGCACCAUACCAGCAACCCACAGCGAUGGCGUCUAAUUCACCA
GCAACACAGGACGCGGCGCUGUAUCCUCAGCCGCCCACUGUGAGACUUAAUCCUACAGCAUCACGUAGUGGACAGGGUGGUGCACUGCA
UGCAGUCAUUGAUGAAGCCAGAAAACAGGGCGAUCUUGAGGCAUGGCGGUUCCUGGUAAUUUUACAACUGGUACAGGCCGGGGAAGAGA
CUCAAGUAGGAGCGCCUGCCCGAGCUGAGACUAGAUGUGAACCUUUCACCAUGAAAAUGUUAAAAGAUAUAAAGGAAGGAGUUAAACAA
UAUGGAUCCAACUCCCCUUAUAUAAGAACAUUAUUAGAUUCCAUUGCUCAUGGAAAUAGACUUACUCCUUAUGACUGGGAAAUUUUGGC
CAAAUCUUCCCUUUCAUCCUCUCAGUAUCUACAGUUUAAAACCUGGUGGAUUGAUGGAGUACAAGAACAGGUACGAAAAAAUCAGGCUA
CUAAGCCCACUGUUAAUAUAGACGCAGACCAAUUGUUAGGAACAGGUCCAAAUUGGAGCACCAUUAACCAACAAUCAGUGAUGCAGAAU
GAGGCUAUUGAACAAGUAAGGGCUAUUUGCCUCAGGGCCUGGGGAAAAAUUCAGGACCCAGGAACAGCUUUCCCUAUUAAUUCAAUUAG
ACAAGGCUCUAAAGAGCCAUAUCCUGACUUUGUGGCAAGAUUACAAGAUGCUGCUCAAAAGUCUAUUACAGAUGACAAUGCCCGAAAAG
UUAUUGUAGAAUUAAUGGCCUAUGAAAAUGCAAAUCCAGAAUGUCAGUCGGCCAUAAAGCCAUUAAAAGGAAAAGUUCCAGCAGGAGUU
GAUGUAAUUACAGAAUAUGUGAAGGCUUGUGAUGGGAUUGGAGGAGCUAUGCAUAAGGCAAUGCUAAUGGCUCAAGCAAUGAGGGGGCU
CACUCUAGGAGGACAAGUUAGAACAUUUGGGAAAAAAUGUUAUAAUUGUGGUCAAAUCGGUCAUCUGAAAAGGAGUUGCCCAGGCUUAA
AUAAACAGAAUAUAAUAAAUCAAGCUAUUACAGCAAAAAAUAAAAAGCCAUCUGGCCUGUGUCCAAAAUGUGGAAAAGCAAAACAUUGG
GCCAAUCAAUGUCAUUCUAAAUUUGAUAAAGAUGGGCAACCAUUGUCUGGAAACAGGAAGAGGGGCCAGCCUCAGGCCCCCCAACAAAC
UGGGGCAUUCCCAGUUAAACUGUUUGUUCCUCAGGGUUUUCAAGGACAACAACCCCUACAGAAAAUACCACCACUUCAGGGAGUCAGCC
AAUUACAACAAUCCAACAGCUGUCCCGCGCCACAGCAGGCAGCACCGCAGUAGAUUUAUGUUCCACCCAAAUGGUCUUUUUACUCCCUG
GAAAGCCCCCACAAAAGAUUCCUAGAGGGGUAUAUGGCCCGCUGCCAGAAGGGAGGGUAGGCCUUUGAGGGAGAUCAAGUCUAAAUUUG
AAGGGAGUCCAAAUUCAUACUGGGGUAAUUUAUUCAGAUUAUAAAGGGGAAUUCAGUUAGUGAUCAGCUCCACUGUUCCCCGGAGUGC
CAAUCCAGGUGAUAGAAUUGCUCAAUUACUGCUUUUGCCUUAUGUUAAAAUUGGGGAAAACAAAAAGGAAAGAACAGGAGGGUUUGGAA
GUACCAACCCUGCAGGAAAAGCUGCUUAUUGGGCUAAUCAGGUCUCAGAGGAUAGACCCGUGUGUACAGUCACUAUUCAGGGAAAGAGU
UUGAAGGAUUAGUGGAUACCCAGGCUGAUGUUUCUGUCAUCGGCAUAGGUACUGCCUCAGAAGUGUAUCAAAGUGCCAUGAUUUUACAU
UGUCCAGGAUCUGAUAAUCAAGAAAGUACGGUUCAGCCUGUGAUCACUUCAUUCCAAUCAAUUUAUGGGGCCGAGACUUGUUACAACAA
UGGCAUGCAGAGAUUACUAUCCCAGCCUCCCUAUACAGCCCCAGGAAUAAAAAAAUCAUGACUAAAAUGGGAUAGCUCCCUAAAAAGGG
ACUAGGAAAGAAGUCCCAAUUGAGGCUGAAAAAAAUCAAAAAAGAAAAGGAAUAGGGCAUCCUUUUUAGGAGCGGUCACUGUAGAGCCU
CCAAAACCCAUUCCAUUAACUUGGGGGAAAAAAAAACAACUGUAUGGUAAAUCAGCAGCGCUUCCAAAACAAAAACUGGAGGCUUUACA
UUUAUUAGCAAAGAAACAAUUAGAAAAAGGACAUUGAGCCUUCAUUUUCGCCUUGGAAUUCUGUUUGUAAUUCAGAAAAAAUCCGGCAG
 
AUGGCGUAUAAUGCCGUAAUUCAACCCAUGGGGGCUCUCCCACCCCGGUUGCCCUCUCCAGCCAUGGUCCCCUUUAAUUAUAAUUGAUC
UGAAGGAUUGCUUUUUUACCAUUCCUCUGGCAAAACAGGAUUUUGAAAAAUUUGCUUUUACCACACCAGCCUAAAUAAUAAAGAACCAG
CCACCAGGUUUCAGUGGAAAGUAUUGCCUCAGGGAAUGCUUAAUAGUUCAACUAUUUGUCAGCUCAAGCUCUGCAACCAGUUAGAGACA
AGUUUUCAGACUGUUACAUCGUUCACUAUGUUGAUAUUUUGUGUGCUGCAGAAACGAGAGACAAAUUAAUUGACCGUUACACAUUUCUG
CAGACAGAGGUUGCCAACGCGGGACUGACAAUAACAUCUGAUAAGAUUCAAACCUCUACUCCUUUCCGUUACUUGGGAAUGCAGGUAGA
GGAAAGGAAAAUUAAACCACAAAAAAUAGAAAUAAGAAAAGACACAUUAAAAGCAUUAAAUGAGUUUCAAAAGUUGCUAGGAGAUACUA
AUUGGAUUUGGAGAUAUUAAUUGGAUUUGGCCAACUCUAGGCAUUCCUACUUAUGCCAUGUCAAAUUUGUUCUCUUUCUUAAGAGGGGA
CUCGGAAUUAAAUAGUGAAAGAACGUUAACUCCAGAGGCAACUAAAGAAAUUAAAUUAAUUGAAGAAAAAAUUCGGUCAGCACAAGUAA
AUAGAAUAGAUCACUUGGCCCCACUCCAAAUUUUGAUUUUUGCUACUGCACAUUCCCUAACAGGCAUCAUUGUUCAAAAUACAGAUCUU
GUGGAGUGGUCCUUCCUUCCUCACAGUACAAUUAAGACUUUUACAUUGUACUUGGAUCAAAUGGCUACAUUAAUUGGUCAGGGAAGAUU
AUGAAUAAUAACAUUGUGUGGAAAUGACCCAGAUAAAAUCACUGUUCCUUUCAACAAGCAACAGGUUAGACAAGCCUUUAUCAAUUCUG
GUGCAUGGCAGAUUGGUCUUGCCGAUUUUGUGGGAAUUAUUGACAAUCGUUACCCCAAAACAAAAAUCUUCCAGUUUUUAAAAUUGACU
ACUUGGAUUUUACCUAAAGUUACCAAACAUAAGCCUUUAAAAAAUGCUCUGGCAGUGUUUACUGAUGGUUCCAGCAAUGGAAAAGUGGC
UUACACCGGGCCAAAAGAAUGAGUCAUCAAAACUCAGUAUCACUUGACUCAAAGAGCAGAGUUGGUUGCCGUCAUUACAGUGUUAACAA
GAUUUUAAUCAGUCUAUUAACAUUGUAUCAGAUUCUGCAUAUGUAGUACAGGCUACAAAGGAUAUUGAGAGAGCCCUAAUCAAAUACAU
UAUGGAUGAUCAGUUAAACCCGCUGUUUAAUUUGUUACAACAAAAUGUAAGAAAAAGAAAUUUCCCAUUUUAUAUUACUCAUAUUCGAG
CACACACUAAUUUACCAGGGCCUUUAACUAAAGCAAAUGAACAAGCUGACUUGCUAGUAUCAUCUGCAUUCAUGGAAGCACAAGAACUU
CAUGCCUUGACUCAUGUAAAUGCAAUAGGAUUAAAAAAUAAAUUUGAUAUCACAUGGAAACAGACAAAAAAUAUUGUACAACAUUGCAC
CCAGUGUCAGAUUCUACACCUGGCCACUCAGGAGGCAAGAGUUAAUCCCAGAGGUCUAUGUCCUAAUGUGUUAUGGCAAAUGGAUGUCA
UGCACGUACCUUCAUUUGGAAAAUUGUCAUUUGUCCAUGUGACAGUUGAUACUUAUUCACAUUUCAUAUGGGCAACCUGCCAGACAGGA
GAAAGUACUUCCCAUGUUAAAAGACAUUUAUUAUCUUGUUUUCCUGUCAUGGGAGUUCCAGAAAAAGUUAAAACAGACAAUGGGCCAGG
UUACUGUAGUAAAGCAGUUCAAAAAUUCUUAAAUCAGUGGAAAAUUACACAUACAAUAGGAAUUCUCUAUAAUUCCCAAGGACAGGCCA
UAAUUGAAAGAACUAAUAGAACACUCAAAGCUCAAUUGGUUAAACAAAAAAAAGGAAAAGACAGGAGUAUAACACUCCCCAGAUGCAAC
UUAAUCUAGCACUCUAUACUUUAAAUGUUUUAAACAUUUAUAGAAAUCAGACCACUACCUCUGCAGAACAACAUCUUACUGGUAAAAGG
AACAGCCCACAUGAAGGAAAACUGAUUUGGUGGAAAGAUAAUAAAAAUAAAACAUGGGAAAUGGGGAAGGUGAUAACGUGGGGGAGAGG
UUUUGCUUGUGUUUCACCAGGAGAAAAUCAGCUUCCUGUUUGGAUACCCACUAGACAUUUAAAGUUCUACAAUGAACUCACUGGAGAUG
CAAAGAAAAGUGUGGAGAUGGAGACACCCCAAUCGACUCGCCAGGUAAACAAAAUGGUGAUAUCAGAAGAACAGAAAAAGUUGCCUUCC
AUCAAGGAAGCAGAGUUGCCAAUAUAGGCACAAUUAAAGAAGCUGACACAGUUAGCUAAAAAAAAAAGCCUAGAGAAUACAAAGGUGAC
ACCAACUCCAGAGAAUAUGCUGCUUGCAGCUCUGAUGAUUGUAUCAACGGUGGUAAGUCUUCCCAAGUCUGCAGGAGCAGCUGCAGCUA
AUUAUACUUACUGGGCCUAUGUGCCUUUCCCACCCUUAAUUCGGGCAGUUACAUAGAUGGAUAAUCCUAUUGAAGUAGAUGUUAAUAAU
AGUGCAUGGGUGCCUGGCCCCACAGAUGACUGUUGCCCUGCCCAACCUGAAGAAGGAAUGAUGAUGAAUAUUUCCAUUGGGUAUCCUUA
UCCUCCUGUUUGCCUAGGGAAGGCACCAGGAUGCUUAAUGCCUACAACCCAAAAUUGGUUGGUAGAAGUACCUACAGUCAGUGCUACCA
GUAGAUUUACUUAUCACAUGGUAAGUGGAAUGUCACAGAUAAAUAAUUUACAGGACCCUUCUUAUCAAAGAUCAUUACAAUGUAGGCCU
AAGGGGAAGGCUUGCCCCAAGGAAAUUCCCAAAGAAUCAAAAAGCCCAGAAGUCUUAGUCUGCGGAGAAUGUGUGGCUGAUACUGCAGU
GUAGUACAAAACAAUGAAUUUUGAACUAUGAUAGACUGGGUCCCUUGAGGCCAAUUAUAUCAUAACUGUACAGGCCAGACUCAUUCAUG
UUCACAGGCCCCAUCCAUCUGGCCCAUUAAUCCAGCCUAUGACGGUGAUGUAACUGAAAGGCUGGACCAGGUUUAUAGAAGGUUAGAAU
CACUCUGUCCAAGGAAAUGGGGUGAAAAGGGAAUUUCAUCACCUUGACCAAAGUUAGUCCUGUUACUGGUCCUGAACAUCCAGAAUUAG
GAAGCUUACUGUGGCCUCACACCACAUUAGAAUUUGUUCUGGAAAUCAAGCUAUAGGAACAAGAGAUCGUAAGUCAUAUUAUACUAUCA
ACCUAAAUUCCAGUCUGACAAUUCCUUUGCAAAAUUGUGUAAAACUCCCUUAUAUUGCUAGUUGUAGGAAAAACAUAGUUAUUAAACCU
GAUUCCCAAACCAUAAUCUGUGAAAAUUGUGGAAUGUUUACUUGCAUUGAUUUGACUUUUAAUUGGCAGCACCGUAUUCUACUAGGAAG
AGCAAGAGAGGGUGUGUGGAUCCUUGUGUCCAUGGACCGACCAUGGGAGGCUUCGCUAUCCAUCCAUAUUUUAACGGAAGUAUUAAAAG
GAAUUCUAACUAGAUCCAAAAGAUUCAUUUUUACUUUGAUGGCAGUGAUUAUGGGCCUCAUUGCAGUCACAGCUACUGCUGCGGCUGCU
GGAAUUGCUUUACACUCCUCUGUUCAAACUGCAGAAUACGUAAAUGAUUGGCAAAAGAAUUCCUCAAAAUUGUGGAAUUCUCAGAUCCA
AAUAGAUCAAAAAUUGGCAAACCAAAUUAAUGAUCUUAGACAAACUGUCAUUUGGAUGGGAGAGGCUCAUGAGCUUGGAAUAUCUUUUU
CAGUUACGAUGUGACUGGAAUACAUCAGAUUUUUGUGUUACACCACAAGCCUAUAAUGAGUCUGAGCAUCACUGGGACAUGGUUAGAUG
CCAUCUGCAAGGAGGAGAAGAUAAUCUUACUUUAGACAUUUCAAAAUUAAAAGAAUUUUUUUUUCUUUGAGACAGAGUCUCGCUCUGU
CGCCCAGGCUGGAGUGCAGUGGCGUGAUCUCAGCUCACUGCAAGUUCCGCCUCCUGGGUUUACACCAUUCUCCUGCCUCAGCCUCCCAA
GUAGUUGGGACUACAGGAGCCCACCACCAUGCCUGGCUAAUUUUUUUGGGUUUUUAAUAGAGAUGGAGUUUCACCGUGUUAGCCAGGA
UGGUCUCGAUCUCCUGACCUUGUGAUCUGCCCACCUUGGCCUCCCAAAGUGCUGGGAUUACAGUCGUGAGCCACCGUGCCCAGCCAAGA
AAAAAUUUUUGAGGCAUCAAAAGCCCAUUUAAAUUUGGUGCCAGGAACGGAGACAAUCGUGAAAGCUGCUGAUAGCCUCACAAAUCUUA
AGCCAGUCACUUGGGUUAAAAGCAUCAGAAGUUUCACUAUUGUAAAUUUCAUAUUAAUCCUUGUAUGCCUGUUCUGUCUGUUGUUAGUC
UACAGGUGUAUCCAGCAGCUCCAAAGAGACAGCAACCAGCAAGAAUGGGCCAUAGUGACGAUGGUGGUUUUGUCAAAAAGAAAAGGGGG
GGAUAUGUAAGGAAAAGAGAGAUCAGACUUUCACUGUGUCUAUGUAGAAAAGGAAGACAUAAGAAACUCCAUUUUGAUCUGUACUAAGA
 
AAAAUUGUUUUGCCUUGAGAUGCUGUUAAUCUGUAACUUUAGCCCCAACCCUGUGCUCACGGAAACAUGUGCUGUAAGGUUUAAGGAU
CUAGGGCUGUGCAGGAUGUACCUUGUUAACAAUAUGUUUGCAGGCAGUAUGUUUGGUAAAAGUCAUCGCCAUUCUCCAUUCUCGAUUAA
CCAGGGGCUCAAUGCACUGUGGAAAGCCACAGGAACCUCUGCCCAAGAAAGCCUGGCUGUUGUGGGAAGUCAGGGACCCCGAAUGGAGG
GACCAGCUGGUGCUGCAUCAGGAAACAUAAAUUGUGAAGAUUUCUUGGACAUUUAUCAGUUUCCAAAAUUAAUACUUUUAUAAUUUCUU
ACACCUGUCUUACUUUAAUCUCUUAAUCCUGUUAUCUUUGUAAGCUGAGGAUAUACGUCACCUCAGGACCACUAUUGUACAAAUUGAUU
GUAAAACAUGUUCACAUGUGUUUGAACAAUAUGAAAUCAGUGCACCUUGAAAAUGAACAGAAUAACAGUGAUUUUAGGGAACAAAGGAA
GACAACCAUAAGGUCUGACUGCCUGAGGGGUCGGGCAAAAAGCCAUAUUUUUCUUCUUGCAGAGAGCCUAUAAAUGGACGUGCAAGUAG
GAGAGAUAUUGCUAAAUUCUUUUCCUAGCAAGGAAUAUAAUACUAAGACCCUAGGGAAAGAAUUGCAUUCCUGGGGGGAGGUCUAUAAA
CGGCCGCUCUGGGAGUGUCUGUCCUAUGUGGUUGAGAUAAGGACUGAGAUACGCCCUGGUCUCCUGCAGUACCCUCAGGCUUACUAGGA
UUGGGAAACCCCAGUCCUGGUAAAUUUGAGGUCAGGCCGGUUCUUUGCUCUGAACCCUGUUUUCUGUUAAGAUGUUUAUCAAGACAAUA
CAUGCACCGCUGAACAUAGACCCUUAUCAGGAGUUUCUGAUUUUGCUCUGGUCCUGUUUCUUCAGAAGCAUGUCAUCUUUGCUCUGCCU
UCUGCCCUUUGAAGCAUGUGAUCUUUGUGACCUACUCCCUGUUCAUACACCCCUCCCCUUUUAAAAUCCCUAAUAAAAACUUGCUGGUU
UUGUGGCUCAGGGGGGCAUCAUGGACCUACCAAUACGUGAUGUCACCCCCGGUGGCCCAGCUGU
**SEQ ID 1198** 
ACAGCACUUUUUUCUUUACCUUGUUUAUGAUGCAGAGACAUUUGUUCACAUGUUUUCCUGCUGGCCCUCUCCCCACUAUUACCCUAUUG
UCCUGCCACAUCCCCCUCUCCGAGAUGGUAGAGAUAAUGAUCAAUAAAUACUGAGGGAACUCAGAGACCGGUGCGGCGCGGGUCCUCCA
UAUGCUGAGCGCCGGUCCCCUGGGCCCACUUUUCUUUCUCUAUACUUUGUCUCUGUUGUCUUUCUUUUCUCAAGUCUCUCGUUCCACCU
GAGGAGAAAUGCCCACAGCUGUGGAGGCGCAGGCCACUCCAUCUGGUGCCCAACGUGGAUGCUUUUCUCUAGGGUGAAGGACUCUCGA
GUGUGGUCAUUGAGGACAAGUCAACGAGAGAUUCCCGAGUACGUCUACAGUGAGCCUUGUGGUAAGCUUGGGCGCUCGGAAGAAGCCAG
GGUUAAUGGGGCAAACUAAAAGUAAAGUCUCUCAUUCCACCUGAUGAGAAACACCCAGAGGUGUGGAGGGGCAGGCCACCCCUUCAGGG
UAGGGUCCCCUCCAUGCAGACCAUAGAGCACAGGUGUGCCCCAAAGAGGAGCAGAGAGAAGGAGGGAGAGGGCCCACGAGAGACUUGGA
AAUGAAUGGCAGGAUUUUAGGCGCUGGACUUGGGUUCGGGGCACCUGGCCUUUCCUUGUGUAUUUCUCCUACUGUCUGCCUAACUAUUU
AAUACAAUAAAAGAAAACCAGCCCCUGGUUCUUGUGGUGUUUCCACCCUCCCGGGUCCCCGCUGGCUGCCUGGCUUCCUCCCGCAGCUC
CUGCUGUGUGUGUAUGUGUGUGUGUGUGCACAUCUGUGGGGCGUAUGUGUGUUCGUCUUUGUAAUUGAGGCUGCAGAGUGGAGAGAGCA
GGGGUUUUCUCUGGGGACCCAGAGAGAAGGAGGCGUUUUCACCACAGCCGAACAGGGCAGGACCCCAGCACCCGGGACCCAGCGGGACU
UUGCCAAGGGGAUGGACCUGGCUGGGCCACGCGGCUGUUUGUGUAGGGAAAAGAAAGAGAGAUCACACUGUUACUGUGUCUAUGUAGAA
AAGGAAGACAUAAACUCCAUUUUGAGCUGUACUAAGAAAAAUUAUUUUGCCUUGACCUGCUGUUAACCUGUAACUGUAGCCCCAACCCU
GUGCUCAAAGAAACAUGUGCUGUAUGGAAUCAAGGUUUAAGGGAUCAAGGGCUGUACAGGAUGUGCCUUGUUAACAAUGUGUUUACAGG
CAGUAUGCUUGGUAAAAGUCAUCGCCAUUCUCCAUUCUCCAUUAAUCAGGGGCACGAUGCACUGCGGAAAGCCACAGGGACCUCUGCCC
GAGAAAGCCUGGGUAUUGUCCAAGGCUUCCCCCCACUGAGACAGCCUGAGAUACGGCCUCGUGGGAAGGGAAAGACCUGACCGUCCCCC
AGCCCGACACCCGUAAAGGGUCUGUGCUGAGGAGGAUUAGUAAAAGGGGAAGGCCUCUUGCAGUUGAGAUAAGAGGAAGGCCUCCGUCU
CCUGCAUGUCCUUGGGAAUGGAAUGUCUUGGUGUAAAACCCGAUAGUACAUUCCUUCUAUUCUGAGAGAAGAAAACCACCCUGUGGCUG
GAGGUGAGAUAUGCUAGCGGCAAUGCUGCUCUGUUACUCUUUGCUACACUGAGAUGUUUGGGUGGAGAGAAGCAUAAAUCUGGCCUAUG
UGCACAUCUGGGCACAGAACCUCCCCUUGAACUUGUGACACAGAUUCCUUUGUUCACAUGUUUUCCUGCUGACCUUCUCCCCACUAUCG
CCCUGUUCUCCCACCGCAUUCCCCUUGCUGAGAUAGUGAAAAUAGUAAUCUGUAGAUACCAAGGGAACUCAGAGACCAUGGCCGGUGCA
CAUCCUCCGUACGCUGAGCGCUGGUCCCCUGGGCCCAUUGUUCUUUCUCUAUACUUUGUCUCUGUGUCUUAUUUCUUUCCUCAGUCUCU
CAUCCCUCCUGACGAGAAAUACCCACAGGUGUGGAGGGGCUGGCCCCCUUCAUCUGAUGCCCAAUGUGGGUGCCUUUCUCUAGGGUGAA
GGUACUCUACAGUGUGGUCAUUGAGGACAAGUUGACGAGAGAGUCCCAAGUACGUCCACGGUCAGCCUUGCGGUAAGCUUGUGUGCUUA
GAGGAACCCAGGGUAACGAUGGGGCAAACUGAAAGUAAAUAUGCCUCUUAUCUCAGCUUUAUUAAAAUUCUUUUAAGAAGAGGGGGAGU
UAGAGCUUCUACAGAAAAUCUAAUUACGCUAUUUCAAACAAUAGAACAAUUCUGCCCAUGGUUUCCAGAACAGGGAACUUUAGAUCUAA
AAGAUUGGGAAAAAAUUGGCAAAGAAUUAAAACAAGCAAAUAGGGAAGGUAAAAUCAUCCCACUUACAGUAUGGAAUGAUUGGGCCAUU
AUUAAAGCAACUUUAGAACCAUUUCAAACAGGAGAAGAUAUUGUUUCAGUUUCUGAUGCCCCUAAAAGCUGUGUAACAGAUUGUGAAGA
AGAGGCAGGGACAGAAUCCCAGCAAGGAACGGAAAGUUCACAUUGUAAAUAUGUAGCAGAGUCUGUAAUGGCUCAGUCAACGCAAAAUG
UUGACUACAGUCAAUUACAGGAGAUAAUAUACCCUGAAUCAUCAAAAUUGGGGGAAGGAGGUCCAGAAUCAUUGGGGCCAUCAGAGCCU
AAACCACGAUCGCCAUCAACUCCUCCUCCCGUGGUUCAGAUGCCUGUAACAUUACAACCUCAAACGCAGGUUAGACAAGCACAAACCCC
AAGAGAAAAUCAAGUAGAAAGGGACAGAGUCUCUAUCCCGGCAAUGCCAACUCAGAUACAGUAUCCACAAUAUCAGCCGGUAGAAAAUA
AGACCCAACCGCUGGUAGUUUAUCAAUACCGGCUGCCAACCGAGCUUCAGUAUCGGCCUCCUUCAGAGGUUCAAUACAGACCUCAAGCG
GUGUGUCCUGUGCCAAAUAGCACGGCACCAUACCAGCAACCCACAGCGAUGGCGUCUAAUUCACCAGCAACACAGGACGCGGCGCUGUA
UCCUCAGCCGCCCACUGUGAGACUUAAUCCUACAGCAUCACGUAGUGGACAGGGUGGUGCACUGCAUGCAGUCAUUGAUGAAGCCAGAA
AACAGGGCGAUCUUGAGGCAUGGCGGUUCCUGGUAAUUUUACAACUGGUACAGGCCGGGGAAGAGACUCAAGUAGGAGCGCCUGCCCGA
GCUGAGACUAGAUGUGAACCUUUCACCAUGAAAAUGUUAAAAGAUAUAAAGGAAGGAGUUAAACAAUAUGGAUCCAACUCCCCUUAUAU
AAGAACAUUAUUAGAUUCCAUUGCUCAUGGAAAUAGACUUACUCCUUAUGACUGGGAAAUUUUGGCCAAAUCUUCCCUUUCAUCCUCUC
AGUAUCUACAGUUUAAAACCUGGUGGAUUGAUGGAGUACAAGAACAGGUACGAAAAAAUCAGGCUACUAAGCCCACUGUUAAUAUAGAC
 
GCAGACCAAUUGUUAGGAACAGGUCCAAAUUGGAGCACCAUUAACCAACAAUCAGUGAUGCAGAAUGAGGCUAUUGAACAAGUAAGGGC
UAUUUGCCUCAGGGCCUGGGGAAAAAUUCAGGACCCAGGAACAGCUUUCCCUAUUAAUUCAAUUAGACAAGGCUCUAAAGAGCCAUAUC
CUGACUUUGUGGCAAGAUUACAAGAUGCUGCUCAAAAGUCUAUUACAGAUGACAAUGCCCGAAAAGUUAUUGUAGAAUUAAUGGCCUAU
GAAAAUGCAAAUCCAGAAUGUCAGUCGGCCAUAAAGCCAUUAAAAGGAAAAGUUCCAGCAGGAGUUGAUGUAAUUACAGAAUAUGUGAA
GGCUUGUGAUGGGAUUGGAGGAGCUAUGCAUAAGGCAAUGCUAAUGGCUCAAGCAAUGAGGGGGCUCACUCUAGGAGGACAAGUUAGAA
CAUUUGGGAAAAAAUGUUAUAAUUGUGGUCAAAUCGGUCAUCUGAAAAGGAGUUGCCCAGGCUUAAAUAAACAGAAUAUAAUAAAUCAA
GCUAUUACAGCAAAAAAUAAAAAGCCAUCUGGCCUGUGUCCAAAAUGUGGAAAAGCAAAACAUUGGGCCAAUCAAUGUCAUUCUAAAUU
UGAUAAAGAUGGGCAACCAUUGUCUGGAAACAGGAAGAGGGGCCAGCCUCAGGCCCCCCAACAAACUGGGGCAUUCCCAGUUAAACUGU
UUGUUCCUCAGGGUUUUCAAGGACAACAACCCCUACAGAAAAUACCACCACUUCAGGGAGUCAGCCAAUUACAACAAUCCAACAGCUGU
CCCGCGCCACAGCAGGCAGCACCGCAGUAGAUUUAUGUUCCACCCAAAUGGUCUUUUUACUCCCUGGAAAGCCCCCACAAAAGAUUCCU
AGAGGGGUAUAUGGCCCGCUGCCAGAAGGGAGGGUAGGCCUUUGAGGGAGAUCAAGUCUAAAUUUGAAGGGAGUCCAAAUUCAUACUGG
GGUAAUUUAUUCAGAUUAUAAAGGGGAAUUCAGUUAGUGAUCAGCUCCACUGUUCCCCGGAGUGCCAAUCCAGGUGAUAGAAUUGCUC
AAUUACUGCUUUUGCCUUAUGUUAAAAUUGGGGAAAACAAAAAGGAAAGAACAGGAGGGUUUGGAAGUACCAACCCUGCAGGAAAAGCU
GCUUAUUGGGCUAAUCAGGUCUCAGAGGAUAGACCCGUGUGUACAGUCACUAUUCAGGGAAAGAGUUUGAAGGAUUAGUGGAUACCCAG
GCUGAUGUUUCUGUCAUCGGCAUAGGUACUGCCUCAGAAGUGUAUCAAAGUGCCAUGAUUUUACAUUGUCCAGGAUCUGAUAAUCAAGA
AAGUACGGUUCAGCCUGUGAUCACUUCAUUCCAAUCAAUUUAUGGGGCCGAGACUUGUUACAACAAUGGCAUGCAGAGAUUACUAUCCC
AGCCUCCCUAUACAGCCCCAGGAAUAAAAAAAUCAUGACUAAAAUGGGAUAGCUCCCUAAAAAGGGACUAGGAAAGAAGUCCCAAUUGA
GGCUGAAAAAAAUCAAAAAAGAAAAGGAAUAGGGCAUCCUUUUUAGGAGCGGUCACUGUAGAGCCUCCAAAACCCAUUCCAUUAACUUG
GGGGAAAAAAAAACAACUGUAUGGUAAAUCAGCAGCGCUUCCAAAACAAAAACUGGAGGCUUUACAUUUAUUAGCAAAGAAACAAUUAG
AAAAAGGACAUUGAGCCUUCAUUUUCGCCUUGGAAUUCUGUUUGUAAUUCAGAAAAAAUCCGGCAGAUGGCGUAUAAUGCCGUAAUUCA
ACCCAUGGGGGCUCUCCCACCCCGGUUGCCCUCUCCAGCCAUGGUCCCCUUUAAUUAUAAUUGAUCUGAAGGAUUGCUUUUUUACCAUU
CCUCUGGCAAAACAGGAUUUUGAAAAAUUUGCUUUUACCACACCAGCCUAAAUAAUAAAGAACCAGCCACCAGGUUUCAGUGGAAAGUA
UUGCCUCAGGGAAUGCUUAAUAGUUCAACUAUUUGUCAGCUCAAGCUCUGCAACCAGUUAGAGACAAGUUUUCAGACUGUUACAUCGUU
CACUAUGUUGAUAUUUUGUGUGCUGCAGAAACGAGAGACAAAUUAAUUGACCGUUACACAUUUCUGCAGACAGAGGUUGCCAACGCGGG
ACUGACAAUAACAUCUGAUAAGAUUCAAACCCUCUACUCCUUUCCGUUACUUGGGAAUGCAGGUAGAGGAAAGGAAAAUUAAACCACAAA
AAAUAGAAAUAAGAAAAGACACAUUAAAAGCAUUAAAUGAGUUUCAAAAGUUGCUAGGAGAUACUAAUUGGAUUUGGAGAUAUUAAUUG
GAUUUGGCCAACUCUAGGCAUUCCUACUUAUGCCAUGUCAAAUUUGUUCUCUUUCUUAAGAGGGGACUCGGAAUUAAAUAGUGAAAGAA
CGUUAACUCCAGAGGCAACUAAAGAAAUUAAAUUAAUUGAAGAAAAAAUUCGGUCAGCACAAGUAAAUAGAAUAGAUCACUUGGCCCCA
CUCCAAAUUUUGAUUUUUGCUACUGCACAUUCCCCUAACAGGCAUCAUUGUUCAAAAUACAGAUCUUGUGGAGUGGUCCUUCCUUCCUCA
CAGUACAAUUAAGACUUUUACAUUGUACUUGGAUCAAAUGGCUACAUUAAUUGGUCAGGGAAGAUUAUGAAUAAUAACAUUGUGUGGAA
AUGACCCAGAUAAAAUCACUGUUCCUUUCAACAAGCAACAGGUUAGACAAGCCUUUAUCAAUUCUGGUGCAUGGCAGAUUGGUCUUGCC
GAUUUUGUGGGAAUUAUUGACAAUCGUUACCCCAAAACAAAAAUCUUCCAGUUUUUAAAAUUGACUACUUGGAUUUUACCUAAAGUUAC
CAAACAUAAGCCUUUAAAAAAUGCUCUGGCAGUGUUUACUGAUGGUUCCAGCAAUGGAAAAGUGGCUUACACCGGGCCAAAAGAAUGAG
UCAUCAAAACUCAGUAUCACUUGACUCAAAGAGCAGAGUUGGUUGCCGUCAUUACAGUGUUAACAAGAUUUUAAUCAGUCUAUUAACAU
UGUAUCAGAUUCUGCAUAUGUAGUACAGGCUACAAAGGAUAUUGAGAGAGCCCUAAUCAAAUACAUUAUGGAUGAUCAGUUAAACCCGC
UGUUUAAUUUGUUACAACAAAAUGUAAGAAAAAGAAAUUUCCCAUUUUAUAUUACUCAUAUUCGAGCACACACUAAUUUACCAGGGCCU
UUAACUAAAGCAAAUGAACAAGCUGACUUGCUAGUAUCAUCUGCAUUCAUGGAAGCACAAGAACUUCAUGCCUUGACUCAUGUAAAUGC
AAUAGGAUUAAAAAAUAAAUUUGAUAUCACAUGGAAACAGACAAAAAAUAUUGUACAACAUUGCACCCAGUGUCAGAUUCUACACCUGG
CCACUCAGGAGGCAAGAGUUAAUCCCAGAGGUCUAUGUCCUAAUGUGUUAUGGCAAAUGGAUGUCAUGCACGUACCUUCAUUUGGAAAA
UUGUCAUUUGUCCAUGUGACAGUUGAUACUUAUUCACAUUUCAUAUGGGCAACCUGCCAGACAGGAGAAAGUACUUCCCAUGUUAAAAG
ACAUUUAUUAUCUUGUUUUCCUGUCAUGGGAGUUCCAGAAAAAGUUAAAACAGACAAUGGGCCAGGUUACUGUAGUAAAGCAGUUCAAA
AAUUCUUAAAUCAGUGGAAAAUUACACAUACAAUAGGAAUUCUCUAUAAUUCCCAAGGACAGGCCAUAAUUGAAAGAACUAAUAGAACA
CUCAAAGCUCAAUUGGUUAAACAAAAAAAAGGAAAAGACAGGAGUAUAACACUCCCCAGAUGCAACUUAAUCUAGCACUCUAUACUUUA
AAUGUUUUAAACAUUUAUAGAAAUCAGACCACUACCUCUGCAGAACAACAUCUUACUGGUAAAAGGAACAGCCCACAUGAAGGAAAACU
GAUUUGGUGGAAAGAUAAUAAAAAUAAAACAUGGGAAAUGGGGAAGGUGAUAACGUGGGGGAGAGGUUUUGCUUGUGUUUCACCAGGAG
AAAAUCAGCUUCCUGUUUGGAUACCCACUAGACAUUUAAAGUUCUACAAUGAACUCACUGGAGAUGCAAAGAAAAGUGUGGAGAUGGAG
ACACCCCAAUCGACUCGCCAGGUAAACAAAAUGGUGAUAUCAGAAGAACAGAAAAAGUUGCCUUCCAUCAAGGAAGCAGAGUUGCCAAU
AUAGGCACAAUUAAAGAAGCUGACACAGUUAGCUAAAAAAAAAAGCCUAGAGAAUACAAAGGUGACACCAACUCCAGAGAAUAUGCUGC
UUGCAGCUCUGAUGAUUGUAUCAACGGUGGUAAGUCUUCCCAAGUCUGCAGGAGCAGCUGCAGCUAAUUAUACUUACUGGGCCUAUGUG
CCUUUCCCACCCUUAAUUCGGGCAGUUACAUAGAUGGAUAAUCCUAUUGAAGUAGAUGUUAAUAAUAGUGCAUGGGUGCCUGGCCCCAC
AGAUGACUGUUGCCCUGCCCAACCUGAAGAAGGAAUGAUGAUGAAUAUUUCCAUUGGGUAUCCUUAUCCUCCUGUUUGCCUAGGGAAGG
CACCAGGAUGCUUAAUGCCUACAACCCAAAAUUGGUUGGUAGAAGUACCUACAGUCAGUGCUACCAGUAGAUUUACUUAUCACAUGGUA
AGUGGAAUGUCACAGAUAAAUAAUUUACAGGACCCUUCUUAUCAAAGAUCAUUACAAUGUAGGCCUAAGGGGAAGGCUUGCCCCAAGGA
 
AAUUCCCAAAGAAUCAAAAAGCCCAGAAGUCUUAGUCUGCGGAGAAUGUGUGGCUGAUACUGCAGUGUAGUACAAAACAAUGAAUUUUG
AACUAUGAUAGACUGGGUCCCUUGAGGCCAAUUAUAUCAUAACUGUACAGGCCAGACUCAUUCAUGUUCACAGGCCCCAUCCAUCUGGC
CCAUUAAUCCAGCCUAUGACGGUGAUGUAACUGAAAGGCUGGACCAGGUUUAUAGAAGGUUAGAAUCACUCUGUCCAAGGAAAUGGGGU
GAAAAGGGAAUUUCAUCACCUUGACCAAAGUUAGUCCUGUUACUGGUCCUGAACAUCCAGAAUUAGGAAGCUUACUGUGGCCUCACACC
ACAUUAGAAUUUGUUCUGGAAAUCAAGCUAUAGGAACAAGAGAUCGUAAGUCAUAUUAUACUAUCAACCUAAAUUCCAGUCUGACAAUU
CCUUUGCAAAAUUGUGUAAAACUCCCUUAUAUUGCUAGUUGUAGGAAAAACAUAGUUAUUAAACCUGAUUCCCAAACCAUAAUCUGUGA
AAAUUGUGGAAUGUUUACUUGCAUUGAUUUGACUUUUAAUUGGCAGCACCGUAUUCUACUAGGAAGAGCAAGAGAGGGUGUGUGGAUCC
UUGUGUCCAUGGACCGACCAUGGGAGGCUUCGCUAUCCAUCCAUAUUUAACGGAAGUAUUAAAGGAAUUCUAACUAGAUCCAAAAGA
UUCAUUUUUACUUUGAUGGCAGUGAUUAUGGGCCUCAUUGCAGUCACAGCUACUGCUGCGGCUGCUGGAAUUGCUUUACACUCCUCUGU
UCAAACUGCAGAAUACGUAAAUGAUUGGCAAAAGAAUUCCUCAAAAUUGUGGAAUUCUCAGAUCCAAAUAGAUCAAAAAUUGGCAAACC
AAAUUAAUGAUCUUAGACAAACUGUCAUUUGGAUGGGAGAGGCUCAUGAGCUUGGAAUAUCUUUUUCAGUUACGAUGUGACUGGAAUAC
AUCAGAUUUUUGUGUUACACCACAAGCCUAUAAUGAGUCUGAGCAUCACUGGGACAUGGUUAGAUGCCAUCUGCAAGGAGGAGAAGAUA
AUCUUACUUUAGACAUUUCAAAAUUAAAAGAAUUUUUUUUUCUUUGAGACAGAGUCUCGCUCUGUCGCCCAGGCUGGAGUGCAGUGGC
GUGAUCUCAGCUCACUGCAAGUUCCGCCUCCUGGGUUUACACCAUUCUCCUGCCUCAGCCUCCCAAGUAGUUGGGACUACAGGAGCCCA
CCACCAUGCCUGGCUAAUUUUUUUUGGGUUUUUAAUAGAGAUGGAGUUUCACCGUGUUAGCCAGGAUGGUCUCGAUCUCCUGACCUUGU
GAUCUGCCCACCUUGGCCUCCCAAAGUGCUGGGAUUACAGUCGUGAGCCACCGUGCCCAGCCAAGAAAAAAUUUUUGAGGCAUCAAAAG
CCCAUUUAAAUUUGGUGCCAGGAACGGAGACAAUCGUGAAAGCUGCUGAUAGCCUCACAAAUCUUAAGCCAGUCACUUGGGUUAAAAGC
AUCAGAAGUUUCACUAUUGUAAAUUUCAUAUUAAUCCUUGUAUGCCUGUUCUGUCUGUUGUUAGUCUACAGGUGUAUCCAGCAGCUCCA
AAGAGACAGCAACCAGCAAGAAUGGGCCAUAGUGACGAUGGUGGUUUUGUCAAAAAGAAAAGGGGGGAUAUGUAAGGAAAAGAGAGAU
CAGACUUUCACUGUGUCUAUGUAGAAAAGGAAGACAUAAGAAACUCCAUUUUGAUCUGUACUAAGAAAAAUUGUUUUGCCUUGAGAUGC
UGUUAAUCUGUAACUUUAGCCCCAACCCUGUGCUCACGGAAACAUGUGCUGUAAGGUUUAAGGAUCUAGGGCUGUGCAGGAUGUACCU
UGUUAACAAUAUGUUUGCAGGCAGUAUGUUUGGUAAAAGUCAUCGCCAUUCUCCAUUCUCGAUUAACCAGGGGCUCAAUGCACUGUGGA
AAGCCACAGGAACCUCUGCCCAAGAAAGCCUGGCUGUUGUGGGAAGUCAGGGACCCCGAAUGGAGGGACCAGCUGGUGCUGCAUCAGGA
AACAUAAAUUGUGAAGAUUUCUUGGACAUUUAUCAGUUUCCAAAAUUAAUACUUUUAUAAUUUCUUACACCUGUCUUACUUUAAUCUCU
UAAUCCUGUUAUCUUUGUAAGCUGAGGAUAUACGUCACCUCAGGACCACUAUUGUACAAAUUGAUUGUAAAACAUGUUCACAUGUGUUU
GAACAAUAUGAAAUCAGUGCACCUUGAAAAUGAACAGAAUAACAGUGAUUUAGGGAACAAAGGAAGACAACCAUAAGGUCUGACUGCC
UGAGGGGUCGGGCAAAAAGCCAUAUUUUCUUCUUGCAGAGAGCCUAUAAAUGGACGUGCAAGUAGGAGAGAUAUUGCUAAAUUCUUUU
CCUAGCAAGGAAUAUAAUACUAAGACCCUAGGGAAAGAAUUGCAUUCCUGGGGGGAGGUCUAUAAACGGCCGCUCUGGGAGUGUCUGUC
CUAUGUGGUUGAGAUAAGGACUGAGAUACGCCCUGGUCUCCUGCAGUACCCUCAGGCUUACUAGGAUUGGGAAACCCCAGUCCUGGUAA
AUUUGAGGUCAGGCCGGUUCUUUGCUCUGAACCCUGUUUUCUGUUAAGAUGUUUAUCAAGACAAUACAUGCACCGCUGAACAUAGACCC
UUAUCAGGAGUUUCUGAUUUUGCUCUGGUCCUGUUUCUUCAGAAGCAUGUCAUCUUUGCUCUGCCUUCUGCCCUUUGAAGCAUGUGAUC
UUUGUGACCUACUCCCUGUUCAUACACCCCUCCCCUUUUAAAAUCCCUAAUAAAAACUUGCUGGUUUUGUGGCUCAGGGGGGCAUCAUG
GACCUACCAAUACGUGAUGUCACCCCCGGUGGCCCAGCUGU
**SEQ ID 1199** 
UUAUGUAUAUGCACAUCAAAAGCACAGCACUUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUGUCUCU
CAUCC
**SEQ ID 1200** 
UUAUGUAUAUGCACAUCAAAAGCACAGCACUUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUGGGUGA
AGGUA
**SEQ ID 1201** 
UUAUGUAUAUGCACAUCAAAAGCACAGCACUUUUUUCUUUACCUUGUUUAUGAUGCAGAGACAUUUGUUCACAUGUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
 
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUG
**SEQ ID 1202**
   AGAGAAAAGCCTCCACGTTGGGCACC
**SEQ ID 1203**
   GTAGGGGTGGGTTGCCCC
**SEQ ID 1204**
   AAACCGCCTTAGGGCTGGAGGTGGGAC
**SEQ ID 1205**
   TGCGGGCAGCAATACTGC
**SEQ ID 1206**
   TAAAGCACTGAGATGTTTATGTGTATGC
**SEQ ID 1207**
   GCACAGCACTTAATCCTTTACATT
**SEQ ID 1208**
   GTTTGTCTGCTGACCCTCTCCC

## Claims

1. A method for diagnosing prostate cancer, the method comprising the step of detecting in a patient sample the level of an expression product of a human endogenous retrovirus (PCAV) located at megabase 20.428 on chromosome 22 wherein the expression product is a mRNA transcript or a polypeptide and wherein the level of expression product in the patient sample is compared to a control sample.

2. The method of claim 1, wherein a mRNA transcript is detected by hybridization, by sequencing, or by a reverse transcriptase polymerase chain reaction.

3. The method of any preceding claim, wherein the method comprises an initial step of: (a) extracting mRNA from the patient sample; (b) removing DNA from the patient sample without removing mRNA; and/or (c) removing or disrupting PCAV DNA, but not PCAV mRNA, in the patient sample.

4. The method of any preceding claim, wherein the mRNA transcript comprises one or more of SEQ IDs 24, 25, 26, 27, 28, 29, 30, 31, 32, 37, 38, 40, 41, 42, 43 and/or 1191.

5. The method of any preceding claim, comprising the steps of: (a) contacting the patient sample with nucleic acid primers and/or probe(s) under hybridizing conditions; and (b) detecting the presence or absence of hybridization in the patient sample.

6. The method of any preceding claim, comprising the steps of: (a) enriching mRNA in the sample relative to DNA to give a mRNA-enriched sample; (b) contacting the mRNA-enriched sample with nucleic acid primers and/or probe(s) under hybridizing conditions; and (c) detecting the presence or absence of hybridization to mRNA present in the mRNA-enriched sample.

7. The method of any preceding claim, comprising the steps of: (a) preparing DNA copies of mRNA in the sample; (b) contacting the DNA copies with nucleic acid primers and/or probe(s) under hybridizing conditions; and (c) detecting the presence or absence of hybridization to said DNA copies.

8. The method of any preceding claim, comprising the step of contacting the patient sample with an antibody which recognizes an expressed polypeptide from the retrovirus.

9. The method of any preceding claim, wherein the patient sample comprises prostate cells.

10. The method of any preceding claim, wherein the patient is an adult human male.

## Patentansprüche

1. Verfahren zur Diagnose von Prostatakrebs, bei dem man in einer Patientenprobe die Menge eines Expressionsproduktes von einem humanen endogenen Retrovirus (PCAV) nachweist, welcher an Megabase 20.428 von Chromosom 22 lokalisiert ist, wobei das Expressionsprodukt ein mRNA-Transkript oder ein Polypeptid ist, und wobei die Menge des Expressionsprodukts in der Patientenprobe mit einer Kontrollprobe verglichen wird.

2. Verfahren nach Anspruch 1, wobei ein mRNA-Transkript durch Hybridisierung, Sequenzierung oder durch eine Reverse Transkriptase-Polymerase-Kettenreaktion nachgewiesen wird.

3. Verfahren nach einem der vorhergehenden Ansprüche, wobei das Verfahren einen ersten Schritt umfasst, bei dem man (a) mRNA aus der Patientenprobe extrahiert; (b) DNA aus der Patientenprobe entfernt, ohne mRNA zu entfernen; und/oder (c) in der Patientenprobe PCAV-DNA entfernt oder abbaut, nicht aber PCA-mRNA.

4. Verfahren nach einem der vorhergehenden Ansprüche, wobei das mRNA-Transkript eine oder mehrere der SEQ ID NOs: 24, 25, 26, 27, 28, 29, 30, 31, 32, 37, 38, 40, 41, 42, 43 und/oder 1191 umfasst.

5. Verfahren nach einem der vorhergehenden Ansprüche, bei dem man (a) die Patientenprobe mit Nukleinsäure-Primern und/oder Sonde(n) unter Hybridisierungsbedingungen in Kontakt bringt; und (b) das Vorhandensein oder Fehlen einer Hybridisierung in der Patientenprobe nachweist.

6. Verfahren nach einem der vorhergehenden Ansprüche, bei dem man (a) die mRNA in der Probe relativ zur DNA anreichert, um eine mRNA-angereicherte Probe zu erhalten; (b) die mRNA-angereicherte Probe mit Nukleinsäure-Primern und/oder Sonde(n) unter Hybridisierungsbedingungen in Kontakt bringt; und (c) das Vorhandensein oder Fehlen einer Hybridisierung an mRNA in der mRNA-angereicherten Probe nachweist.

7. Verfahren nach einem der vorhergehenden Ansprüche, bei dem man (a) DNA-Kopien von mRNA in der Probe herstellt; (b) die DNA-Kopien mit Nukleinsäure-Primern und/oder Sonde(n) unter Hybridisierungsbedingungen in Kontakt bringt; und (c) das Vorhandensein oder Fehlen einer Hybridisierung an die DNA-Kopien nachweist.

8. Verfahren nach einem der vorhergehenden Ansprüche, bei dem man die Patientenprobe mit einem Antikörper in Kontakt bringt, der ein exprimiertes Polypeptid von dem Retrovirus erkennt.

9. Verfahren nach einem der vorhergehenden Ansprüche, wobei die Patientenprobe Prostatazellen umfasst.

10. Verfahren nach einem der vorhergehenden Ansprüche, wobei der Patient ein erwachsener humaner Mann ist.

## Revendications

1. Méthode de diagnostic du cancer de la prostate, la méthode comprenant l'étape consistant à détecter dans un échantillon provenant d'un patient, le taux d'un produit d'expression d'un rétrovirus endogène humain (PCAV) situé au niveau de la mégabase 20 428 sur le chromosome 22, le produit d'expression étant un produit de transcription d'ARNm ou un polypeptide et le taux du produit d'expression dans l'échantillon provenant du patient étant comparé à un échantillon témoin.

2. Méthode selon la revendication 1, dans laquelle un produit de transcription d'ARNm est détecté par hybridation, par séquençage ou par une réaction en chaîne polymérase par transcriptase inverse.

3. Méthode selon l'une quelconque des revendications précédentes, dans laquelle la méthode comprend une étape initiale consistant à : (a) extraire l'ARNm de l'échantillon provenant du patient ; (b) retirer l'ADN de l'échantillon provenant du patient sans retirer l'ARNm ; et/ou (c) retirer ou rompre l'ADN de PCAV mais non l'ARNm de PCAV dans l'échantillon provenant du patient.

4. Méthode selon l'une quelconque des revendications précédentes, dans laquelle le produit de transcription d'ARNm comprend une ou plusieurs des SEQ ID N° : 24, 25, 26, 27, 28, 29, 30, 31, 32, 37, 38, 40, 41, 42, 43 et/ou 1191.

5. Méthode selon l'une quelconque des revendications précédentes, comprenant les étapes consistant à : (a) mettre en contact l'échantillon provenant du patient avec des amorces d'acide nucléique et/ou une/des sonde(s) dans des conditions d'hybridation ; et (b) détecter la présence ou l'absence d'hybridation dans l'échantillon provenant du patient.

6. Méthode selon l'une quelconque des revendications précédentes, comprenant les étapes consistant à : (a) enrichir l'ARNm dans l'échantillon par rapport à l'ADN pour donner un échantillon enrichi en ARNm ; (b) mettre en contact l'échantillon enrichi en ARNm avec des amorces d'acide nucléique et/ou une/des sonde(s) dans des conditions d'hybridation ; et (c) détecter la présence ou l'absence d'hybridation avec l'ARNm présent dans l'échantillon enrichi en ARNm.

7. Méthode selon l'une quelconque des revendications précédentes, comprenant les étapes consistant à : (a) préparer des copies d'ADN d'ARNm dans l'échantillon ; (b) mettre en contact les copies d'ADN avec les amorces d'acide nucléique et/ou la/les sonde(s) dans des conditions d'hybridation ; et (c) détecter la présence ou l'absence d'hybridation avec lesdites copies d'ADN.

8. Méthode selon l'une quelconque des revendications précédentes, comprenant l'étape de mise en contact de l'échantillon provenant du patient avec un anticorps qui reconnaît un polypeptide exprimé du rétrovirus.

9. Méthode selon l'une quelconque des revendications précédentes, dans laquelle l'échantillon provenant du patient comprend des cellules prostatiques.

10. Méthode selon l'une quelconque des revendications précédentes, dans laquelle le patient est un homme adulte.
